(19) Europäisches Patentamt
European Patent Office
Office européen des brevets

(11) EP 4 349 371 A1

(12) **EUROPEAN PATENT APPLICATION**
published in accordance with Art. 153(4) EPC

(43) Date of publication:
**10.04.2024 Bulletin 2024/15**

(21) Application number: **22815326.8**

(22) Date of filing: **01.06.2022**

(51) International Patent Classification (IPC):
***A61K 47/60*** *(2017.01)* ***A61K 47/68*** *(2017.01)*
***A61K 47/65*** *(2017.01)* ***C07K 16/00*** *(2006.01)*
***A61P 35/00*** *(2006.01)*

(52) Cooperative Patent Classification (CPC):
**A61K 31/4745; A61K 45/06; A61K 47/60; A61K 47/65; A61K 47/68; A61P 29/00; A61P 31/00; A61P 35/00; A61P 37/02; C07K 5/1024; C07K 16/00**

(86) International application number:
**PCT/CN2022/096690**

(87) International publication number:
**WO 2022/253284 (08.12.2022 Gazette 2022/49)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **02.06.2021 PCT/CN2021/098024**
**10.06.2021 CN 202110651300**
**14.10.2021 CN 202111198590**
**23.12.2021 CN 202111595515**
**24.12.2021 CN 202111602429**

(71) Applicant: **Bio-Thera Solutions, Ltd.**
**Guangzhou, Guangdong 510530 (CN)**

(72) Inventors:
- **TANG, Weijia**
**Guangzhou, Guangdong 510530 (CN)**
- **ZHOU, Xin**
**Guangzhou, Guangdong 510530 (CN)**
- **MEI, Xingxing**
**Guangzhou, Guangdong 510530 (CN)**
- **YAN, Hui**
**Guangzhou, Guangdong 510530 (CN)**
- **LI, Shuoxu**
**Guangzhou, Guangdong 510530 (CN)**
- **FAN, Jianjun**
**Guangzhou, Guangdong 510530 (CN)**
- **QI, Xuekang**
**Guangzhou, Guangdong 510530 (CN)**
- **YU, Jin-Chen**
**Guangzhou, Guangdong 510530 (CN)**
- **LI, Shengfeng**
**Guangzhou, Guangdong 510530 (CN)**

(74) Representative: **dompatent von Kreisler Selting Werner -**
**Partnerschaft von Patent- und Rechtsanwälten mbB**
**Deichmannhaus am Dom**
**Bahnhofsvorplatz 1**
**50667 Köln (DE)**

Remarks:
The complete document including Reference Table(s) and the Sequence Listing(s) can be downloaded from the EPO website

(54) **DRUG CONJUGATE AND USE THEREOF**

(57) A drug conjugate, such as an antibody drug conjugate, and the use thereof, belonging to the field of biomedicine. In some embodiments, the drug conjugate is a compound of Formula I or a pharmaceutically acceptable salt or a solvate thereof, and can be used for treating cancers, autoimmune diseases, inflammatory diseases or infectious diseases.

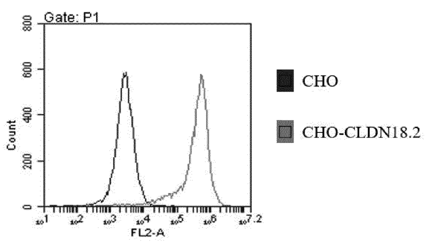


FIG. 1

EP 4 349 371 A1

## Description

### TECHNICAL FIELD

The present invention relates to drug conjugates such as antibody-drug conjugates, linkers and intermediates for preparing the drug conjugates, and use of the drug conjugates.

### BACKGROUND

The targeted treatment of cancer, immunodeficiency, infectious diseases, etc. is currently the main focus of precision medicine. Over the years, there have been numerous reports of using cell surface receptor binding molecules as drug delivery vehicles to form conjugates with cytotoxin molecules for targeted delivery of cytotoxin molecules to attack various pathogenic cells (Allen, T.M. and Cullis, P.R., 2004 Science, 303(5665), 1818-22; Hu, Q.Y, et al. (2016), Chem Soc Rev 45(6): 1691-1719).

An antibody-drug conjugate consists of three parts: an antibody, a cytotoxin molecule, and a linker in between for linking the two (Thomas, A., et al. (2016), Lancet Oncol 17(6): e254-e262). The three components each serve unique functions: the antibody should be able to specifically bind to tumor cells, the cytotoxin molecule should be sufficiently active and have a broad spectrum for the tumor cells, and the linker should be uniquely functional, stable in the blood circulation and effective in releasing the cytotoxin molecule upon reaching the tumor cells (Chari, R.V (2008), Acc Chem Res 41(1): 98-107). Good clinical results can be produced only when the three components are reasonably constructed (Singh, S.K., et al. (2015), Pharm Res 32(11): 3541-3571; Hamilton, G.S. (2015), Biologicals 43(5): 318-332).

### SUMMARY

One or more embodiments of the present invention provide a drug conjugate having a structure shown as Formula I or a stereoisomer thereof or a pharmaceutically acceptable salt or solvate thereof:

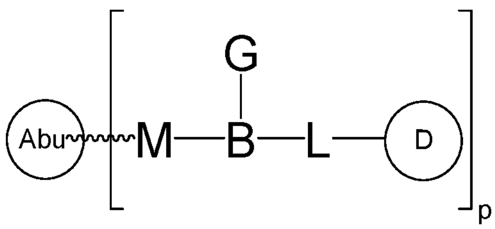

Formula I

wherein

Abu is a polypeptide, such as an antibody or an antigen-binding unit thereof;

D is a drug, such as an anti-cancer drug, a cytotoxic drug, a cell differentiation factor, a stem cell trophic factor, a steroid drug, a drug for treating autoimmune diseases, an anti-inflammatory drug or a drug for treating infectious diseases;

M is

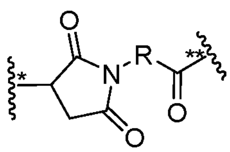

,

wherein * links to Abu, ** links to B, and R is selected from: $-(CH_2)_r$, $-(CHR^m)_r-$, C3-C8 carbocyclyl, $-O-(CH_2)_r-$, arylene, $-(CH_2)_r$-arylene-, -arylene-$(CH_2)$r-, $-(CH_2)_r$-(C3-C8 carbocyclyl)-, -(C3-C8 carbocyclyl)-$(CH_2)_r$, C3-C8 heterocyclyl, $-(CH_2)_r$-(C3-C8 heterocyclyl)-, -(C3-C8 heterocyclyl)-$(CH_2)_r$, $-(CH_2)_rC(O)NR^m(CH_2)_r-$, $-(CH_2CH_2O)_r-$,

$-(CH_2CH_2O)_r-CH_2-$, $-(CH_2)_rC(O)NR^m(CH_2CH_2O)_r-$, $-(CH_2)_rC(O)NR^m(CH_2CH_2O)_r-CH_2-$, $-(CH_2CH_2O)_rC(O)NR^m(CH_2CH_2O)_r-$, $-(CH_2CH_2O)_rC(O)NR^m(CH_2CH_2O)_r-CH_2-$ and $-(CH_2CH_2O)_rC(O)NR^m(CH_2)_r-$; wherein each $R^m$ is independently H, C1-C6 alkyl, C3-C8 carbocyclyl, phenyl or benzyl, and each r is independently 1, 2, 3, 4, 5, 6, 7, 8, 9 or 10;

B is

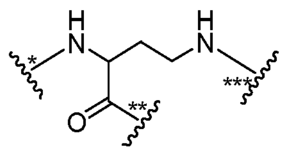

,

e.g.,

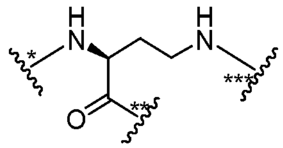

;

wherein * links to M, ** links to L, and *** links to G;

L is -(AA)i-(FF)f-, wherein AA is an amino acid or polypeptide, and i is 1, 2, 3, 4, 5, 6, 7, 8, 9, 10, 11, 12, 13, 14, 15, 16, 17, 18, 19 or 20; each FF is independently

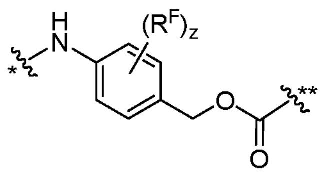

,

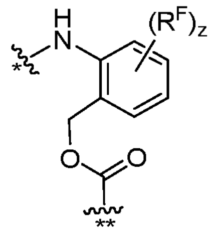

or

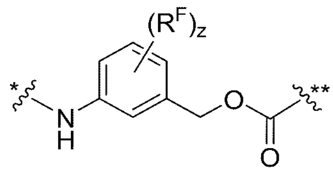

,

wherein each $R^F$ is independently C1-C6 alkyl, C1-C6 alkoxy, $-NO_2$ or halogen; z is 0, 1, 2, 3 or 4; f is 1, 2, 3, 4, 5, 6, 7, 8, 9 or 10; wherein * links to AA, and ** links to D;

Gis

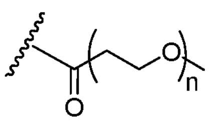

,

wherein n is an integer from 1 to 24, e.g., 1, 2, 3, 4, 5, 6, 7, 8, 9, 10, 11, 12, 13, 14, 15, 16, 17, 18, 19, 20, 21, 22, 23 or 24;

p is 1-10, e.g., 1, 2, 3, 4, 5, 6, 7, 8, 9 or 10.

In one or more embodiments, D is an anti-cancer drug.

In one or more embodiments, D is a tubulin inhibitor, a DNA damaging agent, or a DNA topoisomerase inhibitor.

In one or more embodiments, the tubulin inhibitor is selected from dolastatin, auristatins and maytansinoids.

In one or more embodiments, D is an auristatin, e.g., MMAE (monomethyl auristatin E), MMAF (monomethyl auristatin F), or AF (auristatin F).

In one or more embodiments, D is a DNA damaging agent, e.g., a calicheamicin, a duocarmycin, or the anthramycin derivative PBD (pyrrolobenzodiazepine).

In one or more embodiments, D is a DNA topoisomerase inhibitor or a salt thereof, e.g., irinotecan, irinotecan hydrochloride, camptothecin, 9-aminocamptothecin, 9-nitrocamptothecin, 10-hydroxycamptothecin, 9-chloro-10-hydroxycamptothecin, the camptothecin derivative SN-38, 22-hydroxyacuminatine, topotecan, lurtotecan, belotecan, exatecan, an exatecan derivative, homosilatecan, 6,8-dibromo-2-methyl-3-[2-(D-xylopyranosylamino)phenyl]-4(3*H*)-quinazolinone, 2-cyano-3-(3,4-dihydroxyphenyl)-*N*-(phenylmethyl)-(2*E*)-2-propenamide, 2-cyano-3-(3,4-dihydroxyphenyl)-*N*-(3-hydroxyphenylpropyl)-(E)-2-propenamide, 12-β-D-glucopyranosyl-12,13-dihydro-2,10-dihydroxy-6-[[2-hydroxy-1-(hydroxymethyl)ethyl]amino]-5*H*-indolo[2,3-*a*]pyrrolo[3,4-*c*]carbazole-5, 7(6*H*)-dione, *N*-[2-(dimethylamino)ethyl]-4-acridinecarboxamide dihydrochloride, or *N*-[2-(dimethylamino)ethyl]-4-acridinecarboxamide.

In one or more embodiments, the DNA topoisomerase inhibitor is camptothecin, 10-hydroxycamptothecin, topotecan, belotecan, irinotecan, 22-hydroxyacuminatine, or exatecan, or a salt thereof.

In one or more embodiments, D is a tubulysin, a taxane drug derivative, a leptomycine derivative, CC-1065 or an analog thereof, an amatoxin, a spliceosome inhibitor, a benzodiazepine (PBD) dimer, adriamycin, methotrexate, vincristine, vinblastine, daunorubicin, mitomycin C, melphalan, or a chlorambucil derivative.

In one or more embodiments, D has an amino group or an amino group substituted with one alkyl group, and links to FF through an amide bond.

In one or more embodiments, D is

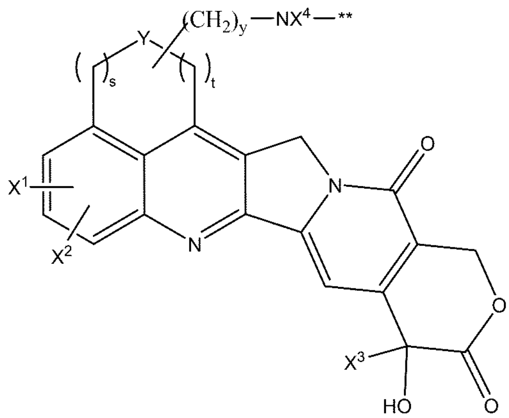,

wherein

$X^1$ and $X^2$ are each independently:

H,

hydroxy,

C1-C6 alkyl,

C1-C6 alkyl substituted with one or more hydroxy, halogen, nitro or cyano groups,

C2-C6 alkenyl,

C2-C6 alkynyl,

C1-C6 alkoxy,

C1-C6 aminoalkoxy,

halogen,

nitro,

cyano,

thiol,

alkylthio,

amino, amino substituted with an amino-protecting group, C1-C6 aminoalkyl optionally substituted at the amino moiety with an amino-protecting group or C1-C6 alkyl,

C1-C6 aminoalkylamino optionally substituted at the amino moiety with an amino-protecting group or C1-C6 alkyl,

C1-C6 alkyl linking to a heterocyclic ring, wherein the heterocyclic ring is optionally substituted with one or more C1-C6 alkyl, C1-C6 alkoxy, amino, halogen, nitro or cyano groups,

C1-C6 alkylamino linking to a heterocyclic ring, wherein the heterocyclic ring is optionally substituted with C1-C6 alkyl or C1-C6 alkoxy, and the amino is optionally substituted with an amino-protecting group, halogen, nitro, cyano or protecting group, amino-substituted heterocyclyl, which is optionally substituted at a nitrogen atom of the heterocyclyl moiety or at the amino moiety with a protecting group or one or more C1-C6 alkyl groups,

heterocyclylamino, which is optionally substituted at a nitrogen atom of the heterocyclic moiety or at the amino moiety with a protecting group or C1-C6 alkyl, carbamoyl optionally substituted with a carbamoyl-protecting group or C1-C6 alkyl, morpholin-1-yl, or

piperidin-1-yl;

$X^3$ is C1-C6 alkyl;

$X^4$ is H, $-(CH_2)_q-CH_3$, $-(CHR^n)_q-CH_3$, C3-C8 carbocyclyl, $-O-(CH_2)_q-CH_3$, arylene-$CH_3$, $-(CH_2)_q$-arylene-$CH_3$, -arylene-$(CH_2)_q-CH_3$, $-(CH_2)_q$-(C3-C8 carbocyclyl)-$CH_3$, -(C3-C8 carbocyclyl)-$(CH_2)_q-CH_3$, C3-C8 heterocyclyl, $-(CH_2)_q$-(C3-C8 heterocyclyl)-$CH_3$, -(C3-C8 heterocyclyl)-$(CH_2)_q-CH_3$,

$-(CH_2)_qC(O)NR^n(CH_2)_q-CH_3$, $-(CH_2CH_2O)_q-CH_3$, $-(CH_2CH_2O)_q-CH_2-CH_3$, $-(CH_2)_qC(O)NR^n(CH_2CH_2O)_q-CH_3$, $-(CH_2)_qC(O)NR^n(CH_2CH_2O)_q-CH_2-CH_3$, $-(CH_2CH_2O)_qC(O)NR^n(CH_2CH_2O)_q-CH_3$, $-(CH_2CH_2O)_qC(O)NR^n(CH_2CH_2O)_q-CH_2-CH_3$ or $-(CH_2CH_2O)_qC(O)NR^n(CH_2)_q-CH_3$; wherein each $R^n$ is independently H, C1-C6 alkyl, C3-C8 carbocyclyl, phenyl or benzyl, and each q is independently 1, 2, 3, 4, 5, 6, 7, 8, 9 or 10;

** links to L;

y is 0, 1 or 2;

Y is O, S or $CR^1R^2$, wherein $R^1$ and $R^2$ are each independently H or C1-C6 alkyl;

s and t are each independently 0, 1 or 2, but not both 0.

In one or more embodiments, $X^4$ is H or C1-C6 alkyl.

In one or more embodiments, the heterocyclyl is azetidine, niverazine, morpholine, pyrrolidine, piperidine, imidazole, thiazole, oxazole or pyridine.

In one or more embodiments, the amino-protecting group is formyl, acetyl, trityl, *t*-butoxycarbonyl, benzyl, or *p*-methoxybenzyloxycarbonyl.

In one or more embodiments, D is

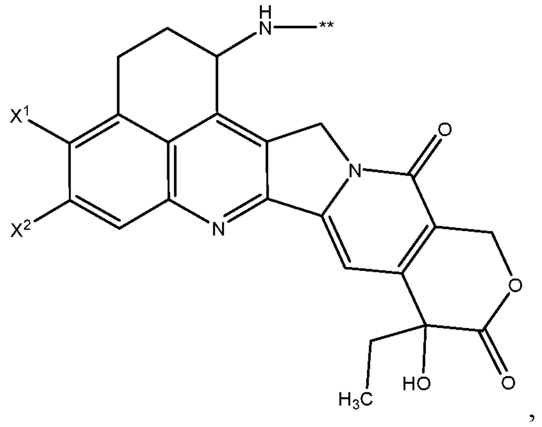

wherein $X^1$ and $X^2$ are each independently C1-C6 alkyl, halogen, or -OH; ** links to L.

In one or more embodiments, D is

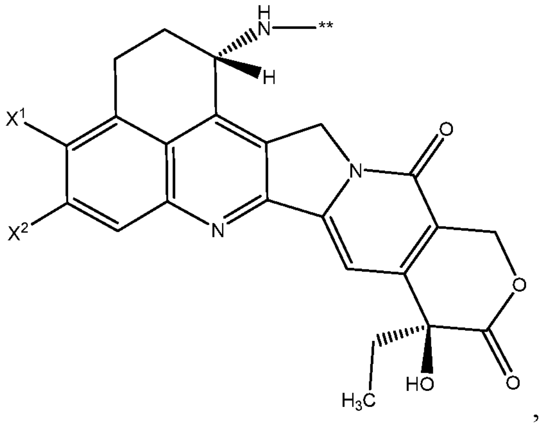

wherein $X^1$ and $X^2$ are each independently C1-C6 alkyl, halogen, or -OH; ** links to L.

In one or more embodiments, $X^1$ and $X^2$ are each $-CH_3$.

In one or more embodiments, $X^1$ and $X^2$ are each independently F, Cl, Br, or I.

In one or more embodiments, $X^1$ and $X^2$ are each F or Cl.

In one or more embodiments, $X^1$ and $X^2$ are each F.

In one or more embodiments, $X^1$ and $X^2$ are each independently $-CH_3$, F, or -OH.

In one or more embodiments, $X^1$ and $X^2$ are each independently F or $-CH_3$.

In one or more embodiments, $X^1$ is $-CH_3$ and $X^2$ is F.

In one or more embodiments, R is $-(CH_2)_r-$.

In one or more embodiments, R is $-(CH_2)_r-$, wherein r is 1 or 5.

In one or more embodiments, each AA is independently selected from the following amino acids and peptide sequences: Val-Cit, Val-Lys, Phe-Lys, Lys-Lys, Ala-Lys, Phe-Cit, Leu-Cit, Ile-Cit, Trp, Cit, Phe-Ala, Phe-Phe-Lys, D-Phe-Phe-Lys, Gly-Phe-Lys, Leu-Ala-Leu, Ile-Ala-Leu, Val-Ala-Val, Ala-Leu-Ala-Leu, β-Ala-Leu-Ala-Leu, and Gly-Phe-Leu-Gly.

In one or more embodiments, i is 1.

In one or more embodiments, AA is Val-Cit, and i is 1.

In one or more embodiments, each FF is independently

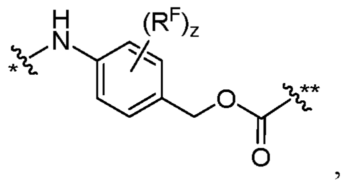

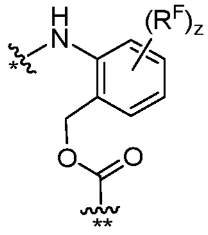

or

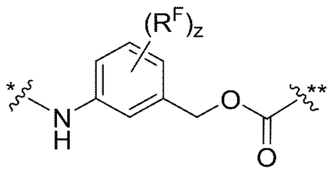

,

wherein * links to AA, and ** links to D, wherein each $R^F$ is independently C1-C6 alkyl, C1-C6 alkoxy, $-NO_2$, or halogen.

In one or more embodiments, the halogen is F.

In one or more embodiments, each $R^F$ is independently $-CH_3$, F, $-NO_2$ or $-OCH_3$.

In one or more embodiments, z is 0.

In one or more embodiments, z is 1 or 2.

In one or more embodiments, f is 1.

In one or more embodiments, each FF is independently

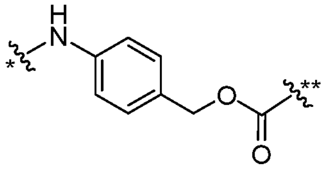

,

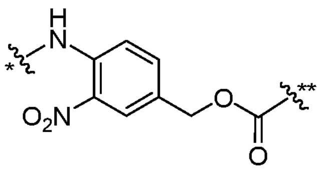

,

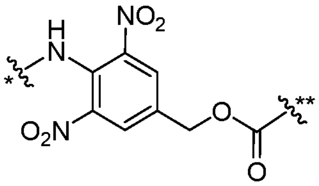

,

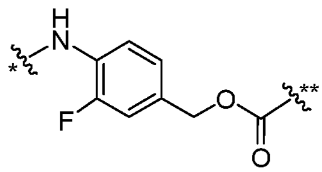

,

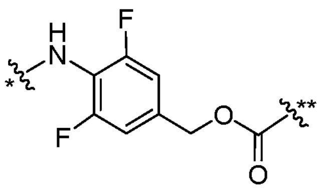

,

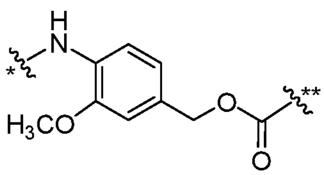

,

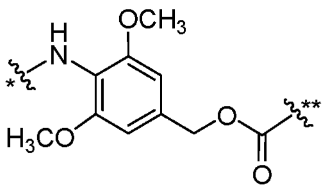

,

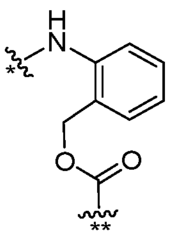

,

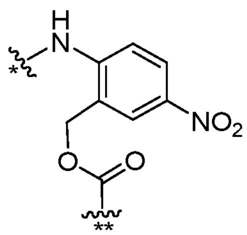

,

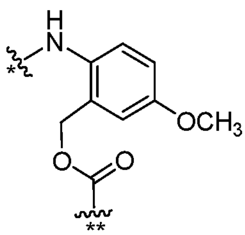

,

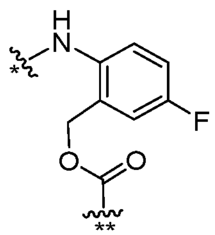

,

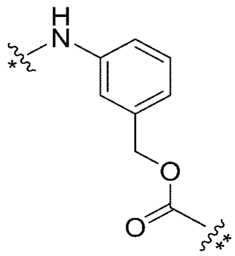

,

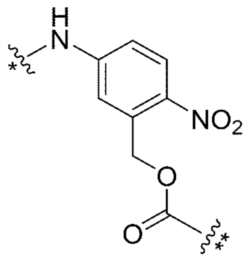

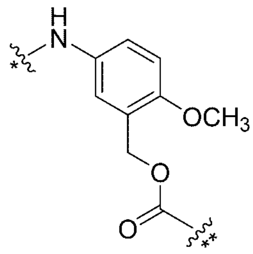

or

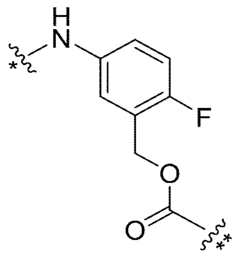

,

wherein * links to AA, and ** links to D.

In one or more embodiments, f is 1.

In one or more embodiments, FF is

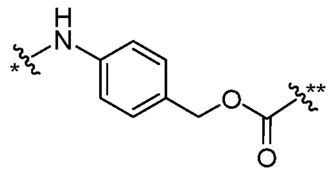

,

and f is 1, wherein * links to AA, and ** links to D.

In one or more embodiments, L is

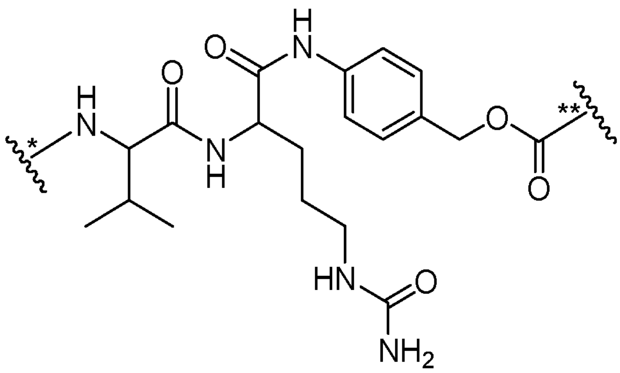

,

wherein * links to B, and ** links to D.

In one or more embodiments, L is

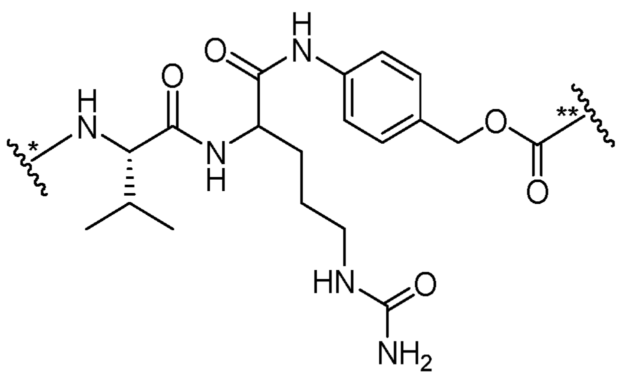

,

wherein * links to B, and ** links to D.

In one or more embodiments, L is

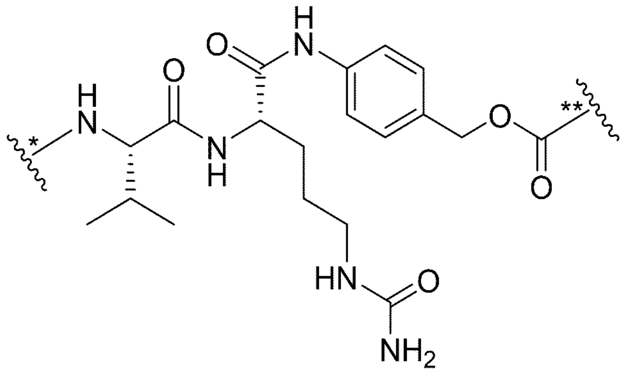

,

wherein * links to B, and ** links to D.

In one or more embodiments, G is

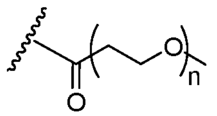

,

and n is 4-12.

In one or more embodiments, n is 4-8.

In one or more embodiments, n is 4.

In one or more embodiments, n is 8.

In one or more embodiments, p is 2-8.

In one or more embodiments, p is 4-8.

In one or more embodiments, p is 6-8.

In one or more embodiments, p is 7-8.

In one or more embodiments, Formula I is:

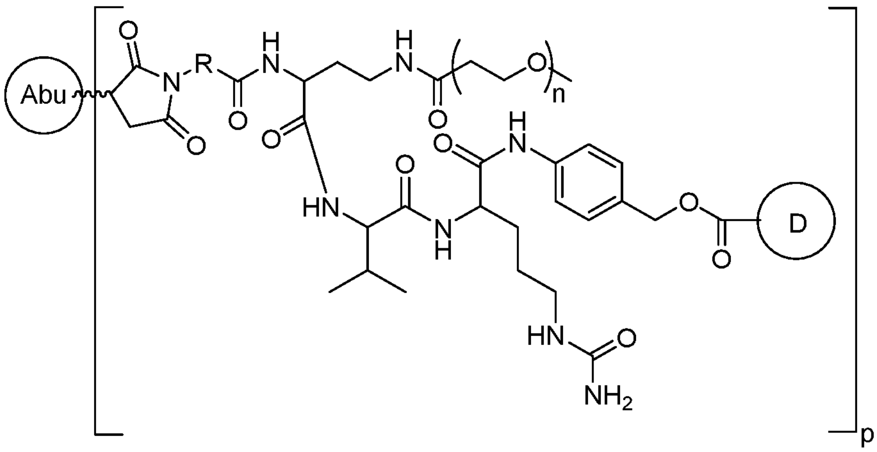

Formula I-1

wherein

Abu is a polypeptide, such as an antibody or an antigen-binding unit thereof;

R is selected from: $-(CH_2)_r-$, $-(CHR^m)_r-$, C3-C8 carbocyclyl, $-O-(CH_2)_r-$, arylene, $-(CH_2)_r$arylene-, -arylene-$(CH_2)$r-, $-(CH_2)_r$-(C3-C8 carbocyclyl)-, -(C3-C8 carbocyclyl)-$(CH_2)_r$-, C3-C8 heterocyclyl, $-(CH_2)_r$(C3-C8 heterocyclyl)-, -(C3-C8 heterocyclyl)-$(CH_2)_r$-, $-(CH_2)_rC(O)NR^m(CH_2)_r-$, $-(CH_2CH_2O)_r-$, $-(CH_2CH_2O)_r-CH_2-$,

$-(CH_2)_rC(O)NR^m(CH_2CH_2O)_r-$, $-(CH_2)_rC(O)NR^m(CH_2CH_2O)_r-CH_2-$, $-(CH_2CH_2O)_rC(O)NR^m(CH_2CH_2O)_r-$, $-(CH_2CH_2O)_rC(O)NR^m(CH_2CH_2O)_r-CH_2-$ and $-(CH_2CH_2O)_rC(O)NR^m(CH_2)_r-$; wherein each $R^m$ is independently H, C1-C6 alkyl, C3-C8 carbocyclyl, phenyl or benzyl, and each r is independently 1, 2, 3, 4, 5, 6, 7, 8, 9 or 10;

D is a drug, such as an anti-cancer drug, a cytotoxic drug, a cell differentiation factor, a stem cell trophic factor, a steroid drug, a drug for treating autoimmune diseases, an anti-inflammatory drug or a drug for treating infectious diseases;

n is an integer from 1 to 24, e.g., 1, 2, 3, 4, 5, 6, 7, 8, 9, 10, 11, 12, 13, 14, 15, 16, 17, 18, 19, 20, 21, 22, 23 or 24;

p is 1-10, e.g., 1, 2, 3, 4, 5, 6, 7, 8, 9 or 10.

In one or more embodiments, D is a tubulin inhibitor, a DNA damaging agent, or a DNA topoisomerase inhibitor.

In one or more embodiments, the tubulin inhibitor is selected from dolastatin, auristatins and maytansinoids.

In one or more embodiments, D is an auristatin, e.g., MMAE (monomethyl auristatin E), MMAF (monomethyl auristatin F), or AF (auristatin F).

In one or more embodiments, D is a DNA damaging agent, e.g., a calicheamicin, a duocarmycin, or the anthramycin derivative PBD (pyrrolobenzodiazepine).

In one or more embodiments, D is a DNA topoisomerase inhibitor or a salt thereof, e.g., irinotecan, irinotecan hydrochloride, camptothecin, 9-aminocamptothecin, 9-nitrocamptothecin, 10-hydroxycamptothecin, 9-chloro-10-hydroxycamptothecin, the camptothecin derivative SN-38, 22-hydroxyacuminatine, topotecan, lurtotecan, belotecan, exatecan, homosilatecan, 6,8-dibromo-2-methyl-3-[2-(D-xylopyranosylamino)phenyl]-4(3*H*)-quinazolinone, 2-cyano-3-(3,4-dihydroxyphenyl)-*N*-(phenylmethyl)-(2*E*)-2-propenamide,

2-cyano-3-(3,4-dihydroxyphenyl)-*N*-(3-hydroxyphenylpropyl)-(*E*)-2-propenamide, 12-β-D-glucopyranosyl-12,13-dihydro-2,10-dihydroxy-6-[[2-hydroxy-1-(hydroxymethyl)ethyl]amino]-5*H*-indolo[2,3-*a*]pyrrolo[3,4-*c*]carbazole-5, 7(6*H*)-dione, *N*-[2-(dimethylamino)ethyl]-4-acridinecarboxamide dihydrochloride, or *N*-[2-(dimethylamino)ethyl]-4-acridinecarboxamide.

In one or more embodiments, the DNA topoisomerase inhibitor is camptothecin, 10-hydroxycamptothecin, topotecan, belotecan, irinotecan, 22-hydroxyacuminatine, or exatecan, or a salt thereof.

In one or more embodiments, D is a tubulysin, a taxane drug derivative, a leptomycine derivative, CC-1065 or an analog thereof, an amatoxin, a spliceosome inhibitor, a benzodiazepine (PBD) dimer, adriamycin, methotrexate, vincristine, vinblastine, daunorubicin, mitomycin C, melphalan, or a chlorambucil derivative.

In one or more embodiments, D is

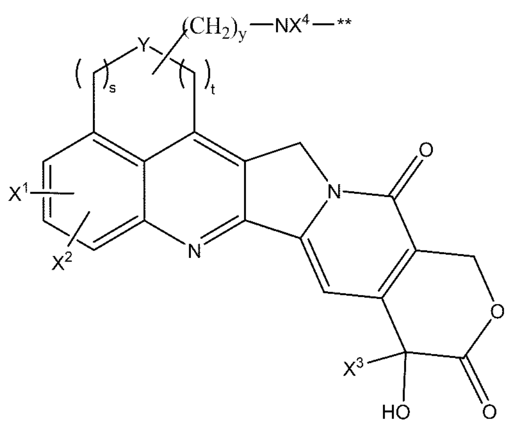
,

wherein

$X^1$ and $X^2$ are each independently:

H,

hydroxy,

C1-C6 alkyl,

C1-C6 alkyl substituted with one or more hydroxy, halogen, nitro or cyano groups,

C2-C6 alkenyl,

C2-C6 alkynyl,

C1-C6 alkoxy,

C1-C6 aminoalkoxy,

halogen,

nitro,

cyano,

thiol,

alkylthio,

amino, amino substituted with an amino-protecting group, C1-C6 aminoalkyl optionally substituted at the amino moiety with an amino-protecting group or C1-C6 alkyl,

C1-C6 aminoalkylamino optionally substituted at the amino moiety with an amino-protecting group or C1-C6 alkyl,

C1-C6 alkyl linking to a heterocyclic ring, wherein the heterocyclic ring is optionally substituted with one or more C1-C6 alkyl, C1-C6 alkoxy, amino, halogen, nitro or cyano groups,

C1-C6 alkylamino linking to a heterocyclic ring, wherein the heterocyclic ring is optionally substituted with C1-C6 alkyl or C1-C6 alkoxy, and the amino is optionally substituted with an amino-protecting group, halogen, nitro, cyano or protecting group,

amino-substituted heterocyclyl, which is optionally substituted at a nitrogen atom of the heterocyclyl moiety or at the amino moiety with a protecting group or one or more C1-C6 alkyl groups,

heterocyclylamino, which is optionally substituted at a nitrogen atom of the heterocyclic moiety or at the amino moiety with a protecting group or C1-C6 alkyl, carbamoyl optionally substituted with a carbamoyl-protecting group or C1-C6 alkyl, morpholin-1-yl, or

piperidin-1-yl;

$X^3$ is C1-C6 alkyl;

$X^4$ is H, $-(CH_2)_q-CH_3$, $-(CHR^n)_q-CH_3$, C3-C8 carbocyclyl, $-O-(CH_2)_q-CH_3$, arylene-$CH_3$, $-(CH_2)_q$-arylene-$CH_3$, -arylene-$(CH_2)_q-CH_3$, $-(CH_2)_q$-(C3-C8 carbocyclyl)-$CH_3$, -(C3-C8 carbocyclyl)-$(CH_2)_q-CH_3$, C3-C8 heterocyclyl, $-(CH_2)_q$-(C3-C8 heterocyclyl)-$CH_3$, -(C3-C8 heterocyclyl)-$(CH_2)_q-CH_3$,

$-(CH_2)_qC(O)NR^n(CH_2)_q-CH_3$, $-(CH_2CH_2O)_q-CH_3$, $-(CH_2CH_2O)_q-CH_2-CH_3$, $-(CH_2)_qC(O)NR^n(CH_2CH_2O)_q-CH_3$, $-(CH_2)_qC(O)NR^n(CH_2CH_2O)_q-CH_2-CH_3$, $-(CH_2CH_2O)_qC(O)NR^n(CH_2CH_2O)_q-CH_3$, $-(CH_2CH_2O)_qC(O)NR^n(CH_2CH_2O)_q-CH_2-CH_3$ or $-(CH_2CH_2O)_qC(O)NR^n(CH_2)_q-CH_3$; wherein each $R^n$ is independently H, C1-C6 alkyl, C3-C8 carbocyclyl, phenyl or benzyl, and each q is independently 1, 2, 3, 4, 5, 6, 7, 8, 9 or 10;

** is point of connection;

y is 0, 1 or 2;

Y is O, S or $CR^1R^2$, wherein $R^1$ and $R^2$ are each independently H or C1-C6 alkyl;

s and t are each independently 0, 1 or 2, but not both 0.

In one or more embodiments, $X^4$ is H or C1-C6 alkyl.

In one or more embodiments, the heterocyclyl is azetidine, niverazine, morpholine, pyrrolidine, piperidine, imidazole, thiazole, oxazole or pyridine.

In one or more embodiments, the amino-protecting group is formyl, acetyl, trityl, *t*-butoxycarbonyl, benzyl, or *p*-methoxybenzyloxycarbonyl.

In one or more embodiments, D is

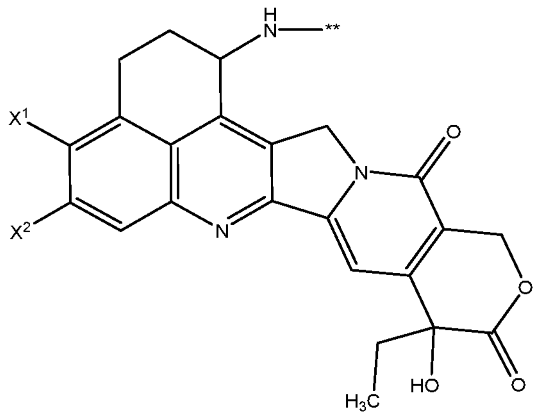

,

wherein $X^1$ and $X^2$ are each independently C1-C6 alkyl, halogen, or -OH; ** is point of connection.

In one or more embodiments, D is

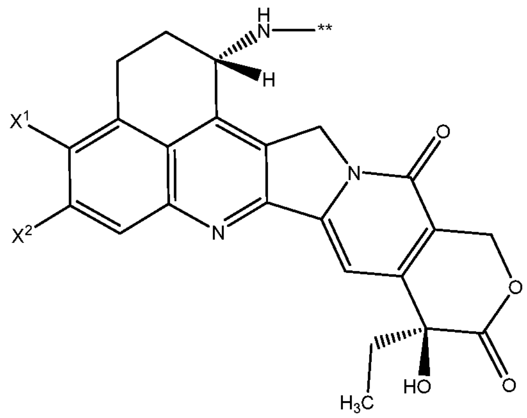

,

wherein $X^1$ and $X^2$ are each independently C1-C6 alkyl, halogen, or -OH; ** is point of connection.

In one or more embodiments, $X^1$ and $X^2$ are each $-CH_3$.

In one or more embodiments, $X^1$ and $X^2$ are each independently F, Cl, Br, or I.

In one or more embodiments, $X^1$ and $X^2$ are each F.

In one or more embodiments, $X^1$ and $X^2$ are each independently $-CH_3$, F, or -OH.

In one or more embodiments, $X^1$ and $X^2$ are each independently F or $-CH_3$.

In one or more embodiments, $X^1$ is $-CH_3$ and $X^2$ is F.

In one or more embodiments, R is $-(CH_2)_r-$.

In one or more embodiments, R is $-(CH_2)_r-$, wherein r is 1 or 5.

In one or more embodiments, n is 4-12.

In one or more embodiments, n is 4-8.

In one or more embodiments, n is 4.

In one or more embodiments, n is 8.

In one or more embodiments, p is 2-8.

In one or more embodiments, p is 4-8.

In one or more embodiments, p is 6-8.

In one or more embodiments, p is 7-8.

In one or more embodiments, Formula I is:

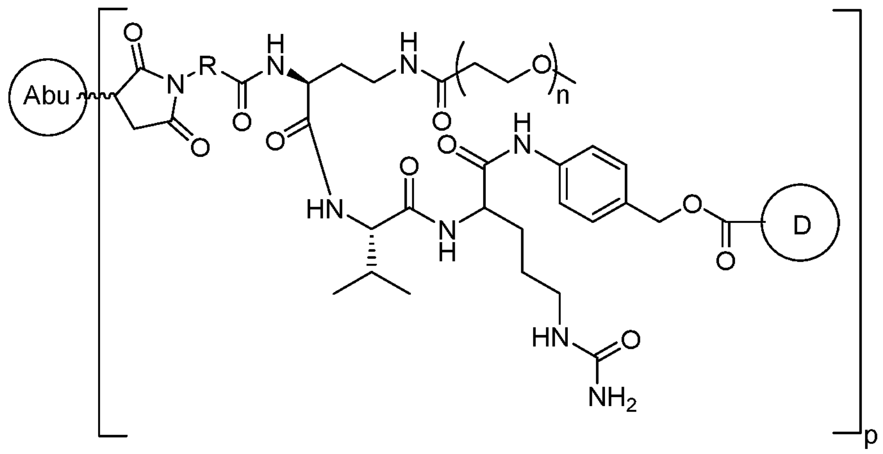

Formula I-2

or

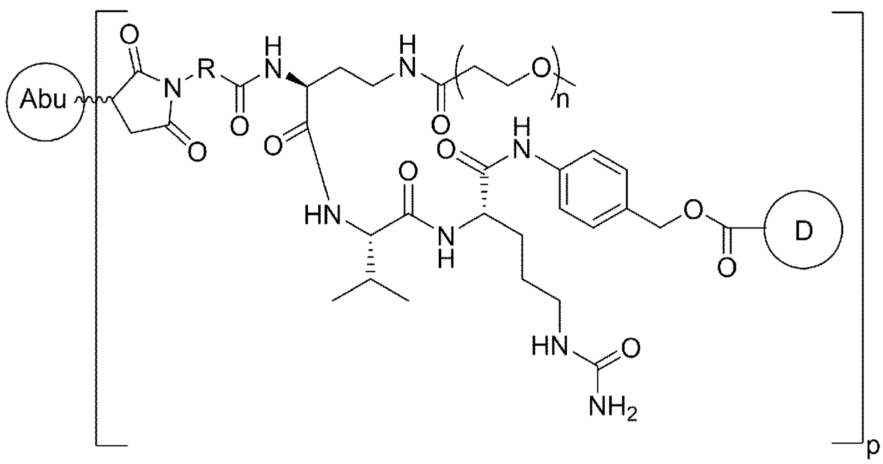

Formula I-2-1

wherein

Abu is a polypeptide, such as an antibody or an antigen-binding unit thereof;

R is selected from: $-(CH_2)_r-$, $-(CHR^m)_r-$, C3-C8 carbocyclyl, $-O-(CH_2)_r-$, arylene, $-(CH_2)_r$arylene-, -arylene-$(CH_2)$r-, $-(CH_2)_r-$(C3-C8 carbocyclyl)-, -(C3-C8 carbocyclyl)-$(CH_2)_r-$, C3-C8 heterocyclyl, $-(CH_2)_r$(C3-C8 heterocyclyl)-, -(C3-C8 heterocyclyl)-$(CH_2)_r-$, $-(CH_2)_rC(O)NR^m(CH_2)_r-$, $-(CH_2CH_2O)_r-$, $-(CH_2CH_2O)_r-CH_2-$, $-(CH_2)_rC(O)NR^m(CH_2CH_2O)_r-$, $-(CH_2)_rC(O)NR^m(CH_2CH_2O)_r-CH_2-$, $-(CH_2CH_2O)_rC(O)NR^m(CH_2CH_2O)_r-$, $-(CH_2CH_2O)_rC(O)NR^m(CH_2CH_2O)_r-CH_2-$ and $-(CH_2CH_2O)_rC(O)NR^m(CH_2)_r-$; wherein each $R^m$ is independently H, C1-C6 alkyl, C3-C8 carbocyclyl, phenyl or benzyl, and each r is independently 1, 2, 3, 4, 5, 6, 7, 8, 9 or 10;

D is a drug, such as an anti-cancer drug, a cytotoxic drug, a cell differentiation factor, a stem cell trophic factor, a steroid drug, a drug for treating autoimmune diseases, an anti-inflammatory drug or a drug for treating infectious diseases;

n is an integer from 1 to 24, e.g., 1, 2, 3, 4, 5, 6, 7, 8, 9, 10, 11, 12, 13, 14, 15, 16, 17, 18, 19, 20, 21, 22, 23 or 24;

p is 1-10, e.g., 1, 2, 3, 4, 5, 6, 7, 8, 9 or 10.

In one or more embodiments, D is a tubulin inhibitor, a DNA damaging agent, or a DNA topoisomerase inhibitor.

In one or more embodiments, the tubulin inhibitor is selected from dolastatin, auristatins and maytansinoids.

In one or more embodiments, D is an auristatin, e.g., MMAE (monomethyl auristatin E), MMAF (monomethyl auristatin F), or AF (auristatin F).

In one or more embodiments, D is a DNA damaging agent, e.g., a calicheamicin, a duocarmycin, or the anthramycin derivative PBD (pyrrolobenzodiazepine).

In one or more embodiments, D is a DNA topoisomerase inhibitor or a salt thereof, e.g., irinotecan, irinotecan hydrochloride, camptothecin, 9-aminocamptothecin, 9-nitrocamptothecin, 10-hydroxycamptothecin, 9-chloro-10-hydroxycamptothecin, the camptothecin derivative SN-38, 22-hydroxyacuminatine, topotecan, lurtotecan, belotecan, exatecan, homosilatecan, 6,8-dibromo-2-methyl-3-[2-(D-xylopyranosylamino)phenyl]-4(3*H*)-quinazolinone, 2-cyano-3-(3,4-dihydroxyphenyl)-*N*-(phenylmethyl)-(2*E*)-2-propenamide, 2-cyano-3-(3,4-dihydroxyphenyl)-N-(3-hydroxyphenylpropyl)-(*E*)-2-propenamide, 12-β-D-glucopyranosyl-12,13-dihydro-2,10-dihydroxy-6-[[2-hydroxy-1-(hydroxymethyl)ethyl]amino]-5*H*-indolo[2,3-*a*]pyrrolo[3,4-*c*]carbazole-5, 7(6*H*)-dione, *N*-[2-(dimethylamino)ethyl]-4-acridinecarboxamide dihydrochloride, or *N*-[2-(dimethylamino)ethyl]-4-acridinecarboxamide.

In one or more embodiments, the DNA topoisomerase inhibitor is camptothecin, 10-hydroxycamptothecin, topotecan, belotecan, irinotecan, 22-hydroxyacuminatine, or exatecan , or a salt thereof.

In one or more embodiments, D is a tubulysin, a taxane drug derivative, a leptomycine derivative, CC-1065 or an analog thereof, an amatoxin, a spliceosome inhibitor, a benzodiazepine (PBD) dimer, adriamycin, methotrexate, vincristine, vinblastine, daunorubicin, mitomycin C, melphalan, or a chlorambucil derivative.

In one or more embodiments, D is

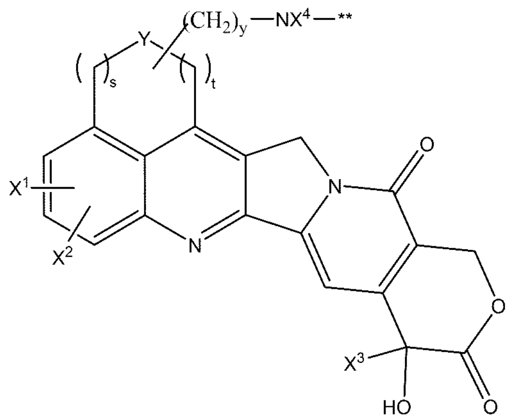,

wherein

$X^1$ and $X^2$ are each independently:

H,

hydroxy,

C1-C6 alkyl,

C1-C6 alkyl substituted with one or more hydroxy, halogen, nitro or cyano groups,

C2-C6 alkenyl,

C2-C6 alkynyl,

C1-C6 alkoxy,

C1-C6 aminoalkoxy,

halogen,

nitro,

cyano,

thiol,

alkylthio,

amino, amino substituted with an amino-protecting group, C1-C6 aminoalkyl optionally substituted at the amino moiety with an amino-protecting group or C1-C6 alkyl,

C1-C6 aminoalkylamino optionally substituted at the amino moiety with an amino-protecting group or C1-C6 alkyl,

C1-C6 alkyl linking to a heterocyclic ring, wherein the heterocyclic ring is optionally substituted with one or more C1-C6 alkyl, C1-C6 alkoxy, amino, halogen, nitro or cyano groups,

C1-C6 alkylamino linking to a heterocyclic ring, wherein the heterocyclic ring is optionally substituted with C1-C6 alkyl or C1-C6 alkoxy, and the amino is optionally substituted with an amino-protecting group, halogen, nitro, cyano or protecting group,

amino-substituted heterocyclyl, which is optionally substituted at a nitrogen atom of the heterocyclyl moiety or at the amino moiety with a protecting group or one or more C1-C6 alkyl groups,

heterocyclylamino, which is optionally substituted at a nitrogen atom of the heterocyclic moiety or at the amino moiety with a protecting group or C1-C6 alkyl, carbamoyl optionally substituted with a carbamoyl-protecting group or C1-C6 alkyl, morpholin-1-yl, or

piperidin-1-yl;

$X^3$ is C1-C6 alkyl;

$X^4$ is H, $-(CH_2)_q-CH_3$, $-(CHR^n)_q-CH_3$, C3-C8 carbocyclyl, $-O-(CH_2)_q-CH_3$, arylene-$CH_3$, $-(CH_2)_q$-arylene-$CH_3$, -arylene-$(CH_2)_q-CH_3$, $-(CH_2)_q$-(C3-C8 carbocyclyl)-$CH_3$, -(C3-C8 carbocyclyl)-$(CH_2)_q-CH_3$, C3-C8 heterocyclyl, $-(CH_2)_q$-(C3-C8 heterocyclyl)-$CH_3$, -(C3-C8 heterocyclyl)-$(CH_2)_q-CH_3$, $-(CH_2)_qC(O)NR^n(CH_2)_q-CH_3$, $-(CH_2CH_2O)_q-CH_3$, $-(CH_2CH_2O)_q-CH_2-CH_3$, $-(CH_2)_qC(O)NR^n(CH_2CH_2O)_q-CH_3$, $-(CH_2)_qC(O)NR^n(CH_2CH_2O)_q-CH_2-CH_3$, $-(CH_2CH_2O)_qC(O)NR^n(CH_2CH_2O)_q-CH_3$, $-(CH_2CH_2O)_qC(O)NR^n(CH_2CH_2O)_q-CH_2-CH_3$ or $-(CH_2CH_2O)_qC(O)NR^n(CH_2)_q-CH_3$; wherein each $R^n$ is independently H, C1-C6 alkyl, C3-C8 carbocyclyl, phenyl or benzyl, and each q is independently 1, 2, 3, 4, 5, 6, 7, 8, 9 or 10;

** is point of connection;

y is 0, 1 or 2;

Y is O, S or $CR^1R^2$, wherein $R^1$ and $R^2$ are each independently H or C1-C6 alkyl;

s and t are each independently 0, 1 or 2, but not both 0.

In one or more embodiments, $X^4$ is H or C1-C6 alkyl.

In one or more embodiments, the heterocyclyl is azetidine, niverazine, morpholine, pyrrolidine, piperidine, imidazole, thiazole, oxazole or pyridine.

In one or more embodiments, the amino-protecting group is formyl, acetyl, trityl, t-butoxycarbonyl, benzyl, or *p*-methoxybenzyloxycarbonyl.

In one or more embodiments, D is

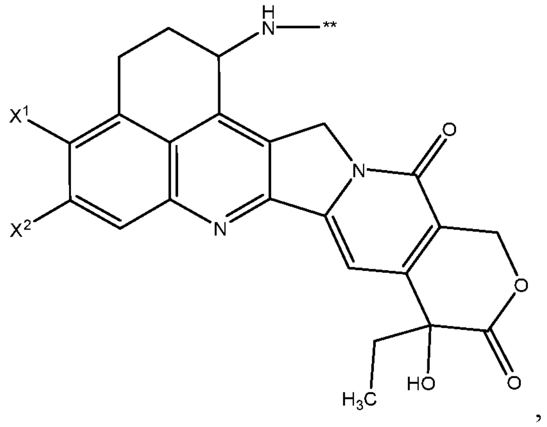

wherein $X^1$ and $X^2$ are each independently C1-C6 alkyl, helogen, or -OH; ** is point of connection.

In one or more embodiments, D is

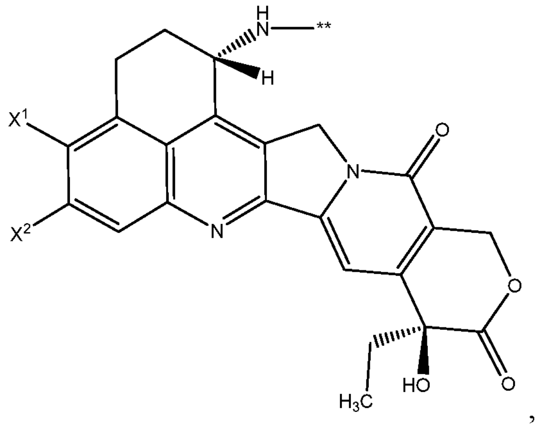

wherein $X^1$ and $X^2$ are each independently C1-C6 alkyl, halogen, or -OH; ** is point of connection.

In one or more embodiments, $X^1$ and $X^2$ are each $-CH_3$.

In one or more embodiments, $X^1$ and $X^2$ are each independently F, Cl, Br, or I.

In one or more embodiments, $X^1$ and $X^2$ are each F.

In one or more embodiments, $X^1$ and $X^2$ are each independently $-CH_3$, F, or -OH.

In one or more embodiments, $X^1$ and $X^2$ are each independently F or $-CH_3$.

In one or more embodiments, $X^1$ is $-CH_3$ and $X^2$ is F.

In one or more embodiments, R is $-(CH_2)_r-$.

In one or more embodiments, R is $-(CH_2)_r-$, wherein r is 1 or 5.

In one or more embodiments, n is 4-12.

In one or more embodiments, n is 4-8.

In one or more embodiments, n is 4.

In one or more embodiments, n is 8.

In one or more embodiments, p is 2-8.

In one or more embodiments, p is 4-8.

In one or more embodiments, p is 6-8.

In one or more embodiments, p is 7-8.

In one or more embodiments, Formula I is:

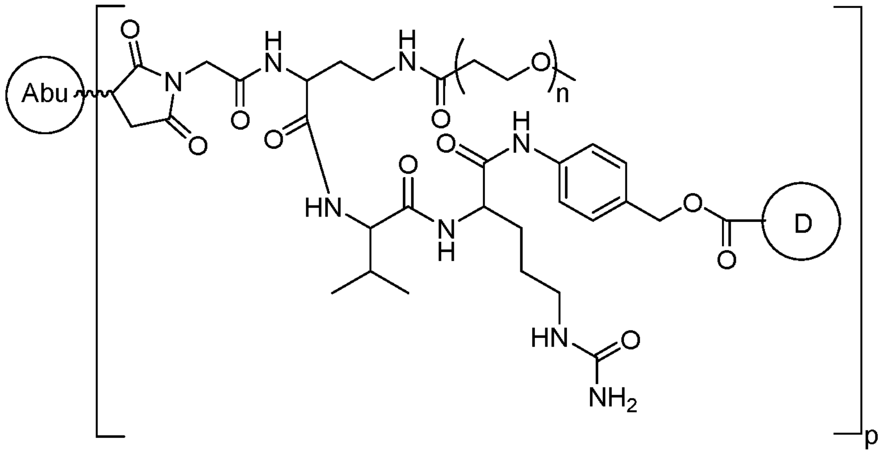

Formula I-3

wherein

Abu is a polypeptide, such as an antibody or an antigen-binding unit thereof;

D is a drug, such as an anti-cancer drug, a cytotoxic drug, a cell differentiation factor, a stem cell trophic factor, a steroid drug, a drug for treating autoimmune diseases, an anti-inflammatory drug or a drug for treating infectious diseases;

n is an integer from 1 to 24, e.g., 1, 2, 3, 4, 5, 6, 7, 8, 9, 10, 11, 12, 13, 14, 15, 16, 17, 18, 19, 20, 21, 22, 23, or 24;

p is 1-10, e.g., 1, 2, 3, 4, 5, 6, 7, 8, 9, or 10.

In one or more embodiments, wherein Formula I is:

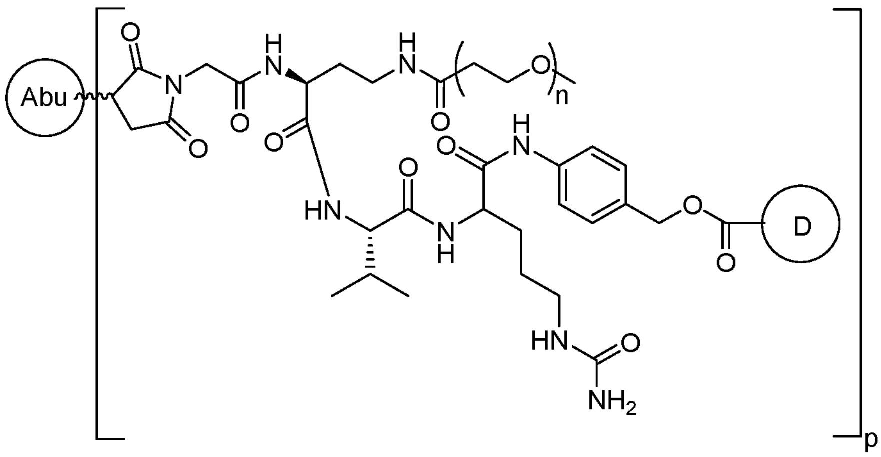

Formula I-4

or

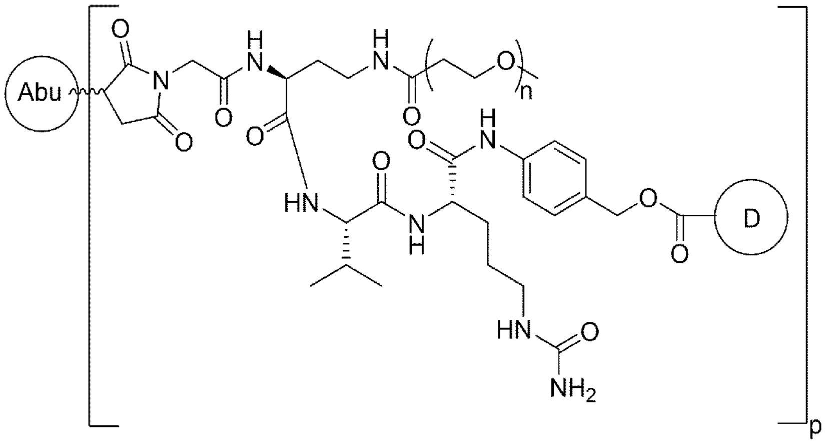

Formula I-4-1

wherein

Abu is a polypeptide, such as an antibody or an antigen-binding unit thereof;

D is a drug, such as an anti-cancer drug, a cytotoxic drug, a cell differentiation factor, a stem cell trophic factor, a steroid drug, a drug for treating autoimmune diseases, an anti-inflammatory drug or a drug for treating infectious diseases;

n is an integer from 1 to 24, e.g., 1, 2, 3, 4, 5, 6, 7, 8, 9, 10, 11, 12, 13, 14, 15, 16, 17, 18, 19, 20, 21, 22, 23 or 24;

p is 1-10, e.g., 1, 2, 3, 4, 5, 6, 7, 8, 9 or 10.

In one or more embodiments, D is a tubulin inhibitor, a DNA damaging agent, or a DNA topoisomerase inhibitor.

In one or more embodiments, the tubulin inhibitor is selected from dolastatin, auristatins and maytansinoids.

In one or more embodiments, D is an auristatin, e.g., MMAE (monomethyl auristatin E), MMAF (monomethyl auristatin F), or AF (auristatin F).

In one or more embodiments, D is a DNA damaging agent, e.g., a calicheamicin, a duocarmycin, the anthramycin derivative PBD (pyrrolobenzodiazepine), or a DNA topoisomerase inhibitor.

In one or more embodiments, D is a DNA topoisomerase inhibitor or a salt thereof, e.g., irinotecan, irinotecan hydrochloride, camptothecin, 9-aminocamptothecin, 9-nitrocamptothecin, 10-hydroxycamptothecin, 9-chloro-10-hydroxycamptothecin, the camptothecin derivative SN-38, 22-hydroxyacuminatine, topotecan, lurtotecan, belotecan, exatecan, homosilatecan, 6,8-dibromo-2-methyl-3-[2-(D-xylopyranosylamino)phenyl]-4(3*H*)-quinazolinone, 2-cyano-3-(3,4-dihydroxyphenyl)-*N*-(phenylmethyl)-(2*E*)-2-propenamide, 2-cyano-3-(3,4-dihydroxyphenyl)-*N*-(3-hydroxyphenylpropyl)-(*E*)-2-propenamide, 12-β-D-glucopyranosyl-12,13-dihydro-2,10-dihydroxy-6-[[2-hydroxy-1-(hydroxymethyl)ethyl]amino]-5*H*-indolo[2,3-*a*]pyrrolo[3,4-*c*]carbazole-5, 7(6*H*)-dione, *N*-[2-(dimethylamino)ethyl]-4-acridinecarboxamide dihydrochloride, or *N*-[2-(dimethylamino)ethyl]-4-acridinecarboxamide.

In one or more embodiments, the DNA topoisomerase inhibitor is camptothecin, 10-hydroxycamptothecin, topotecan, belotecan, irinotecan, 22-hydroxyacuminatine, or exatecan, or a salt thereof.

In one or more embodiments, D is a tubulysin, a taxane drug derivative, a leptomycine derivative, CC-1065 or an analog thereof, an amatoxin, a spliceosome inhibitor, a benzodiazepine (PBD) dimer, adriamycin, methotrexate, vincristine, vinblastine, daunorubicin, mitomycin C, melphalan, or a chlorambucil derivative.

In one or more embodiments, D is

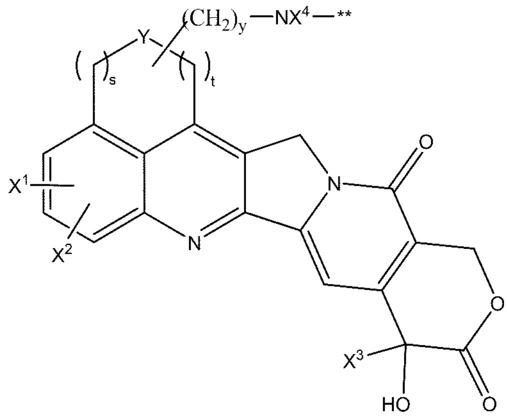

,

wherein

$X^1$ and $X^2$ are each independently:

H,

hydroxy,

C1-C6 alkyl,

C1-C6 alkyl substituted with one or more hydroxy, halogen, nitro or cyano groups,

C2-C6 alkenyl,

C2-C6 alkynyl,

C1-C6 alkoxy,

C1-C6 aminoalkoxy,

halogen,

nitro,

cyano,

thiol,

alkylthio,

amino, amino substituted with an amino-protecting group, C1-C6 aminoalkyl optionally substituted at the amino moiety with an amino-protecting group or C1-C6 alkyl,

C1-C6 aminoalkylamino optionally substituted at the amino moiety with an amino-protecting group or C1-C6 alkyl,

C1-C6 alkyl linking to a heterocyclic ring, wherein the heterocyclic ring is optionally substituted with one or more C1-C6 alkyl, C1-C6 alkoxy, amino, halogen, nitro or cyano groups,

C1-C6 alkylamino linking to a heterocyclic ring, wherein the heterocyclic ring is optionally substituted with C1-C6 alkyl or C1-C6 alkoxy, and the amino is optionally substituted with an amino-protecting group, halogen, nitro, cyano or protecting group, amino-substituted heterocyclyl, which is optionally substituted at a nitrogen atom of the heterocyclyl moiety or at the amino moiety with a protecting group or one or more C1-C6 alkyl groups,

heterocyclylamino, which is optionally substituted at a nitrogen atom of the heterocyclic moiety or at the amino moiety with a protecting group or C1-C6 alkyl, carbamoyl optionally substituted with a carbamoyl-protecting group or C1-C6 alkyl, morpholin-1-yl, or

piperidin-1-yl;

$X^3$ is C1-C6 alkyl;

$X^4$ is H, $-(CH_2)_q-CH_3$, $-(CHR^n)_q-CH_3$, C3-C8 carbocyclyl, $-O-(CH_2)_q-CH_3$, arylene-$CH_3$, $-(CH_2)_q$-arylene-$CH_3$, -arylene-$(CH_2)_q-CH_3$, $-(CH_2)_q$-(C3-C8 carbocyclyl)-$CH_3$, -(C3-C8 carbocyclyl)-$(CH_2)_q-CH_3$, C3-C8 heterocyclyl, $-(CH_2)_q$-(C3-C8 heterocyclyl)-$CH_3$, -(C3-C8 heterocyclyl)-$(CH_2)_q-CH_3$, $-(CH_2)_qC(O)NR^n(CH_2)_q-CH_3$, $-(CH_2CH_2O)_q-CH_3$, $-(CH_2CH_2O)_q-CH_2-CH_3$, $-(CH_2)_qC(O)NR^n(CH_2CH_2O)_q-CH_3$, $-(CH_2)_qC(O)NR^n(CH_2CH_2O)_q-CH_2-CH_3$, $-(CH_2CH_2O)_qC(O)NR^n(CH_2CH_2O)_q-CH_3$, $-(CH_2CH_2O)_qC(O)NR^n(CH_2CH_2O)_q-CH_2-CH_3$ or $-(CH_2CH_2O)_qC(O)NR^n(CH_2)_q-CH_3$; wherein each $R^n$ is independently H, C1-C6 alkyl, C3-C8 carbocyclyl, phenyl or benzyl, and each q is independently 1, 2, 3, 4, 5, 6, 7, 8, 9 or 10;

** is point of connection;

y is 0, 1 or 2;

Y is O, S or $CR^1R^2$, wherein $R^1$ and $R^2$ are each independently H or C1-C6 alkyl;

s and t are each independently 0, 1 or 2, but not both 0.

In one or more embodiments, $X^4$ is H or C1-C6 alkyl.

In one or more embodiments, the heterocyclyl is azetidine, niverazine, morpholine, pyrrolidine, piperidine, imidazole, thiazole, oxazole or pyridine.

In one or more embodiments, the amino-protecting group is formyl, acetyl, trityl, *t*-butoxycarbonyl, benzyl, or *p*-methoxybenzyloxycarbonyl.

In one or more embodiments, D is

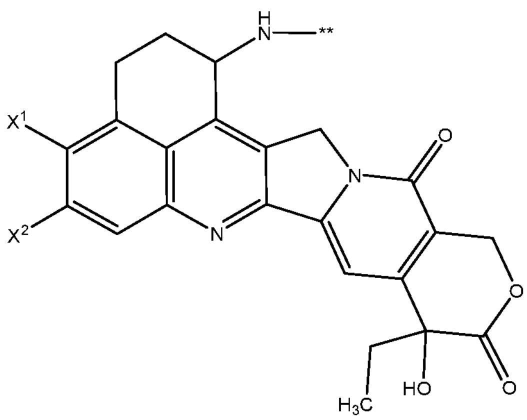

,

wherein $X^1$ and $X^2$ are each independently C1-C6 alkyl, halogen, or -OH; ** is point of connection.

In one or more embodiments, D is

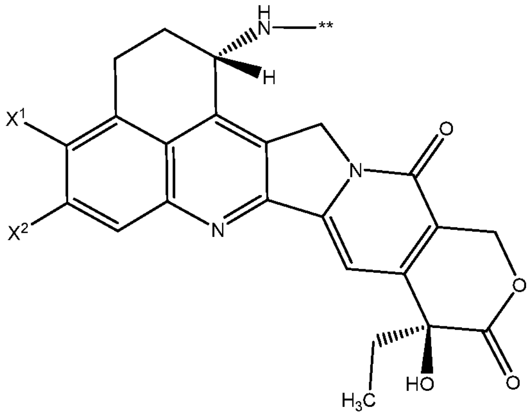

,

wherein $X^1$ and $X^2$ are each independently C1-C6 alkyl, halogen, or -OH; ** is point of connection.

In one or more embodiments, $X^1$ and $X^2$ are each $-CH_3$.

In one or more embodiments, $X^1$ and $X^2$ are each independently F, Cl, Br, or I.

In one or more embodiments, $X^1$ and $X^2$ are each F.

In one or more embodiments, $X^1$ and $X^2$ are each independently $-CH_3$, F, or -OH.

In one or more embodiments, $X^1$ and $X^2$ are each independently F or $-CH_3$.

In one or more embodiments, $X^1$ is $-CH_3$ and $X^2$ is F.

In one or more embodiments, n is 4-12.

In one or more embodiments, n is 4-8.

In one or more embodiments, n is 4.

In one or more embodiments, n is 8.

In one or more embodiments, p is 2-8.

In one or more embodiments, p is 4-8.

In one or more embodiments, p is 6-8.

In one or more embodiments, p is 7-8.

In one or more embodiments, Formula I is:

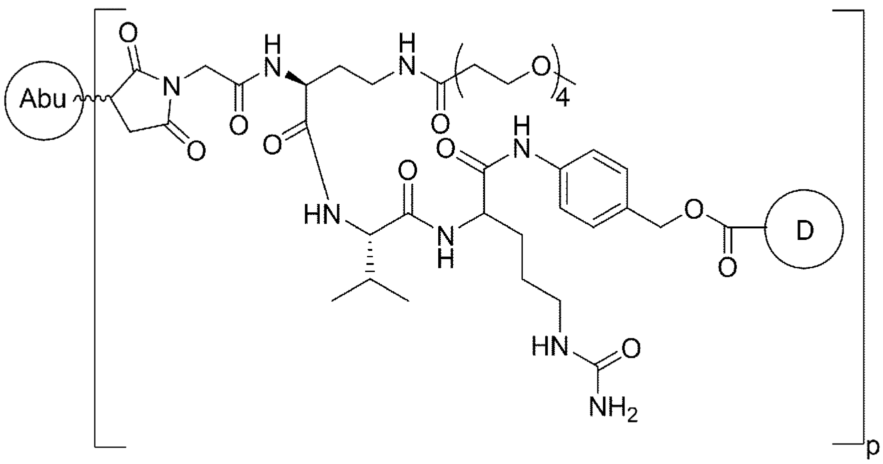

Formula I-5

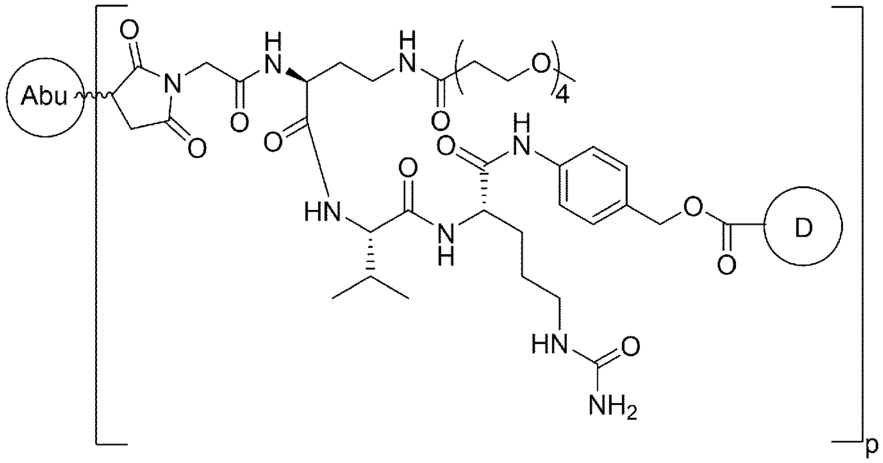

Formula I-5-1

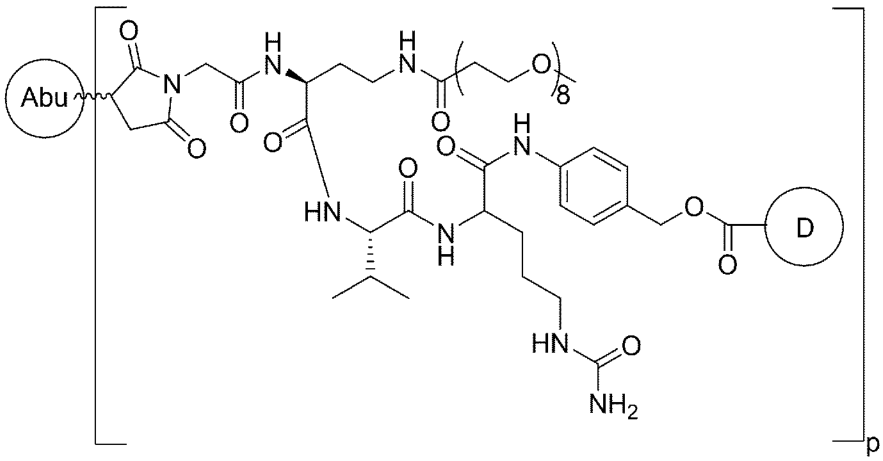

Formula I-6

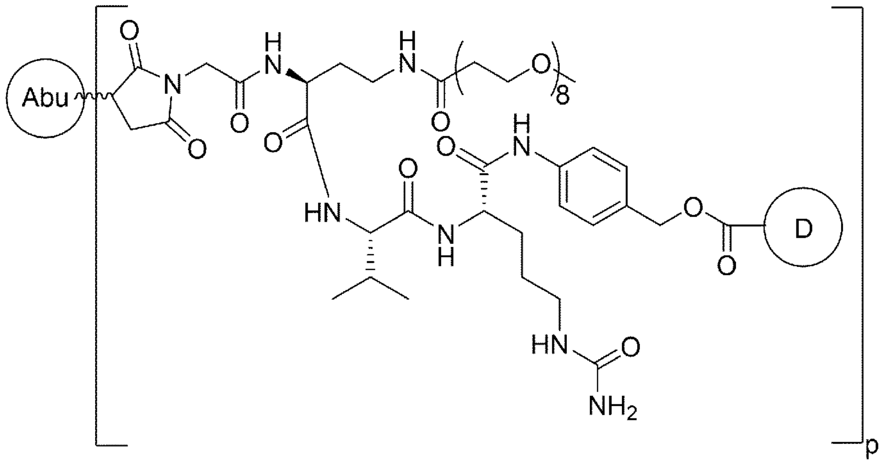

Formula I-6-1

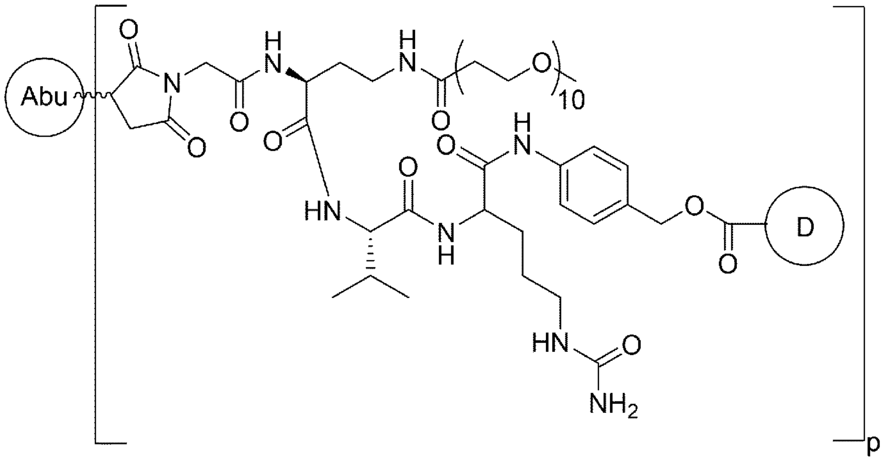

Formula I-7

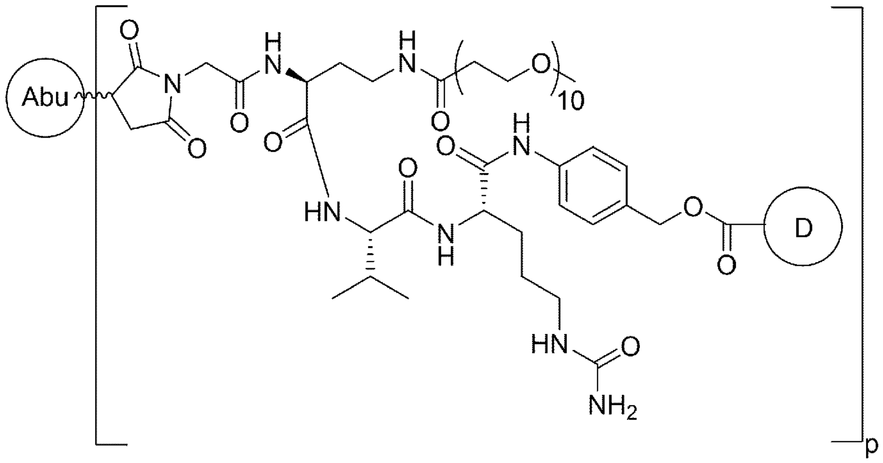

Formula I-7-1

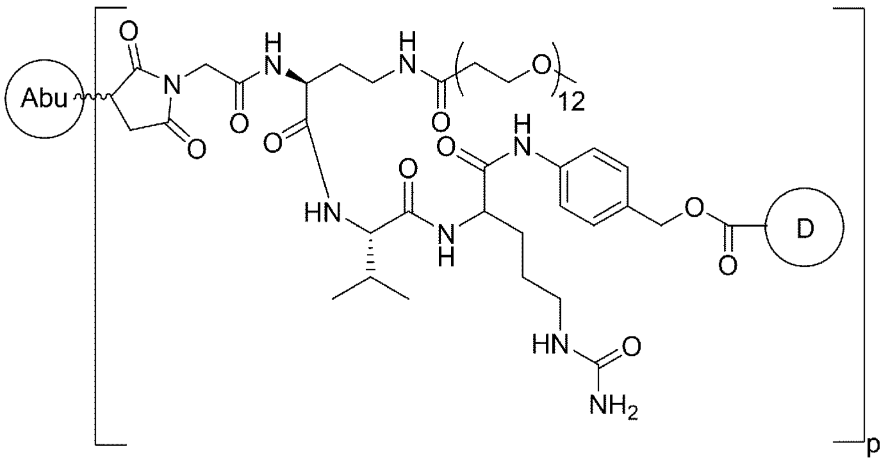

Formula I-8

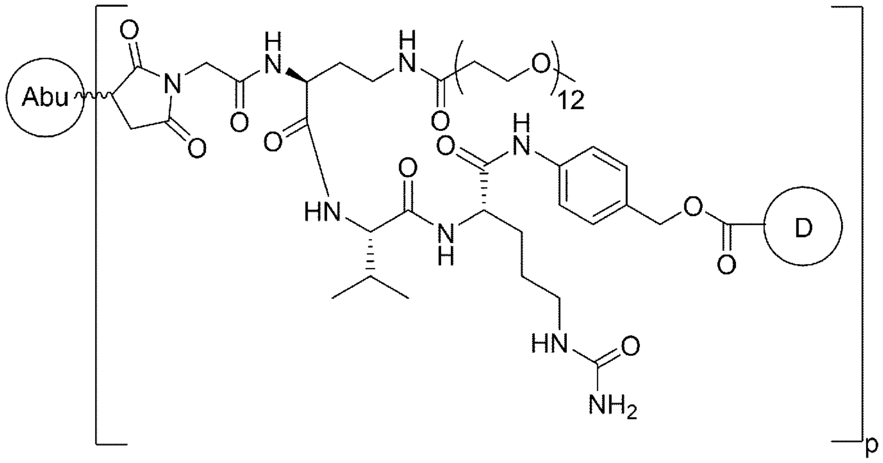

Formula I-8-1

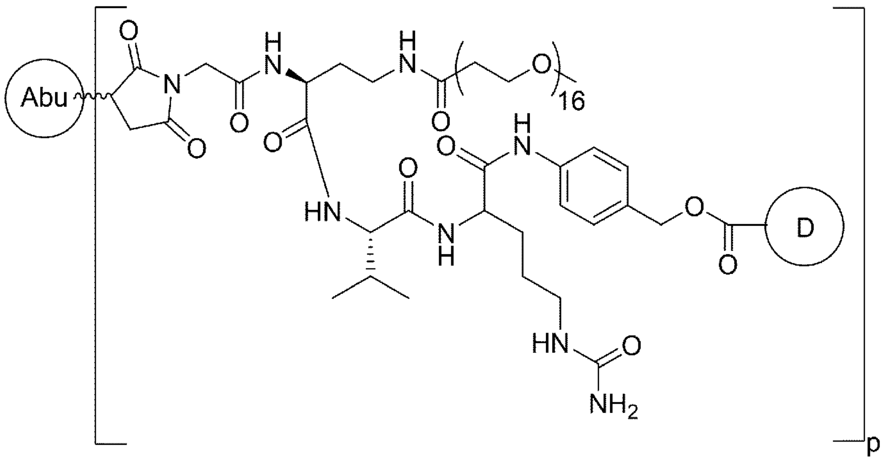

Formula I-9

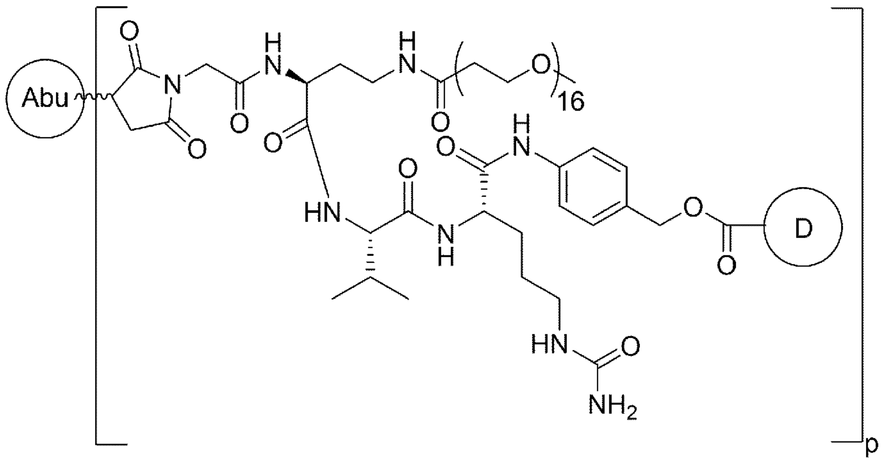

Formula I-9-1

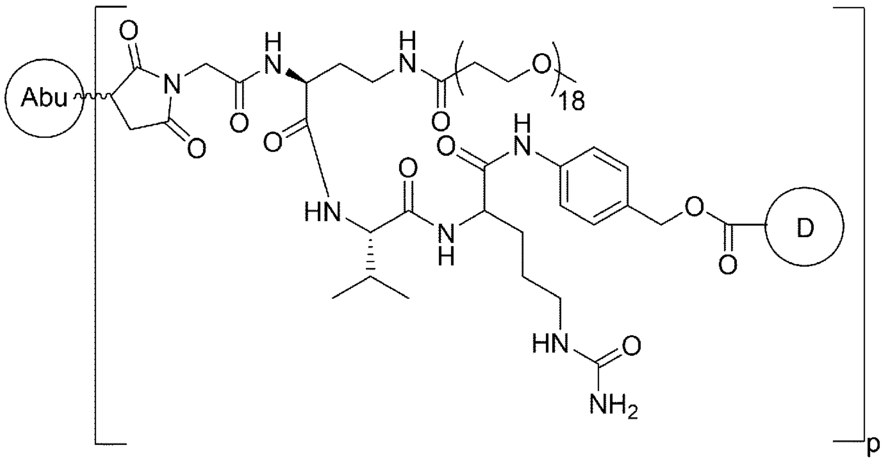

Formula I-10

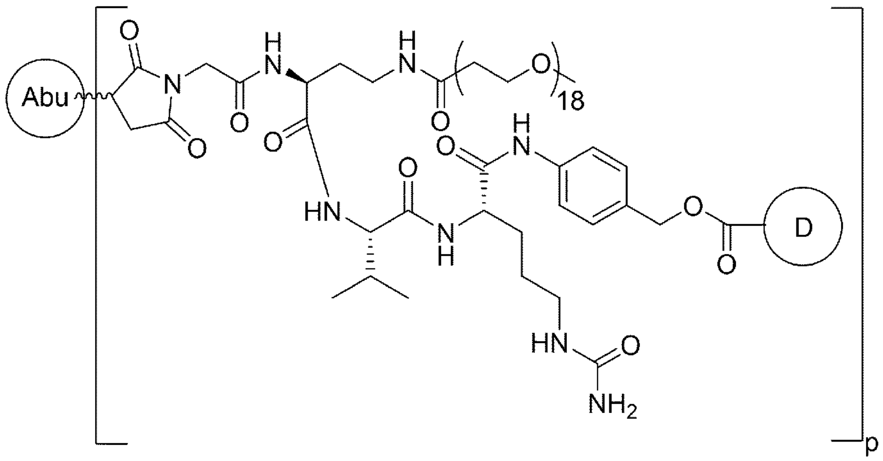

Formula I-10-1

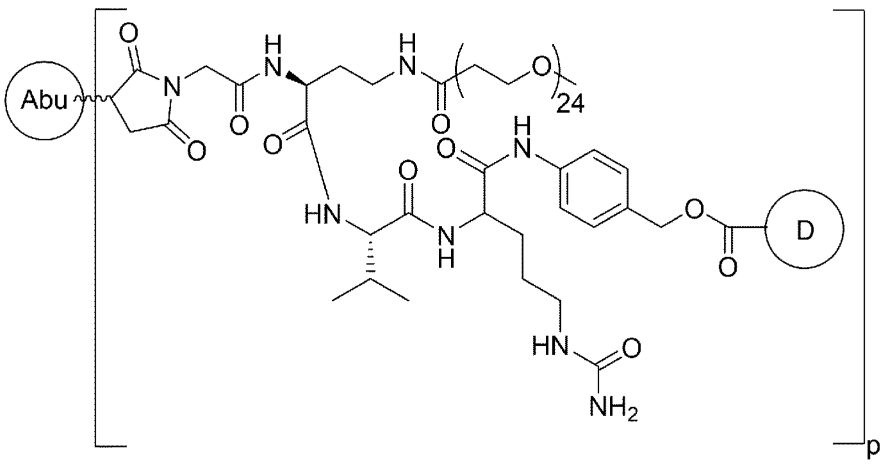

Formula I-11

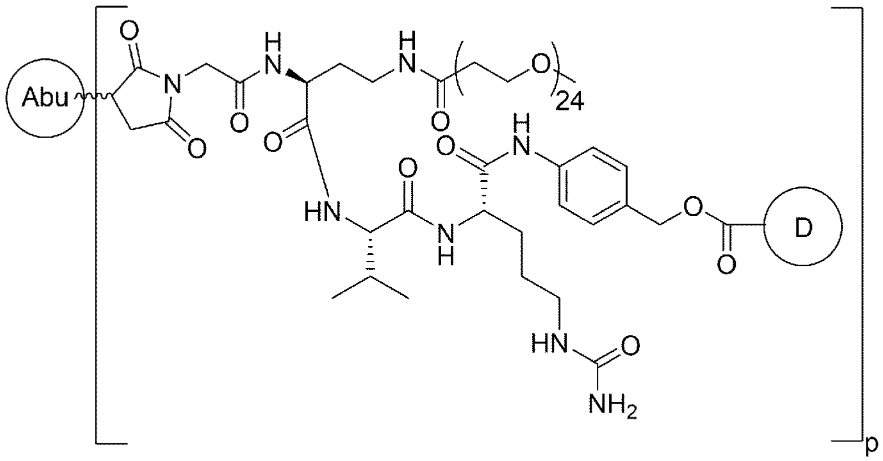

Formula I-11-1

wherein

Abu is a polypeptide, such as an antibody or an antigen-binding unit thereof;

D is a drug, such as an anti-cancer drug, a cytotoxic drug, a cell differentiation factor, a stem cell trophic factor, a steroid drug, a drug for treating autoimmune diseases, an anti-inflammatory drug or a drug for treating infectious diseases;

p is 1-10, e.g., 1, 2, 3, 4, 5, 6, 7, 8, 9 or 10.

In one or more embodiments, D is a tubulin inhibitor, a DNA damaging agent, or a DNA topoisomerase inhibitor.

In one or more embodiments, the tubulin inhibitor is selected from dolastatin, auristatins and maytansinoids.

In one or more embodiments, D is an auristatin, e.g., MMAE (monomethyl auristatin E), MMAF (monomethyl auristatin F), or AF (auristatin F).

In one or more embodiments, D is a DNA damaging agent, e.g., a calicheamicin, a duocarmycin, or the anthramycin derivative PBD (pyrrolobenzodiazepine).

In one or more embodiments, D is a DNA topoisomerase inhibitor or a salt thereof, e.g., irinotecan, irinotecan hydrochloride, camptothecin, 9-aminocamptothecin, 9-nitrocamptothecin, 10-hydroxycamptothecin, 9-chloro-10-hydroxycamptothecin, the camptothecin derivative SN-38, 22-hydroxyacuminatine, topotecan, lurtotecan, belotecan, exatecan, homosilatecan, 6,8-dibromo-2-methyl-3-[2-(D-xylopyranosylamino)phenyl]-4(3*H*)-quinazolinone, 2-cyano-3-(3,4-dihydroxyphenyl)-*N*-(phenylmethyl)-(2*E*)-2-propenamide, 2-cyano-3-(3,4-dihydroxyphenyl)-*N*-(3-hydroxyphenylpropyl)-(*E*)-2-propenamide, 12-β-D-glucopyranosyl-12,13-dihydro-2,10-dihydroxy-6-[[2-hydroxy-1-(hydroxymethyl)ethyl]amino]-5*H*-indolo[2,3-*a*]pyrrolo[3,4-*c*]carbazole-5, 7(6*H*)-dione, *N*-[2-(dimethylamino)ethyl]-4-acridinecarboxamide dihydrochloride, or *N*-[2-(dimethylamino)ethyl]-4-acridinecarboxamide.

In one or more embodiments, the DNA topoisomerase inhibitor is camptothecin, 10-hydroxycamptothecin, topotecan, belotecan, irinotecan, 22-hydroxyacuminatine, or exatecan, or a salt thereof.

In one or more embodiments, D is a tubulysin, a taxane drug derivative, a leptomycine derivative, CC-1065 or an analog thereof, an amatoxin, a spliceosome inhibitor, a benzodiazepine (PBD) dimer, adriamycin, methotrexate, vincristine, vinblastine, daunorubicin, mitomycin C, melphalan, or a chlorambucil derivative.

In one or more embodiments, D is

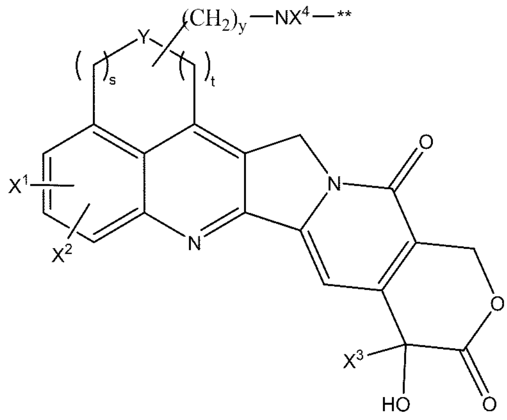

wherein

$X^1$ and $X^2$ are each independently:

H,

hydroxy,

C1-C6 alkyl,

C1-C6 alkyl substituted with one or more hydroxy, halogen, nitro or cyano groups,

C2-C6 alkenyl,

C2-C6 alkynyl,

C1-C6 alkoxy,

C1-C6 aminoalkoxy,

halogen,

nitro,

cyano,

thiol,

alkylthio,

amino, amino substituted with an amino-protecting group, C1-C6 aminoalkyl optionally substituted at the amino moiety with an amino-protecting group or C1-C6 alkyl,

C1-C6 aminoalkylamino optionally substituted at the amino moiety with an amino-protecting group or C1-C6 alkyl,

C1-C6 alkyl linking to a heterocyclic ring, wherein the heterocyclic ring is optionally substituted with one or more C1-C6 alkyl, C1-C6 alkoxy, amino, halogen, nitro or cyano groups,

C1-C6 alkylamino linking to a heterocyclic ring, wherein the heterocyclic ring is optionally substituted with C1-C6 alkyl or C1-C6 alkoxy, and the amino is optionally substituted with an amino-protecting group, halogen, nitro, cyano or protecting group, amino-substituted heterocyclyl, which is optionally substituted at a nitrogen atom of the heterocyclyl moiety or at the amino moiety with a protecting group or one or more C1-C6 alkyl groups,

heterocyclylamino, which is optionally substituted at a nitrogen atom of the heterocyclic moiety or at the amino moiety with a protecting group or C1-C6 alkyl, carbamoyl optionally substituted with a carbamoyl-protecting group or C1-C6 alkyl, morpholin-1-yl, or

piperidin-1-yl;

$X^3$ is C1-C6 alkyl;

$X^4$ is H, $-(CH_2)_q-CH_3$, $-(CHR^n)_q-CH_3$, C3-C8 carbocyclyl, $-O-(CH_2)_q-CH_3$, arylene-$CH_3$, $-(CH_2)_q$-arylene-$CH_3$, -arylene-$(CH_2)_q-CH_3$, $-(CH_2)_q$-(C3-C8 carbocyclyl)-$CH_3$, -(C3-C8 carbocyclyl)-$(CH_2)_q-CH_3$, C3-C8 heterocyclyl, $-(CH_2)_q$-(C3-C8 heterocyclyl)-$CH_3$, -(C3-C8 heterocyclyl)-$(CH_2)_q-CH_3$, $-(CH_2)_qC(O)NR^n(CH_2)_q-CH_3$, $-(CH_2CH_2O)_q-CH_3$, $-(CH_2CH_2O)_q-CH_2-CH_3$, $-(CH_2)_qC(O)NR^n(CH_2CH_2O)_q-CH_3$, $-(CH_2)_qC(O)NR^n(CH_2CH_2O)_q-CH_2-CH_3$, $-(CH_2CH_2O)_qC(O)NR^n(CH_2CH_2O)_q-CH_3$, $-(CH_2CH_2O)_qC(O)NR^n(CH_2CH_2O)_q-CH_2-CH_3$ or $-(CH_2CH_2O)_qC(O)NR^n(CH_2)_q-CH_3$; wherein each $R^n$ is independently H, C1-C6 alkyl, C3-C8 carbocyclyl, phenyl or benzyl, and each q is independently 1, 2, 3, 4, 5, 6, 7, 8, 9 or 10;

** is point of connection;

y is 0, 1 or 2;

Y is O, S or $CR^1R^2$, wherein $R^1$ and $R^2$ are each independently H or C1-C6 alkyl;

s and t are each independently 0, 1 or 2, but not both 0.

In one or more embodiments, $X^4$ is H or C1-C6 alkyl.

In one or more embodiments, the heterocyclyl is azetidine, niverazine, morpholine, pyrrolidine, piperidine, imidazole, thiazole, oxazole or pyridine.

In one or more embodiments, the amino-protecting group is formyl, acetyl, trityl, t-butoxycarbonyl, benzyl, or *p*-methoxybenzyloxycarbonyl.

In one or more embodiments, D is

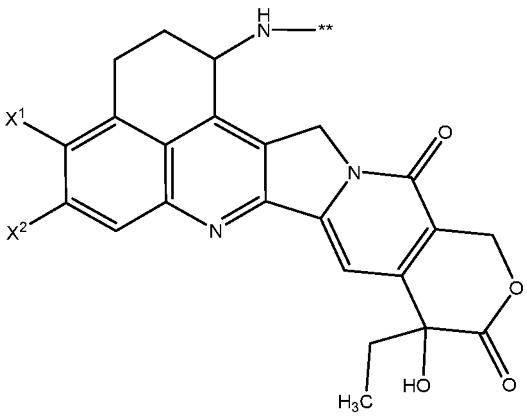

,

wherein $X^1$ and $X^2$ are each independently C1-C6 alkyl, halogen, or -OH; ** is point of connection.

In one or more embodiments, D is

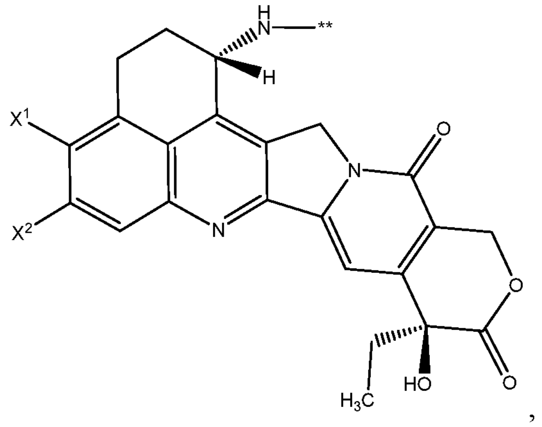

wherein $X^1$ and $X^2$ are each independently C1-C6 alkyl, halogen, or -OH; ** is point of connection.

In one or more embodiments, $X^1$ and $X^2$ are each $-CH_3$.

In one or more embodiments, $X^1$ and $X^2$ are each independently F, Cl, Br, or I.

In one or more embodiments, $X^1$ and $X^2$ are each F.

In one or more embodiments, $X^1$ and $X^2$ are each independently $-CH_3$, F, or -OH.

In one or more embodiments, $X^1$ and $X^2$ are each independently F or $-CH_3$.

In one or more embodiments, $X^1$ is $-CH_3$ and $X^2$ is F.

In one or more embodiments, p is 2-8.

In one or more embodiments, p is 4-8.

In one or more embodiments, p is 6-8.

In one or more embodiments, p is 7-8.

In one or more embodiments, Formula I is:

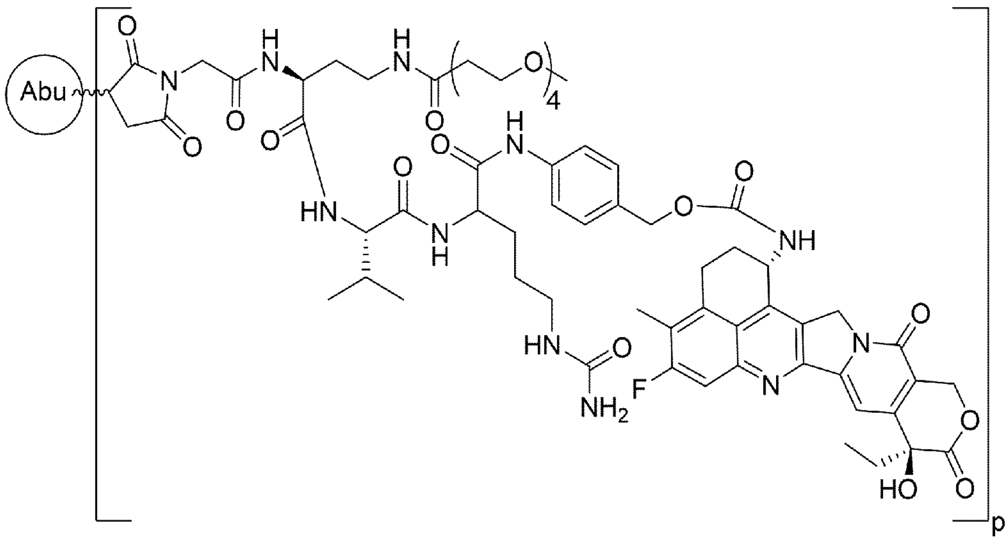

Formula I-12

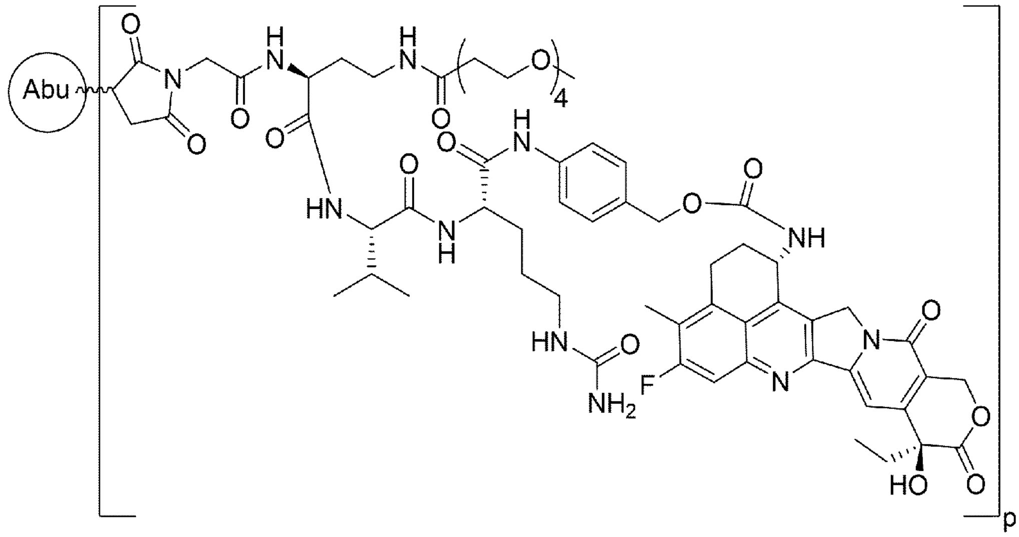

Formula I-12-1

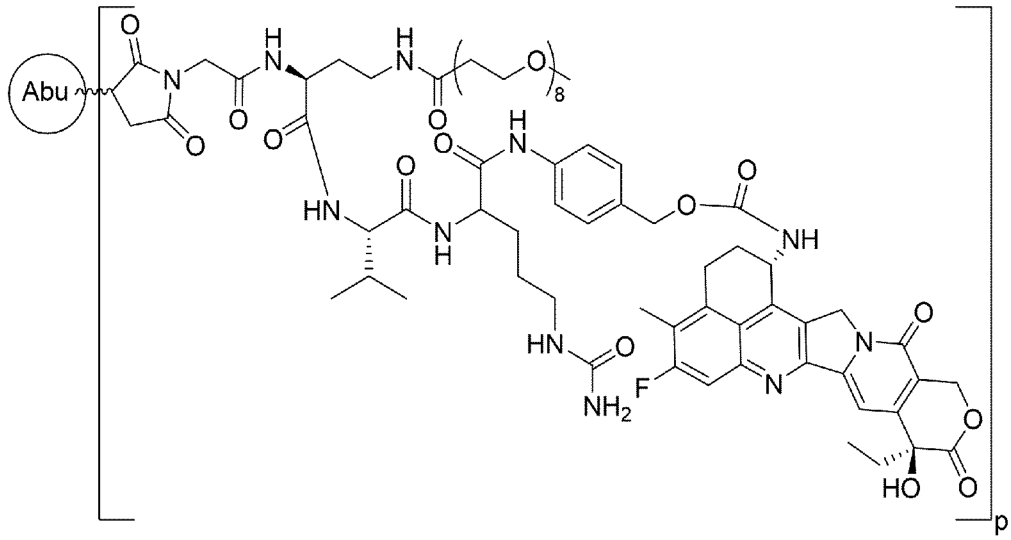

Formula I-13

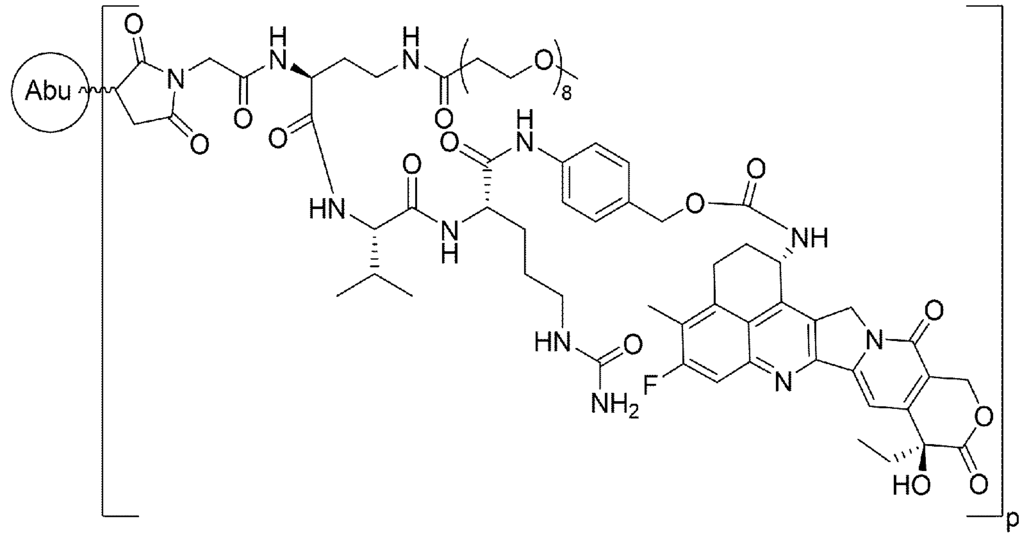

Formula I-13-1

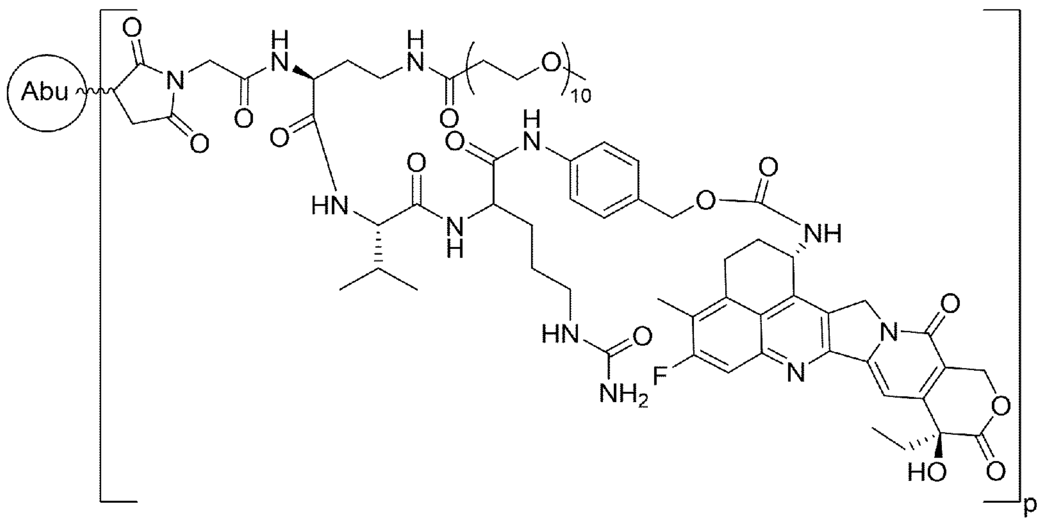

Formula I-14

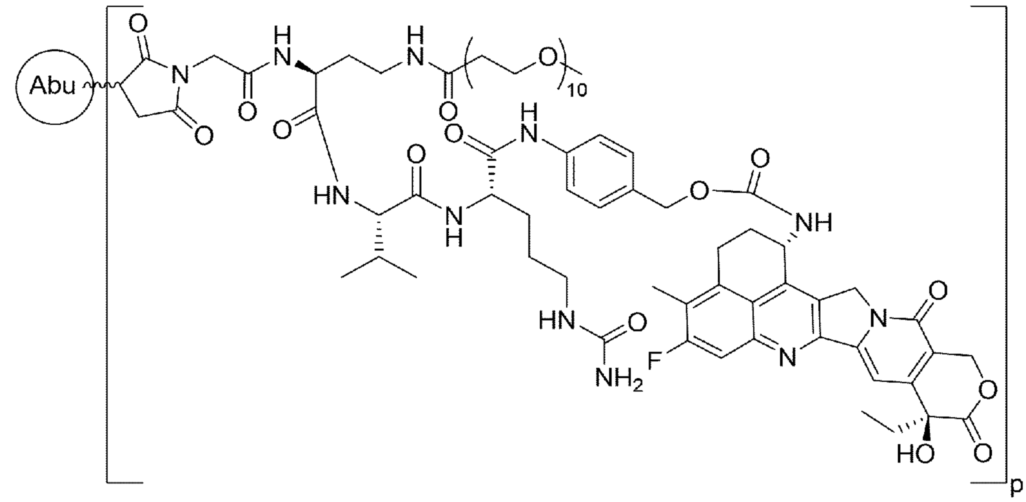

Formula I-14-1

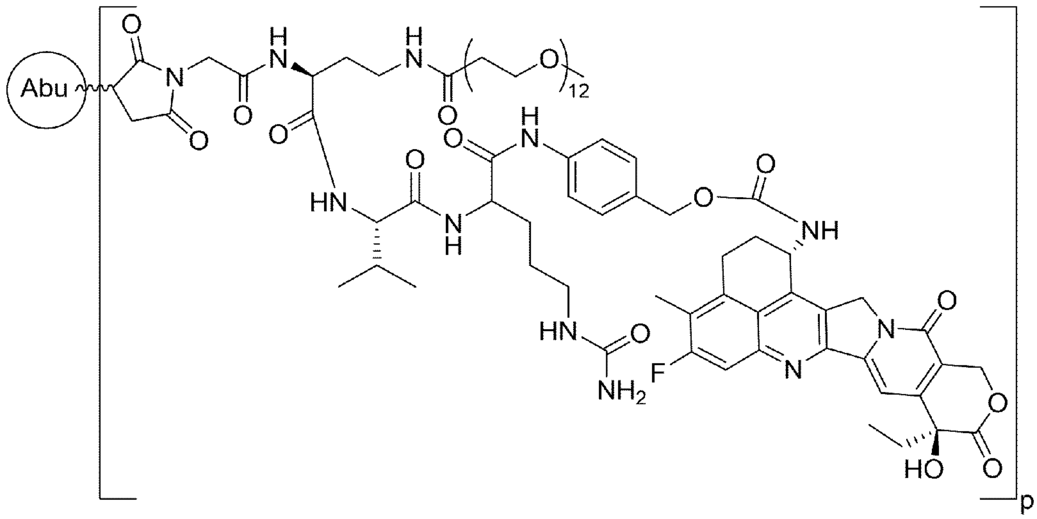

Formula I-15

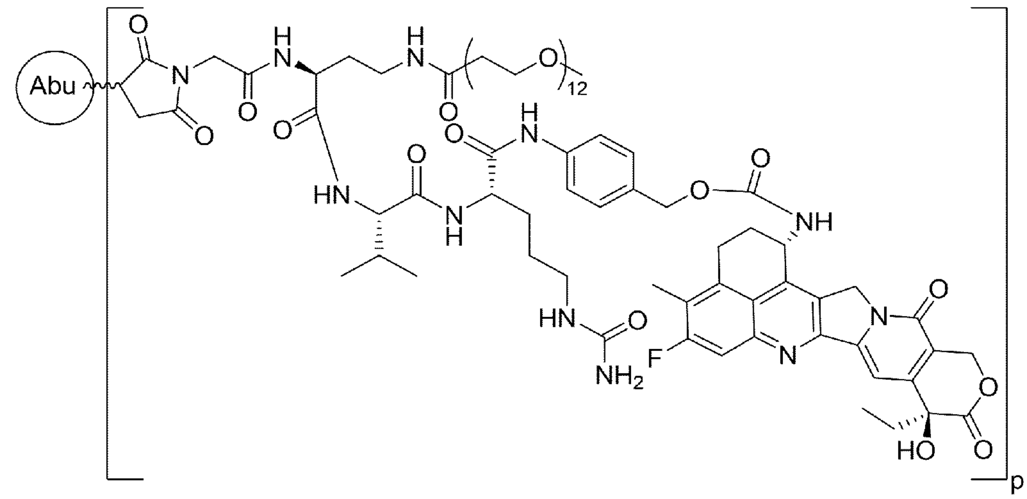

Formula I-15-1

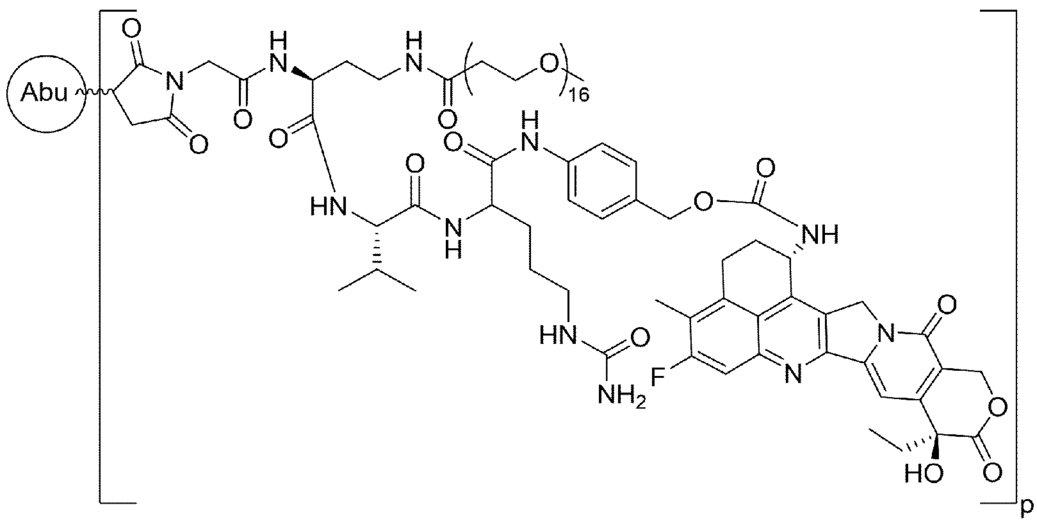

Formula I-16

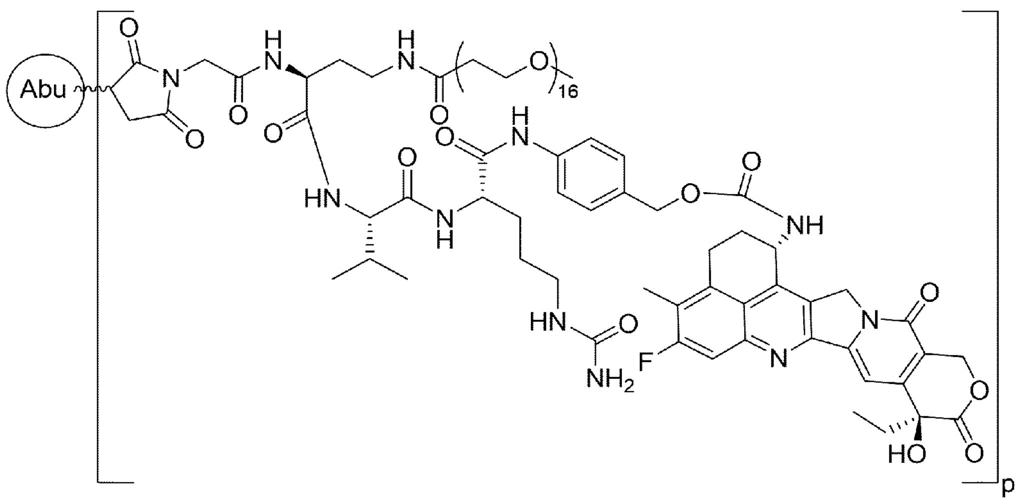

Formula I-16-1

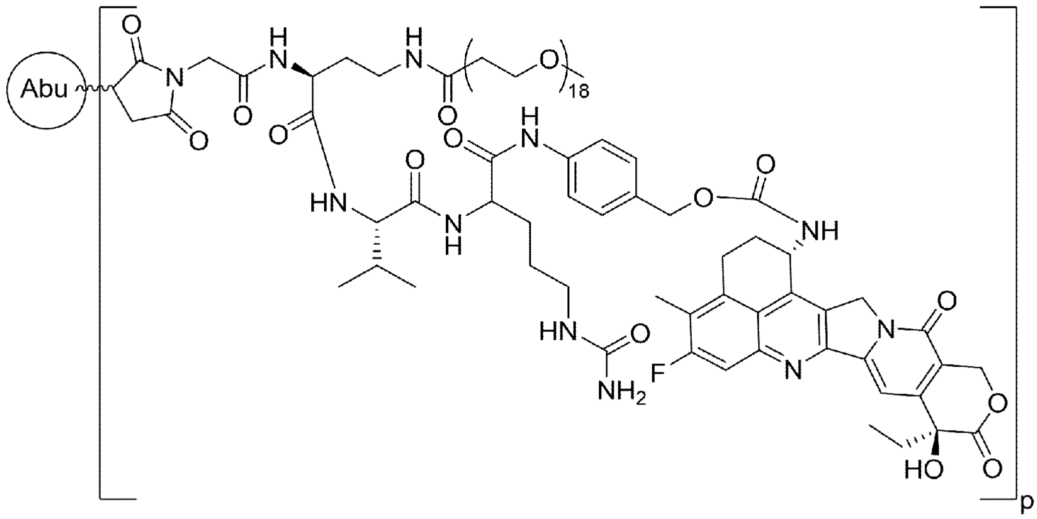

Formula I-17

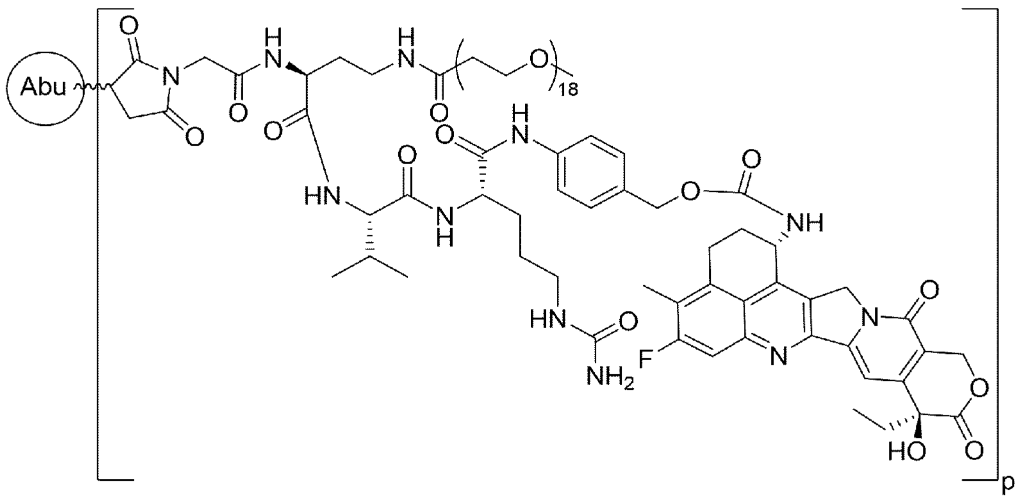

Formula I-17-1

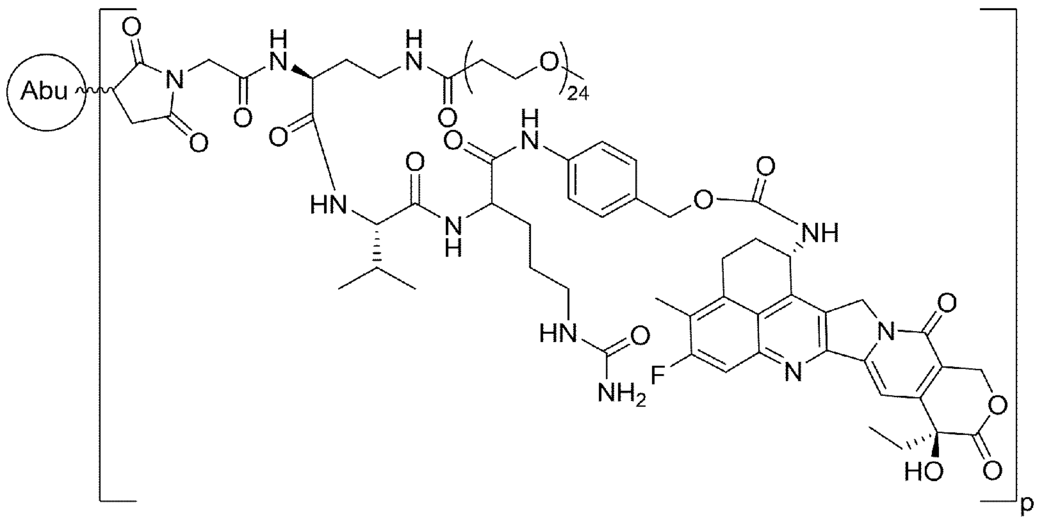

Formula I-18

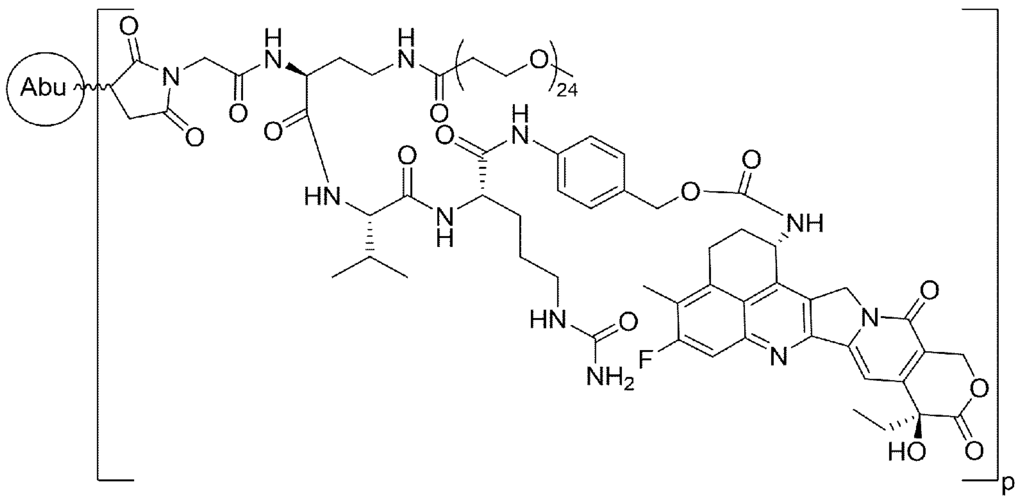

Formula I-18-1

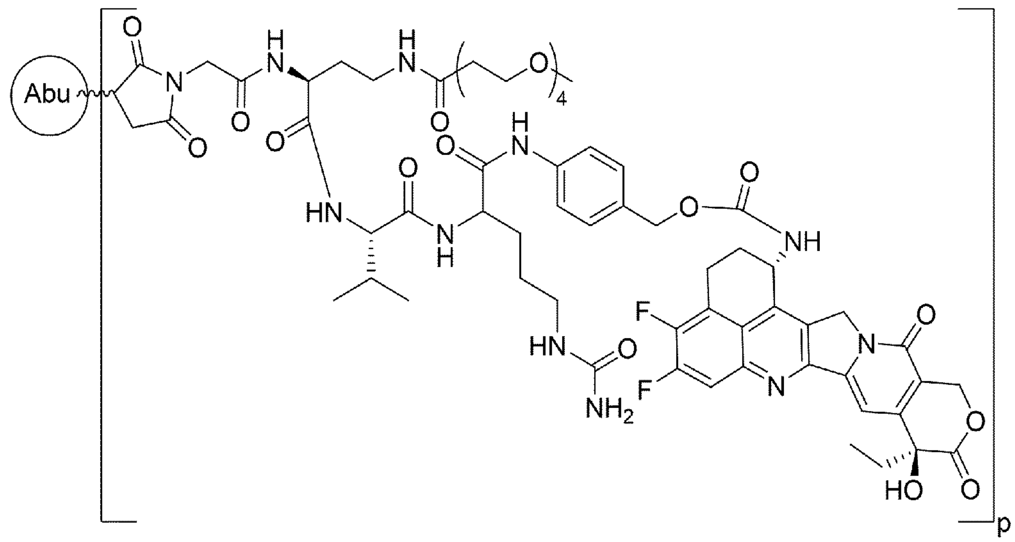

Formula I-19

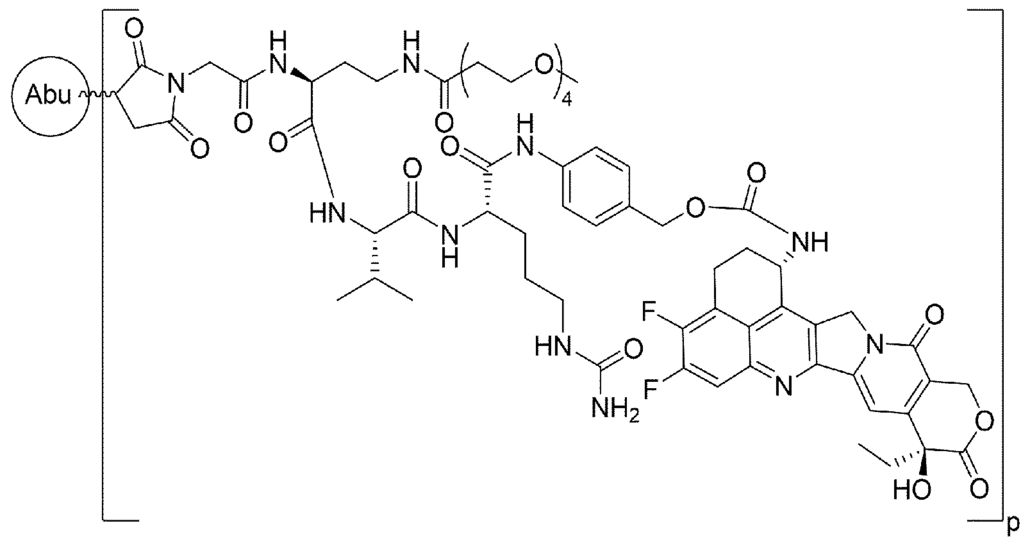

Formula I-19-1

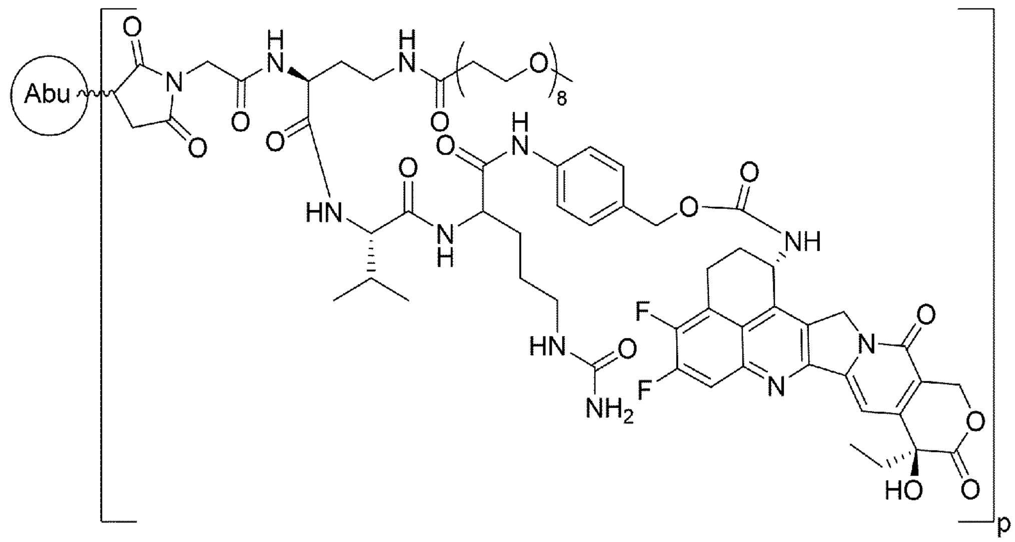

Formula I-20

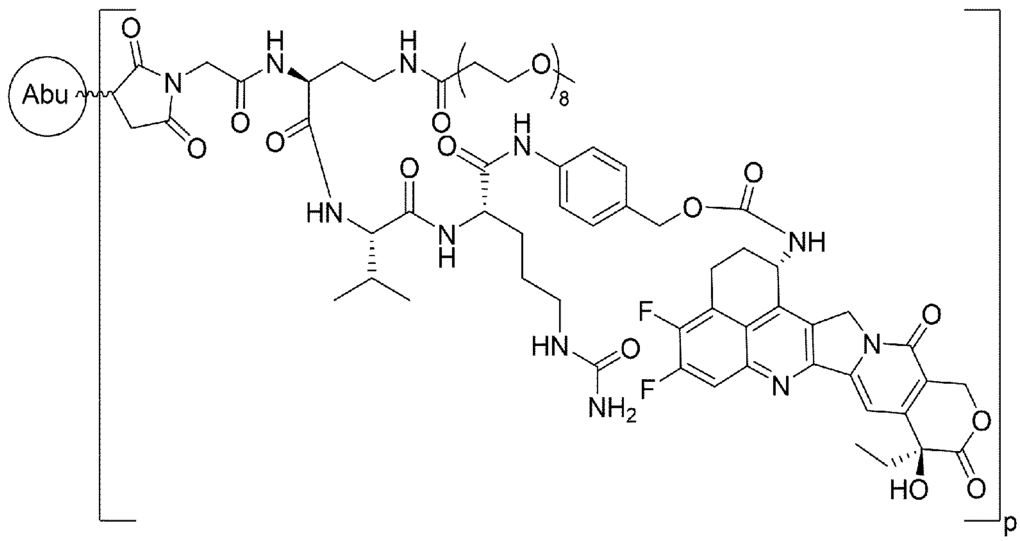

Formula I-20-1

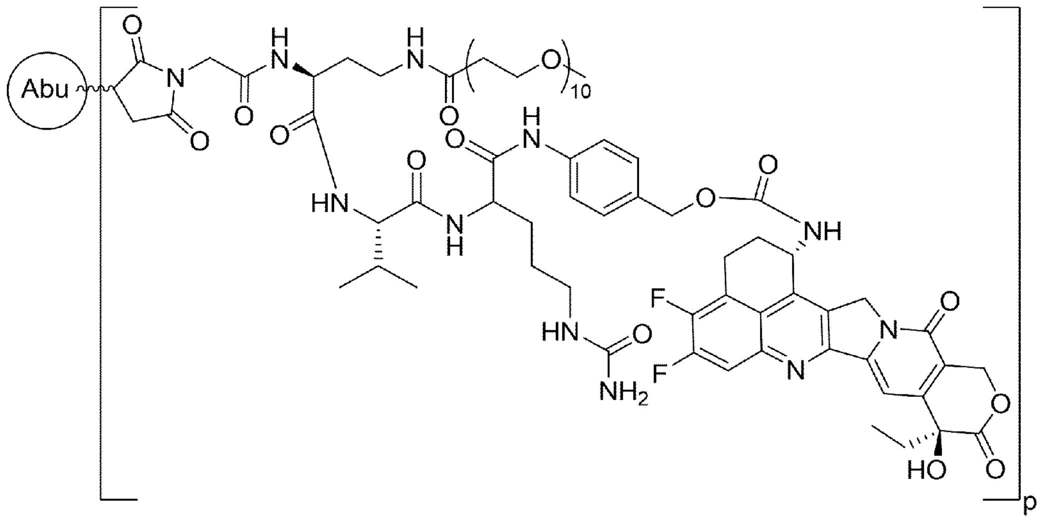

Formula I-21

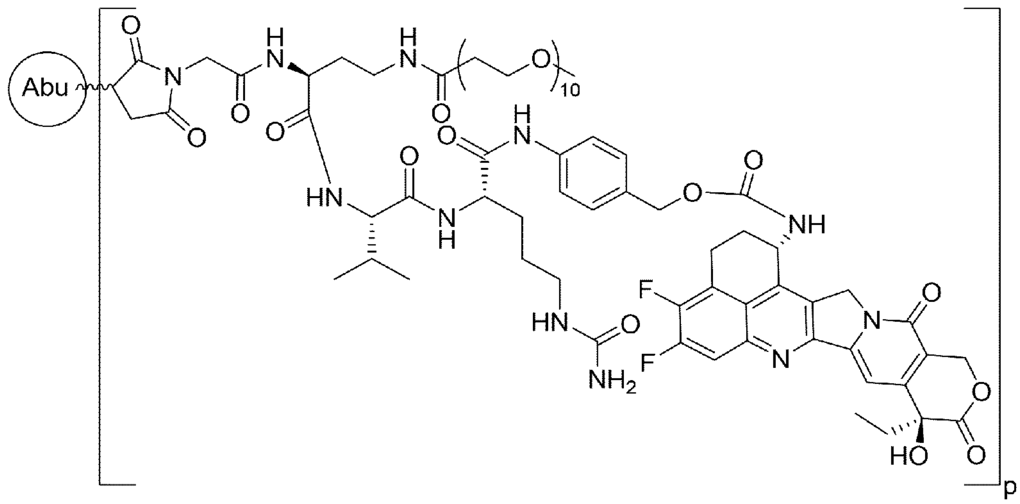

Formula I-21-1

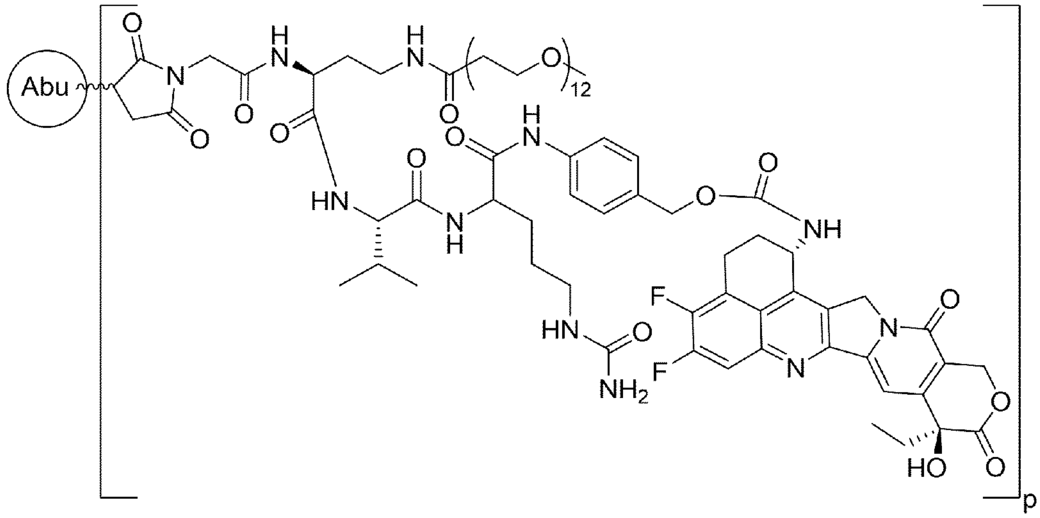

Formula I-22

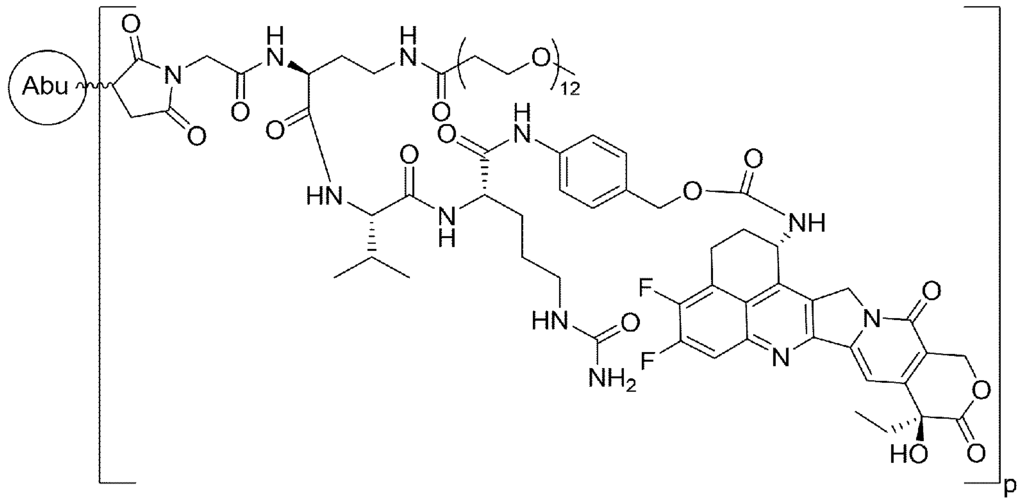

Formula I-22-1

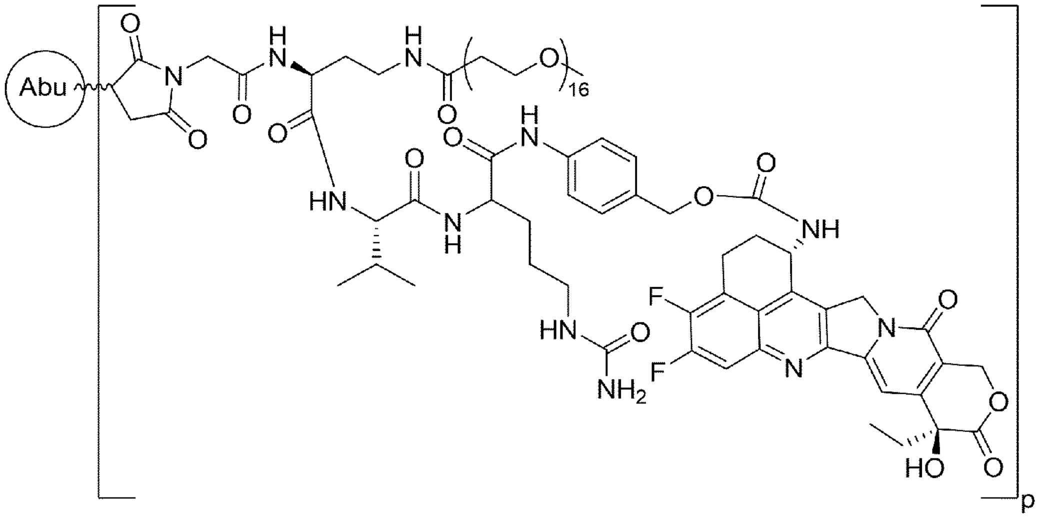

Formula I-23

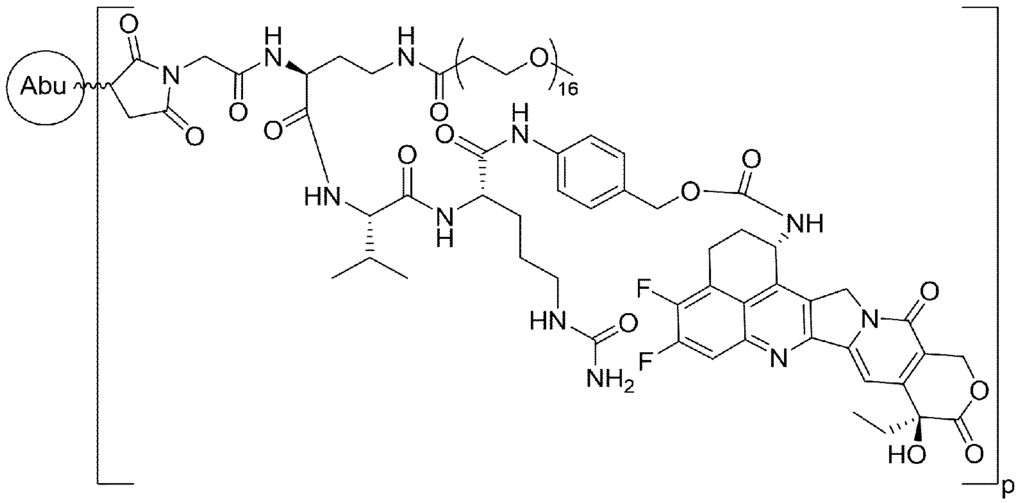

Formula I-23-1

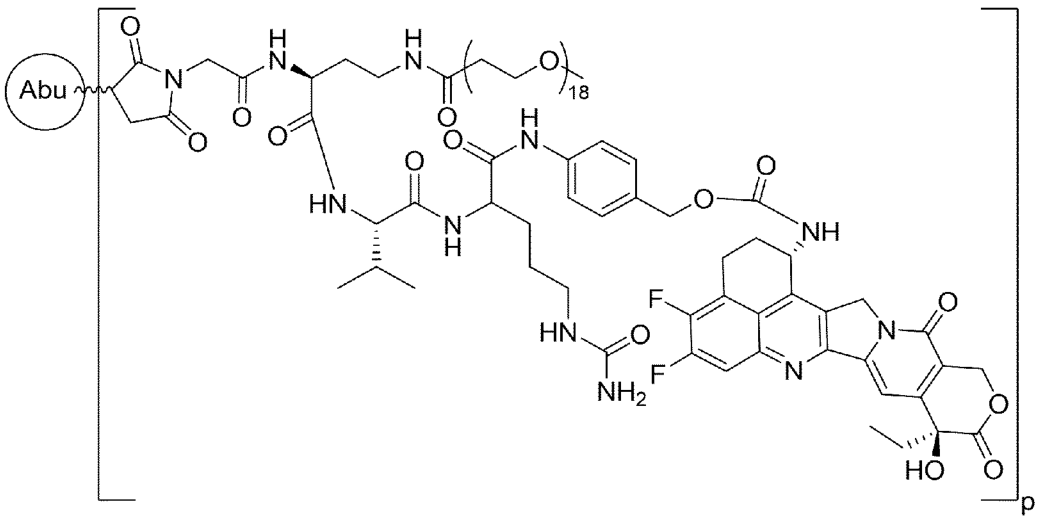

Formula I-24

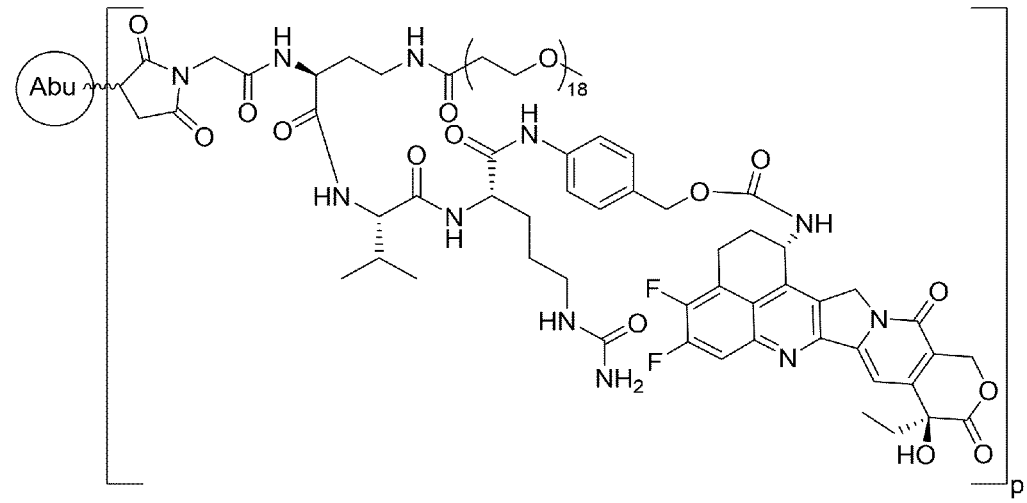

Formula I-24-1

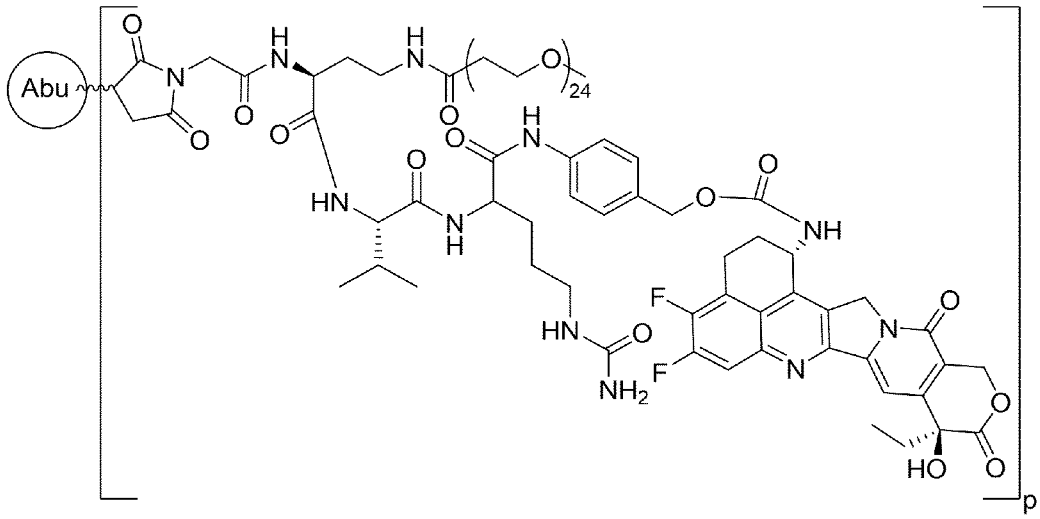

Formula I-25

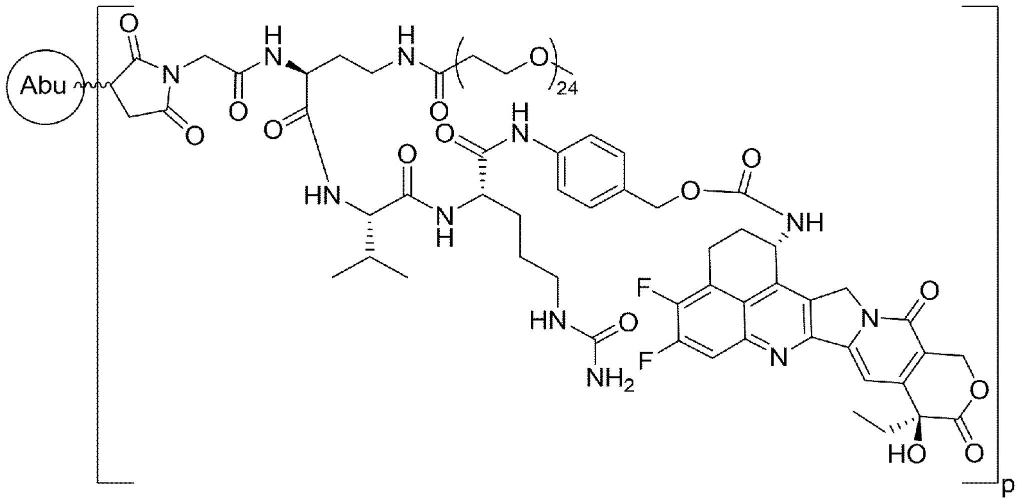

Formula I-25-1

wherein

Abu is a polypeptide, such as an antibody or an antigen-binding unit thereof;

p is 1-10, e.g., 1, 2, 3, 4, 5, 6, 7, 8, 9 or 10.

In one or more embodiments, the Abu is a polypeptide containing a cysteine in the sequence, and connected to other parts of the drug conjugate (such as M of Formula I) through the sulfur atom of cysteine.

In one or more embodiments, the Abu is a polypeptide containing a Fc region. In one or more embodiments, the Abu is connected to other parts of the drug conjugate (such as M of Formula I) through the Fc region.

In one or more embodiments, the binding target for Abu is selected from: HER2, TROP-2, Nection-4, B7H3, B7H4, CLDN18, BMPR1B, E16, STEAP1, 0772P, MPF, Napi3b, Sema5b, PSCAhlg, ETBR, MSG783, STEAP2, TrpM4, CRIPTO, CD20, CD21, CD22, CD30, FcRH2, NCA, MDP, IL20Rα, Brevican, EphB2R, ASLG659, PSCA, GEDA, BAFF-R, CD79a, CD79b, CXCR5, HLA-DOB, P2X5, CD72, LY64, FcRH1, IRTA2, TENB2, PMEL17, TMEFF1, GDNF-Ra1, Ly6E, TMEM46, Ly6G6D, LGR5, RET, LY6K, GPR19, GPR54, ASPHD1, tyrosinase, TMEM118, EpCAM, ROR1, GPR172A, and FRalpha(FRα).

In one or more embodiments, the binding target for Abu is HER2, TROP-2, CLDN18.2, B7H3, or FRα.

In one or more embodiments, the Abu is an antibody or an antigen-binding unit thereof, the drug conjugate is an antibody-drug conjugate.

In one or more embodiments, the Abu is an antibody or an antigen-binding unit thereof containing a Fc region. In one or more embodiments, the Fc region is a human IgG1, IgG2, IgG3, or IgG4 Fc region.

In one or more embodiments, the Abu is connected to other parts of the drug conjugate (such as M of Formula I) through the Fc region.

In one or more embodiments, the Abu is a single domain antibody.

In one or more embodiments, the Abu is a Fab fragment.

In one or more embodiments, the Abu is a single chain antibody.

In one or more embodiments, the Abu is a whole antibody.

In one or more embodiments, the Abu is an anti-HER2 antibody, an anti-Trop2 antibody, an anti-CLDN18.2 antibody, an anti-B7H3 antibody, or an anti-FRα antibody. In one or more embodiments, the Abu is an anti-HER2 monoclonal antibody, an anti-Trop2 monoclonal antibody, an anti-CLDN18.2 monoclonal antibody, an anti-B7H3 monoclonal antibody, or an anti-FRα monoclonal antibody.

In one or more embodiments, Abu is trastuzumab, pertuzumab, panitumumab, nimotuzumab, matuzumab, rituximab, or cetuximab.

In one or more embodiments, the Abu is an anti-CLDN18.2 antibody or an antigen-binding unit thereof, wherein the anti-CLDN18.2 antibody or the antigen-binding unit thereof comprises one or more of (a) - (f), wherein:

(a) VH CDR1, which comprises or consists of an amino acid sequence set forth in SEQ ID NO: 7;

(b) VH CDR2, which comprises or consists of an amino acid sequence set forth in SEQ ID NO: 8;

(c) VH CDR3, which comprises or consists of an amino acid sequence set forth in SEQ ID NO: 9;

(d) VL CDR1, which comprises or consists of an amino acid sequence set forth in SEQ ID NO: 10;

(e) VL CDR2, which comprises or consists of an amino acid sequence set forth in SEQ ID NO: 11;

(f) VL CDR3, which comprises or consists of an amino acid sequence set forth in SEQ ID NO: 12.

In one or more embodiments, the anti-CLDN18.2 antibody or the antigen-binding unit thereof comprises the following CDRs:

(a) VH CDR1, which comprises or consists of an amino acid sequence set forth in SEQ ID NO: 7;

(b) VH CDR2, which comprises or consists of an amino acid sequence set forth in SEQ ID NO: 8;

(c) VH CDR3, which comprises or consists of an amino acid sequence set forth in SEQ ID NO: 9;

(d) VL CDR1, which comprises or consists of an amino acid sequence set forth in SEQ ID NO: 10;

(e) VL CDR2, which comprises or consists of an amino acid sequence set forth in SEQ ID NO: 11;

(f) VL CDR3, which comprises or consists of an amino acid sequence set forth in SEQ ID NO: 12.

In one or more embodiments, the anti-CLDN18.2 antibody or the antigen-binding unit thereof comprises a VH CDR1 set forth in SEQ ID NO: 7, a VH CDR2 set forth in SEQ ID NO: 8, a VH CDR3 set forth in SEQ ID NO: 9, a VL CDR1 set forth in SEQ ID NO: 10, a VL CDR2 set forth in SEQ ID NO: 11, and a VL CDR3 set forth in SEQ ID NO: 12.

In one or more embodiments, the anti-CLDN18.2 antibody or the antigen-binding unit thereof comprises a heavy chain variable region and a light chain variable region; wherein the heavy chain variable region comprises a sequence set forth in SEQ ID NO: 13, or a sequence having at least 90% identity to the sequence set forth in SEQ ID NO: 13, or an amino acid sequence having one or more conservative amino acid substitutions as compared to the sequence set forth in SEQ ID NO: 13; and/or

wherein the light chain variable region comprises a sequence set forth in SEQ ID NO: 14, or a sequence having at least 90% identity to the sequence set forth in SEQ ID NO: 14, or an amino acid sequence having one or more conservative amino acid substitutions as compared to the sequence set forth in SEQ ID NO: 14.

In one or more embodiments, the anti-CLDN18.2 antibody or the antigen-binding unit thereof comprises a heavy chain constant region, wherein the heavy chain constant region comprises an amino acid sequence set forth in SEQ ID NO: 15 or 16.

In one or more embodiments, the anti-CLDN18.2 antibody or the antigen-binding unit thereof comprises a light chain constant region, wherein the light chain constant region comprises an amino acid sequence set forth in SEQ ID NO: 17.

In one or more embodiments, the anti-CLDN18.2 antibody or the antigen-binding unit thereof comprises a heavy chain constant region and a light chain constant region, wherein the heavy chain constant region comprises an amino acid sequence set forth in SEQ ID NO: 15; wherein the light chain constant region comprises an amino acid sequence set forth in SEQ ID NO: 17.

In one or more embodiments, the anti-CLDN18.2 antibody or the antigen-binding unit thereof comprises a heavy chain constant region and a light chain constant region, wherein the heavy chain constant region comprises an amino acid sequence set forth in SEQ ID NO: 16; wherein the light chain constant region comprises an amino acid sequence set forth in SEQ ID NO: 17.

In one or more embodiments, the anti-CLDN18.2 antibody is H239H-2b-K-6a-1 antibody, comprising a heavy chain variable region having an amino acid sequence set forth in SEQ ID NO: 13, a heavy chain constant region having an amino acid sequence set forth in SEQ ID NO: 15, a light chain variable region having an amino acid sequence set forth in SEQ ID NO: 14, and a light chain constant region having an amino acid sequence set forth in SEQ ID NO: 17.

In one or more embodiments, the anti-CLDN18.2 antibody is H239H-2b-K-6a-2 antibody, comprising a heavy chain variable region having an amino acid sequence set forth in SEQ ID NO: 13, a heavy chain constant region having an amino acid sequence set forth in SEQ ID NO: 16, a light chain variable region having an amino acid sequence set forth in SEQ ID NO: 14, and a light chain constant region having an amino acid sequence set forth in SEQ ID NO: 17.

In one or more embodiments, the antibody comprises a sequence having at least 80% identity to the H239H-2b-K-6a-1 antibody, or an amino acid sequence having one or more conservative amino acid substitutions as compared to the H239H-2b-K-6a-1 antibody.In one or more embodiments, the antibody comprises a sequence having at least 80% identity to the H239H-2b-K-6a-2 antibody, or an amino acid sequence having one or more conservative amino acid substitutions as compared to the H239H-2b-K-6a-2 antibody.In one or more embodiments, the Abu is H239H-2b-K-6a-1 antibody, which comprises a light chain having an amino acid sequence set forth in SEQ ID NO: 20 and a heavy chain having an amino acid sequence set forth in SEQ ID NO: 18.

In one or more embodiments, the Abu is H239H-2b-K-6a-2 antibody, which comprises a light chain having an amino acid sequence set forth in SEQ ID NO: 20 and a heavy chain having an amino acid sequence set forth in SEQ ID NO: 19.

In one or more embodiments, the Abu is an anti-B7H3 antibody or an antigen-binding unit thereof.

In one or more embodiments, the anti-B7H3 antibody comprises a heavy chain having an amino acid sequence set forth in SEQ ID NO: 21 and a light chain having an amino acid sequence set forth in SEQ ID NO: 22.

In one or more embodiments, the Abu is an anti-FRα antibody or an antigen-binding unit thereof.

In one or more embodiments, the anti-FRα antibody comprises a heavy chain having an amino acid sequence set forth in SEQ ID NO: 23 and a light chain having an amino acid sequence set forth in SEQ ID NO: 24.

In one or more embodiments, the antibody comprises a sequence having at least 80% identity to any of the antibodies described above, or an amino acid sequence having one or more conservative amino acid substitutions as compared to any of the antibodies described above.

In one or more embodiments, p is 2-8.

In one or more embodiments, p is 4-8.

In one or more embodiments, p is 6-8.

In one or more embodiments, p is 7-8.

One or more embodiments provide a linker precursor for forming a drug conjugate, such as an antibody-drug conjugate, the linker precursor being a compound of Formula II or a stereoisomer thereof or a pharmaceutically acceptable salt or solvate thereof:

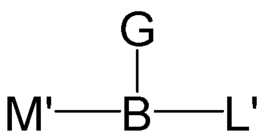

Formula II

wherein

M' is

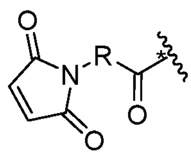

,

wherein * links to B, and R is selected from: $-(CH_2)_r-$, $-(CHR^m)_r-$, C3-C8 carbocyclyl, $-O-(CH_2)_r-$, arylene, $-(CH_2)_r$-arylene-, -arylene-$(CH_2)$r-, $-(CH_2)_r$-(C3-C8 carbocyclyl)-, -(C3-C8 carbocyclyl)-$(CH_2)_r$, C3-C8 heterocyclyl, $-(CH_2)_r$-(C3-C8 heterocyclyl)-, -(C3-C8 heterocyclyl)-$(CH_2)_r$, $-(CH_2)_rC(O)NR^m(CH_2)_r-$, $-(CH_2CH_2O)_r-$, $-(CH_2CH_2O)_r-CH_2-$, $-(CH_2)_rC(O)NR^m(CH_2CH_2O)_r-$, $-(CH_2)_rC(O)NR^m(CH_2CH_2O)_r-CH_2-$, $-(CH_2CH_2O)_rC(O)NR^m(CH_2CH_2O)_r-$, $-(CH_2CH_2O)_rC(O)NR^m(CH_2CH_2O)_r-CH_2-$ and $-(CH_2CH_2O)_rC(O)NR^m(CH_2)_r-$; wherein each $R^m$ is independently H, C1-C6 alkyl, C3-C8 carbocyclyl, phenyl or benzyl, and each r is independently 1, 2, 3, 4, 5, 6, 7, 8, 9 or 10;

B is

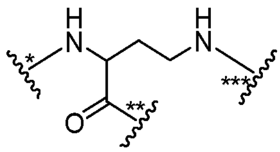

;

wherein * links to M', ** links to L', and *** links to G;

L' is -(AA)i-(FF')f-, wherein AA is an amino acid or polypeptide, and i is 1, 2, 3, 4, 5, 6, 7, 8, 9, 10, 11, 12, 13, 14, 15, 16, 17, 18, 19 or 20; each FF' is independently

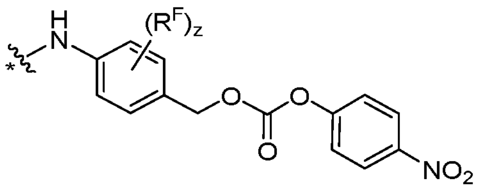 , 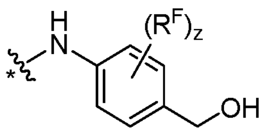 , 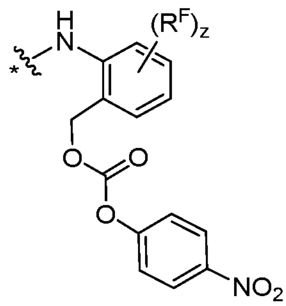 ,

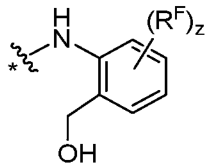 , 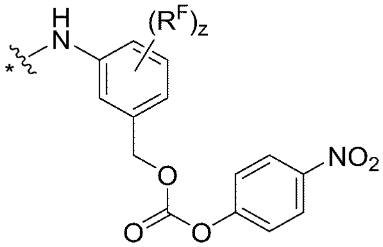 or 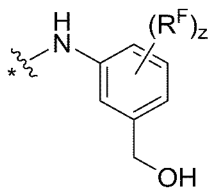 ,

wherein each

$R^F$ is independently C1-C6 alkyl, C1-C6 alkoxy, $-NO_2$ or halogen; z is 0, 1, 2, 3 or 4; f is 1, 2, 3, 4, 5, 6, 7, 8, 9 or 10; wherein * links to AA;

Gis

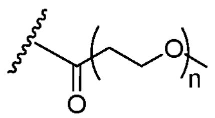

,

wherein n is 1-24, e.g., 1, 2, 3, 4, 5, 6, 7, 8, 9, 10, 11, 12, 13, 14, 15, 16, 17, 18, 19, 20, 21, 22, 23 or 24.

In one or more embodiments, R is $-(CH_2)_r-$.

In one or more embodiments, R is $-(CH_2)_r-$, wherein r is 1 or 5.

In one or more embodiments, each AA is independently selected from the following amino acids and peptide sequences: Val-Cit, Val-Lys, Phe-Lys, Lys-Lys, Ala-Lys, Phe-Cit, Leu-Cit, Ile-Cit, Trp, Cit, Phe-Ala, Phe-Phe-Lys, D-Phe-Phe-Lys, Gly-Phe-Lys, Leu-Ala-Leu, Ile-Ala-Leu, Val-Ala-Val, Ala-Leu-Ala-Leu, β-Ala-Leu-Ala-Leu, and Gly-Phe-Leu-Gly.

In one or more embodiments, i is 1.

In one or more embodiments, AA is Val-Cit, and i is 1.

In one or more embodiments, each FF' is independently

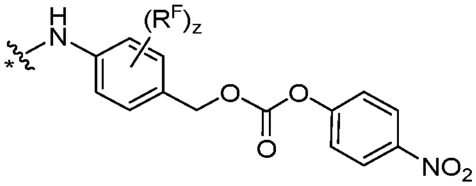, 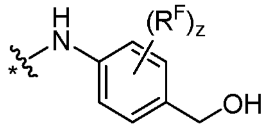, 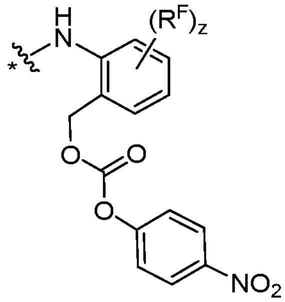,

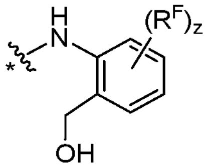, 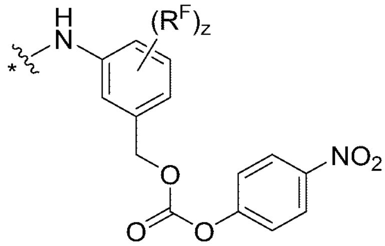 or 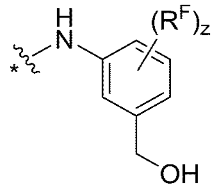,

wherein * links to AA; wherein each $R^F$ is independently C1-C6 alkyl, C1-C6 alkoxy, $-NO_2$ or halogen.

In one or more embodiments, $R^F$ is F.

In one or more embodiments, z is 0.

In one or more embodiments, z is 1 or 2.

In one or more embodiments, f is 1.

In one or more embodiments, B is

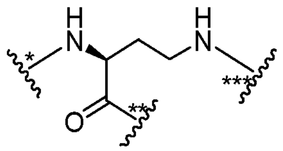,

wherein * links to M', ** links to L' and *** links to G.

In one or more embodiments, each FF' is independently

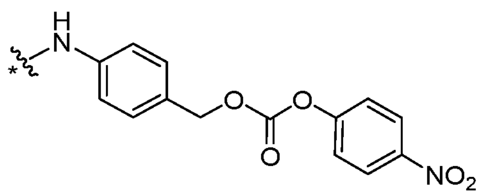, 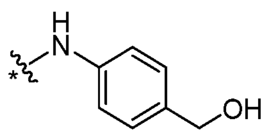,

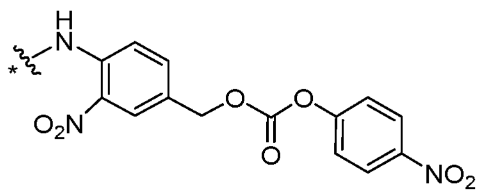, 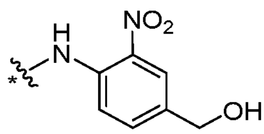,

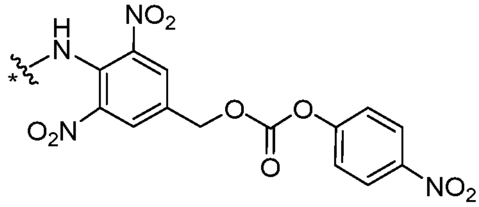 , 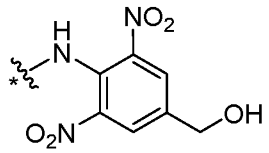 ,

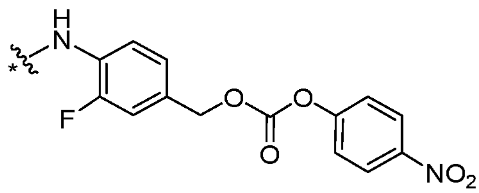 , 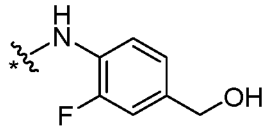 ,

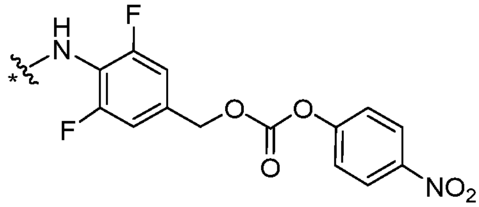 , 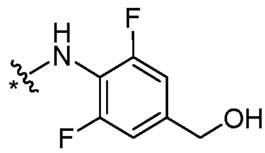 ,

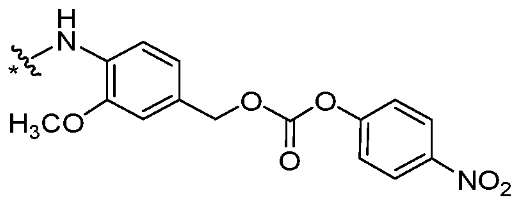 , 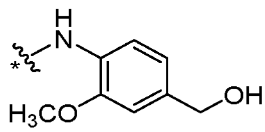 ,

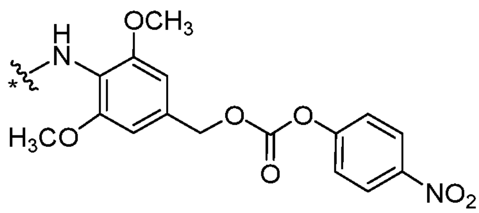 , 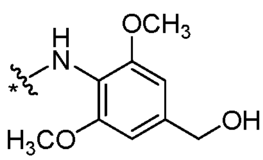 , 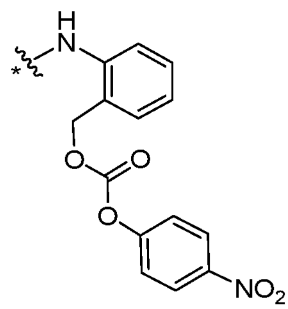 ,

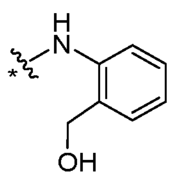 , 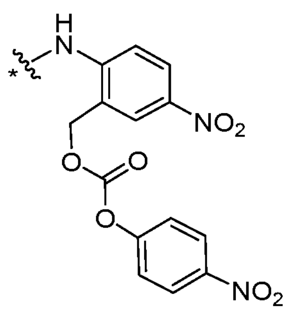 , 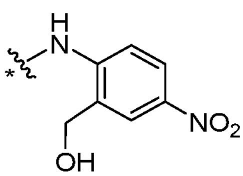 , 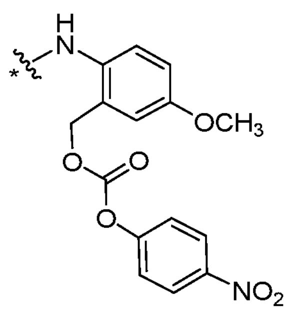 ,

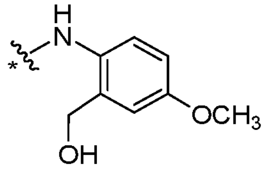, 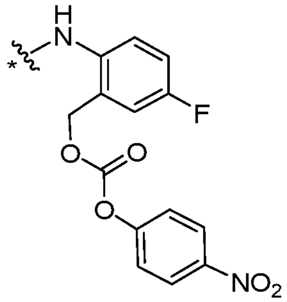, 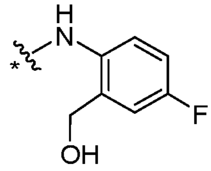,

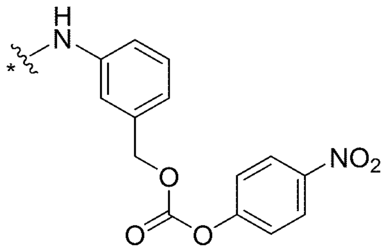, 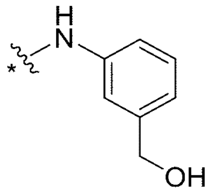, 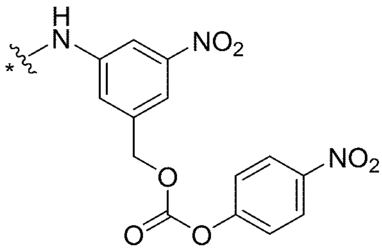,

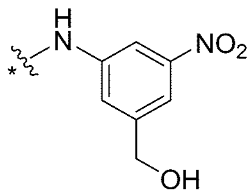, 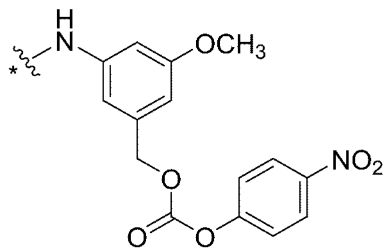, 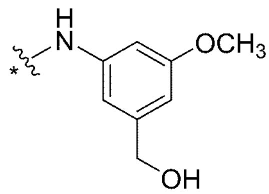,

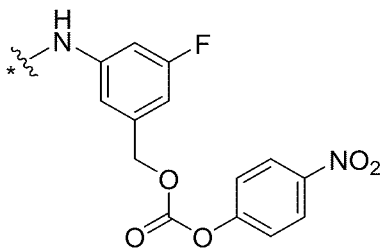 or 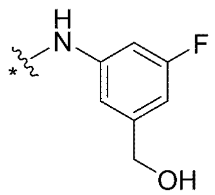,

wherein * links to AA.

In one or more embodiments, f is 1.

In one or more embodiments, FF' is

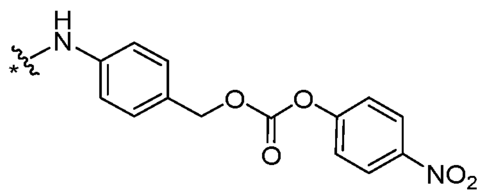

or

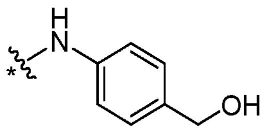,

and f is 1, wherein * links to AA.

In one or more embodiments, L' is

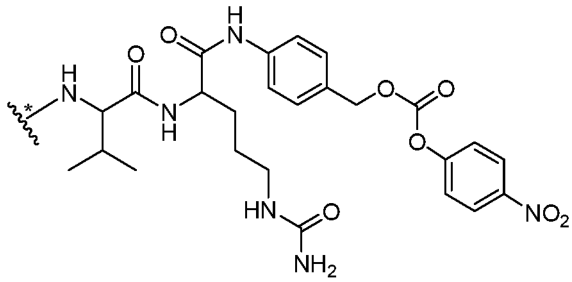

or

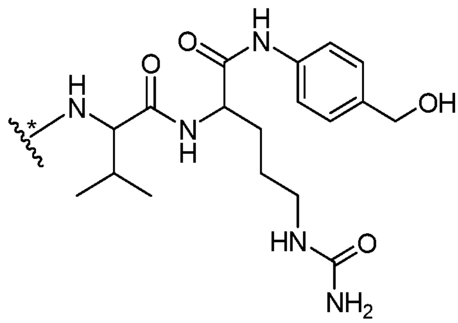,

wherein * links to B.

In one or more embodiments, L' is

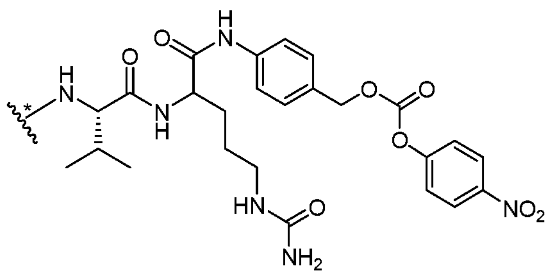

or

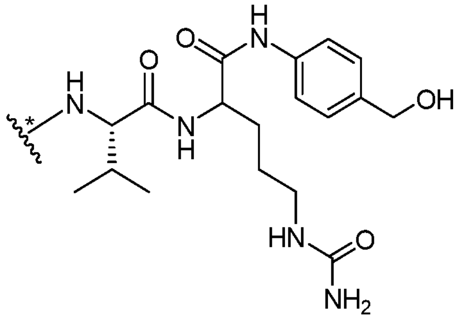

,

wherein * links to B.

In one or more embodiments, L' is

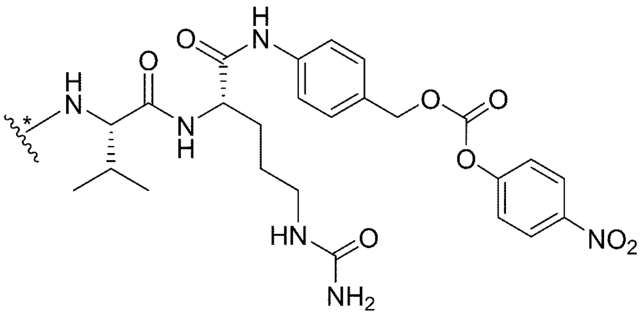

or

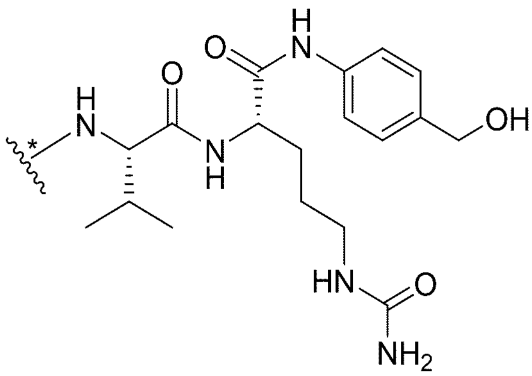

,

wherein * links to B.

In one or more embodiments, R is $-(CH_2)_r-$.

In one or more embodiments, R is $-(CH_2)_r-$, wherein r is 1 or 5.

In one or more embodiments, n is 4-12.

In one or more embodiments, n is 4-8.

In one or more embodiments, n is 4.

In one or more embodiments, n is 8.

In one or more embodiments, Formula II is:

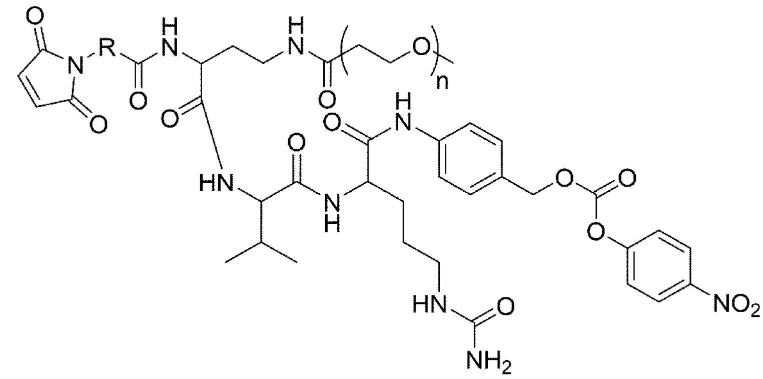

Formula II-1A

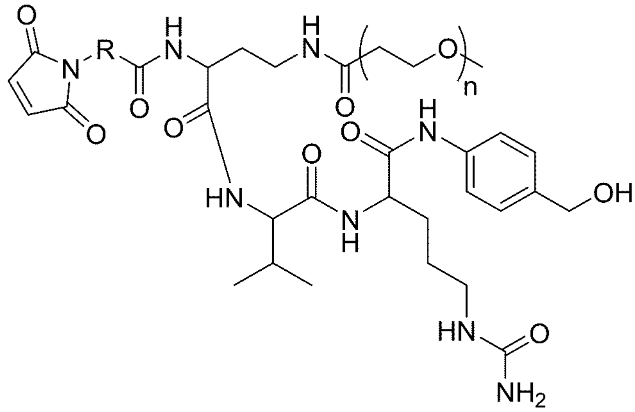

Formula II-1B

wherein

R is selected from: $-(CH_2)_r-$, $-(CHR^m)_r-$, C3-C8 carbocyclyl, $-O-(CH_2)_r-$, arylene, $-(CH_2)_r$arylene-, -arylene-$(CH_2)$r-, $-(CH_2)_r$-(C3-C8 carbocyclyl)-, -(C3-C8 carbocyclyl)-$(CH_2)_r-$, C3-C8 heterocyclyl, $-(CH_2)_r$(C3-C8 heterocyclyl)-, -(C3-C8 heterocyclyl)-$(CH_2)_r-$, $-(CH_2)_rC(O)NR^m(CH_2)_r-$, $-(CH_2CH_2O)_r-$, $-(CH_2CH_2O)_r-CH_2-$, $-(CH_2)_rC(O)NR^m(CH_2CH_2O)_r-$, $-(CH_2)_rC(O)NR^m(CH_2CH_2O)_r-CH_2-$, $-(CH_2CH_2O)_rC(O)NR^m(CH_2CH_2O)_r-$, $-(CH_2CH_2O)_rC(O)NR^m(CH_2CH_2O)_r-CH_2-$ and $-(CH_2CH_2O)_rC(O)NR^m(CH_2)_r-$; wherein each $R^m$ is independently H, C1-C6 alkyl, C3-C8 carbocyclyl, phenyl or benzyl, and each r is independently 1, 2, 3, 4, 5, 6, 7, 8, 9 or 10;

n is an integer from 1 to 24, e.g., 1, 2, 3, 4, 5, 6, 7, 8, 9, 10, 11, 12, 13, 14, 15, 16, 17, 18, 19, 20, 21, 22, 23 or 24.

In one or more embodiments, R is $-(CH_2)_r-$.

In one or more embodiments, R is $-(CH_2)_r-$, wherein r is 1 or 5.

In one or more embodiments, n is 4-12.

In one or more embodiments, n is 4-8.

In one or more embodiments, n is 4.

In one or more embodiments, n is 8.

In one or more embodiments, Formula II is:

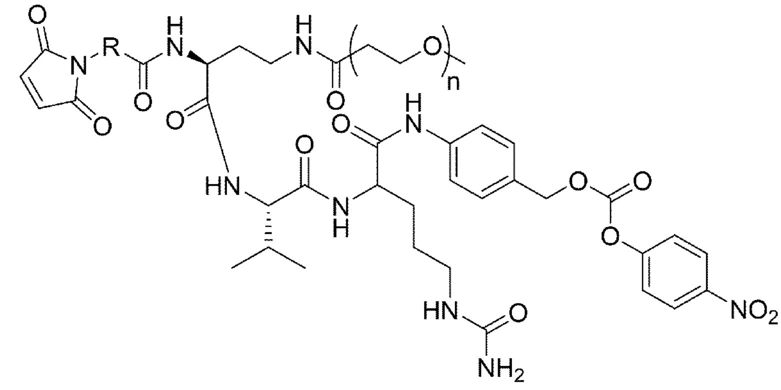

Formula II-2A

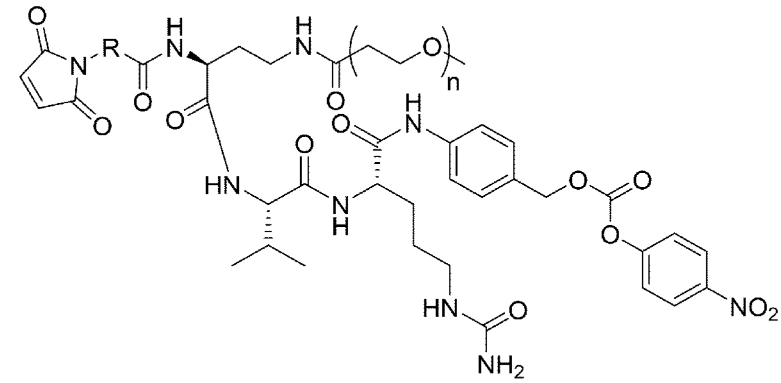

Formula II-2A-1

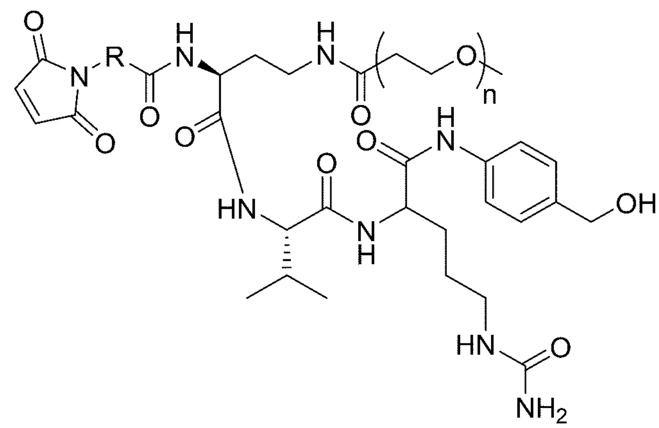

Formula II-2B

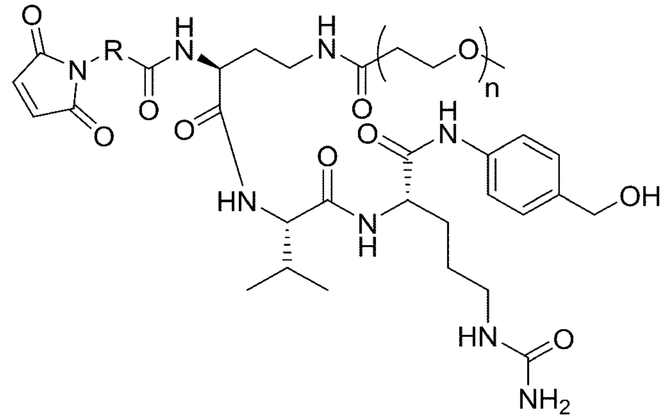

Formula II-2B-1

wherein

R is selected from: $-(CH_2)_r-$, $-(CHR^m)_r-$, C3-C8 carbocyclyl, $-O-(CH_2)_r-$, arylene, $-(CH_2)_r$arylene-, -arylene-$(CH_2)$r-, $-(CH_2)_r-$(C3-C8 carbocyclyl)-, -(C3-C8 carbocyclyl)-$(CH_2)_r-$, C3-C8 heterocyclyl, $-(CH_2)_r$(C3-C8 heterocyclyl)-, -(C3-C8 heterocyclyl)-$(CH_2)_r-$, $-(CH_2)_rC(O)NR^m(CH_2)_r-$, $-(CH_2CH_2O)_r-$, $-(CH_2CH_2O)_r-CH_2-$, $-(CH_2)_rC(O)NR^m(CH_2CH_2O)_r-$, $-(CH_2)_rC(O)NR^m(CH_2CH_2O)_r-CH_2-$, $-(CH_2CH_2O)_rC(O)NR^m(CH_2CH_2O)_r$, $-(CH_2CH_2O)_rC(O)NR^m(CH_2CH_2O)_rCH_2-$ and $-(CH_2CH_2O)_rC(O)NR^m(CH_2)_r-$; wherein each $R^m$ is independently H, C1-C6 alkyl, C3-C8 carbocyclyl, phenyl or benzyl, and each r is independently 1, 2, 3, 4, 5, 6, 7, 8, 9 or 10;

n is an integer from 1 to 24, e.g., 1, 2, 3, 4, 5, 6, 7, 8, 9, 10, 11, 12, 13, 14, 15, 16, 17, 18, 19, 20, 21, 22, 23 or 24.

In one or more embodiments, R is $-(CH_2)_r-$.

In one or more embodiments, R is $-(CH_2)_r-$, wherein r is 1 or 5.

In one or more embodiments, n is 4-12.

In one or more embodiments, n is 4-8.

In one or more embodiments, n is 4.

In one or more embodiments, n is 8.

In one or more embodiments, Formula II is:

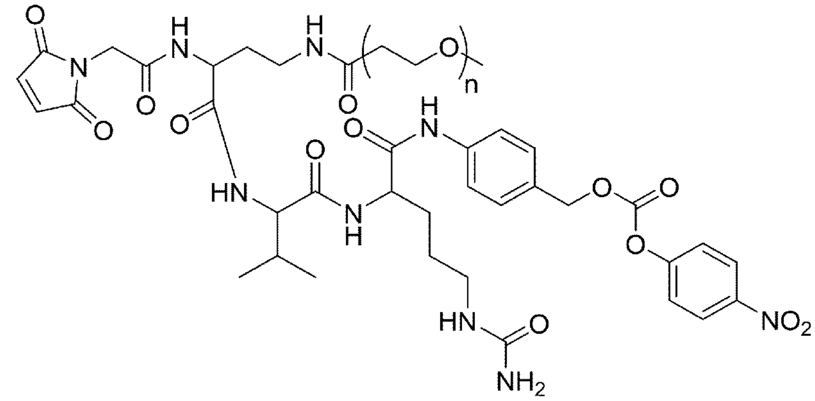

Formula II-3A

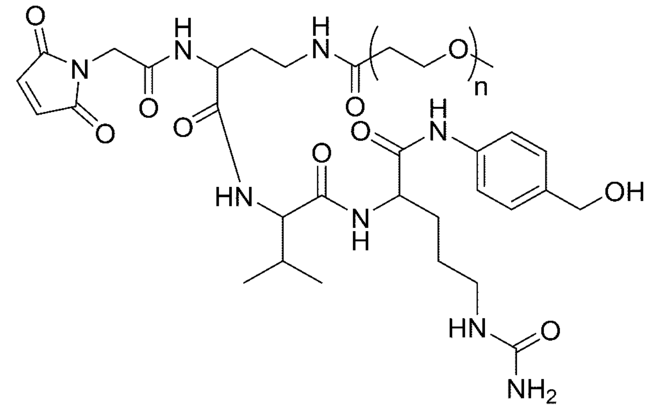

Formula II-3B

wherein n is an integer from 1 to 24, e.g., 1, 2, 3, 4, 5, 6, 7, 8, 9, 10, 11, 12, 13, 14, 15, 16, 17, 18, 19, 20, 21, 22, 23 or 24.

In one or more embodiments, n is 4-12.

In one or more embodiments, n is 4-8.

In one or more embodiments, n is 4.

In one or more embodiments, n is 8.

In one or more embodiments, Formula II is:

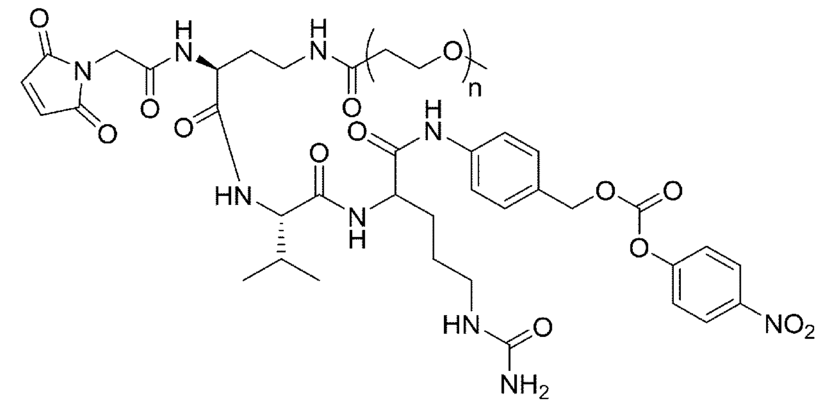

Formula II-4A

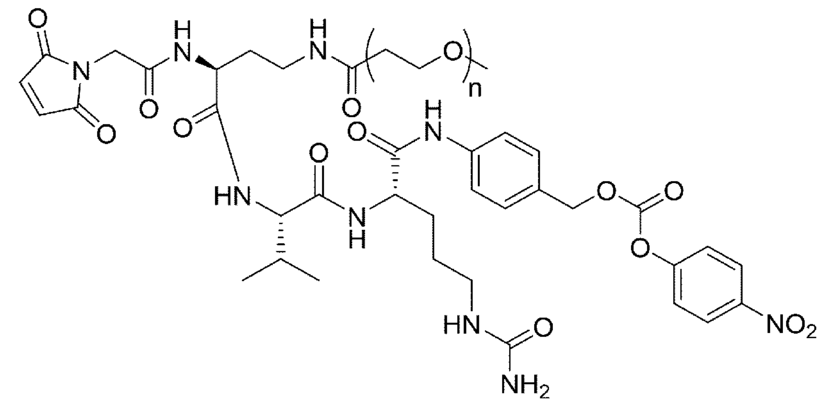

Formula II-4A-1

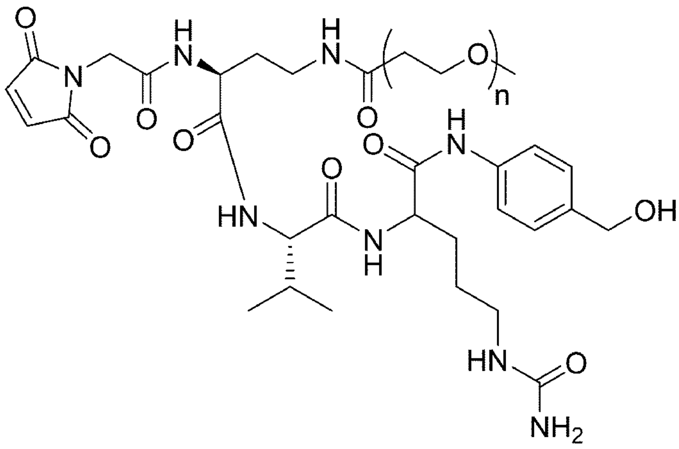

Formula II-4B

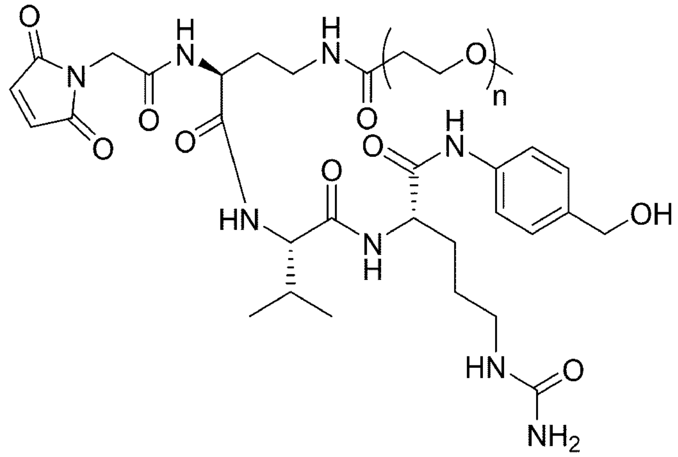

Formula II-4B-1

wherein n is an integer from 1 to 24, e.g., 1, 2, 3, 4, 5, 6, 7, 8, 9, 10, 11, 12, 13, 14, 15, 16, 17, 18, 19, 20, 21, 22, 23 or 24.

In one or more embodiments, n is 4-12.

In one or more embodiments, n is 4-8.

In one or more embodiments, n is 4.

In one or more embodiments, n is 8.

In one or more embodiments, Formula II has the following structures:

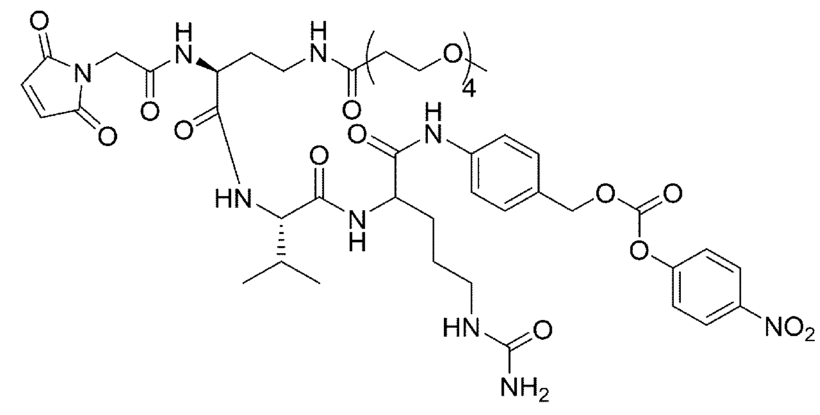

Formula II-5A

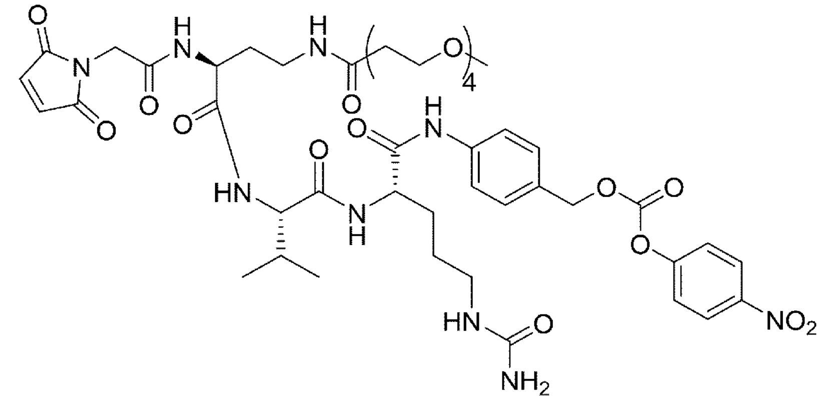

Formula II-5A-1

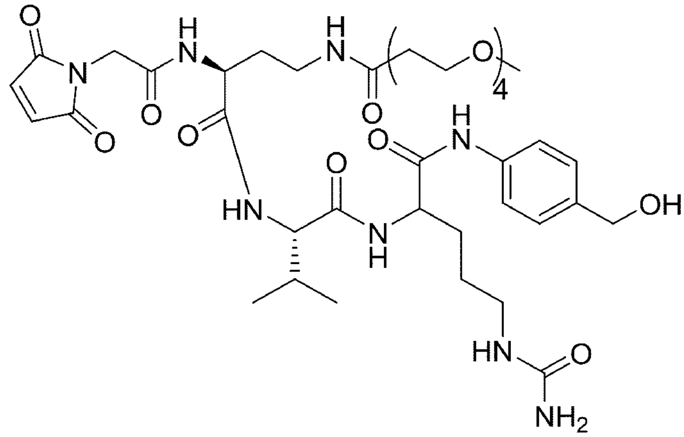

Formula II-5B

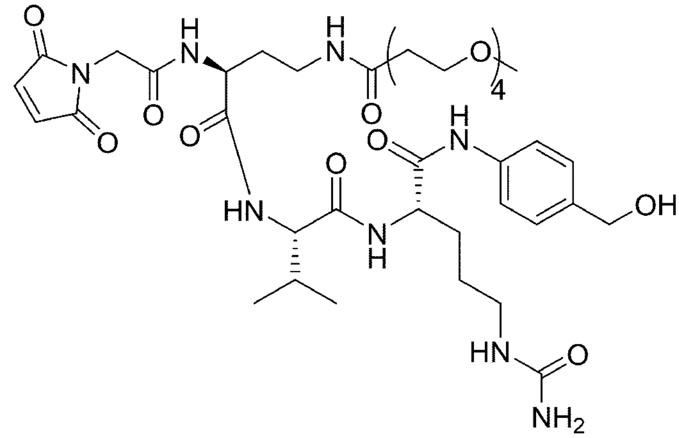

Formula II-5B-1

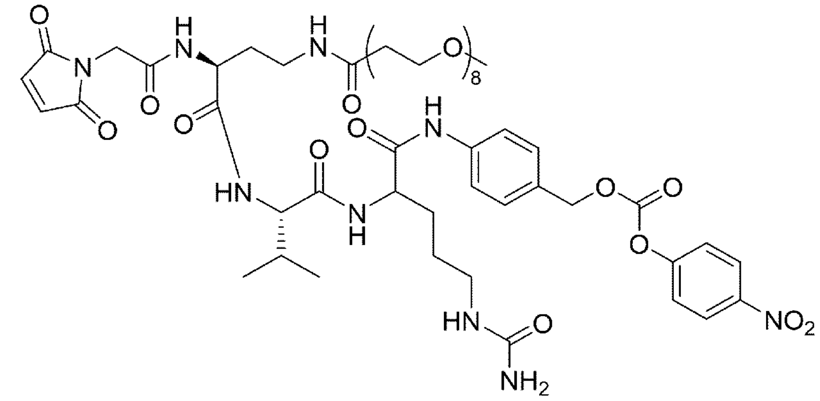

Formula II-6A

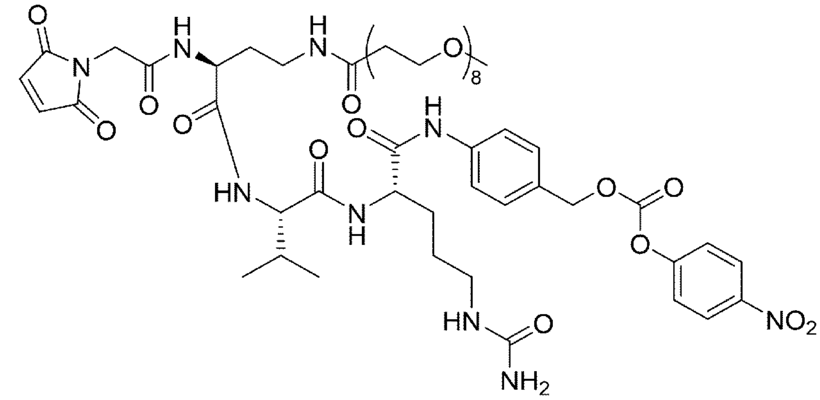

Formula II-6A-1

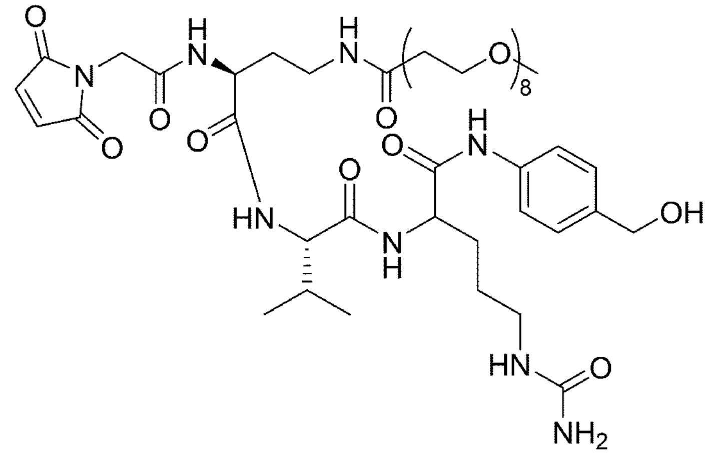

Formula II-6B

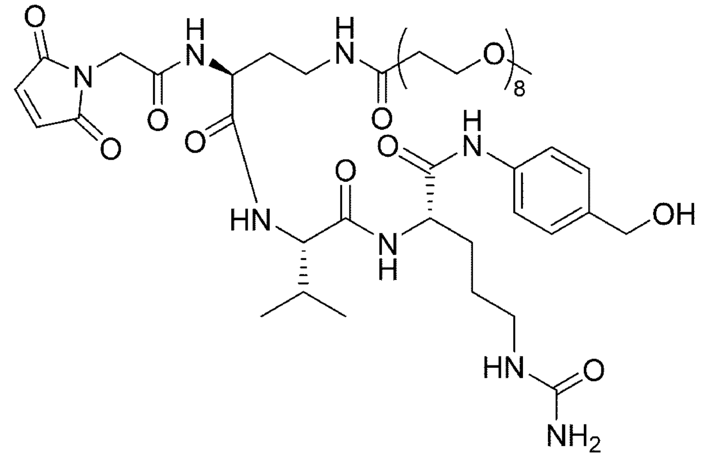

Formula II-6B-1

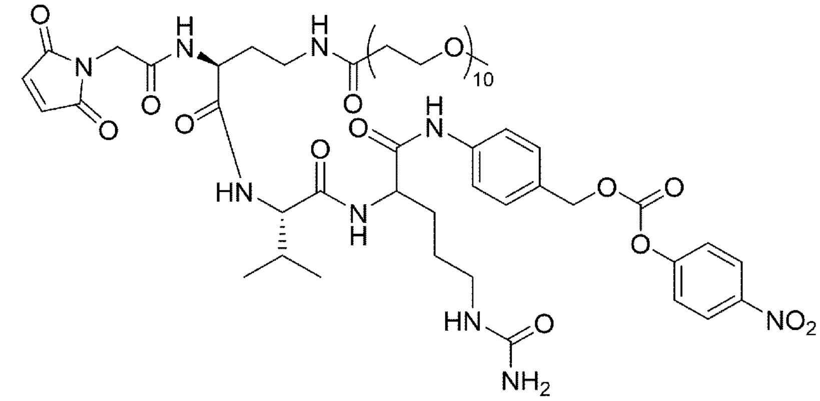

Formula II-7A

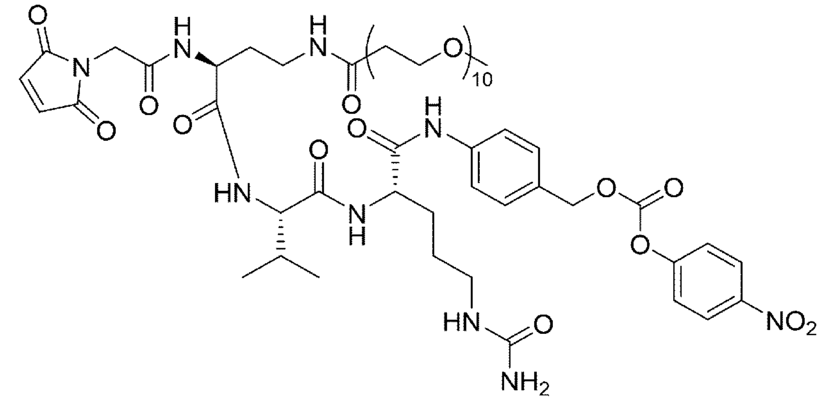

Formula II-7A-1

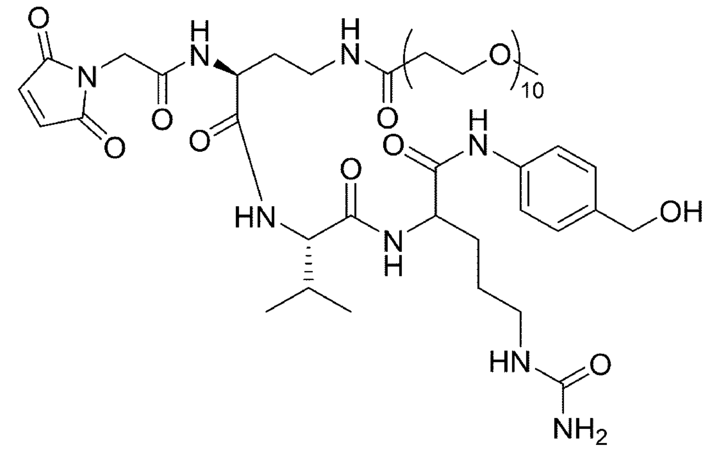

Formula II-7B

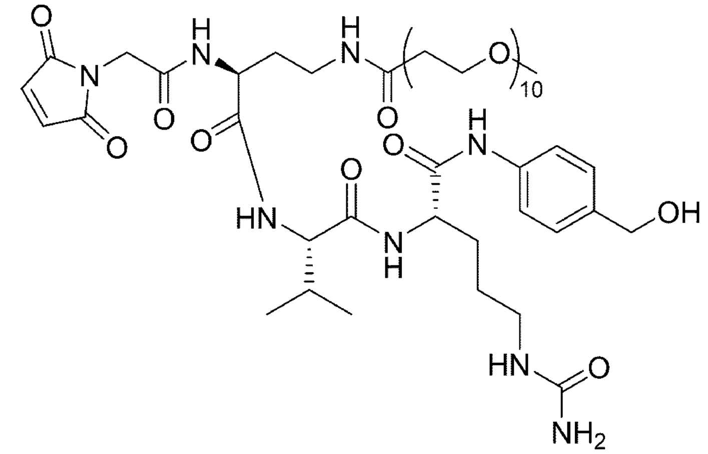

Formula II-7B-1

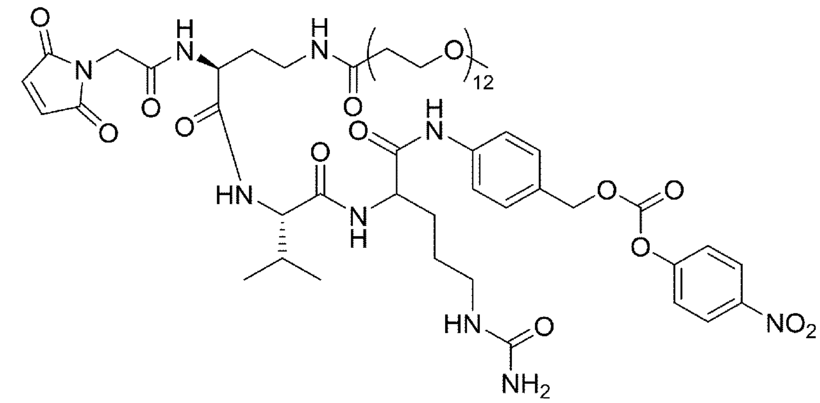

Formula II-8A

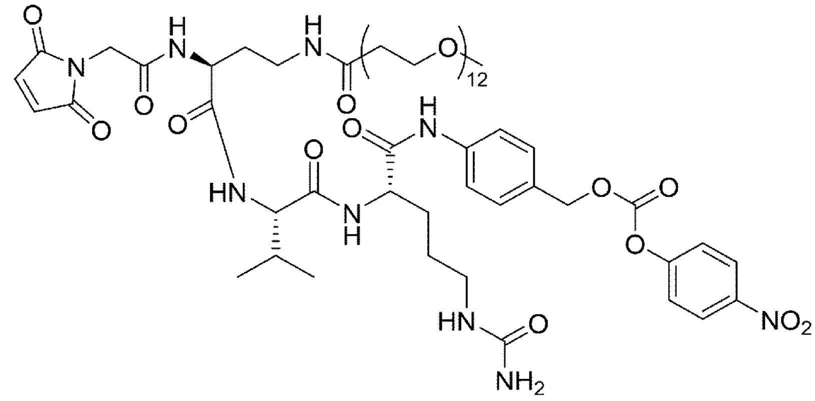

Formula II-8A-1

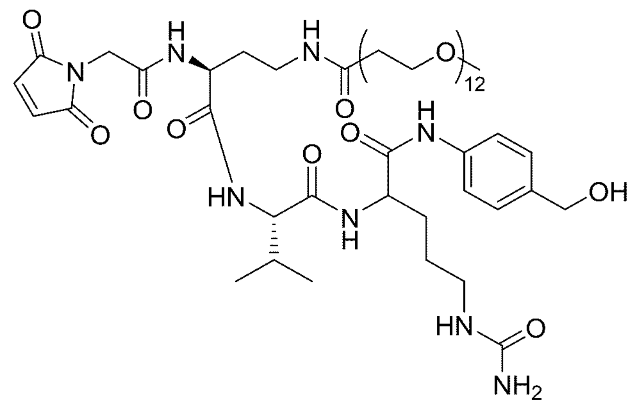

Formula II-8B

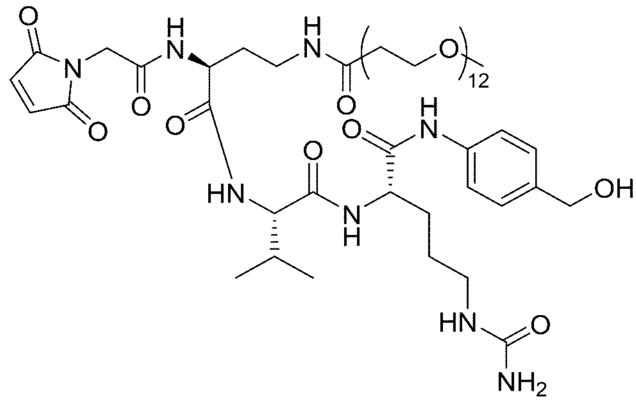

Formula II-8B-1

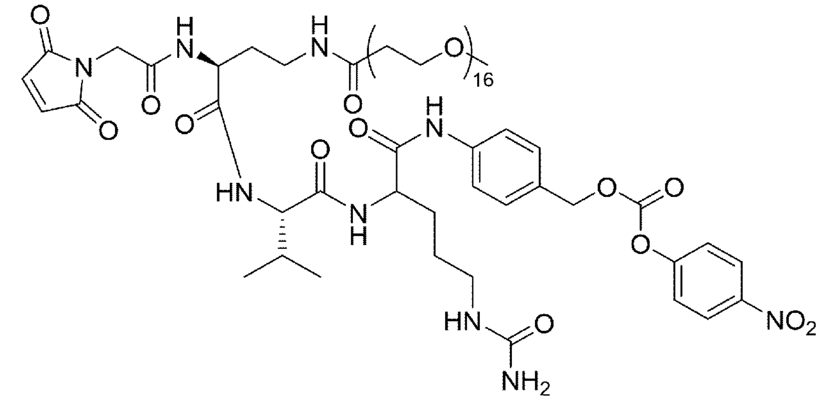

Formula II-9A

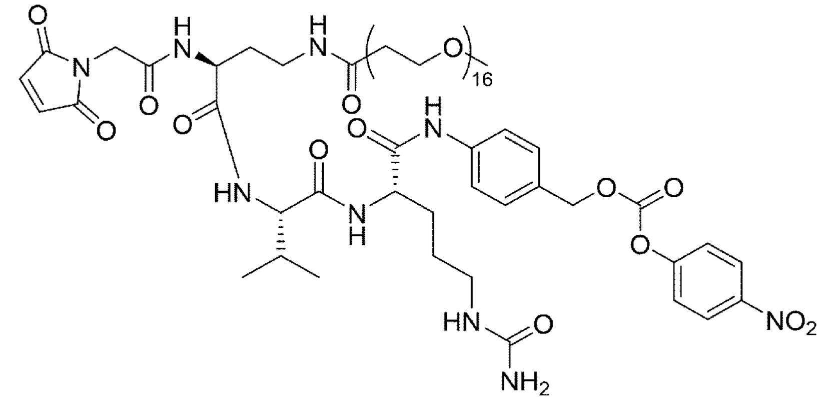

Formula II-9A-1

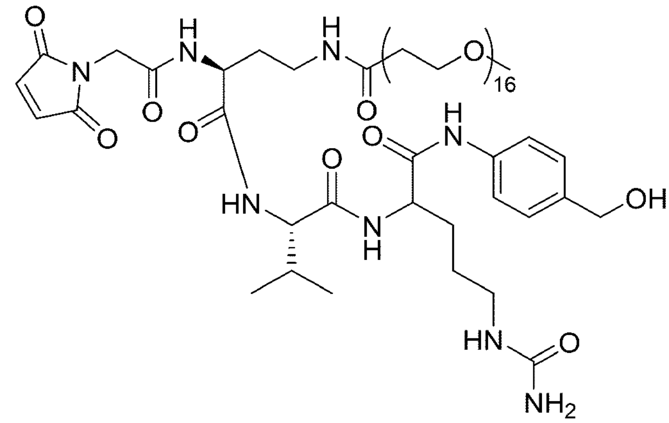

Formula II-9B

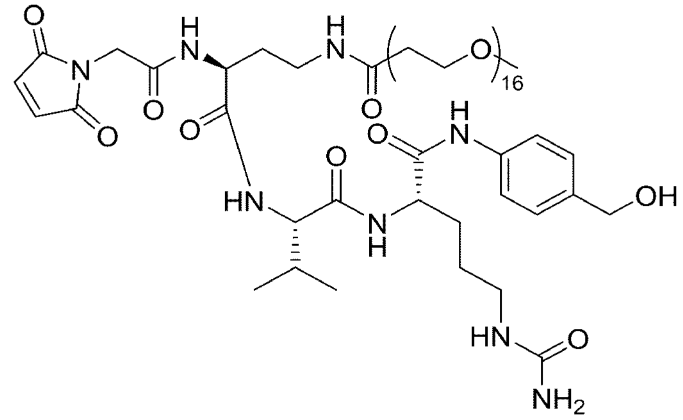

Formula II-9B-1

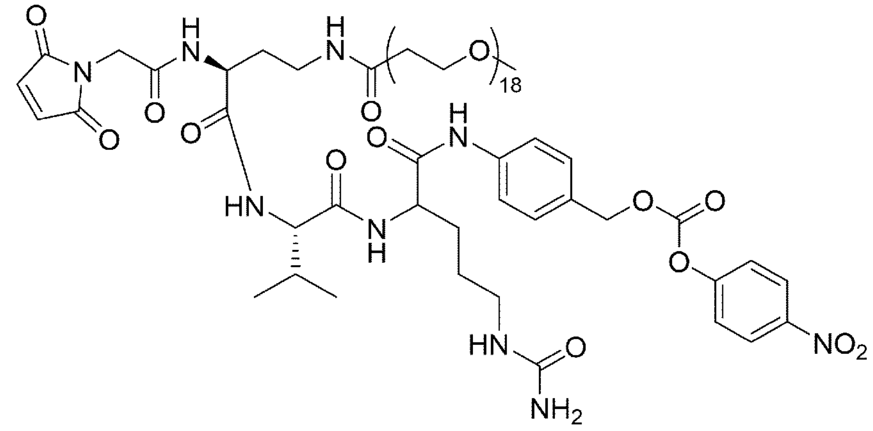

Formula II-10A

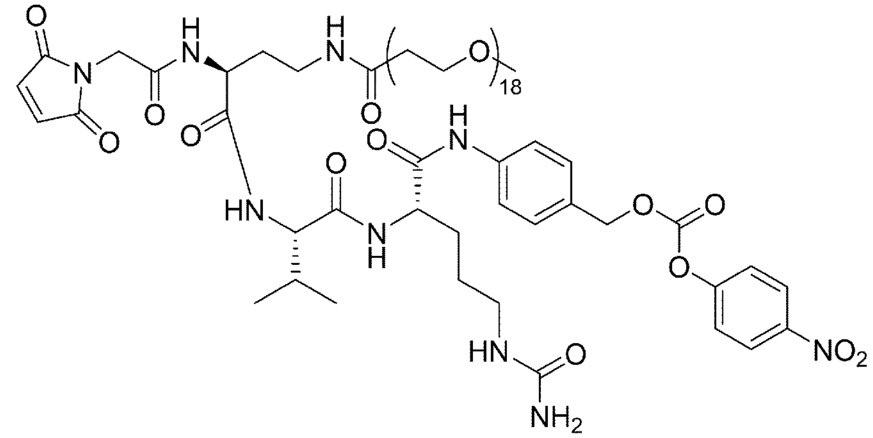

Formula II-10A-1

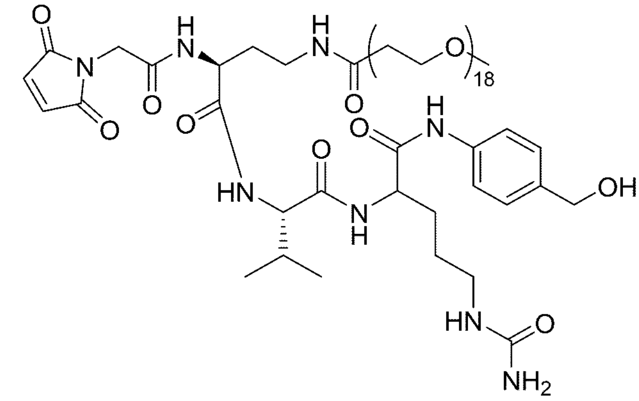

Formula II-10B

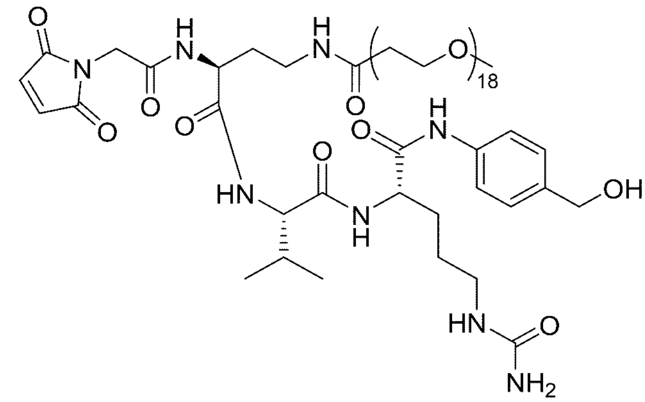

Formula II-10B-1

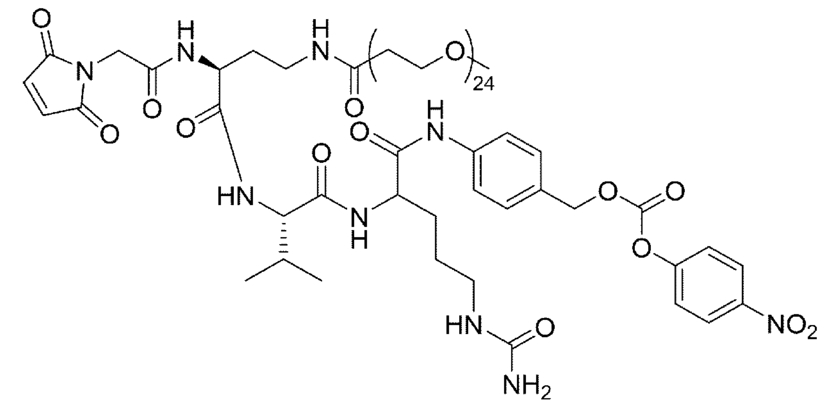

Formula II-11A

or

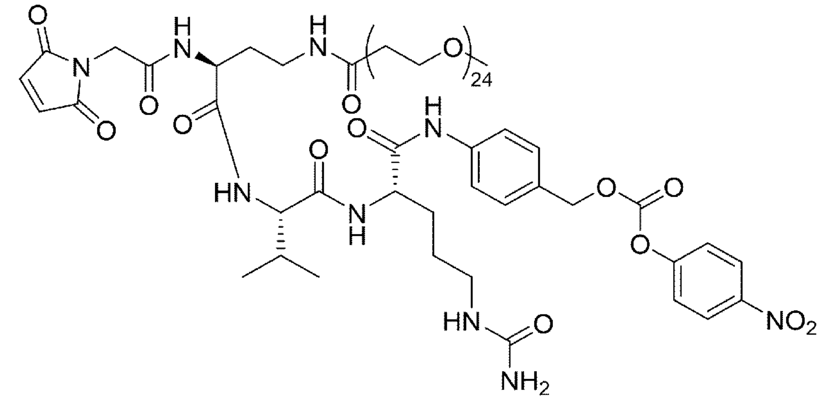

Formula II-11A-1

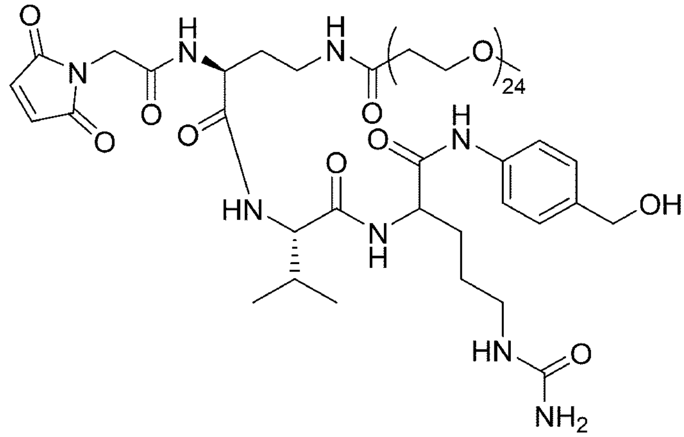

Formula II-11B

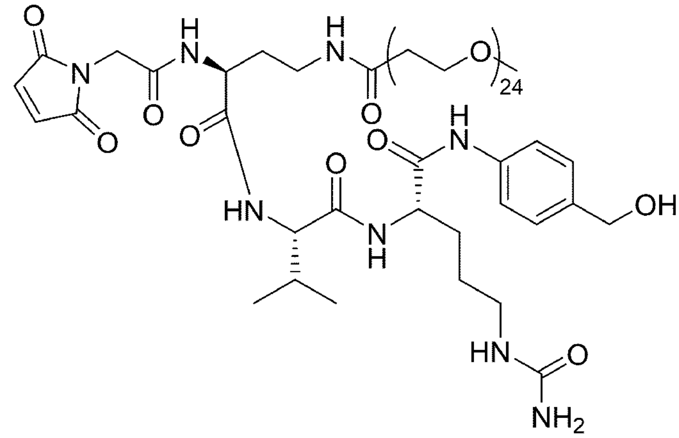

Formula II-11B-1

One or more embodiments provide an intermediate for forming a drug conjugate, such as an antibody-drug conjugate, the intermediate being a compound of Formula III or a stereoisomer thereof or a pharmaceutically acceptable salt or solvate thereof:

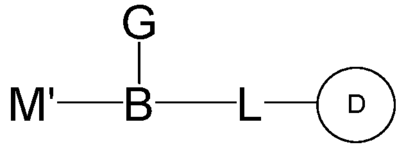

Formula III

D is a drug, such as an anti-cancer drug, a cytotoxic drug, a cell differentiation factor, a stem cell trophic factor, a steroid drug, a drug for treating autoimmune diseases, an anti-inflammatory drug or a drug for treating infectious diseases;

M' is

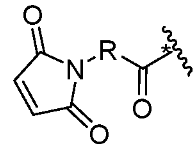

,

wherein * links to B, and R is selected from: $-(CH_2)_r-$, $-(CHR^m)_r-$, C3-C8 carbocyclyl, $-O-(CH_2)_r-$, arylene, $-(CH_2)_r$-arylene-, -arylene-$(CH_2)$r-, $-(CH_2)_r$-(C3-C8 carbocyclyl)-, -(C3-C8 carbocyclyl)-$(CH_2)_r$-, C3-C8 heterocyclyl, $-(CH_2)_r$-(C3-C8 heterocyclyl)-, -(C3-C8 heterocyclyl)-$(CH_2)_r$-, $-(CH_2)_rC(O)NR^m(CH_2)_r-$, $-(CH_2CH_2O)_r-$, $-(CH_2CH_2O)_r-CH_2-$, $-(CH_2)_rC(O)NR^m(CH_2CH_2O)_r-$, $-(CH_2)_rC(O)NR^m(CH_2CH_2O)_r-CH_2-$, $-(CH_2CH_2O)_rC(O)NR^m(CH_2CH_2O)_r-$, $-(CH_2CH_2O)_rC(O)NR^m(CH_2CH_2O)$r-$CH_2$- and $-(CH_2CH_2O)_rC(O)NR^m(CH_2)_r-$; wherein each $R^m$ is independently H, C1-C6 alkyl, C3-C8 carbocyclyl, phenyl or benzyl, and each r is independently 1, 2, 3, 4, 5, 6, 7, 8, 9 or 10;

B is

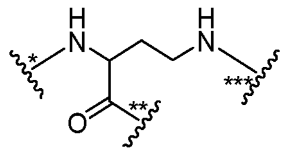

,

wherein * links to M', ** links to L, and *** links to G;

L is $-(AA)_i-(FF)_f-$, wherein AA is an amino acid or polypeptide, and i is 1, 2, 3, 4, 5, 6, 7, 8, 9, 10, 11, 12, 13, 14, 15, 16, 17, 18, 19 or 20; each FF is independently

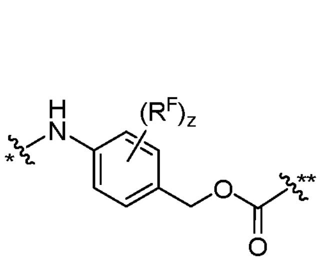

,

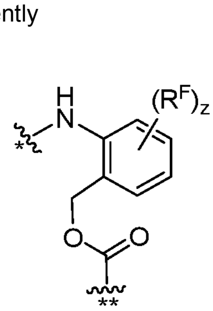

or

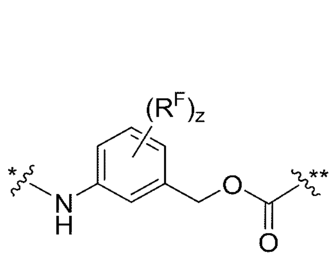

,

wherein

each $R^F$ is independently C1-C6 alkyl, C1-C6 alkoxy, $-NO_2$ or halogen; z is 0, 1, 2, 3 or 4; f is 1, 2, 3, 4, 5, 6, 7, 8, 9 or 10; wherein * links to AA; ** links to D;

G is

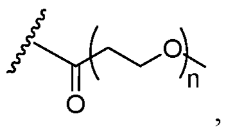

wherein n is 1-24, e.g., 1, 2, 3, 4, 5, 6, 7, 8, 9, 10, 11, 12, 13, 14, 15, 16, 17, 18, 19, 20, 21, 22, 23 or 24.

In one or more embodiments, D is an anti-cancer drug.

In one or more embodiments, D is a tubulin inhibitor, a DNA damaging agent, or a DNA topoisomerase inhibitor.

In one or more embodiments, the tubulin inhibitor is selected from dolastatin, auristatins and maytansinoids.

In one or more embodiments, D is an auristatin selected from MMAE (monomethyl auristatin E), MMAF (monomethyl auristatin F) and AF (auristatin F).

In one or more embodiments, D is a DNA damaging agent selected from calicheamicins, duocarmycins, and the anthramycin derivative PBD (pyrrolobenzodiazepine).

In one or more embodiments, D is a DNA topoisomerase inhibitor or a salt thereof selected from irinotecan, irinotecan hydrochloride, camptothecin, 9-aminocamptothecin, 9-nitrocamptothecin, 10-hydroxycamptothecin, 9-chloro-10-hydroxycamptothecin, the camptothecin derivative SN-38, 22-hydroxyacuminatine, topotecan, lurtotecan, belotecan, exatecan, homosilatecan, 6,8-dibromo-2-methyl-3-[2-(D-xylopyranosylamino)phenyl]-4(3*H*)-quinazolinone, 2-cyano-3-(3,4-dihydroxyphenyl)-*N*-(phenylmethyl)-(2*E*)-2-propenamide, 2-cyano-3-(3,4-dihydroxyphenyl)-N-(3-hydroxyphenylpropyl)-(*E*)-2-propenamide, 12-β-D-glucopyranosyl-12,13-dihydro-2,10-dihydroxy-6-[[2-hydroxy-1-(hydroxymethyl)ethyl]amino]-5*H*-indolo[2,3-*a*]pyrrolo[3,4-*c*]carbazole-5, 7(6*H*)-dione, *N*-[2-(dimethylamino)ethyl]-4-acridinecarboxamide dihydrochloride, and *N*-[2-(dimethylamino)ethyl]-4-acridinecarboxamide.

In one or more embodiments, the DNA topoisomerase inhibitor is camptothecin, 10-hydroxycamptothecin, topotecan, belotecan, irinotecan, 22-hydroxyacuminatine, or exatecan, or a salt thereof.

In one or more embodiments, D is

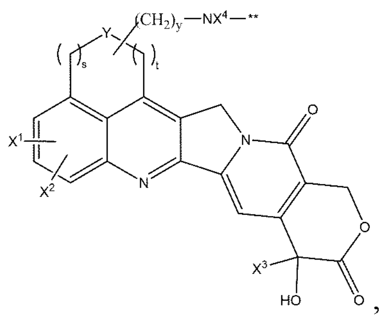

wherein

$X^1$ and $X^2$ are each independently:

H,
hydroxy,
C1-C6 alkyl,
C1-C6 alkyl substituted with one or more hydroxy, halogen, nitro or cyano groups,
C2-C6 alkenyl,
C2-C6 alkynyl,
C1-C6 alkoxy,
C1-C6 aminoalkoxy,
halogen,
nitro,
cyano,
thiol,
alkylthio,
amino, amino substituted with an amino-protecting group, C1-C6 aminoalkyl optionally substituted at the amino

moiety with an amino-protecting group or C1-C6 alkyl,

C1-C6 aminoalkylamino optionally substituted at the amino moiety with an amino-protecting group or C1-C6 alkyl,

C1-C6 alkyl linking to a heterocyclic ring, wherein the heterocyclic ring is optionally substituted with one or more C1-C6 alkyl, C1-C6 alkoxy, amino, halogen, nitro or cyano groups,

C1-C6 alkylamino linking to a heterocyclic ring, wherein the heterocyclic ring is optionally substituted with C1-C6 alkyl or C1-C6 alkoxy, and the amino is optionally substituted with an amino-protecting group, halogen, nitro, cyano or protecting group, amino-substituted heterocyclyl, which is optionally substituted at a nitrogen atom of the heterocyclyl moiety or at the amino moiety with a protecting group or one or more C1-C6 alkyl groups,

heterocyclylamino, which is optionally substituted at a nitrogen atom of the heterocyclic moiety or at the amino moiety with a protecting group or C1-C6 alkyl, carbamoyl optionally substituted with a carbamoyl-protecting group or C1-C6 alkyl, morpholin-1-yl, or

piperidin-1-yl;

$X^3$ is C1-C6 alkyl;

$X^4$ is H, $-(CH_2)_q-CH_3$, $-(CHR^n)_q-CH_3$, C3-C8 carbocyclyl, $-O-(CH_2)_q-CH_3$, arylene-$CH_3$, $-(CH_2)_q$-arylene-$CH_3$, -arylene-$(CH_2)_q-CH_3$, $-(CH_2)_q$-(C3-C8 carbocyclyl)-$CH_3$, -(C3-C8 carbocyclyl)-$(CH_2)_q-CH_3$, C3-C8 heterocyclyl, $-(CH_2)_q$-(C3-C8 heterocyclyl)-$CH_3$, -(C3-C8 heterocyclyl)-$(CH_2)_q-CH_3$, $-(CH_2)_qC(O)NR^n(CH_2)_q-CH_3$, $-(CH_2CH_2O)_q-CH_3$, $-(CH_2CH_2O)_q-CH_2-CH_3$, $-(CH_2)_qC(O)NR^n(CH_2CH_2O)_q-CH_3$, $-(CH_2)_qC(O)NR^n(CH_2CH_2O)_q-CH_2-CH_3$, $-(CH_2CH_2O)_qC(O)NR^n(CH_2CH_2O)_q-CH_3$, $-(CH_2CH_2O)_qC(O)NR^n(CH_2CH_2O)_q-CH_2-CH_3$ or $-(CH_2CH_2O)_qC(O)NR^n(CH_2)_q-CH_3$; wherein each $R^n$ is independently H, C1-C6 alkyl, C3-C8 carbocyclyl, phenyl or benzyl, and each q is independently 1, 2, 3, 4, 5, 6, 7, 8, 9 or 10;

** links to L;

y is 0, 1 or 2;

Y is O, S or $CR^1R^2$, wherein $R^1$ and $R^2$ are each independently H or C1-C6 alkyl;

s and t are each independently 0, 1 or 2, but not both 0.

In one or more embodiments, $X^4$ is H or C1-C6 alkyl.

In one or more embodiments, the heterocyclyl is azetidine, niverazine, morpholine, pyrrolidine, piperidine, imidazole, thiazole, oxazole or pyridine.

In one or more embodiments, the amino-protecting group is formyl, acetyl, trityl, *t*-butoxycarbonyl, benzyl, or *p*-methoxybenzyloxycarbonyl.

In one or more embodiments, D is

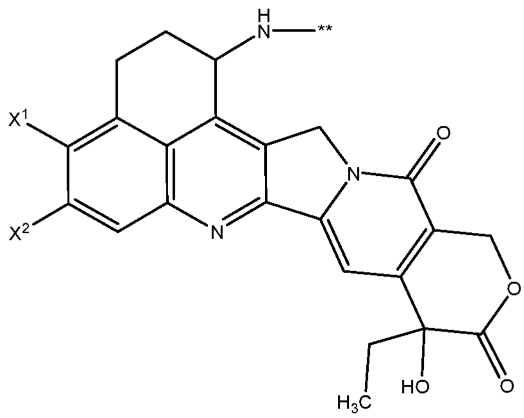

,

wherein $X^1$ and $X^2$ are each independently C1-C6 alkyl, halogen, or -OH; ** links to L.

In one or more embodiments, D is

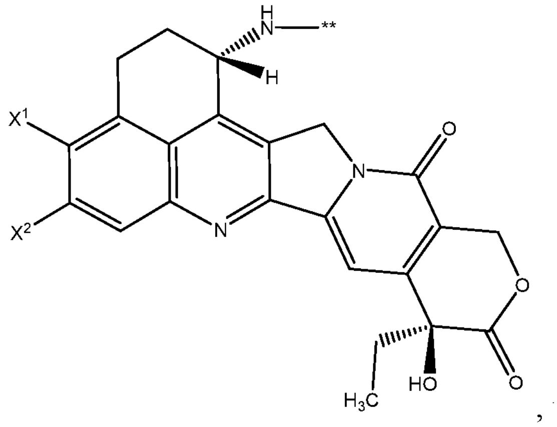

,

wherein $X^1$ and $X^2$ are each independently C1-C6 alkyl, halogen, or -OH; ** links to L.

In one or more embodiments, $X^1$ and $X^2$ are each $-CH_3$.

In one or more embodiments, $X^1$ and $X^2$ are each independently F, C1, Br, or I.

In one or more embodiments, $X^1$ and $X^2$ are each F.

In one or more embodiments, $X^1$ and $X^2$ are each independently $-CH_3$, F, or -OH.

In one or more embodiments, $X^1$ and $X^2$ are each independently F or $-CH_3$.

In one or more embodiments, $X^1$ is $-CH_3$ and $X^2$ is F.

In one or more embodiments, R is $-(CH_2)_r-$.

In one or more embodiments, r is 1 or 5.

In one or more embodiments, B is

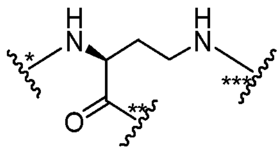

wherein * links to M', ** links to L, and *** links to G.

In one or more embodiments, each AA is independently selected from the following amino acids and peptide sequences: Val-Cit, Val-Lys, Phe-Lys, Lys-Lys, Ala-Lys, Phe-Cit, Leu-Cit, Ile-Cit, Trp, Cit, Phe-Ala, Phe-Phe-Lys, D-Phe-Phe-Lys, Gly-Phe-Lys, Leu-Ala-Leu, Ile-Ala-Leu, Val-Ala-Val, Ala-Leu-Ala-Leu, β-Ala-Leu-Ala-Leu, and Gly-Phe-Leu-Gly.

In one or more embodiments, i is 1.

In one or more embodiments, AA is Val-Cit, and i is 1.

In one or more embodiments, each FF is independently

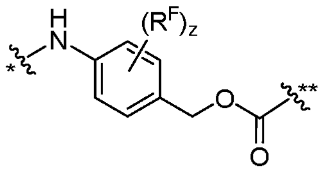

,

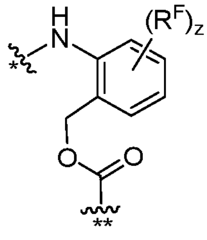

or

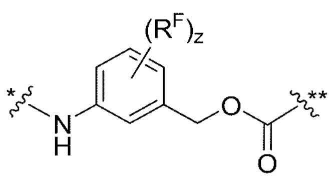

,

wherein * links to AA, and ** links to D, wherein $R^F$ is C1-C6 alkyl, C1-C6 alkoxy, $-NO_2$ or halogen.

In one or more embodiments, the halogen is F.

In one or more embodiments, each $R^F$ is independently $-CH_3$, F, $-NO_2$ or $-OCH_3$.

In one or more embodiments, z is 0.

In one or more embodiments, z is 1 or 2.

In one or more embodiments, f is 1.

In one or more embodiments, FF is

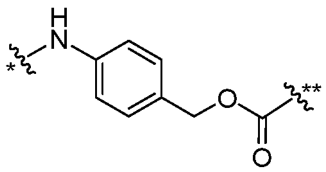
,

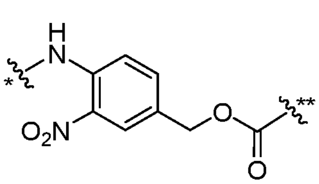
,
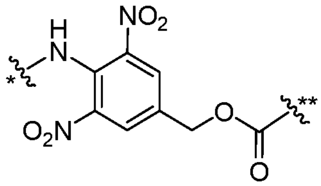
,
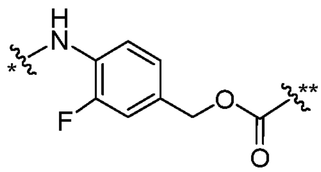
,

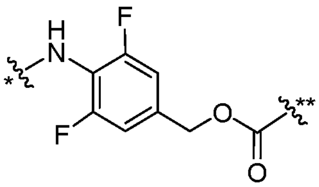
,
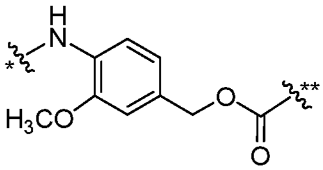
,
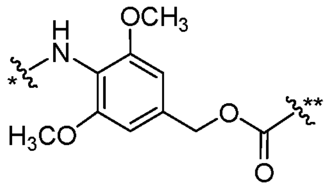
,

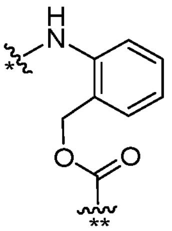
,
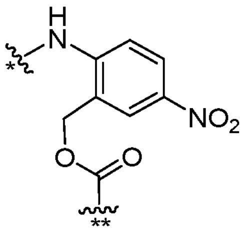
,
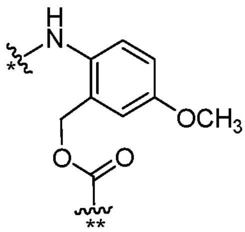
,
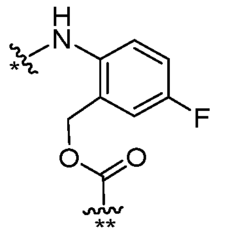
,

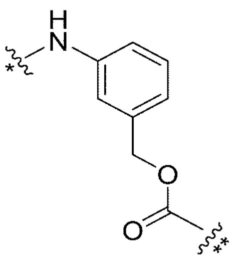
,
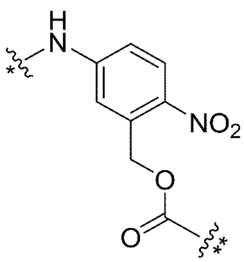
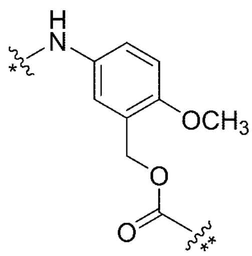
or
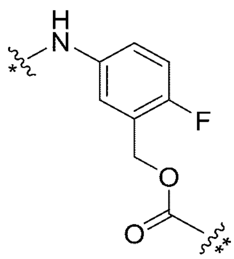
;

f is 1; wherein * links to AA, and ** links to D.

In one or more embodiments, FF is

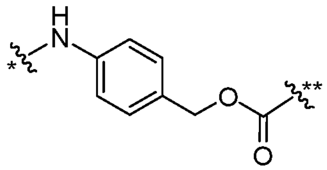

,

and f is 1; wherein * links to AA, and ** links to D.

In one or more embodiments, L is

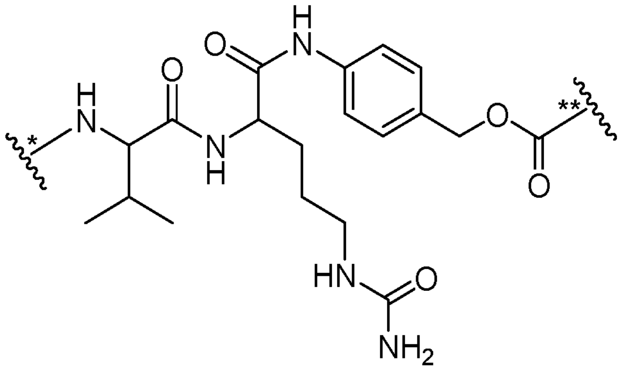

,

wherein * links to B, and ** links to D.

In one or more embodiments, L is

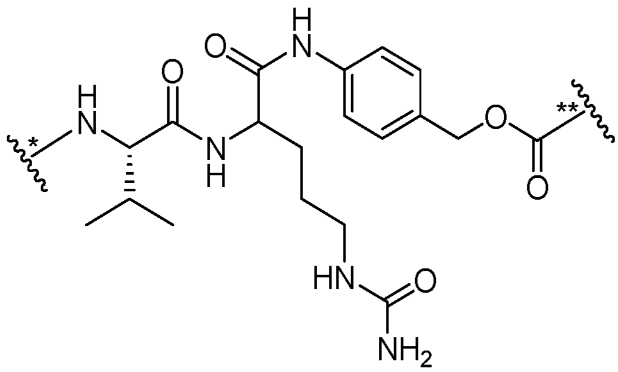

,

wherein * links to B, and ** links to D.

In one or more embodiments, L is

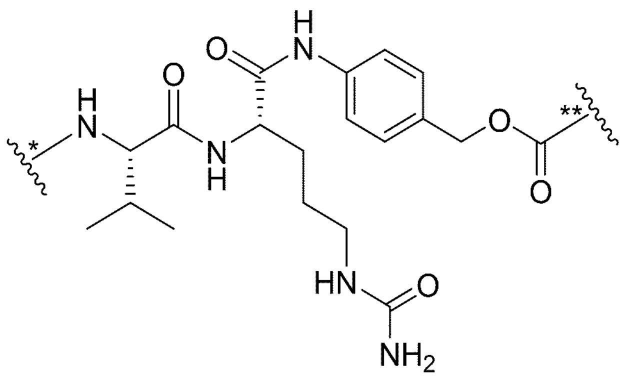

,

wherein * links to B, and ** links to D.

In one or more embodiments, n is 4-12.

In one or more embodiments, n is 4-8.

In one or more embodiments, n is 4.

In one or more embodiments, n is 8.

In one or more embodiments, Formula III is:

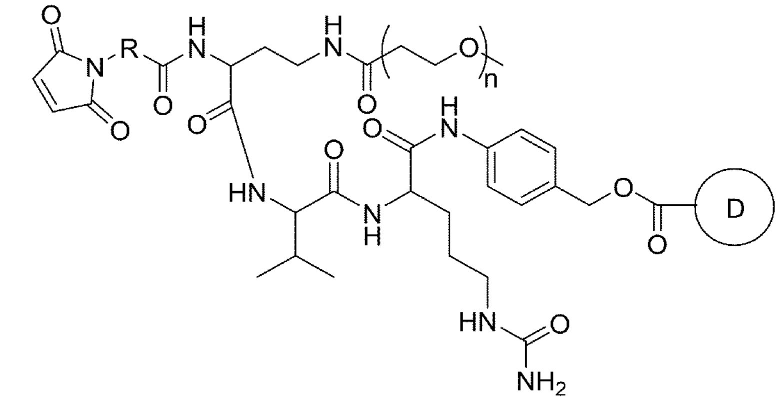

Formula III-1

wherein

R is selected from: $-(CH_2)_r-$, $-(CHR^m)r-$, C3-C8 carbocyclyl, $-O-(CH_2)_r-$, arylene, $-(CH_2)_r$arylene-, -arylene-$(CH_2)r-$, $-(CH_2)_r$-(C3-C8 carbocyclyl)-, -(C3-C8 carbocyclyl)-$(CH_2)_r-$, C3-C8 heterocyclyl, $-(CH_2)_r$-(C3-C8 heterocyclyl)-, -(C3-C8 heterocyclyl)-$(CH_2)_r-$, $-(CH_2)_rC(O)NR^m(CH_2)_r-$, $-(CH_2CH_2O)_r-$, $-(CH_2CH_2O)_rCH_2-$, $-(CH_2)_rC(O)NR^m(CH_2CH_2O)_r-$, $-(CH_2)_rC(O)NR^m(CH_2CH_2O)_r-CH_2-$, $-(CH_2CH_2O)_rC(O)NR^m(CH_2CH_2O)_r-$, $-(CH_2CH_2O)_rC(O)NR^m(CH_2CH_2O)_r-CH_2-$ and $-(CH_2CH_2O)_rC(O)NR^m(CH_2)_r-$; wherein each $R^m$ is independently H, C1-C6 alkyl, C3-C8 carbocyclyl, phenyl or benzyl, and each r is independently 1, 2, 3, 4, 5, 6, 7, 8, 9 or 10;

D is a drug, such as an anti-cancer drug, a cytotoxic drug, a cell differentiation factor, a stem cell trophic factor, a steroid drug, a drug for treating autoimmune diseases, an anti-inflammatory drug or a drug for treating infectious diseases;

n is an integer from 1 to 24, e.g., 1, 2, 3, 4, 5, 6, 7, 8, 9, 10, 11, 12, 13, 14, 15, 16, 17, 18, 19, 20, 21, 22, 23 or 24. In one or more embodiments, D is a tubulin inhibitor, a DNA damaging agent, or a DNA topoisomerase inhibitor.

In one or more embodiments, the tubulin inhibitor is selected from dolastatin, auristatins and maytansinoids.

In one or more embodiments, D is an auristatin, e.g., MMAE (monomethyl auristatin E), MMAF (monomethyl auristatin F), or AF (auristatin F).

In one or more embodiments, D is a DNA damaging agent, e.g., a calicheamicin, a duocarmycin, or the anthramycin derivative PBD (pyrrolobenzodiazepine).

In one or more embodiments, D is a DNA topoisomerase inhibitor or a salt thereof, e.g., irinotecan, irinotecan hydrochloride, camptothecin, 9-aminocamptothecin, 9-nitrocamptothecin, 10-hydroxycamptothecin, 9-chloro-10-hydroxycamptothecin, the camptothecin derivative SN-38, 22-hydroxyacuminatine, topotecan, lurtotecan, belotecan, exatecan, homosilatecan, 6,8-dibromo-2-methyl-3-[2-(D-xylopyranosylamino)phenyl]-4(3*H*)-quinazolinone, 2-cyano-3-(3,4-dihydroxyphenyl)-*N*(phenylmethyl)-(2*E)*-2-propenamide, 2-cyano-3-(3,4-dihydroxyphenyl)-*N*-(3-hydroxyphenylpropyl)-(*E*)-2-propenamide, 12-(β-D-glucopyranosyl-12,13-dihydro-2,10-dihydroxy-6-[[2-hydroxy-1-(hydroxymethyl)ethyl]amino]-5*H*-indolo[2,3-*a*]pyrrolo[3,4-*c*]carbazole-5, 7(6*H*)-dione, *N*-[2-(dimethylamino)ethyl]-4-acridinecarboxamide dihydrochloride, or *N*-[2-(dimethylamino)ethyl]-4-acridinecarboxamide.

In one or more embodiments, the DNA topoisomerase inhibitor is camptothecin, 10-hydroxycamptothecin, topotecan, belotecan, irinotecan, 22-hydroxyacuminatine, or exatecan, or a salt thereof.

In one or more embodiments, D is a tubulysin, a taxane drug derivative, a leptomycine derivative, CC-1065 or an analog thereof, an amatoxin, a spliceosome inhibitor, a benzodiazepine (PBD) dimer, adriamycin, methotrexate, vincristine, vinblastine, daunorubicin, mitomycin C, melphalan, or a chlorambucil derivative.

In one or more embodiments, D is

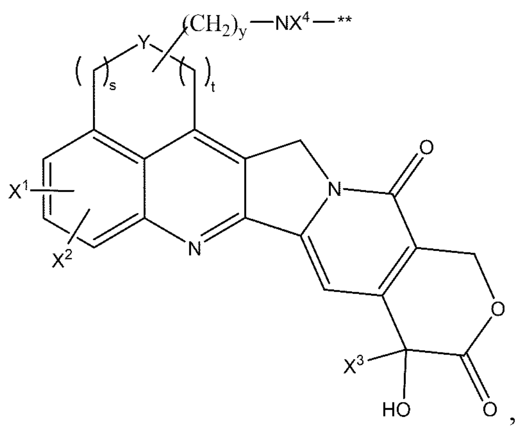

wherein
$X^1$ and $X^2$ are each independently:

H,

hydroxy,

C1-C6 alkyl,

C1-C6 alkyl substituted with one or more hydroxy, halogen, nitro or cyano groups,

C2-C6 alkenyl,

C2-C6 alkynyl,

C1-C6 alkoxy,

C1-C6 aminoalkoxy,

halogen,

nitro,

cyano,

thiol,

alkylthio,

amino, amino substituted with an amino-protecting group, C1-C6 aminoalkyl optionally substituted at the amino moiety with an amino-protecting group or C1-C6 alkyl,

C1-C6 aminoalkylamino optionally substituted at the amino moiety with an amino-protecting group or C1-C6 alkyl,

C1-C6 alkyl linking to a heterocyclic ring, wherein the heterocyclic ring is optionally substituted with one or more C1-C6 alkyl, C1-C6 alkoxy, amino, halogen, nitro or cyano groups,

C1-C6 alkylamino linking to a heterocyclic ring, wherein the heterocyclic ring is optionally substituted with C1-C6 alkyl or C1-C6 alkoxy, and the amino is optionally substituted with an amino-protecting group, halogen, nitro, cyano or protecting group, amino-substituted heterocyclyl, which is optionally substituted at a nitrogen atom of the heterocyclyl moiety or at the amino moiety with a protecting group or one or more C1-C6 alkyl groups,

heterocyclylamino, which is optionally substituted at a nitrogen atom of the heterocyclic moiety or at the amino

moiety with a protecting group or C1-C6 alkyl, carbamoyl optionally substituted with a carbamoyl-protecting group or C1-C6 alkyl, morpholin-1-yl, or

piperidin-1-yl;

$X^3$ is C1-C6 alkyl;

$X^4$ is H, $-(CH_2)_q-CH_3$, $-(CHR^n)_q-CH_3$, C3-C8 carbocyclyl, $-O-(CH_2)_q-CH_3$, arylene-$CH_3$, $-(CH_2)_q$-arylene-$CH_3$, -arylene-$(CH_2)_q-CH_3$, $-(CH_2)_q$-(C3-C8 carbocyclyl)-$CH_3$, -(C3-C8 carbocyclyl)-$(CH_2)_q-CH_3$, C3-C8 heterocyclyl, $-(CH_2)_q$-(C3-C8 heterocyclyl)-$CH_3$, -(C3-C8 heterocyclyl)-$(CH_2)_q-CH_3$, $-(CH_2)_qC(O)NR^n(CH_2)_q-CH_3$, $-(CH_2CH_2O)_q-CH_3$, $-(CH_2CH_2O)_q-CH_2-CH_3$, $-(CH_2)_qC(O)NR^n(CH_2CH_2O)_q-CH_3$, $-(CH_2)_qC(O)NR^n(CH_2CH_2O)_q-CH_2-CH_3$, $-(CH_2CH_2O)_qC(O)NR^n(CH_2CH_2O)_q-CH_3$, $-(CH_2CH_2O)_qC(O)NR^n(CH_2CH_2O)_q-CH_2-CH_3$ or $-(CH_2CH_2O)_qC(O)NR^n(CH_2)_q-CH_3$; wherein each $R^n$ is independently H, C1-C6 alkyl, C3-C8 carbocyclyl, phenyl or benzyl, and each q is independently 1, 2, 3, 4, 5, 6, 7, 8, 9 or 10;

** is point of connection;

y is 0, 1 or 2;

Y is O, S or $CR^1R^2$, wherein $R^1$ and $R^2$ are each independently H or C1-C6 alkyl;

s and t are each independently 0, 1 or 2, but not both 0.

In one or more embodiments, $X^4$ is H or C1-C6 alkyl.

In one or more embodiments, the heterocyclyl is azetidine, niverazine, morpholine, pyrrolidine, piperidine, imidazole, thiazole, oxazole or pyridine.

In one or more embodiments, the amino-protecting group is formyl, acetyl, trityl, *t*-butoxycarbonyl, benzyl, or *p*-methoxybenzyloxycarbonyl.

In one or more embodiments, D is

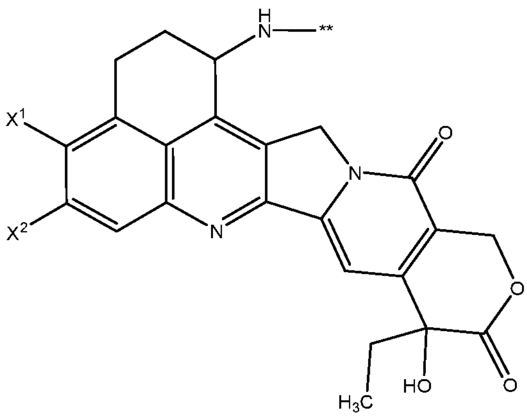

,

wherein $X^1$ and $X^2$ are each independently C1-C6 alkyl, halogen, or -OH; ** is point of connection.

In one or more embodiments, D is

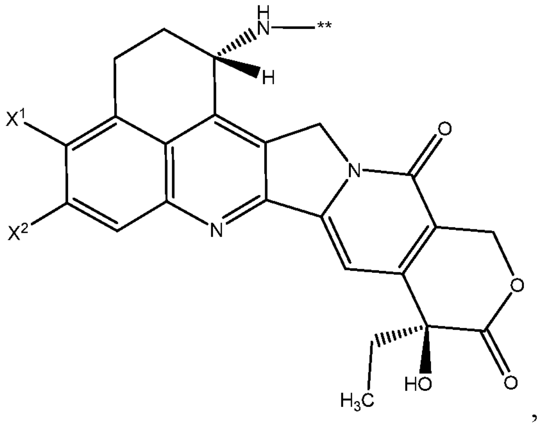

wherein $X^1$ and $X^2$ are each independently C1-C6 alkyl, halogen, or -OH; ** is point of connection.

In one or more embodiments, $X^1$ and $X^2$ are each $-CH_3$.

In one or more embodiments, $X^1$ and $X^2$ are each independently F, Cl, Br, or I.

In one or more embodiments, $X^1$ and $X^2$ are each F.

In one or more embodiments, $X^1$ and $X^2$ are each independently $-CH_3$, F, or -OH.

In one or more embodiments, $X^1$ and $X^2$ are each independently F or $-CH_3$.

In one or more embodiments, $X^1$ is $-CH_3$ and $X^2$ is F.

In one or more embodiments, R is $-(CH_2)_r-$.

In one or more embodiments, R is $-(CH_2)_r-$, wherein r is 1 or 5.

In one or more embodiments, n is 4-12.

In one or more embodiments, n is 4-8.

In one or more embodiments, n is 4.

In one or more embodiments, n is 8.

In one or more embodiments, Formula III is:

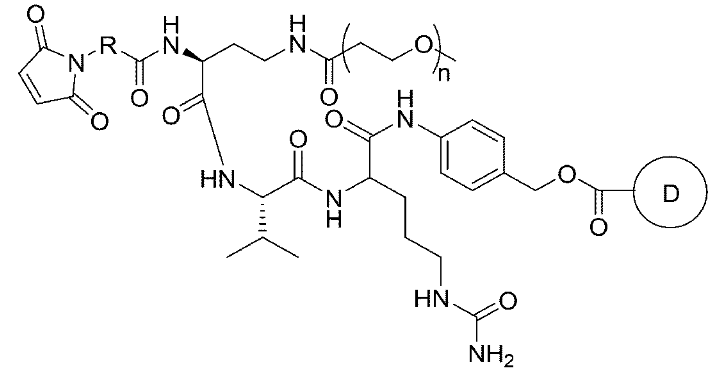

Formula III-2

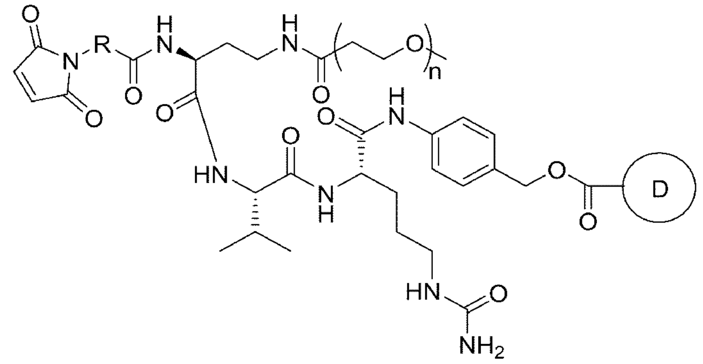

Formula III-2-1

wherein

R is selected from: $-(CH_2)_r-$, $-(CHR^m)r-$, C3-C8 carbocyclyl, $-O-(CH_2)_r-$, arylene, $-(CH_2)_r$arylene-, -arylene-$(CH_2)r-$, $-(CH_2)_r$-(C3-C8 carbocyclyl)-, -(C3-C8 carbocyclyl)-$(CH_2)_r-$, C3-C8 heterocyclyl, $-(CH_2)_r$(C3-C8 heterocyclyl)-, -(C3-C8 heterocyclyl)-$(CH_2)_r-$, $-(CH_2)_rC(O)NR^m(CH_2)_r-$, $-(CH_2CH_2O)_r-$, $-(CH_2CH_2O)_rCH_2-$, $-(CH_2)_rC(O)NR^m(CH_2CH_2O)_r-$, $-(CH_2)_rC(O)NR^m(CH_2CH_2O)_r-CH_2-$, $-(CH_2CH_2O)_rC(O)NR^m(CH_2CH_2O)_r-$, $-(CH_2CH_2O)_rC(O)NR^m(CH_2CH_2O)_r-CH_2-$ and $-(CH_2CH_2O)_rC(O)NR^m(CH_2)_r-$; wherein each $R^m$ is independently H, C1-C6 alkyl, C3-C8 carbocyclyl, phenyl or benzyl, and each r is independently 1, 2, 3, 4, 5, 6, 7, 8, 9 or 10;

D is a drug, such as an anti-cancer drug, a cytotoxic drug, a cell differentiation factor, a stem cell trophic factor, a steroid drug, a drug for treating autoimmune diseases, an anti-inflammatory drug or a drug for treating infectious diseases;

n is an integer from 1 to 24, e.g., 1, 2, 3, 4, 5, 6, 7, 8, 9, 10, 11, 12, 13, 14, 15, 16, 17, 18, 19, 20, 21, 22, 23 or 24.

In one or more embodiments, D is a tubulin inhibitor, a DNA damaging agent, or a DNA topoisomerase inhibitor.

In one or more embodiments, the tubulin inhibitor is selected from dolastatin, auristatins and maytansinoids.

In one or more embodiments, D is an auristatin, e.g., MMAE (monomethyl auristatin E), MMAF (monomethyl auristatin F), or AF (auristatin F).

In one or more embodiments, D is a DNA damaging agent, e.g., a calicheamicin, a duocarmycin, or the anthramycin derivative PBD (pyrrolobenzodiazepine).

In one or more embodiments, D is a DNA topoisomerase inhibitor or a salt thereof, e.g., irinotecan, irinotecan hydrochloride, camptothecin, 9-aminocamptothecin, 9-nitrocamptothecin, 10-hydroxycamptothecin, 9-chloro-10-hydroxycamptothecin, the camptothecin derivative SN-38, 22-hydroxyacuminatine, topotecan, lurtotecan, belotecan, exatecan, homosilatecan, 6,8-dibromo-2-methyl-3-[2-(D-xylopyranosylamino)phenyl]-4(3*H*)-quinazolinone, 2-cyano-3-(3,4-dihydroxyphenyl)-*N*-(phenylmethyl)-(2E)-2-propenamide, 2-cyano-3-(3,4-dihydroxyphenyl)-*N*-(3-hydroxyphenylpropyl)-(*E*)-2-propenamide, 12-(β-D-glucopyranosyl-12,13-dihydro-2,10-dihydroxy-6-[[2-hydroxy-1-(hydroxymethyl)ethyl]amino]-5*H*-indolo[2,3-*a*]pyrrolo[3,4-*c*]carbazole-5, 7(6*H*)-dione, *N*-[2-(dimethylamino)ethyl]-4-acridinecarboxamide dihydrochloride, or *N*-[2-(dimethylamino)ethyl]-4-acridinecarboxamide.

In one or more embodiments, the DNA topoisomerase inhibitor is camptothecin, 10-hydroxycamptothecin, topotecan, belotecan, irinotecan, 22-hydroxyacuminatine, or exatecan , or a salt thereof.

In one or more embodiments, D is a tubulysin, a taxane drug derivative, a leptomycine derivative, CC-1065 or an analog thereof, an amatoxin, a spliceosome inhibitor, a benzodiazepine (PBD) dimer, adriamycin, methotrexate, vincristine, vinblastine, daunorubicin, mitomycin C, melphalan, or a chlorambucil derivative.

In one or more embodiments, D is

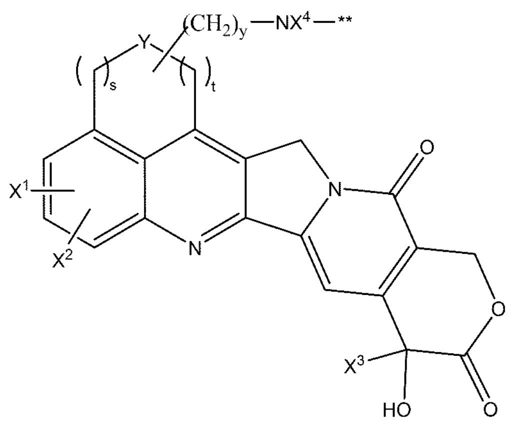

wherein
$X^1$ and $X^2$ are each independently:

H,

hydroxy,

C1-C6 alkyl,

C1-C6 alkyl substituted with one or more hydroxy, halogen, nitro or cyano groups,

C2-C6 alkenyl,

C2-C6 alkynyl,

C1-C6 alkoxy,

C1-C6 aminoalkoxy,

halogen,

nitro,

cyano,

thiol,

alkylthio,

amino, amino substituted with an amino-protecting group, C1-C6 aminoalkyl optionally substituted at the amino moiety with an amino-protecting group or C1-C6 alkyl,

C1-C6 aminoalkylamino optionally substituted at the amino moiety with an amino-protecting group or C1-C6 alkyl,

C1-C6 alkyl linking to a heterocyclic ring, wherein the heterocyclic ring is optionally substituted with one or more C1-C6 alkyl, C1-C6 alkoxy, amino, halogen, nitro or cyano groups,

C1-C6 alkylamino linking to a heterocyclic ring, wherein the heterocyclic ring is optionally substituted with C1-C6 alkyl or C1-C6 alkoxy, and the amino is optionally substituted with an amino-protecting group, halogen, nitro, cyano or protecting group,

amino-substituted heterocyclyl, which is optionally substituted at a nitrogen atom of the heterocyclyl moiety or at the amino moiety with a protecting group or one or more C1-C6 alkyl groups,

heterocyclylamino, which is optionally substituted at a nitrogen atom of the heterocyclic moiety or at the amino moiety with a protecting group or C1-C6 alkyl, carbamoyl optionally substituted with a carbamoyl-protecting group or C1-C6 alkyl,

morpholin-1-yl, or

piperidin-1-yl;

$X^3$ is C1-C6 alkyl;

$X^4$ is H, $-(CH_2)_q-CH_3$, $-(CHR^n)_q-CH_3$, C3-C8 carbocyclyl, $-O-(CH_2)_q-CH_3$, arylene-$CH_3$, $-(CH_2)_q$-arylene-$CH_3$, -arylene-$(CH_2)_q-CH_3$, $-(CH_2)_q$-(C3-C8 carbocyclyl)-$CH_3$, -(C3-C8 carbocyclyl)-$(CH_2)_q-CH_3$, C3-C8 heterocyclyl, $-(CH_2)_q$-(C3-C8 heterocyclyl)-$CH_3$, -(C3-C8 heterocyclyl)-$(CH_2)_q-CH_3$, $-(CH_2)_qC(O)NR^n(CH_2)_q-CH_3$, $-(CH_2CH_2O)_q-CH_3$, $-(CH_2CH_2O)_q-CH_2-CH_3$, $-(CH_2)_qC(O)NR^n(CH_2CH_2O)_q-CH_3$, $-(CH_2)_qC(O)NR^n(CH_2CH_2O)_q-CH_2-CH_3$, $-(CH_2CH_2O)_qC(O)NR^n(CH_2CH_2O)_q-CH_3$, $-(CH_2CH_2O)_qC(O)NR^n(CH_2CH_2O)_q-CH_2-CH_3$ or $-(CH_2CH_2O)_qC(O)NR^n(CH_2)_q-CH_3$; wherein each $R^n$ is independently H, C1-C6 alkyl, C3-C8 carbocyclyl, phenyl or benzyl, and each q is independently 1, 2, 3, 4, 5, 6, 7, 8, 9 or 10;

** is point of connection;

y is 0, 1 or 2;

Y is O, S or $CR^1R^2$, wherein $R^1$ and $R^2$ are each independently H or C1-C6 alkyl;

s and t are each independently 0, 1 or 2, but not both 0.

In one or more embodiments, $X^4$ is H or C1-C6 alkyl.

In one or more embodiments, the heterocyclyl is azetidine, niverazine, morpholine, pyrrolidine, piperidine, imidazole, thiazole, oxazole or pyridine.

In one or more embodiments, the amino-protecting group is formyl, acetyl, trityl, *t*-butoxycarbonyl, benzyl, or *p*-methoxybenzyloxycarbonyl.

In one or more embodiments, D is

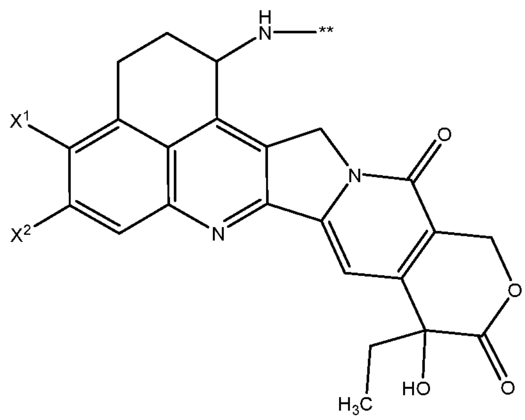

,

wherein $X^1$ and $X^2$ are each independently C1-C6 alkyl, halogen, or -OH; ** is point of connection.

In one or more embodiments, D is

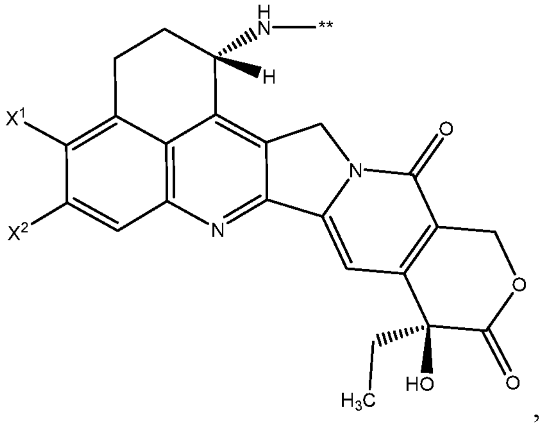

wherein $X^1$ and $X^2$ are each independently C1-C6 alkyl, halogen, or -OH; ** is point of connection.

In one or more embodiments, $X^1$ and $X^2$ are each $-CH_3$.

In one or more embodiments, $X^1$ and $X^2$ are each independently F, Cl, Br, or I.

In one or more embodiments, $X^1$ and $X^2$ are each F.

In one or more embodiments, $X^1$ and $X^2$ are each independently $-CH_3$, F, or -OH.

In one or more embodiments, $X^1$ and $X^2$ are each independently F or $-CH_3$.

In one or more embodiments, $X^1$ is $-CH_3$ and $X^2$ is F.

In one or more embodiments, R is $-(CH_2)_r-$.

In one or more embodiments, R is $-(CH_2)_r-$, wherein r is 1 or 5.

In one or more embodiments, n is 4-12.

In one or more embodiments, n is 4-8.

In one or more embodiments, n is 4.

In one or more embodiments, n is 8.

In one or more embodiments, Formula III is:

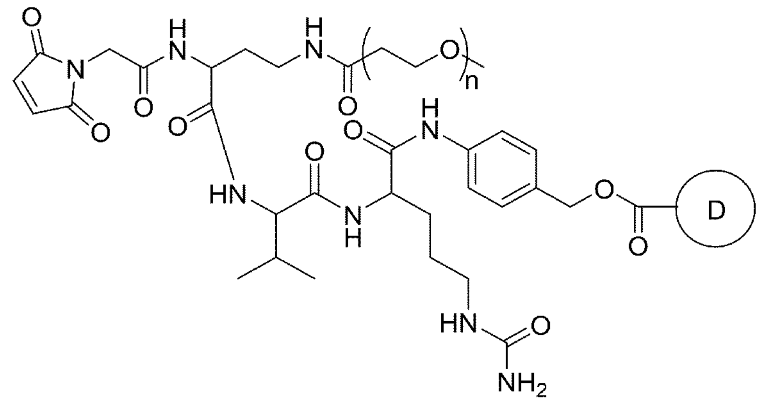

Formula III-3

wherein

D is a drug, such as an anti-cancer drug, a cytotoxic drug, a cell differentiation factor, a stem cell trophic factor, a steroid drug, a drug for treating autoimmune diseases, an anti-inflammatory drug or a drug for treating infectious diseases;

n is an integer from 1 to 24, e.g., 1, 2, 3, 4, 5, 6, 7, 8, 9, 10, 11, 12, 13, 14, 15, 16, 17, 18, 19, 20, 21, 22, 23 or 24.

In one or more embodiments, D is a tubulin inhibitor, a DNA damaging agent, or a DNA topoisomerase inhibitor.

In one or more embodiments, the tubulin inhibitor is selected from dolastatin, auristatins and maytansinoids.

In one or more embodiments, D is an auristatin, e.g., MMAE (monomethyl auristatin E), MMAF (monomethyl auristatin F), or AF (auristatin F).

In one or more embodiments, D is a DNA damaging agent, e.g., a calicheamicin, a duocarmycin, or the anthramycin derivative PBD (pyrrolobenzodiazepine).

In one or more embodiments, D is a DNA topoisomerase inhibitor or a salt thereof, e.g., irinotecan, irinotecan hydrochloride, camptothecin, 9-aminocamptothecin, 9-nitrocamptothecin, 10-hydroxycamptothecin, 9-chloro-10-hydroxycamptothecin, the camptothecin derivative SN-38, 22-hydroxyacuminatine, topotecan, lurtotecan, belotecan, exatecan, homosilatecan, 6,8-dibromo-2-methyl-3-[2-(D-xylopyranosylamino)phenyl]-4(3*H*)-quinazolinone, 2-cyano-3-(3,4-dihydroxyphenyl)-*N*-(phenylmethyl)-(2E)-2-propenamide, 2-cyano-3-(3,4-dihydroxyphenyl)-*N*-(3-hydroxyphenylpropyl)-(*E*)-2-propenamide, 12-(β-D-glucopyranosyl-12,13-dihydro-2,10-dihydroxy-6-[[2-hydroxy-1-(hydroxymethyl)ethyl]amino]-5*H*-indolo[2,3-*a*]pyrrolo[3,4-*c*]carbazole-5, 7(6*H*)-dione, *N*-[2-(dimethylamino)ethyl]-4-acridinecarboxamide dihydrochloride, or *N*-[2-(dimethylamino)ethyl]-4-acridinecarboxamide.

In one or more embodiments, the DNA topoisomerase inhibitor is camptothecin, 10-hydroxycamptothecin, topotecan, belotecan, irinotecan, 22-hydroxyacuminatine, or exatecan, or a salt thereof.

In one or more embodiments, D is a tubulysin, a taxane drug derivative, a leptomycine derivative, CC-1065 or an analog thereof, an amatoxin, a spliceosome inhibitor, a benzodiazepine (PBD) dimer, adriamycin, methotrexate, vincristine, vinblastine, daunorubicin, mitomycin C, melphalan, or a chlorambucil derivative.

In one or more embodiments, D is

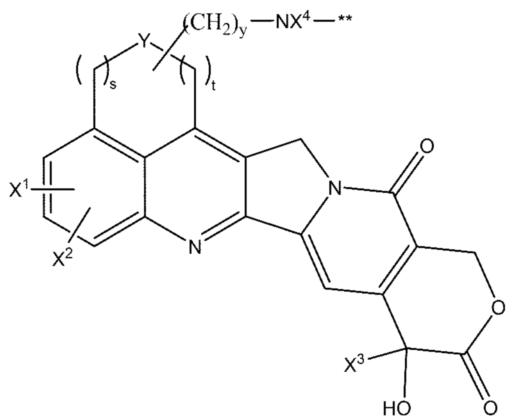,

wherein

$X^1$ and $X^2$ are each independently:

H,

hydroxy,

C1-C6 alkyl,

C1-C6 alkyl substituted with one or more hydroxy, halogen, nitro or cyano groups,

C2-C6 alkenyl,

C2-C6 alkynyl,

C1-C6 alkoxy,

C1-C6 aminoalkoxy,

halogen,

nitro,

cyano,

thiol,

alkylthio,

amino, amino substituted with an amino-protecting group, C1-C6 aminoalkyl optionally substituted at the amino moiety with an amino-protecting group or C1-C6 alkyl,

C1-C6 aminoalkylamino optionally substituted at the amino moiety with an amino-protecting group or C1-C6 alkyl,

C1-C6 alkyl linking to a heterocyclic ring, wherein the heterocyclic ring is optionally substituted with one or more C1-C6 alkyl, C1-C6 alkoxy, amino, halogen, nitro or cyano groups,

C1-C6 alkylamino linking to a heterocyclic ring, wherein the heterocyclic ring is optionally substituted with C1-C6 alkyl or C1-C6 alkoxy, and the amino is optionally substituted with an amino-protecting group, halogen, nitro, cyano or protecting group, amino-substituted heterocyclyl, which is optionally substituted at a nitrogen atom of the heterocyclyl moiety or at the amino moiety with a protecting group or one or more C1-C6 alkyl groups,

heterocyclylamino, which is optionally substituted at a nitrogen atom of the heterocyclic moiety or at the amino moiety with a protecting group or C1-C6 alkyl, carbamoyl optionally substituted with a carbamoyl-protecting group or C1-C6 alkyl,

morpholin-1-yl, or

piperidin-1-yl;

$X^3$ is C1-C6 alkyl;

$X^4$ is H, $-(CH2)_q-CH_3$, $-(CHR^n)_q-CH_3$, C3-C8 carbocyclyl, $-O-(CH_2)_q-CH_3$, arylene-$CH_3$, $-(CH_2)_q$-arylene-$CH_3$, -arylene-$(CH_2)_q-CH_3$, $-(CH_2)_q$-(C3-C8 carbocyclyl)-$CH_3$, -(C3-C8 carbocyclyl)-$(CH_2)_q-CH_3$, C3-C8 heterocyclyl, $-(CH_2)_q$-(C3-C8 heterocyclyl)-$CH_3$, -(C3-C8 heterocyclyl)-$(CH_2)_q-CH_3$, $-(CH_2)_qC(O)NR^n(CH_2)_q-CH_3$, $-(CH_2CH_2O)_q-CH_3$, $-(CH_2CH_2O)_q-CH_2-CH_3$, $-(CH_2)_qC(O)NR^n(CH_2CH_2O)_q-CH_3$, $-(CH_2)_qC(O)NR^n(CH_2CH_2O)_q-CH_2-CH_3$, $-(CH_2CH_2O)_qC(O)NR^n(CH_2CH_2O)_q-CH_3$, $-(CH_2CH_2O)_qC(O)NR^n(CH_2CH_2O)_q-CH_2-CH_3$ or $-(CH_2CH_2O)_qC(O)NR^n(CH_2)_q-CH_3$; wherein each $R^n$ is independently H, C1-C6 alkyl, C3-C8 carbocyclyl, phenyl or benzyl, and each q is independently 1, 2, 3, 4, 5, 6, 7, 8, 9 or 10;

** is point of connection;

y is 0, 1 or 2;

Y is O, S or $CR^1R^2$, wherein $R^1$ and $R^2$ are each independently H or C1-C6 alkyl;

s and t are each independently 0, 1 or 2, but not both 0.

In one or more embodiments, $X^4$ is H or C1-C6 alkyl.

In one or more embodiments, the heterocyclyl is azetidine, niverazine, morpholine, pyrrolidine, piperidine, imidazole, thiazole, oxazole or pyridine.

In one or more embodiments, the amino-protecting group is formyl, acetyl, trityl, *t*-butoxycarbonyl, benzyl, or *p*-methoxybenzyloxycarbonyl.

In one or more embodiments, D is

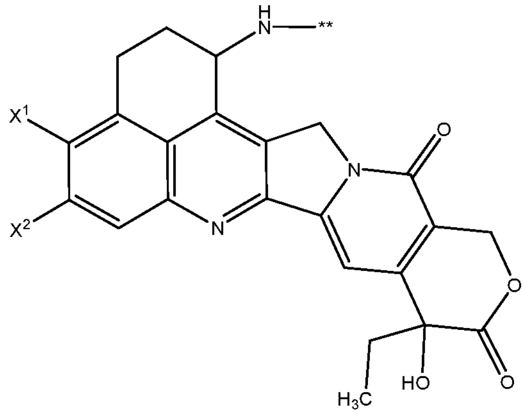

,

wherein $X^1$ and $X^2$ are each independently C1-C6 alkyl, halogen, or -OH; ** is point of connection.

In one or more embodiments, D is

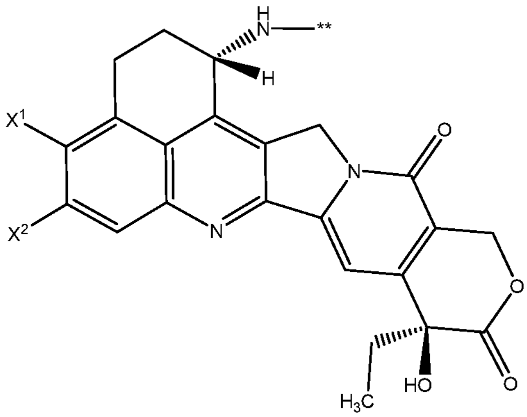

,

wherein $X^1$ and $X^2$ are each independently C1-C6 alkyl, halogen, or -OH; ** is point of connection.

In one or more embodiments, $X^1$ and $X^2$ are each $-CH_3$.

In one or more embodiments, $X^1$ and $X^2$ are each independently F, Cl, Br, or I.

In one or more embodiments, $X^1$ and $X^2$ are each F.

In one or more embodiments, $X^1$ and $X^2$ are each independently $-CH_3$, F, or -OH.

In one or more embodiments, $X^1$ and $X^2$ are each independently F or $-CH_3$.

In one or more embodiments, $X^1$ is $-CH_3$ and $X^2$ is F.

In one or more embodiments, n is 4-12.

In one or more embodiments, n is 4-8.

In one or more embodiments, n is 4.

In one or more embodiments, n is 8.

In one or more embodiments, Formula III is:

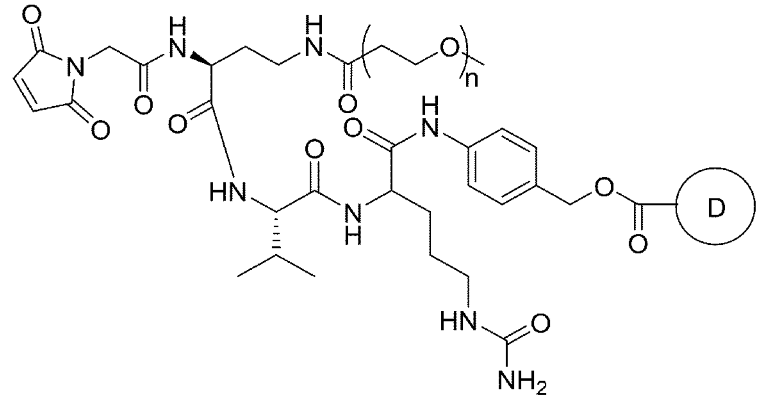

Formula III-4

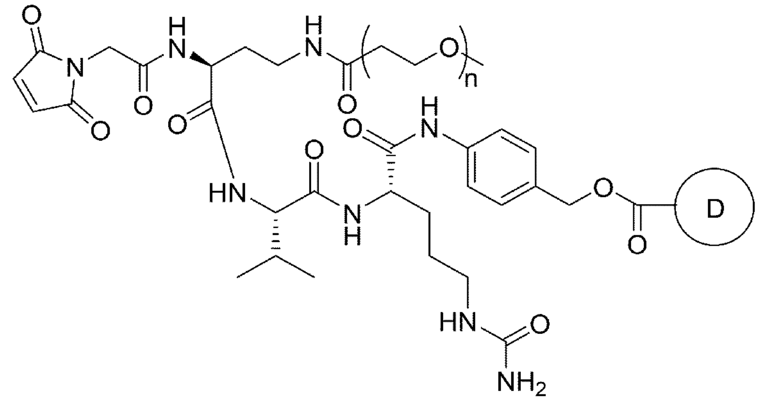

Formula III-4-1

wherein

D is a drug, such as an anti-cancer drug, a cytotoxic drug, a cell differentiation factor, a stem cell trophic factor, a steroid drug, a drug for treating autoimmune diseases, an anti-inflammatory drug or a drug for treating infectious diseases;

n is an integer from 1 to 24, e.g., 1, 2, 3, 4, 5, 6, 7, 8, 9, 10, 11, 12, 13, 14, 15, 16, 17, 18, 19, 20, 21, 22, 23 or 24.

In one or more embodiments, D is a tubulin inhibitor, a DNA damaging agent, or a DNA topoisomerase inhibitor.
In one or more embodiments, the tubulin inhibitor is selected from dolastatin, auristatins and maytansinoids.
In one or more embodiments, D is an auristatin, e.g., MMAE (monomethyl auristatin E), MMAF (monomethyl auristatin F), or AF (auristatin F).
In one or more embodiments, D is a DNA damaging agent, e.g., a calicheamicin, a duocarmycin, or the anthramycin derivative PBD (pyrrolobenzodiazepine).
In one or more embodiments, D is a DNA topoisomerase inhibitor or a salt thereof, e.g., irinotecan, irinotecan hydrochloride, camptothecin, 9-aminocamptothecin, 9-nitrocamptothecin, 10-hydroxycamptothecin, 9-chloro-10-hydroxycamptothecin, the camptothecin derivative SN-38, 22-hydroxyacuminatine, topotecan, lurtotecan, belotecan, exatecan, homosilatecan, 6,8-dibromo-2-methyl-3-[2-(D-xylopyranosylamino)phenyl]-4(3*H*)-quinazolinone, 2-cyano-3-(3,4-dihydroxyphenyl)-*N*-(phenylmethyl)-(2*E*)-2-propenamide, 2-cyano-3-(3,4-dihydroxyphenyl)-*N*-(3-hydroxyphenylpropyl)-(*E*)-2-propenamide, 12-(β-D-glucopyranosyl-12,13-dihydro-2,10-dihydroxy-6-[[2-hydroxy-1-(hydroxymethyl)ethyl]amino]-5*H*-indolo[2,3-*a*]pyrrolo[3,4-*c*]carbazole-5, 7(6*H*)-dione, *N*-[2-(dimethylamino)ethyl]-4-aciidinecarboxamide dihydrochloride, or *N*-[2-(dimethylamino)ethyl]-4-acridinecarboxamide.
In one or more embodiments, the DNA topoisomerase inhibitor is camptothecin, 10-hydroxycamptothecin, topotecan, belotecan, irinotecan, 22-hydroxyacuminatine, or exatecan, or a salt thereof.
In one or more embodiments, D is a tubulysin, a taxane drug derivative, a leptomycine derivative, CC-1065 or an analog thereof, an amatoxin, a spliceosome inhibitor, a benzodiazepine (PBD) dimer, adriamycin, methotrexate,

vincristine, vinblastine, daunorubicin, mitomycin C, melphalan, or a chlorambucil derivative.

In one or more embodiments, D is

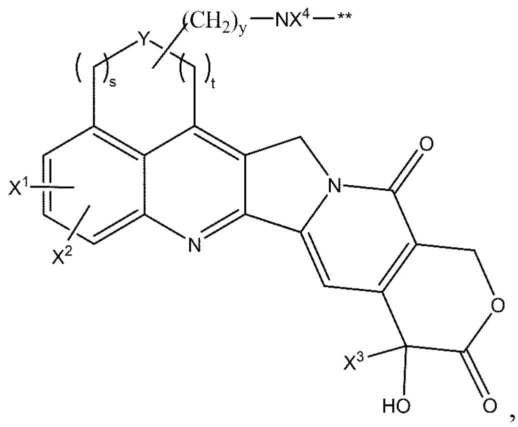

wherein

$X^1$ and $X^2$ are each independently:

H,

hydroxy,

C1-C6 alkyl,

C1-C6 alkyl substituted with one or more hydroxy, halogen, nitro or cyano groups,

C2-C6 alkenyl,

C2-C6 alkynyl,

C1-C6 alkoxy,

C1-C6 aminoalkoxy,

halogen,

nitro,

cyano,

thiol,

alkylthio,

amino, amino substituted with an amino-protecting group, C1-C6 aminoalkyl optionally substituted at the amino moiety with an amino-protecting group or C1-C6 alkyl,

C1-C6 aminoalkylamino optionally substituted at the amino moiety with an amino-protecting group or C1-C6 alkyl,

C1-C6 alkyl linking to a heterocyclic ring, wherein the heterocyclic ring is optionally substituted with one or more C1-C6 alkyl, C1-C6 alkoxy, amino, halogen, nitro or cyano groups,

C1-C6 alkylamino linking to a heterocyclic ring, wherein the heterocyclic ring is optionally substituted with C1-C6 alkyl or C1-C6 alkoxy, and the amino is optionally substituted with an amino-protecting group, halogen, nitro, cyano or protecting group, amino-substituted heterocyclyl, which is optionally substituted at a nitrogen atom of the heterocyclyl moiety or at the amino moiety with a protecting group or one or more C1-C6 alkyl groups,

heterocyclylamino, which is optionally substituted at a nitrogen atom of the heterocyclic moiety or at the amino moiety with a protecting group or C1-C6 alkyl,

carbamoyl optionally substituted with a carbamoyl-protecting group or C1-C6 alkyl, morpholin-1-yl, or

piperidin-1-yl;

$X^3$ is C1-C6 alkyl;

$X^4$ is H, $-(CH_2)_q-CH_3$, $-(CHR^n)_q-CH_3$, C3-C8 carbocyclyl, $-O-(CH_2)_q-CH_3$, arylene-$CH_3$, $-(CH_2)_q$-arylene-$CH_3$, -arylene-$(CH_2)_q-CH_3$, $-(CH_2)_q$-(C3-C8 carbocyclyl)-$CH_3$, -(C3-C8 carbocyclyl)-$(CH_2)_q-CH_3$, C3-C8 heterocyclyl, $-(CH_2)_q$-(C3-C8 heterocyclyl)-$CH_3$, -(C3-C8 heterocyclyl)-$(CH_2)_q-CH_3$, $-(CH_2)_qC(O)NR^n(CH_2)_q-CH_3$, $-(CH_2CH_2O)_q-CH_3$, $-(CH_2CH_2O)_q-CH_2-CH_3$, $-(CH_2)_qC(O)NR^n(CH_2CH_2O)_q-CH_3$, $-(CH_2)_qC(O)NR^n(CH_2CH_2O)_q-CH_2-CH_3$, $-(CH_2CH_2O)_qC(O)NR^n(CH_2CH_2O)_q-CH_3$, $-(CH_2CH_2O)_qC(O)NR^n(CH_2CH_2O)_q-CH_2-CH_3$ or $-(CH_2CH_2O)_qC(O)NR^n(CH_2)_q-CH_3$; wherein each $R^n$ is independently H, C1-C6 alkyl, C3-C8 carbocyclyl, phenyl or benzyl, and each q is independently 1, 2, 3, 4, 5, 6, 7, 8, 9 or 10;

** is point of connection;

y is 0, 1 or 2;

Y is O, S or $CR^1R^2$, wherein $R^1$ and $R^2$ are each independently H or C1-C6 alkyl;

s and t are each independently 0, 1 or 2, but not both 0.

In one or more embodiments, $X^4$ is H or C1-C6 alkyl.

In one or more embodiments, the heterocyclyl is azetidine, niverazine, morpholine, pyrrolidine, piperidine, imidazole, thiazole, oxazole or pyridine.

In one or more embodiments, the amino-protecting group is formyl, acetyl, trityl, *t*-butoxycarbonyl, benzyl, or *p*-methoxybenzyloxycarbonyl.

In one or more embodiments, D is

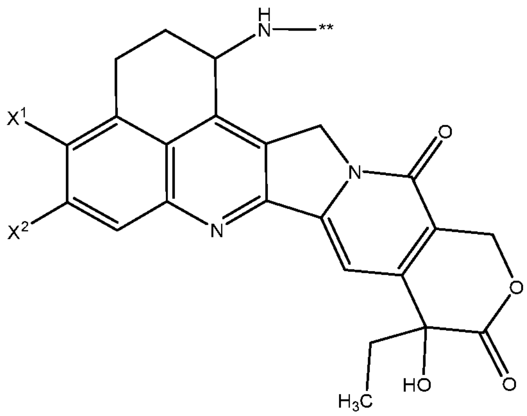

,

wherein $X^1$ and $X^2$ are each independently C1-C6 alkyl, halogen, or -OH; ** is point of connection.

In one or more embodiments, D is

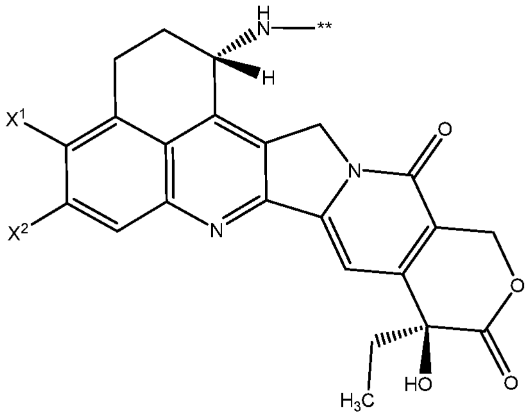

,

wherein $X^1$ and $X^2$ are each independently C1-C6 alkyl, halogen, or -OH; ** is point of connection.

In one or more embodiments, $X^1$ and $X^2$ are each $-CH_3$.

In one or more embodiments, $X^1$ and $X^2$ are each independently F, Cl, Br, or I.

In one or more embodiments, $X^1$ and $X^2$ are each F.

In one or more embodiments, $X^1$ and $X^2$ are each independently $-CH_3$, F, or -OH.

In one or more embodiments, $X^1$ and $X^2$ are each independently F or $-CH_3$.

In one or more embodiments, $X^1$ is $-CH_3$ and $X^2$ is F.

In one or more embodiments, n is 4-12.

In one or more embodiments, n is 4-8.

In one or more embodiments, n is 4.

In one or more embodiments, n is 8.

In one or more embodiments, Formula III is selected from the following structures:

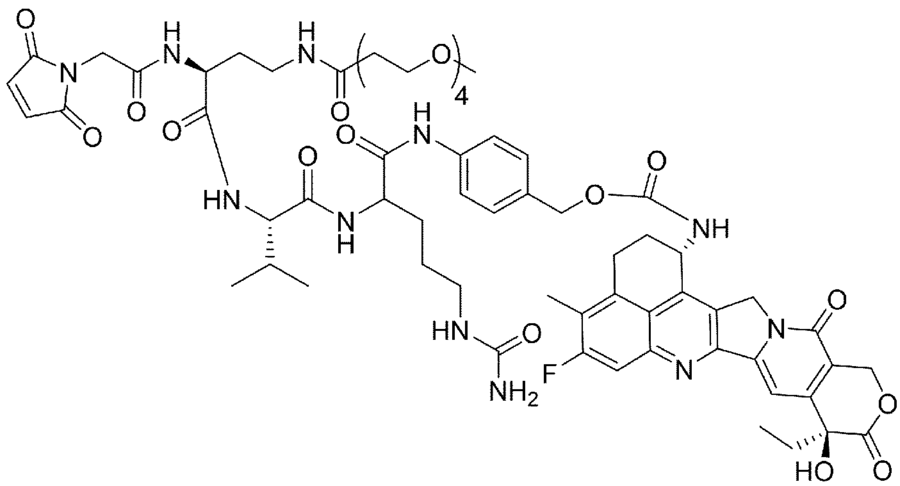

Formula III-5

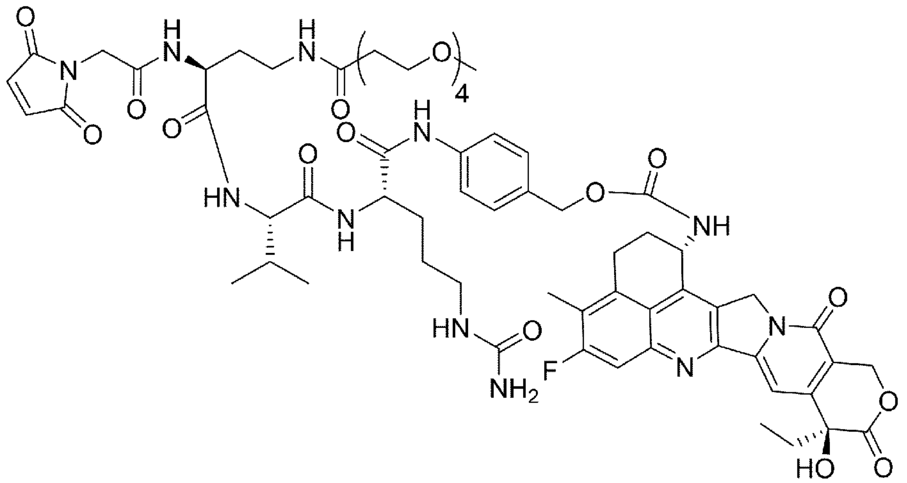

Formula III-5-1

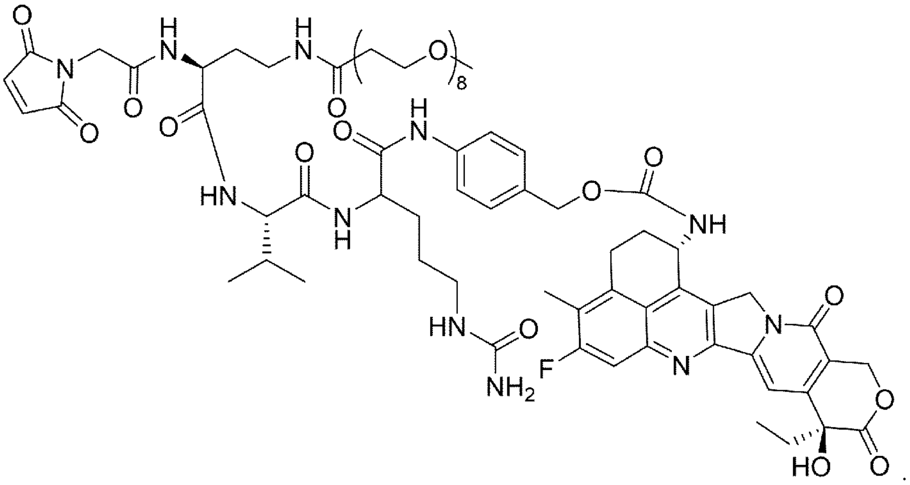

Formula III-6

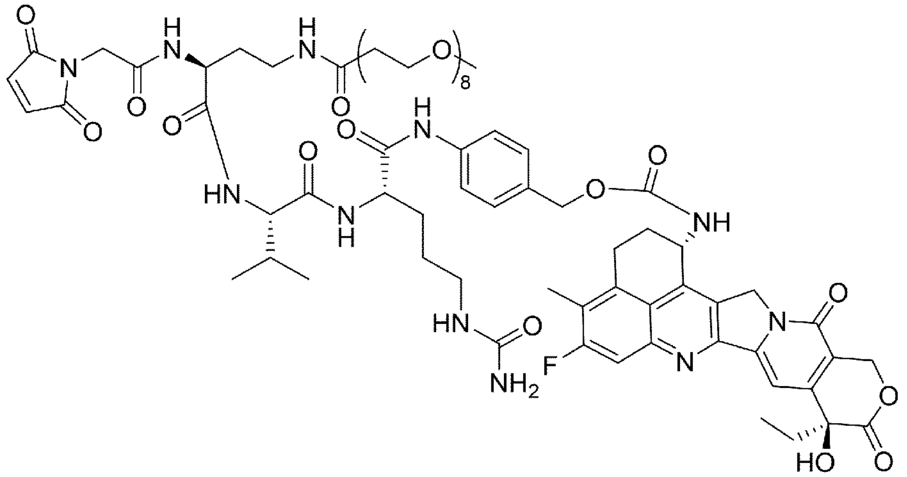

Formula III-6-1

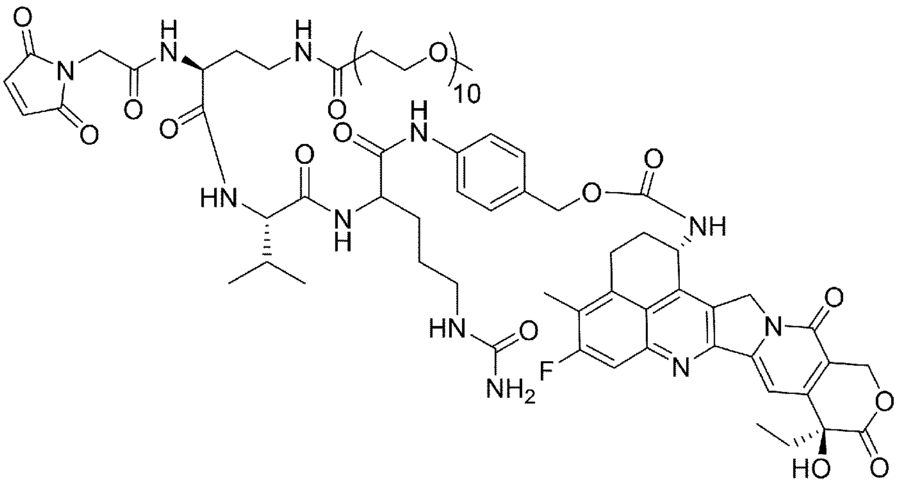

Formula III-7

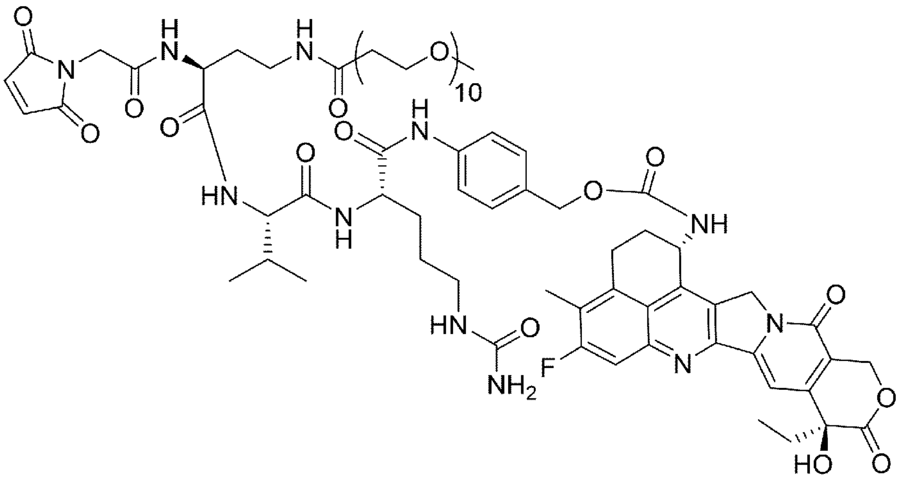

Formula III-7-1

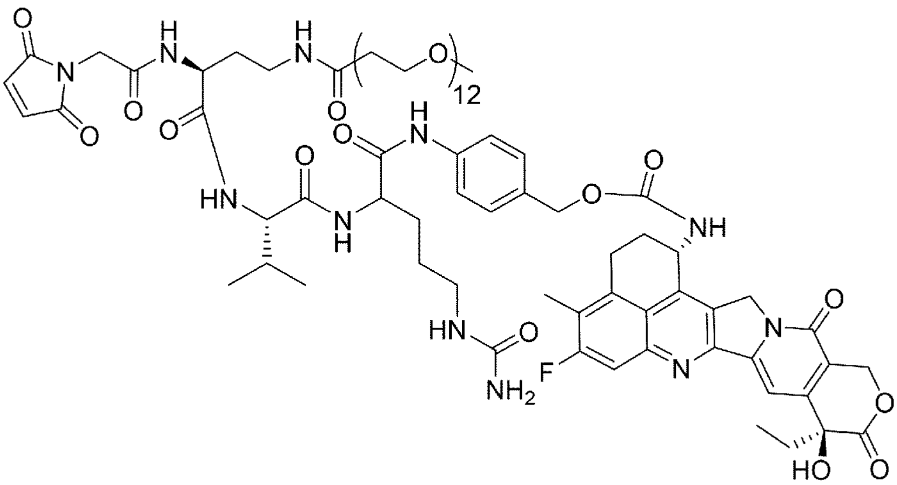

Formula III-8

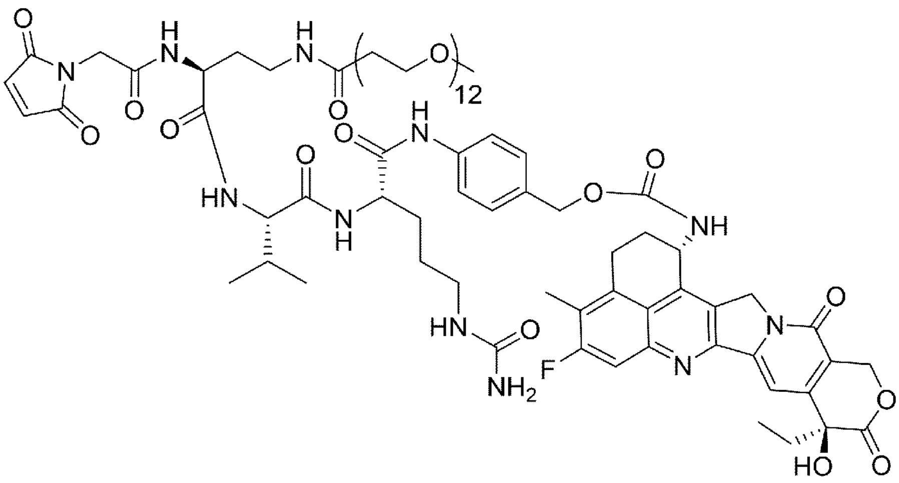

Formula III-8-1

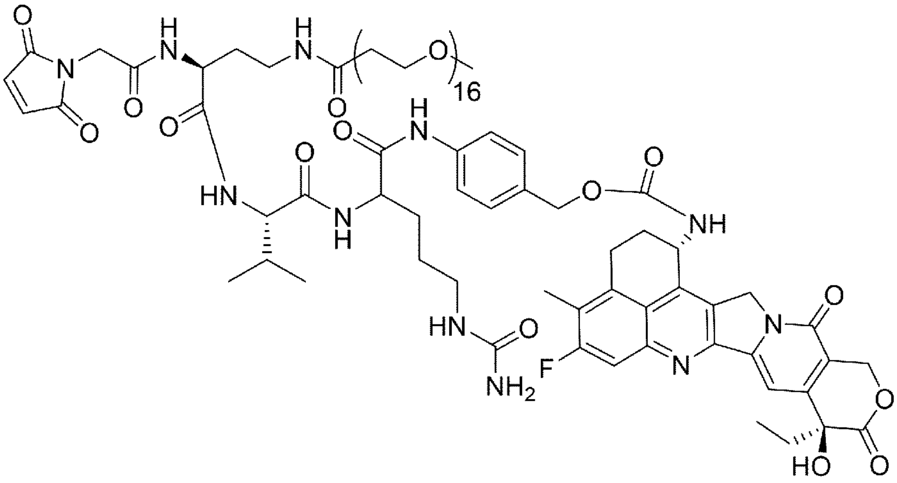

Formula III-9

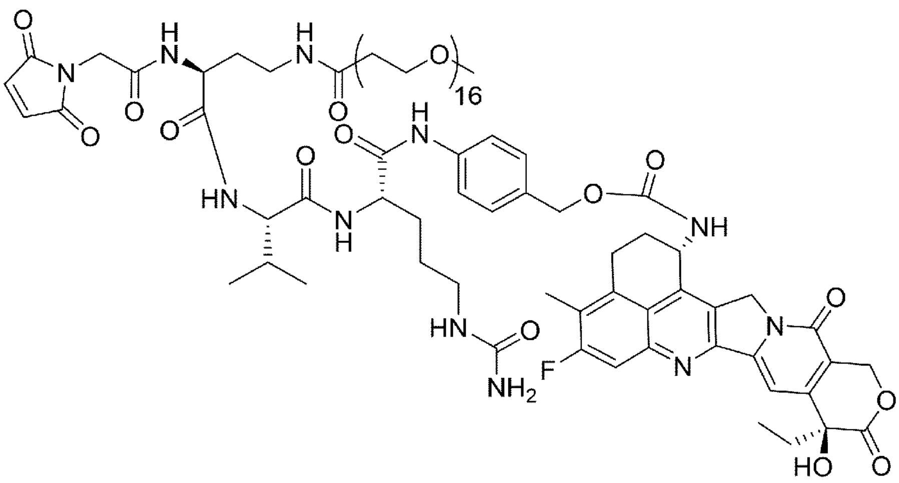

Formula III-9-1

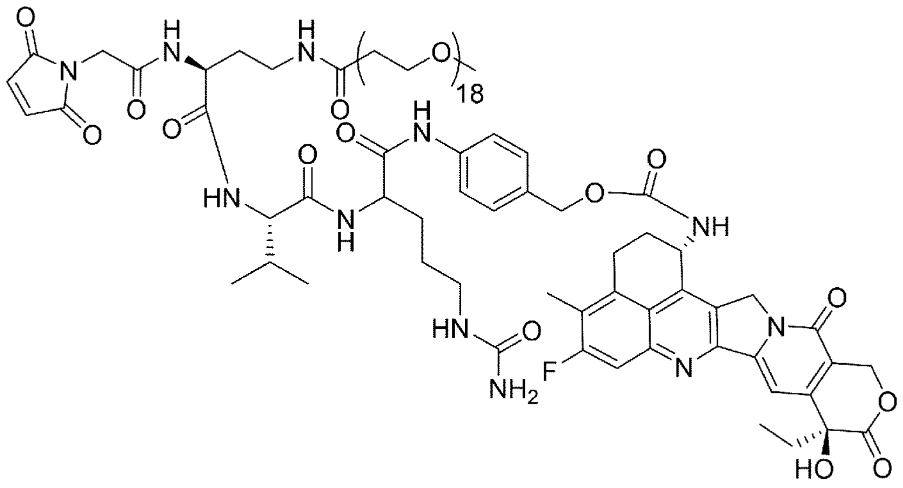

Formula III-10

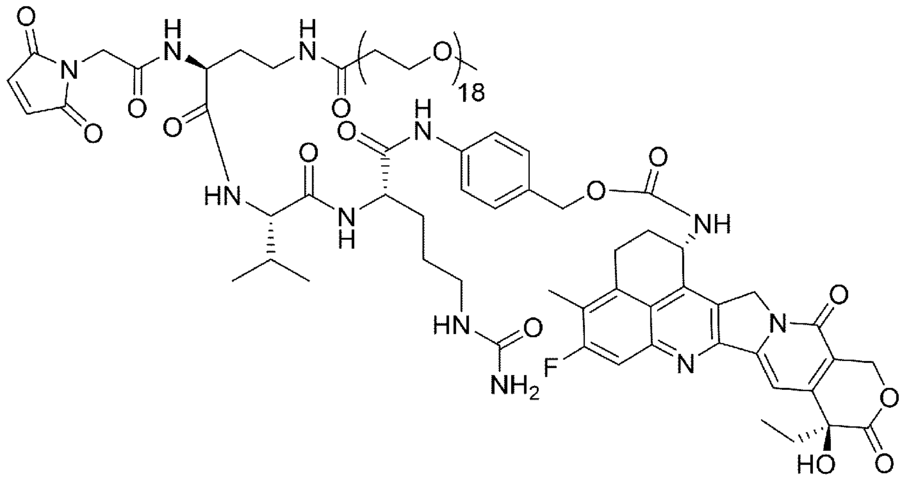

Formula III-10-1

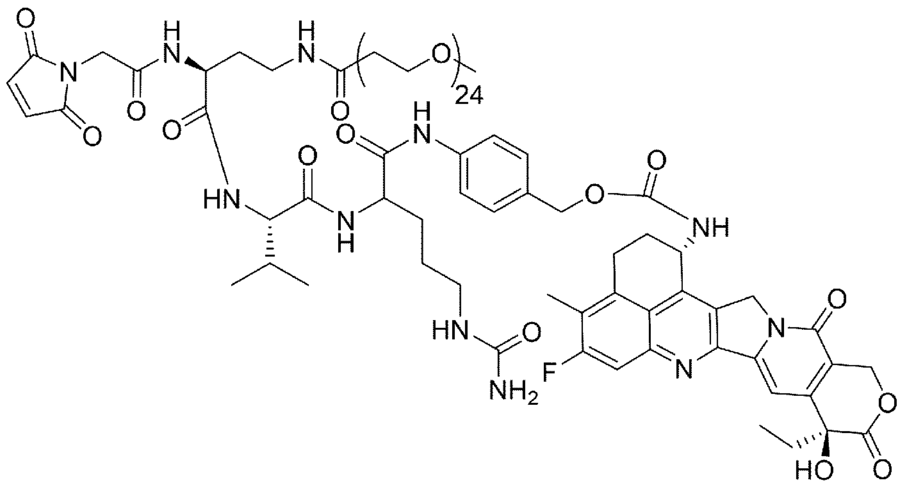

Formula III-11

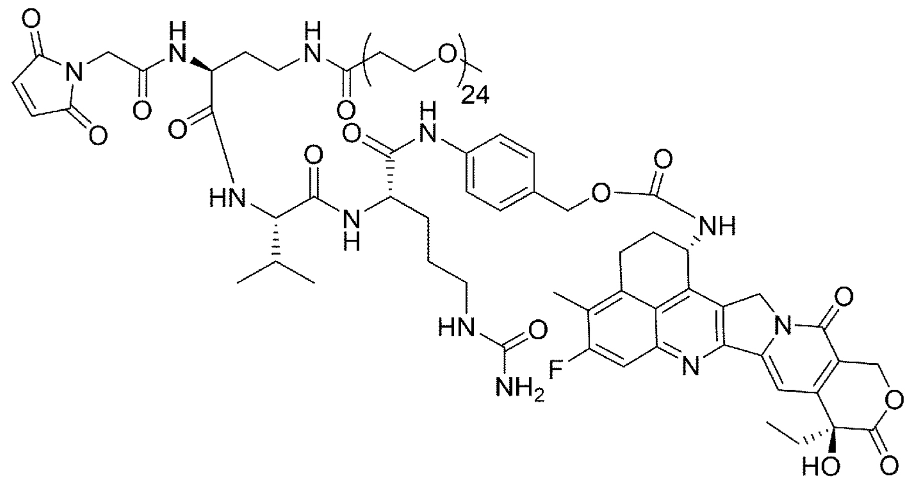

Formula III-11-1

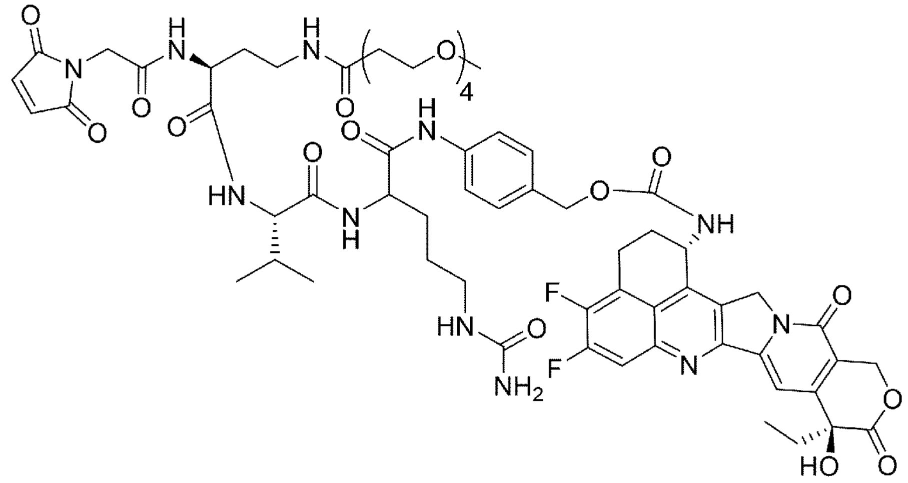

Formula III-12

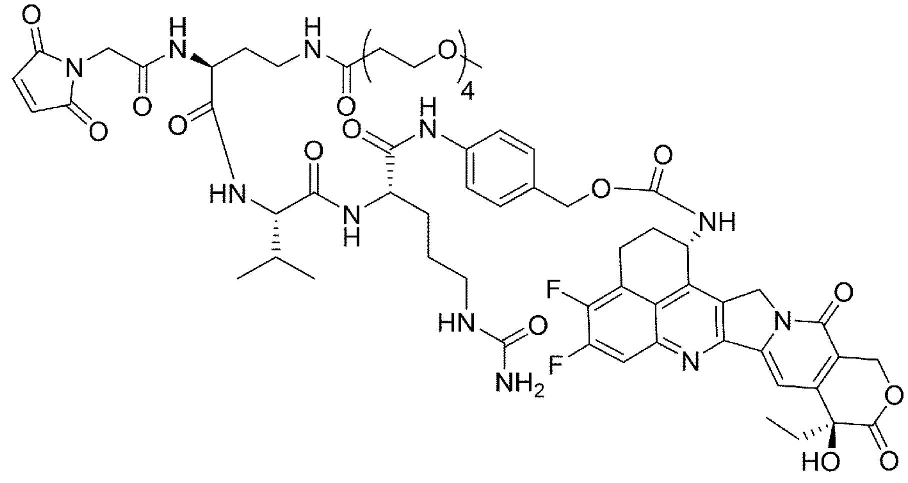

Formula III-12-1

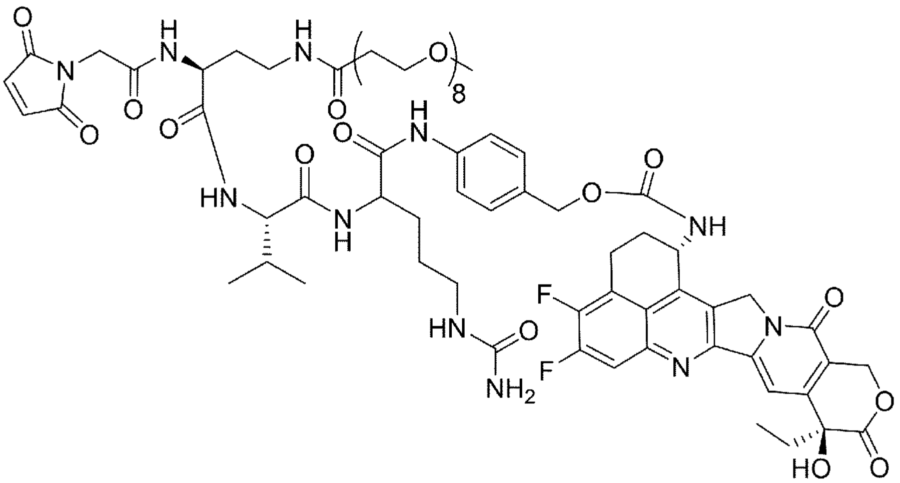

Formula III-13

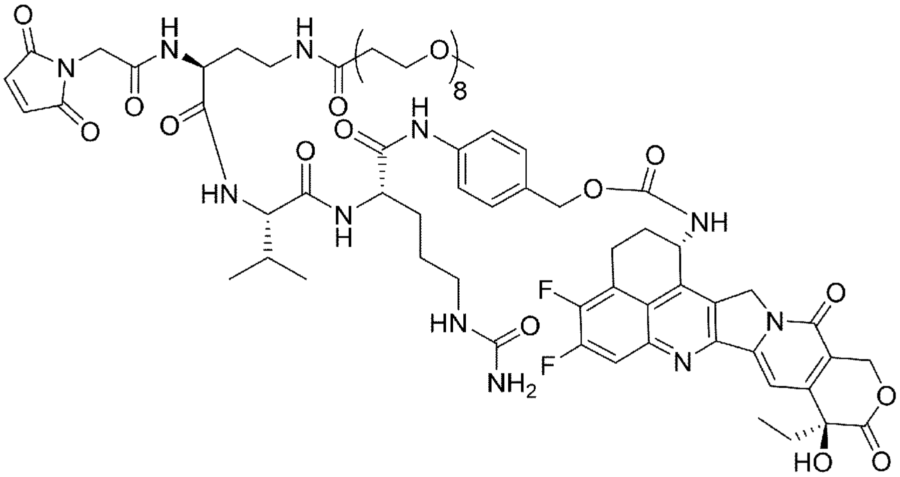

Formula III-13-1

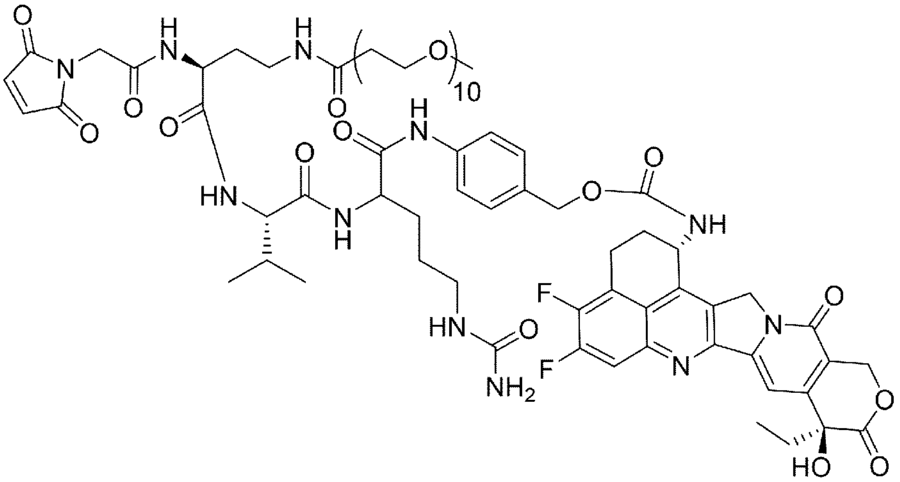

Formula III-14

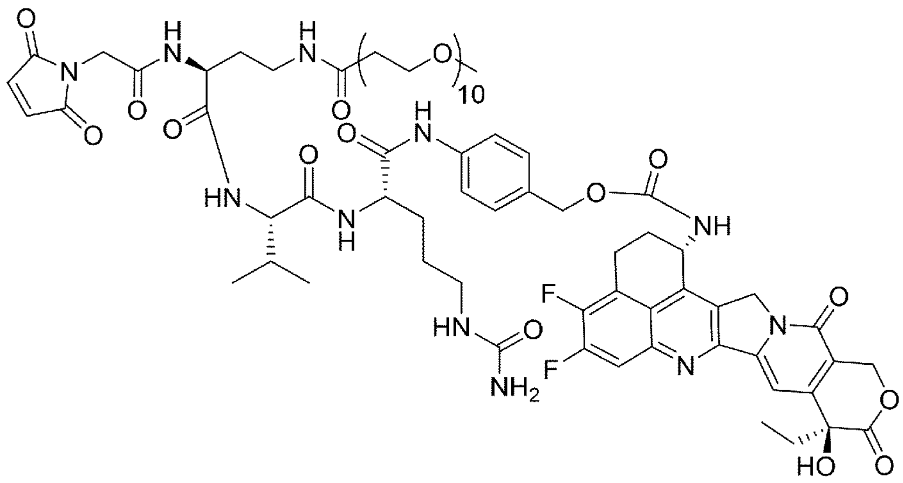

Formula III-14-1

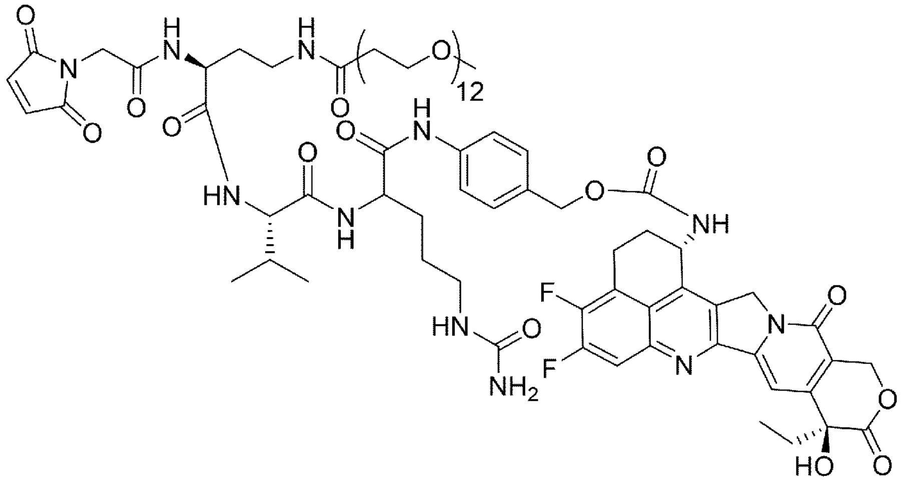

Formula III-15

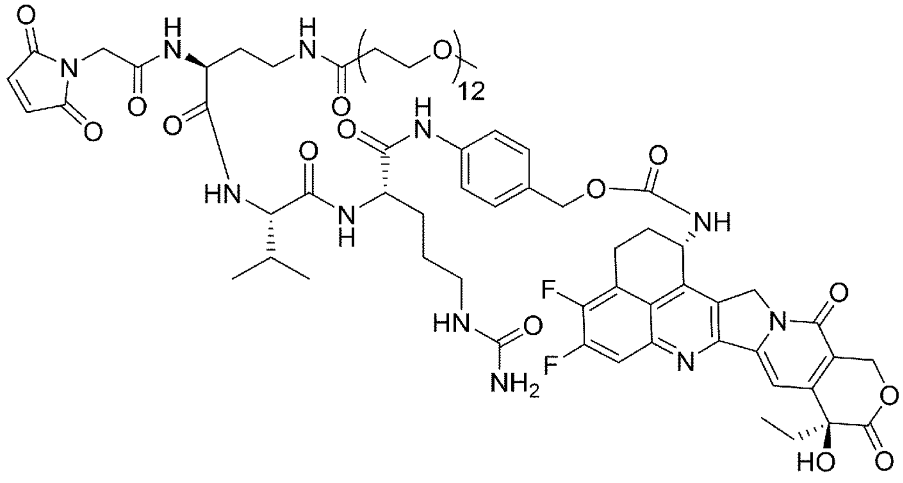

Formula III-15-1

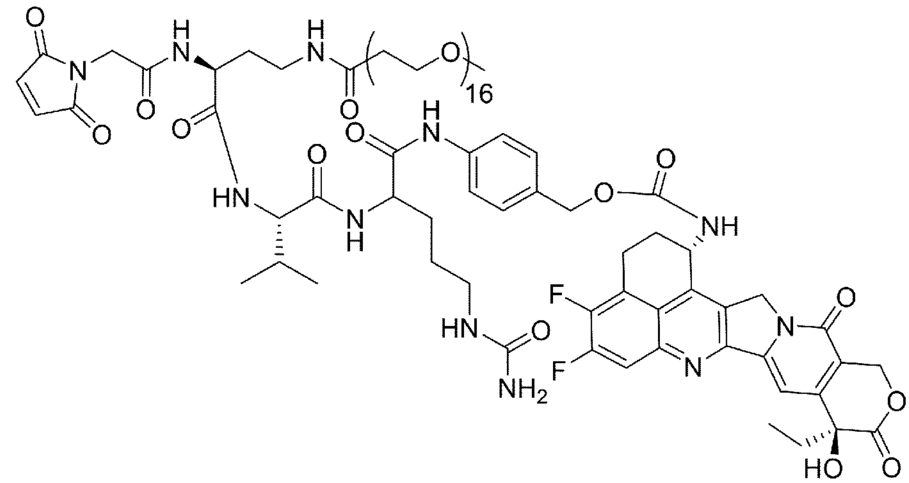

Formula III-16

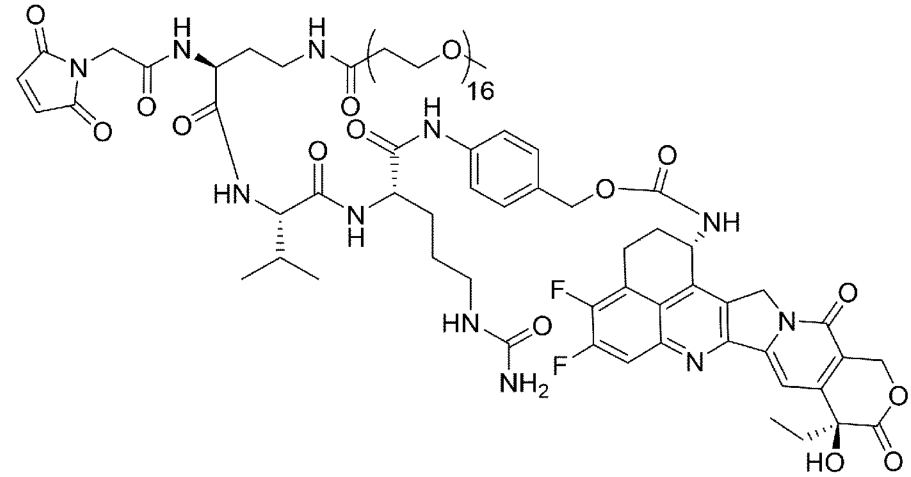

Formula III-16-1

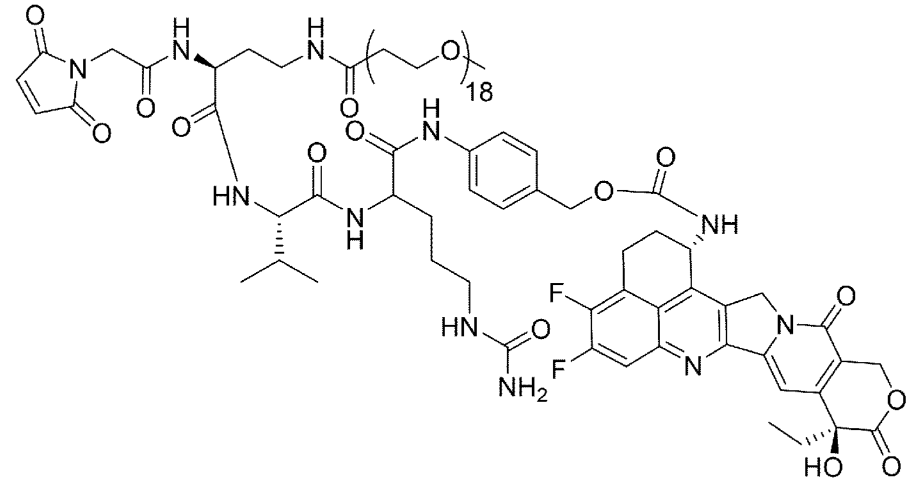

Formula III-17

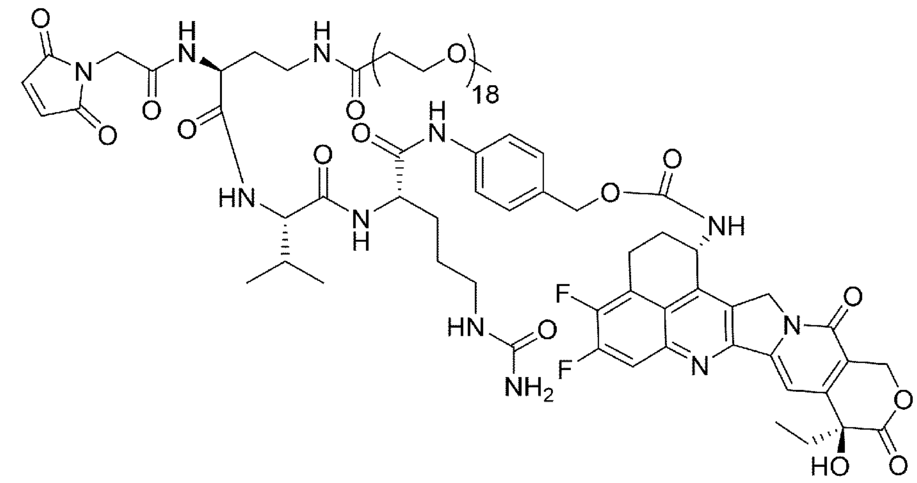

Formula III-17-1

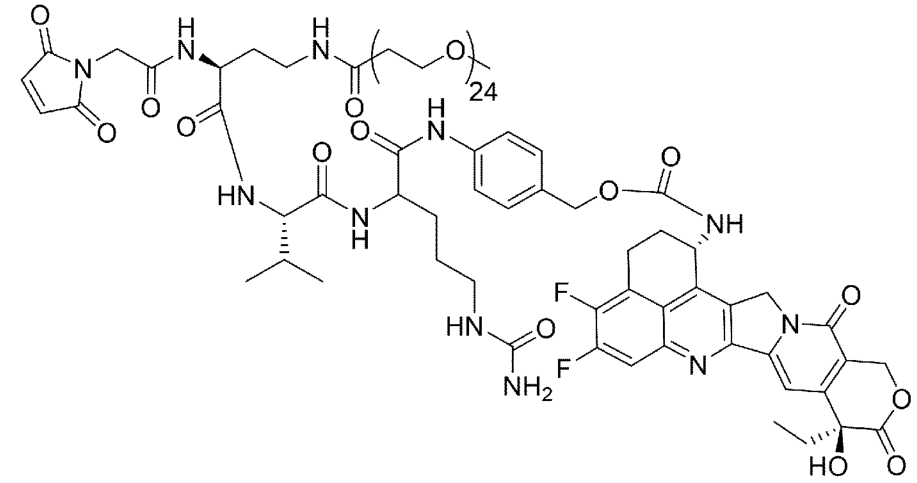

Formula III-18

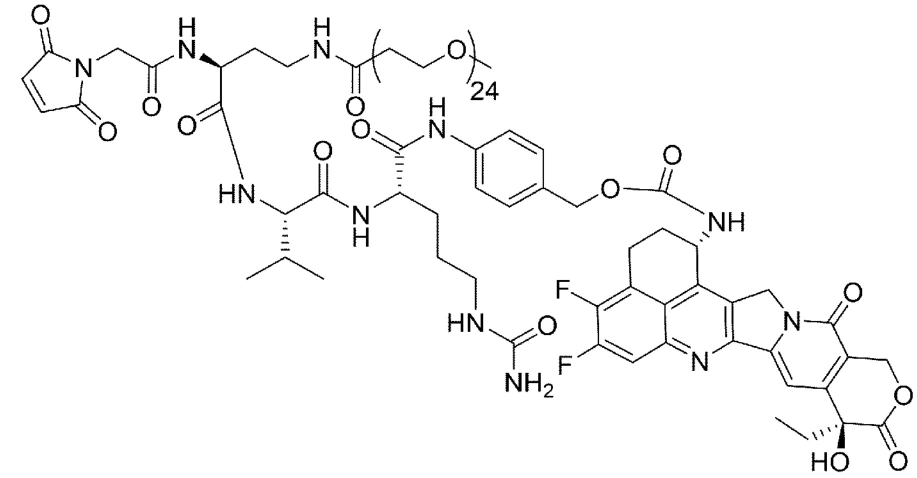

Formula III-18-1

One or more embodiments provide the compound (1*S*,9*S*)-1-amino-9-ethyl-4,5-difluoro-9-hydroxy-1,2,3,9,12,15-hexahydro-10*H*,13*H*-b enzo[*de*]pyrano[3',4':6,7]indolo[1,2-*b*]quinoline-10,13 or a pharmaceutically acceptable salt or solvate thereof.

One or more embodiments provide a pharmaceutical composition comprising the drug conjugate, such as the antibody-drug conjugate, or a pharmaceutically acceptable salt or solvate thereof, and a pharmaceutically acceptable carrier, an excipient and/or an adjuvant, and optionally other anti-cancer drugs. The pharmaceutical composition may be administered by any convenient route, e.g., by infusion or bolus injection, by absorption through epithelial or cutaneous mucosa (e.g., oral mucosa, rectal and intestinal mucosa), and may be co-administered with other biologically active agents. Accordingly, the pharmaceutical composition may be administered intravenously, subcutaneously, orally, rectally, parenterally, intracisternally, intravaginally, intraperitoneally, topically (e.g. through powder, ointment, drops or transdermal patch), buccally or by oral or nasal spray.

In some embodiments, the term "pharmaceutically acceptable carrier" refers generally to any type of non-toxic solid, semi-solid, or liquid filler, diluent, encapsulating material, formulation additive, etc.

The term "carrier" refers to a diluent, adjuvant, excipient or carrier that can be administered to a patient together with the active ingredient. Such pharmaceutical carriers can be sterile liquids, such as water and oils, including oils originated from petroleum, animal, plant or synthesis, such as peanut oil, soybean oil, mineral oil, sesame oil, and the like. When the pharmaceutical composition is administered intravenously, water is a preferred carrier. A saline aqueous solution, a glucose aqueous solution, and a glycerol solution can also be used as a liquid carrier, especially for injection. Suitable pharmaceutical excipients include starch, glucose, lactose, sucrose, gelatin, malt, rice, flour, chalk, silica gel, sodium stearate, glycerol monostearate, talc, sodium chloride, skimmed milk powder, glycerol, propylene, glycol, water, ethanol, etc. If desired, the composition may also contain a small amount of a wetting or emulsifying agent, or a pH buffering agent such as an acetate, citrate or phosphate salt. Antibacterial agents such as phenylmethanol or methylparaben, antioxidants such as ascorbic acid or sodium bisulfite, chelating agents such as ethylenediaminetetraacetic acid, and tonicity adjusting agents such as sodium chloride or dextrose are also contemplated. These composition may come in the form of solutions, suspensions, emulsions, tablets, pills, capsules, powders, sustained release formulations, etc. The composition may be formulated as a suppository with a conventional binder and carrier such as a triglyceride. Oral formulations may comprise a standard carrier such as pharmaceutical grade mannitol, lactose, starch, magnesium stearate, sodium saccharin, cellulose, and magnesium carbonate. Examples of suitable pharmaceutical carriers are described in Remington's Pharmaceutical Sciences by E. W. Martin, which is hereby incorporated herein by reference. Such compositions will contain a clinically effective dose of an antibody or antigen-binding fragment, preferably in purified form, together with an appropriate amount of a carrier, to provide a form suitable for administration to a patient. The formulation should be suitable for the administration mode. The formulation may be encapsulated in an ampoule, a disposable syringe, or a multi-dose vial made of glass or plastic.

In some embodiments, the composition is formulated into a pharmaceutical composition suitable for intravenous injection into a human body according to conventional steps. A composition for intravenous administration is usually a solution in sterile isotonic aqueous buffer. The composition may also comprise a solubilizer and a local anesthetic such as lidocaine to alleviate the pain at the injection site. In general, the active ingredients are provided in a unit dosage form individually or as a mixture, for example, the active ingredients are encapsulated in sealed containers (such as ampoule bottles or sachets) that can indicate the amount of the active agent, in the form of lyophilized powder or anhydrous concentrate. Where the composition is administered by infusion, the composition can be dispensed in infusion bottles containing sterile, pharmaceutical grade water or saline. Where the composition is administrated by injection, an ampoule bottle containing sterile water or saline for injection can be used, so that the active ingredients can be mixed before administration.

One or more embodiments provide use of the drug conjugate in preparing a medicament for treating cancer, autoimmune diseases, inflammatory diseases or infectious diseases.

In one or more embodiments, the drug conjugate is administered in combination with other anti-cancer drugs.

One or more embodiments provide use of the linker or the intermediate or the pharmaceutically acceptable salt or solvate thereof in preparing a drug conjugate, such as an antibody-drug conjugate, or a pharmaceutically acceptable salt or solvate thereof.

The pharmaceutically acceptable salts include those derived from the drug conjugates, such as the antibody-drug conjugates, with a variety of organic and inorganic counterions well known in the art, and salts as examples only include organic or inorganic salts such as sodium salt, potassium salt, calcium salt, magnesium salt, ammonium salt, isopropylamine, trimethylamine, diethylamino, triethylamine, tripropylamine, ethanolamine, 2-dimethylaminoethanol, 2-diethylaminoethanol, dicyclohexylamine, lysine, arginine, histidine, caffeine, procaine, choline, betaine, ethylenediamine, glucosamine, methylglucamine, theobromine, purine piperazine, piperidine, N-ethyl, piperidine, polyamine resin, and tetraalkylammonium salt , etc,when the molecule contains an acidic functional group; and organic or inorganic acid salts such as hydrochloride salt, hydrobromide salt, tartrate salt, mesylate salt, acetate salt, maleate salt and oxalate salt when the molecule contains a basic functional group. Other non-limiting examples of acids include sulfuric acid, nitric acid, phosphoric acid, propionic acid, glycolic acid, pyruvic acid, malonic acid, succinic acid, fumaric acid, tartaric acid,

citric acid, benzoic acid cinnamic acid, mandelic acid, methanesulfonic acid, ethanesulfonic acid, *p*-toluenesulfonic acid salicylic acid, etc. The solvate includes hydrates. The salts can usually be prepared by conventional methods by reacting, for example, appropriate acids or bases with the ADC of the present invention.

One or more embodiments provide a method for treating cancer comprising administering to a patient an effective amount of an antibody-drug conjugate. The "effective amount" refers to the amount of an active compound or medicament that results in a biological or drug response of tissues, systems, animals, individuals and humans which is being sought by researchers, vets, doctors or other clinical doctors, including the treatment of a disease.

Generally, a suitable dose may range from about 0.1 mg/kg to 100 mg/kg, and administration may be performed at a frequency of, for example, once monthly, once every two weeks, once every three weeks, twice every three weeks, three times every four weeks, once weekly, or twice weekly, etc. For example, the administration may be performed by intravenous infusion, intravenous bolus injection, subcutaneous injection, intramuscular injection, etc.

One or more embodiments provide a drug conjugate, such as an antibody-drug conjugate, for use as a medicament.

One or more embodiments provide use of a drug conjugate, such as an antibody-drug conjugate, or a pharmaceutical composition containing a drug conjugate, such as an antibody-drug conjugate, in the preparation of a medicament for treating and/or preventing diseases.

One or more embodiments provide a drug conjugate, such as an antibody-drug conjugate, for use in treating cancer, autoimmune diseases, inflammatory diseases or infectious diseases; the cancer includes triple-negative breast cancer, glioblastoma, medulloblastoma, urothelial cancer, breast cancer, head and neck cancer, renal cancer (clear cell renal cell carcinoma and papillary renal cell carcinoma), ovarian cancer (e.g., ovarian adenocarcinoma and ovarian teratocarcinoma), pancreatic cancer, gastric cancer, Kaposi's sarcoma, lung cancer (e.g., small cell lung cancer and non-small cell lung cancer), cervical cancer, colorectal cancer, esophageal cancer, oral squamous cell carcinoma, prostate cancer, thyroid cancer, bladder cancer, glioma, hepatobiliary cancer, colorectal cancer, T-cell lymphoma, uterine cancer, liver cancer, endometrial cancer, salivary gland carcinoma, esophageal cancer, melanoma, neuroblastoma, sarcoma (e.g., synovialsarcoma or carcinosarcoma), colon cancer, rectal cancer, colorectal cancer, leukemia (e.g., acute lymphocytic leukemia, acute myelocytic leukemia, acute promyelocytic leukemia, chronic myelocytic leukemia, chronic lymphocytic leukemia), bone cancer, skin cancer (e.g., basal cell carcinoma or squamous cell carcinoma), pancreatic cancer, malignant melanoma, small intestine cancer, testicular embryonal carcinoma, placental choriocarcinoma, testicular cancer, lymphoma (e.g., Hodgkin's lymphoma, non-Hodgkin's lymphoma, or recurrent anaplastic large cell lymphoma).

One or more embodiments provide a product comprising a drug conjugate or a pharmaceutical composition;

a container; and

a package insert, instruction or label indicating that the compound or composition is for treating cancer, autoimmune diseases, inflammatory diseases, or infectious diseases.

## BRIEF DESCRIPTION OF THE DRAWINGS

FIG. 1 shows the flow cytometry assay results for CHO-CLDN18.2 cells, CHO cells and CHO-CLDN18.2 cells being depicted from left to right in the figure.

FIG. 2 shows the dose curve of ADC8, ADC10, ADC11, and ADC12 on CHO-CLDN18.2 cells proliferation inhibition.

FIG. 3 shows the bystander effect of ADC 1.

FIG. 4 shows the bystander effect of ADC4.

FIG. 5 shows the bystander effect of ADC9, ADC 11 and ADC 12.

FIG. 6 shows the bystander effect of ADC 14.

FIG. 7 shows the bystander effect of ADC 16.

FIG. 8 shows changes in the plasma concentration of ADC4 in rats.

FIG. 9 shows the effect of ADC4 in inhibiting tumor growth.

FIG. 10 shows the effect of ADC1 and ADC2 in inhibiting tumor growth.

FIG. 11 shows the effect of ADC4 and ADC6 in inhibiting tumor growth.

FIG. 12 shows the effect of ADC9, ADC11 and ADC12 in inhibiting tumor growth.

FIG. 13 shows the tumor weights (mean ± standard error) of mice in each group of ADC9, ADC11, ADC12, and ADC13 in the GA0006 xenograft model.

FIG. 14 shows the in vivo tumor inhibitory activity of ADC14.

FIG. 15 shows the in vivo tumor inhibitory effect of ADC16.

FIG. 16 shows the in vivo tumor inhibitory effect of ADC16.

## DETAILED DESCRIPTION

The "alkyl" refers to a saturated aliphatic hydrocarbyl group, and this term includes linear and branched hydrocarbyl groups. For example, C1-C20 alkyl, such as C1-C6 alkyl. C1-C20 alkyl refers to an alkyl group containing 1 to 20 carbon atoms, e.g., an alkyl group containing 1 carbon atom, 2 carbon atoms, 3 carbon atoms, 4 carbon atoms, 5 carbon atoms, 6 carbon atoms, 7 carbon atoms, 8 carbon atoms, 9 carbon atoms, 10 carbon atoms, 11 carbon atoms, 12 carbon atoms, 13 carbon atoms, 14 carbon atoms, 15 carbon atoms, 16 carbon atoms, 17 carbon atoms, 18 carbon atoms, 19 carbon atoms, or 20 carbon atoms. Non-limiting examples of alkyl include methyl, ethyl, *n*-propyl, isopropyl, *n*-butyl, isobutyl, *sec*-butyl, *tert*-butyl, *n*-pentyl, neopentyl, *n*-hexyl, etc. The alkyl may be unsubstituted or substituted with one or more substituents including, but not limited to, alkyl, alkoxy, cyano, hydroxy, carbonyl, carboxy, aryl, heteroaryl, amino, halogen, sulfonyl, sulfinyl, phosphono, etc.

The "carbocyclyl" refers to a stable non-aromatic monocyclic or polycyclic hydrocarbyl radical consisting of carbon and hydrogen atoms only, and may comprise a fused or bridged ring system, contains 3 to 15 carbon atoms, for example, 3 to 10 (e.g., 3, 4, 5, 6, 7, 8, 9 or 10) carbon atoms. It is saturated or unsaturated, and links to the remainder of the molecule through a single bond. Monocyclic radicals include, for example, cyclopropyl, cyclobutyl, cyclopentyl, cyclohexyl, cycloheptyl and cyclooctyl. Polycyclic radicals include, for example, adamantyl, norbornyl, and decahydronaphthyl. When specifically indicated in the specification, the carbocyclyl may be optionally substituted with one or more substituents independently selected from: alkyl, halogen, haloalkyl, cyano, nitro, oxo, aryl, aralkyl, carbocyclyl, carbocyclylalkyl, heterocyclyl, heterocyclylalkyl, heteroaryl and heteroarylalkyl.

The "aryl" refers to an all-carbon monocyclic or all-carbon fused ring having a completely conjugated $\pi$-electron system, and typically has 5-14 carbon atoms, e.g., 6, 10, 12, or 14 carbon atoms. Aryl may be unsubstituted or substituted with one or more substituents including, but not limited to, alkyl, alkoxy, cyano, hydroxy, carboxy, aryl, aralkyl, amino, halogen, sulfonyl, sulfinyl, phosphono, etc. Examples of unsubstituted aryl include, but are not limited to, phenyl, naphthyl, and anthracenyl.

The "heterocyclyl" refers to a stable 3 to 18 membered aromatic or nonaromatic ring group consisting of 2 to 8 (e.g., 2, 3, 4, 5, 6, 7 or 8) carbon atoms and 1 to 6 (1, 2, 3, 4, 5 or 6) heteroatoms selected from nitrogen, oxygen and sulfur. Unless otherwise specifically stated in the specification, heterocyclyl may be a monocyclic, bicyclic, tricyclic or tetracyclic ring system, and may include fused or bridged ring systems; and the nitrogen, carbon or sulfur atoms in heterocyclyl may be optionally oxidized; the nitrogen atoms are optionally quaternized; and heterocyclyl may be partially or fully saturated. Examples of such heterocyclyl include, but are not limited to, dioxolanyl, dioxinyl, thienyl[1,3]dithianyl, decahydroisoquinolinyl, imidazolinyl, imidazolidinyl, isothiazolidinyl, isoxazolidinyl, morpholinyl, octahydroindolyl, octahydroisoindolyl, 2-oxopiperazinyl, 2-oxopiperidinyl, 2-oxopyrrolidinyl, oxazolidinyl, piperidinyl, piperazinyl, 4-piperidinonyl, pyrrolidinyl, pyrazolidinyl, quinuclidinyl, thiazolidinyl, 1,2,4-thiadiazol-5(4*H*)-ylidene, tetrahydrofuranyl, trioxacyclohexyl, trithianyl, triazinanyl, tetrahydropyranyl, thiomorpholinyl (thiomorpholinyl), 1-oxo-thiomorpholinyl, and 1,1-dioxo-thiomorpholinyl. When specifically stated in the specification, heterocyclyl may be optionally substituted with one or more substituents selected from: alkyl, alkenyl, halogen, haloalkyl, cyano, oxo, thioxo, nitro, aryl, aralkyl, cycloalkyl, cycloalkylalkyl, optionally substituted heterocyclyl, optionally substituted heterocyclylalkyl, optionally substituted heteroaryl, and optionally substituted heteroarylalkyl.

The "alkoxy" refers to formula -O-(alkyl), wherein alkyl is the alkyl defined herein. Non-limiting examples of alkoxy are methoxy, ethoxy, *n*-propoxy, 1-methylethoxy (isopropoxy), *n*-butoxy, isobutoxy, *sec*-butoxy, and *tert*-butoxy. Alkoxy may be substituted or unsubstituted.

The "halogen" refers to fluoro (F), chloro (Cl), bromo (Br), or iodo (I).

The "amino" refers to $-NH_2$.

The "cyano" refers to -CN.

The "nitro" refers to $-NO_2$.

The "hydroxy" refers to -OH.

The "carboxyl" refers to -COOH.

The "thiol" refers to -SH.

The "carbonyl" refers to C=O.

The "polypeptide" refers to a molecule consisting of two or more amino acid monomers linearly linked by amide bonds (also referred to as peptide bonds), and may include dipeptides, tripeptides, tetrapeptides, oligopeptides, etc.

The "amino acid" refers to an organic compound containing both amino and carboxyl, such as α-amino acids and β-amino acids. Amino acids include alanine (three-letter code: Ala, one-letter code: A), arginine (Arg, R), asparagine (Asn, N), aspartic acid (Asp, D), cysteine (Cys, C), glutamine (Gln, Q), glutamic acid (Glu, E), glycine (Gly, G), histidine (His, H), isoleucine (Ile, I), leucine (Leu, L), lysine (Lys, K), methionine (Met, M), phenylalanine (Phe, F), proline (Pro, P), serine (Ser, S), threonine (Thr, T), tryptophan (Trp, W), tyrosine (Tyr, Y), valine (Val, V), sarcosine (Sar), theanine (Thea), hydroxyproline (Hypro), hydroxylysine (Hylys), β-aminoisobutyric acid (β-AiBA), citrulline (Cit), β-alanine (β-Ala), etc.

Those skilled in the art will appreciate that when an amino acid or polypeptide is a constituent part of a molecule (such as an antibody or ADC), the amino acid or polypeptide refers to the amino acid residue or polypeptide residue (whether written or not), that is, when it's connected with other parts of the molecule, some of its groups (such as a hydrogen atom of its amino group and/or a hydroxyl group of a carboxyl group) are lost due to the formation of covalent bonds (such as amide bonds) with other parts of the molecule.

Minor variations in the amino acid sequences of antibodies or immunoglobulin molecules are contemplated by the present disclosure, provided that the identity of the amino acid sequences is maintained to be at least 75%, such as at least 80%, 90%, 95%, and as another example 99%. In one or more embodiments, the variations are conservative amino acid substitutions. Conservative amino acid substitutions are ones that occur within a family of amino acids that are related in their side chains. Genetically encoded amino acids are generally classified into the following categories: (1) acidic amino acids are aspartate and glutamate; (2) basic amino acids are lysine, arginine and histidine; (3) non-polar amino acids are alanine, valine, leucine, isoleucine, proline, phenylalanine, methionine, tryptophan; and (4) uncharged polar amino acids are glycine, asparagine, glutamine, cysteine, serine, threonine and tyrosine. Amino acids of the other families include (i) serine and threonine of the aliphatic-hydroxy family; (ii) asparagine and glutamine of the amide-containing family; (iii) alanine, valine, leucine and isoleucine of the aliphatic family; and (iv) phenylalanine, tryptophan and tyrosine of the aromatic family. In one or more embodiments, conservative amino acid substitution groups are valine-leucine-isoleucine, phenylalanine-tyrosine, lysine-arginine, alanine-valine, glutamic acid-aspartic acid, and asparagine-glutamine. For example, it is reasonable to expect that the single replacement of leucine with isoleucine or valine, aspartate with glutamate, threonine with serine, or the similar replacement of an amino acid with a structurally related amino acid, will not have a major effect on the binding or properties of the resulting molecule, particularly when the replacement does not involve an amino acid within a binding site. Whether an amino acid change results in a functional peptide can be readily determined by determining the specific activity of the polypeptide derivative. Fragments or analogs of antibodies or immunoglobulin molecules can be readily prepared by those of ordinary skill in the art.

In one or more embodiments, the amino acid substitutions have the following effects: (1) reducing susceptibility to proteolysis, (2) reducing susceptibility to oxidation, (3) altering the binding affinity for formation of protein complexes, (4) altering binding affinity, and (5) conferring or improving other physicochemical or functional properties of such analogs. Analogs can include various muteins whose sequences differ from the naturally occurring peptide sequences. For example, single or multiple amino acid substitutions (preferably conservative amino acid substitutions) may be made in the naturally occurring sequence (preferably in a portion of the polypeptide outside the domains that form intermolecular contacts). A conservative amino acid substitution should not significantly alter the structural characteristics of the parent sequence (e.g., a replacement amino acid should not tend to disrupt a helix that occurs in the parent sequence, or disrupt other types of secondary structures that characterize the parent sequence). Examples of secondary and tertiary structures of artificially recognized polypeptides are described in Proteins: Structures and Molecular Principles (Ed. Creighton, W. H. Freeman and Company, New York (1984)); Introduction to Protein Structure (Eds. C. Branden and J. Tooze, Garland Publishing, New York, N.Y.(1991)); and Thornton et al., Nature 354:105(1991).

The number of amino acids of conservative amino acid substitutions of VL or VH is about 1, about 2, about 3, about 4, about 5, about 6, about 8, about 9, about 10, about 11, about 13, about 14, about 15 conservative amino acid substitutions, or a range between any two of these values (inclusive) or any value therein. The number of amino acids of conservative amino acid substitutions of a heavy chain constant region, a light chain constant region, a heavy chain, or a light chain is about 1, about 2, about 3, about 4, about 5, about 6, about 8, about 9, about 10, about 11, about 13, about 14, about 15, about 18, about 19, about 22, about 24, about 25, about 29, about 31, about 35, about 38, about

41, about 45 conservative amino acid substitutions, or a range between any two of these values (inclusive) or any value therein.

The term "recombinant" , with regard to a polypeptide or polynucleotide, is intended to refer to a polypeptide or polynucleotide that does not occur in nature, and non-limiting examples can be combined to produce a polynucleotide or polypeptide that does not normally occur.

"Homology" , "identity" or "similarity" refers to sequence similarity between two peptides or between two nucleic acid molecules. Homology or identity can be determined by comparing the positions that can be aligned in the sequences. When a position of the compared sequences is occupied by the same base or amino acid, the molecules are homologous or identical at that position. The degree of homology between the sequences is a function of the number of matching or homologous positions shared by the sequences. " At least 80% identity " refers to about 80% identity, about 81% identity, about 82% identity, about 83% identity, about 85% identity, about 86% identity, about 87% identity, about 88% identity, about 90% identity, about 91% identity, about 92% identity, about 94% identity, about 95% identity, about 98% identity, about 99% identity, or a range between any two of these values (inclusive) or any value therein. "At least 90% identity" refers to about 90% identity, about 91% identity, about 92% identity, about 93% identity, about 95% identity, about 96% identity, about 97% identity, about 98% identity, about 99% identity, or a range between any two of these values (inclusive) or any value therein.

"Antibody" or "antigen-binding fragment" refers to a polypeptide or polypeptide complex that specifically recognizes and binds to an antigen. The antibody may be an intact antibody and any antigen-binding fragment or single chain thereof. The term "antibody" thus includes any protein or peptide comprising, in its molecule, at least a portion of an immunoglobulin molecule that has biological activity for binding to an antigen. The antibody and the antigen-binding antigen-binding fragment include, but are not limited to, complementarity determining regions (CDRs) of a heavy or light chain or a ligand binding portion thereof, heavy chain variable regions (VHs), light chain variable regions (VLs), heavy chain constant regions (CHs), light chain constant regions (CLs), framework regions (FRs) or any portion thereof, or at least a portion of a binding protein. The CDRs include CDRs of light chain variable region (VL CDR1-3) and CDRs of heavy chain variable region (VH CDR1-3). An antibody or an antigen-binding unit thereof can specifically recognize and bind to polypeptides or polypeptide complexes of one or more (e.g., two) antigens. The antibody or the antigen-binding unit thereof that specifically recognizes and binds to multiple (e.g., two) antigens can be referred to as a multispecific (e.g., bispecific) antibody or antigen-binding unit thereof.

The term "antibody fragment" or "antigen-binding fragment" refers to a part of an antibody, and the composition of the antibody fragment of the present invention may be similar to F(ab')2, F(ab)2, Fab', Fab, Fv, scFv and the like in monospecific antibody fragments. Regardless of its structure, the antibody fragment binds to the same antigen recognized by an intact antibody. The term " antibody fragment " includes aptamers, mirror image isomers, and bivalent diabodies. The term " antigen-binding fragment" also includes any synthetic or genetically engineered protein that functions as an antibody by binding to a specific antigen to form a complex.

The term " antibody " includes a wide variety of polypeptides that can be biochemically distinguished. Those skilled in the art will appreciate that the classes of heavy chains include gamma, mu, alpha, delta, or epsilon ($\gamma$, $\mu$, $\alpha$, $\delta$, or $\varepsilon$), and some subclasses (e.g., $\gamma$ 1- $\gamma$ 4). The nature of this chain determines the "type" of the antibody as IgG, IgM, IgA, IgG or IgE. Immunoglobulin subclasses (isotypes), such as IgG1, IgG2, IgG3, IgG4, IgG5, etc., have been well characterized and the functional specificity imparted is also known. All types of immunoglobulins are within the scope of the present invention. In one or more embodiments, the immunoglobulin molecule is of the IgG class. Two heavy chains and two light chains are connected in a " Y " configuration through disulfide bonds, wherein the light chain starts at the opening of "Y" configuration and extends through the variable region to surround the heavy chain.

The antibodies, antigen-binding fragments or derivatives disclosed herein include, but are not limited to, polyclonal antibodies, monoclonal antibodies, multispecific antibodies, fully human antibodies, humanized antibodies, primatized antibodies, chimeric antibodies, single-chain antibodies, epitope-binding fragments (e.g., Fab analogs, Fab' analogs and F(ab')2 analogs), and single chain Fv (scFv) analogs.

Light chains can be classified into kappa ( $\kappa$ ) or lambda ( $\lambda$ ). Each heavy chain may connect with a $\kappa$ or $\lambda$ light chain. In general, when an immunoglobulin is produced by a hybridoma, a B cell or a genetically engineered host cell, the light and heavy chains are connected by covalent bonds, and the "tail" portions of the two heavy chains are connected by covalent disulfide bonds or non-covalent bonds. In the heavy chains, the amino acid sequence extends from the N terminus at the forked end of the Y configuration to the C terminus at the bottom of each chain. The immunoglobulin $\kappa$ light chain variable region is V $\kappa$ ; and the immunoglobulin $\lambda$ light chain variable region is V $\lambda$.

Both the light and heavy chains are divided into regions of structural and functional homology. The terms " constant" and "variable" are used in accordance with function. The light chain variable region (VL) and the heavy chain variable region (VH) determine the antigen recognition and specificity. The light chain constant region (CL) and the heavy chain constant region (CH) impart important biological properties such as secretion, transplacental movement, Fc receptor binding, complement fixation, etc. By convention, the numbering of amino acids in the constant regions increases as they become further away from the antigen-binding site of the antibody or amino terminus. The N-terminal

portion is the variable region, and the C-terminal portion is the constant region; the CH3 and CL domains actually comprise the carboxyl termini of the heavy chain and light chain, respectively.

The antibodies disclosed herein may be derived from any animal, including fish, birds and mammals. Preferably, the antibody is derived from a human being, a mouse, a donkey, a rabbit, a goat, a camel, a llama, a horse, or a chicken source. In another embodiment, the variable region may be derived from a condricthoid source (e.g., from a shark).

The "heavy chain constant region" comprises at least one of a CH1 domain, a hinge (e.g., upper, middle, and/or lower hinge region) domain, a CH2 domain, a CH3 domain, or a variant or a fragment. The heavy chain constant regions of the antibody may be derived from different immunoglobulin molecules. For example, the heavy chain constant regions of the polypeptide may comprise a CH1 domain derived from an IgG1 molecule and a hinge region derived from an IgG3 molecule. In another embodiment, the heavy chain constant region may comprise a hinge region derived partially from an IgG1 molecule and partially from an IgG3 molecule. In another embodiment, a portion of the heavy chain may comprise a chimeric hinge region derived partially from an IgG1 molecule and partially from an IgG4 molecule.

"Light chain constant region" includes a part of amino acid sequence from the light chain of an antibody. Preferably, the light chain constant region comprises at least one of a constant $\kappa$ domain or a constant $\lambda$ domain. "Light chain-heavy chain pair" refers to a collection of light and heavy chains that can form dimers through disulfide bonds between the CL domain of the light chain and the CH1 domain of the heavy chain.

The term "antibody-drug conjugate" or "ADC" refers to a binding polypeptide (e.g., an antibody or antigen-binding unit thereof) that is linked to one or more chemical drugs, which may optionally be a therapeutic agent or a cytotoxic agent. In a preferred embodiment, the ADC comprises an antibody, a drug (e.g. a cytotoxic drug), and a linker capable of attaching or coupling the drug to the antibody. Non-limiting examples of drugs that can be included in the ADC are mitotic inhibitors, anti-tumor antibiotics, immunomodulators, vectors for gene therapy, alkylating agents, anti-angiogenic agents, anti-metabolites, boron-containing agents, chemoprotectants, hormones, anti-hormonal agents, corticosteroids, photoactive therapeutic agents, oligonucleotides, radionuclide agents, topoisomerase inhibitors, kinase inhibitors (e.g., TEC-family kinase inhibitors and serine/threonine kinase inhibitors), and radiosensitizers.

The term "drug to antibody ratio" or "DAR" refers to the number of drugs (e.g., exatecan) that are attached to the antibody in the ADC. The DAR of an ADC may be in the range of 1 to 10, but a higher load (e.g., 20) is also possible depending on the number of connection sites on the antibody. The term DAR may be used when referring to the number of drugs loaded onto a single antibody, or alternatively, when referring to an average or mean DAR for a set of ADCs. In some embodiments, the value is selected from 1, 2, 3, 4, 5, 6, 7, 8, 9, or 10. In considering the mean binding number of small molecule drugs, i.e., the mean drug binding number of an antibody, or the mean drug to antibody ratio, the value is selected from about 0 to about 10, or about 2 to about 8. In some embodiments, the drug to antibody ratio is about 3 to about 6. In other embodiments, the drug to antibody ratio is about 6 to about 8, or about 7 to about 8. DAR values may be denoted herein by p.

The DAR value of ADC can be measured by ultraviolet-visible absorption spectroscopy (UV-Vis), high performance liquid chromatography-hydrophobic interaction chromatography (HPLC-HIC), reversed-phase high performance liquid chromatography (RP-HPLC), liquid chromatography-mass spectrometry (LC-MS), etc. These techniques are described in Ouyang, J. Methods Mol Biol, 2013, 1045: p. 275-83.

Other chemical terms herein are used according to conventional usage in the art, such as the McGraw-Hill Dictionary of Chemical Terms (Ed. Parker, S., McGraw-Hill, San Francisco (1985)).

All the publications, patents, and patent applications cited herein are incorporated by reference in their entirety for all purposes.

**Antibody Moiety**

The antibody of the present invention may be any antibody suitable for preparing an antibody-drug conjugate. It may have a complete antibody structure, or may also include antibody fragments (polyclonal and monoclonal antibodies), such as Fab, Fab', F(ab)'2, and Fv.

The antibody is capable of specifically binding to antigens, such as tumor-specific antigens. Tumor antigens can be used for identifying tumor cells, or can also be potential indicators for tumor treatment, or targets for tumor treatment. The choice of specific antibody is therefore made primarily based on the type of disease, and the cells and tissues that are targeted.

Examples of tumor antigens well known in the art include, but are not limited to, EGFR, HER2, CD20, CD30, CD33, CD47, CD52, CD133, CEA, VEGF, TROP2, B7H3, FRalpha (FR$\alpha$) , Nectin-4, B7H4, CLDN18, BMPR1B, E16, STEAP1, 0772P, MPF, Napi3b, Sema5b, PSCAhlg, ETBR, MSG783, STEAP2, TrpM4, CRIPTO, CD21, CD22, FcRH2, NCA, MDP, IL20R$\alpha$, Brevican, EphB2R, ASLG659, PSCA, GEDA, BAFF-R, CD79a, CD79b, CXCR5, HLA-DOB, P2X5, CD72, LY64, FcRH1, IRTA2, TENB2, PMEL17, TMEFF1, GDNF-Ra1, Ly6E, TMEM46, Ly6G6D, LGR5, RET, LY6K, GPR19, GPR54, ASPHD1, tyrosinase, TMEM118, EpCAM, ROR1, GPR172A, etc.

In one or more embodiments, the antibody may be a humanized monoclonal antibody.

In some embodiments, the antibody is an anti-EGFR antibody, including but not limited to: cetuximab, a human murine chimeric monoclonal antibody against EGFR; panitumumab, a fully humanized monoclonal antibody; and nimotuzumab, a humanized monoclonal antibody against EGFR. EGFR is overexpressed in many tumor tissues, such as tissues of metastatic colorectal cancer and head and neck cancer.

In one or more embodiments, the antibody is an anti-EGFR antibody, and the heavy and light chain sequences are SEQ ID NO: 1 and SEQ ID NO: 2, respectively.

The amino acid sequences corresponding to the respective sequence numbers are shown in Table 1.

Table 1

| | |
|---|---|
| SEQ ID NO:1 | QVQESGPGLVKPSETLSLTCTVSGGSVSSGDYYWTWIRQSPGKGLEWIGHIYYS GNTNYNPSLKSRLTISIDTSKTQFSLKLSSVTAADTAIYYCVRDRVTGAFDIWGQ GTLVTVSSASTKGPSVFPLAPSSKSTSGGTAALGCLVKDYFPEPVTVSWNSGAL TSGVHTFPAVLQSSGLYSLSSVVTVPSSSLGTQTYICNVNHKPSNTKVDKKVEP KSCDKTHTCPPCPAPELLGGPSVFLFPPKPKDTLMISRTPEVTCVVVDVSHEDPE VKFNWYVDGVEVHNAKTKPREEQYNSTYRVVSVLTVLHQDWLNGKEYKCK VSNKALPAPIEKTISKAKGQPREPQVYTLPPSRDELTKNQVSLTCLVKGFYPSDI AVEWESNGQPENNYKTTPPVLDSDGSFFLYSKLTVDKSRWQQGNVFSCSVMH EALHNHYTQKSLSLSPGK |
| SEQ ID NO:2 | DIQMTQSPSSLSASVGDRVTITCQASQDISNYLNWYQQKPGKAPKLLIYDASNL ETGVPSRFSGSGSGTDFTFTISSLQPEDIATYFCQHFDHLPLAFGGGTKVEIKRTV AAPSVFIFPPSDEQLKSGTASVVCLLNNFYPREAKVQWKVDNALQSGNSQESV TEQDSKDSTYSLSSTLTLSKADYEKHKVYACEVTHQGLSSPVTKSFNRGEC |

In one or more embodiments, the antibody is an anti-HER2 antibody capable of specifically acting on human epidermal growth factor receptor 2, such as trastuzumab, pertuzumab, margetuximab, and ZW25. The anti-HER2 antibody may be modified. For example, one or more amino acid sequences are changed, expanded or reduced to achieve a corresponding purpose, e.g., to enhance antibody-dependent cell-mediated cytotoxicity.

In one or more embodiments, the anti-HER2 antibody is trastuzumab, and the heavy and light chain sequences are SEQ ID NO: 3 and SEQ ID NO: 4, respectively.

The amino acid sequences corresponding to the respective sequence numbers are shown in Table 2.

Table 2

| | |
|---|---|
| SEQ ID NO:3 | EVQLVESGGGLVQPGGSLRLSCAASGFNIKDTYIHWVRQAPGKGLEWVARIYPT NGYTRYADSVKGRFTISADTSKNTAYLQMNSLRAEDTAVYYCSRWGGDGFYAM DYWGQGTLVTVSSASTKGPSVFPLAPSSKSTSGGTAALGCLVKDYFPEPVTVSW NSGALTSGVHTFPAVLQSSGLYSLSSVVTVPSSSLGTQTYICNVNHKPSNTKVDK KVEPKSCDKTHTCPPCPAPELLGGPSVFLFPPKPKDTLMISRTPEVTCVVVDVSH EDPEVKFNWYVDGVEVHNAKTKPREEQYNSTYRVVSVLTVLHQDWLNGKEY KCKVSNKALPAPIEKTISKAKGQPREPQVYTLPPSREEMTKNQVSLTCLVKGFYP SDIAVEWESNGQPENNYKTTPPVLDSDGSFFLYSKLTVDKSRWQQGNVFSCSVM HEALHNHYTQKSLSLSPGK |
| SEQ ID NO:4 | DIQMTQSPSSLSASVGDRVTITCRASQDVNTAVAWYQQKPGKAPKLLIYSASFLY SGVPSRFSGSRSGTDFTLTISSLQPEDFATYYCQQHYTTPPTFGQGTKVEIKRTVA APSVFIFPPSDEQLKSGTASVVCLLNNFYPREAKVQWKVDNALQSGNSQESVTE QDSKDSTYSLSSTLTLSKADYEKHKVYACEVTHQGLSSPVTKSFNRGEC |

In one or more embodiments, the antibody is an anti-Trop2 antibody capable of specifically acting on Trop2 protein, wherein the Trop2 protein refers to trophoblast cell surface glycoprotein antigen 2. In one or more embodiments, the anti-Trop2 antibody is a fully human monoclonal antibody; in another embodiment, the anti-Trop2 antibody is a humanized monoclonal antibody.

In one or more embodiments, the anti-Trop2 antibody is an antibody published in the following patent documents: WO2021147993A1 (e.g., PD3), CN101264325B (e.g., RS7, hRS7), CN105849126B (e.g., hTINA1 H1L1, hTINA1 H2L1, hTINA1 H2L2, hTINA1 H3L3), CN110903395A (e.g., M1, M2, M3), CN113896796A (e.g., 4D3, 7F11), US20130089872A

(e.g., K5-70, K5-107), K5-116-2-1, T6-16, T5-86) US5840854A (e.g., BR110), US20130122020A (e.g., 3E9, 6G11, 7E6, 15E2, 18B1), US20120237518A (e.g., 77220, KM4097, KM4590).

In one or more embodiments, the anti-Trop2 antibody is purchasable, including LS-C126418, LS-C178765, LS-C126416, LS-C126417 (LifeSpan BioSciences, Inc., Seattle, WA); 10428-MM01, 10428-MM02, 10428-R001, 10428-R030 (Sino Biological Inc., Beijing, China); MR54 (eBioscience, San Diego, CA); Sc-376181, sc-376746, Santa Cruz Biotechnology (Santa Cruz, CA); MM0588-49D6 (Novus Biologicals, Littleton, CO); ab79976 and ab89928 (Cambridge, MA).

In one or more embodiments, the anti-Trop2 antibody is the anti-Trop2 antibodies 162-25.3 and 162-46.2 published by Lipinski et al. (1981, Proc Natl. Acad Sci USA, 78:5147-50) or the Pr1E11 anti-Trop2 antibody published by Ikeda et al. (2015, Biochem Biophys Res Comm 458:877-82), which recognizes a unique epitope on Trop2.

In one or more embodiments, the antibody is an hRS9 antibody, the heavy and light chain sequences of which are SEQ ID NO: 5 and SEQ ID NO: 6, respectively.

The amino acid sequences corresponding to the respective sequence numbers are shown in Table 3.

Table 3

| | |
|---|---|
| SEQ ID NO: 5 | QVQLQQSGSELKKPGASVKVSCKASGYTFTNYGMNWVKQAPGQGLKWMGWINTYTGEPTYTDDFKGRFAFSLDTSVSTAYLQISSLKADDTAVYFCARGGFGSSYWYFDVWGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK |
| SEQ ID NO:6 | DIQLTQSPSSLSASVGDRVSITCKASQDVSIAVAWYQQKPGKAPKLLIYSASYRYTGVPDRFSGSGSGTDFTLTISSLQPEDFAVYYCQQHYITPLTFGAGTKVEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC |

In one or more embodiments, the antibody is an anti-CLDN18.2 antibody or an antigen-binding unit thereof, wherein the anti-CLDN18.2 antibody or the antigen-binding unit thereof has one or more of the following properties: a) specifically binding to CLDN18.2; b) high affinity; c) high ADCC activity; and d) high CDC activity.

In one or more embodiments, the anti-CLDN18.2 antibody or the antigen-binding unit thereof is a murine antibody, a chimeric antibody, or a humanized antibody.

In one or more embodiments, the anti-CLDN18.2 antibody or the antigen-binding unit thereof is Fab, Fab', F(ab')2, Fv, disulfide-linked Fv, scFv, single domain antibody or bispecific antibody. In one or more embodiments, the anti-CLDN18.2 antibody is a multispecific antibody (eg, a bispecific antibody).

In one or more embodiments, the anti-CLDN18.2 antibody can be a monoclonal antibody.

In one or more embodiments, the antibody comprises a heavy chain constant region, e.g., IgG1, IgG2, IgG3, IgG4, IgA, IgE, IgM, or IgD constant region. In one or more embodiments, the anti-CLDN18.2 antibody or the antigen-binding unit thereof comprises an immunoglobulin heavy chain constant domain selected from a human IgG constant domain, a human IgA constant domain, a human IgE constant domain, a human IgM constant domain, and a human IgD constant domain. In one or more embodiments, the anti-CLDN18.2 antibody or the antigen-binding unit thereof comprises an IgG1 heavy chain constant region, an IgG2 heavy chain constant region, an IgG3 heavy chain constant region, or an IgG4 heavy chain constant region. In one or more embodiments, the heavy chain constant region is an IgG1 heavy chain constant region or an IgG4 heavy chain constant region. In one or more embodiments, the antibody or the antigen-binding unit thereof comprises a light chain constant region, such as a κ light chain constant region or a λ light chain constant region. In one or more embodiments, the antibody or its antigen-binding unit comprises a κ light chain constant region.

The anti-CLDN18.2 antibody of the present invention comprises a humanized antibody or an antigen-binding unit thereof. The antibody or the antigen-binding unit thereof is suitable for administration to a human without causing a deleterious immune response in the human to the administered immunoglobulin. In one or more embodiments, the anti-CLDN18.2 antibody or the antigen-binding unit thereof is selected from the anti-CLDN18.2 antibody or the antigen-binding unit thereof described in WO2020043044, US2021403552, WO2021238831, WO2021160154, and WO2021111003. In one or more embodiments, the anti-CLDN18.2 antibody or the antigen-binding unit thereof is selected from zolbetuximab, claudiximab, TST001, AB011, M108, or NBL-015 or the antigen-binding unit thereof.

In one or more embodiments, the anti-CLDN18.2 antibody or the antigen-binding unit thereof comprises one or more of (a)-(f), wherein:

(a) VH CDR1, which comprises or consists of an amino acid sequence set forth in SEQ ID NO: 7;

(b) VH CDR2, which comprises or consists of an amino acid sequence set forth in SEQ ID NO: 8;

(c) VH CDR3, which comprises or consists of an amino acid sequence set forth in SEQ ID NO: 9;

(d) VL CDR1, which comprises or consists of an amino acid sequence set forth in SEQ ID NO: 10;

(e) VL CDR2, which comprises or consists of an amino acid sequence set forth in SEQ ID NO: 11;

(f) VL CDR3, which comprises or consists of an amino acid sequence set forth in SEQ ID NO: 12.

In one or more embodiments, the anti-CLDN18.2 antibody or the antigen-binding unit thereof comprises or consists of the following CDRs:

(a) VH CDR1, which comprises or consists of an amino acid sequence set forth in SEQ ID NO: 7;

(b) VH CDR2, which comprises or consists of an amino acid sequence set forth in SEQ ID NO: 8;

(c) VH CDR3, which comprises or consists of an amino acid sequence set forth in SEQ ID NO: 9;

(d) VL CDR1, which comprises or consists of an amino acid sequence set forth in SEQ ID NO: 10;

(e) VL CDR2, which comprises or consists of an amino acid sequence set forth in SEQ ID NO: 11;

(f) VL CDR3, which comprises or consists of an amino acid sequence set forth in SEQ ID NO: 12.

In one or more embodiments, the anti-CLDN18.2 antibody or the antigen-binding unit thereof comprises or consists of a VH CDR1 set forth in SEQ ID NO: 7, a VH CDR2 set forth in SEQ ID NO: 8, a VH CDR3 set forth in SEQ ID NO: 9, a VL CDR1 set forth in SEQ ID NO: 10, a VL CDR2 set forth in SEQ ID NO: 11, and a VL CDR3 set forth in SEQ ID NO: 12.

In one or more embodiments, the anti-CLDN18.2 antibody or the antigen-binding unit thereof is a humanized antibody or an antigen-binding unit thereof. In one or more embodiments, the heavy chain variable region of the anti-CLDN18.2 antibody or the antigen-binding unit thereof comprises a sequence set forth in SEQ ID NO: 13, or a sequence having at least 90% identity to the sequence set forth in SEQ ID NO: 13, or an amino acid sequence having one or more conservative amino acid substitutions as compared to the sequence set forth in SEQ ID NO: 13; and/or

the light chain variable region of the anti-CLDN18.2 antibody or the antigen-binding unit thereof comprises a sequence set forth in SEQ ID NO: 14, or a sequence having at least 90% identity to the sequence set forth in SEQ ID NO: 14, or an amino acid sequence having one or more conservative amino acid substitutions as compared to the sequence set forth in SEQ ID NO: 14.

In one or more embodiments, the anti-CLDN18.2 antibody or the antigen-binding unit thereof comprises a heavy chain constant region, wherein the heavy chain constant region comprises an amino acid sequence set forth in SEQ ID NO: 15 or 16.

In one or more embodiments, the anti-CLDN18.2 antibody or the antigen-binding unit thereof comprises a heavy chain constant region and a light chain constant region, wherein the heavy chain constant region comprises an amino acid sequence set forth in SEQ ID NO: 15; wherein the light chain constant region comprises an amino acid sequence set forth in SEQ ID NO: 17.

In one or more embodiments, the anti-CLDN18.2 antibody or the antigen-binding unit thereof comprises a heavy chain constant region and a light chain constant region, wherein the heavy chain constant region comprises an amino acid sequence set forth in SEQ ID NO: 16; wherein the light chain constant region comprises an amino acid sequence set forth in SEQ ID NO: 17.

In one or more embodiments, the humanized anti-CLDN18.2 antibody is H239H-2b-K-6a-1 antibody, comprising

a heavy chain variable region having an amino acid sequence set forth in SEQ ID NO: 13, a heavy chain constant region having an amino acid sequence set forth in SEQ ID NO: 15, a light chain variable region having an amino acid sequence set forth in SEQ ID NO: 14, and a light chain constant region having an amino acid sequence set forth in SEQ ID NO: 17.

In one or more embodiments, the humanized anti-CLDN18.2 antibody is H239H-2b-K-6a-2 antibody, comprising a heavy chain variable region having an amino acid sequence set forth in SEQ ID NO: 13, a heavy chain constant region having an amino acid sequence set forth in SEQ ID NO: 16, a light chain variable region having an amino acid sequence set forth in SEQ ID NO: 14, and a light chain constant region having an amino acid sequence set forth in SEQ ID NO: 17.

In one or more embodiments, the antibody comprises a sequence having at least 80% identity to any of the antibodies described above, or an amino acid sequence having one or more conservative amino acid substitutions as compared to any of the antibodies described above.

In one or more embodiments, the H239H-2b-K-6a-1 antibody comprising a light chain having an amino acid sequence set forth in SEQ ID NO: 20 and a heavy chain having an amino acid sequence set forth in SEQ ID NO: 18.

In one or more embodiments, the H239H-2b-K-6a-2 antibody comprising a light chain having an amino acid sequence set forth in SEQ ID NO: 20 and a heavy chain having an amino acid sequence set forth in SEQ ID NO: 19.

In one or more embodiments, the antibody is an anti-B7H3 antibody or an antigen-binding unit thereof.

In one or more embodiments, the anti-B7H3 antibody comprises a heavy chain having an amino acid sequence set forth in SEQ ID NO: 21 and a light chain having an amino acid sequence set forth in SEQ ID NO: 22.

In one or more embodiments, the antibody is an anti-FRα antibody or an antigen-binding unit thereof.

In one or more embodiments, the anti-FRα antibody comprises a heavy chain having an amino acid sequence set forth in SEQ ID NO: 23 and a light chain having an amino acid sequence set forth in SEQ ID NO: 24.

Antibodies can be prepared by using conventional recombinant DNA techniques. Vectors and cell lines for antibody production can be selected, constructed and cultured using techniques well known to those skilled in the art. These techniques are described in various laboratory manuals and main publications, such as Recombinant DNA Technology for Production of Protein Therapeutics in Cultured Mammalian Cells, D. L. Hacker, F. M. Wurm, Reference Module in Life Sciences, 2017, which is incorporated by reference in its entirety, including the supplements.

In one or more embodiments, the DNA encoding the antibody can be designed and synthesized according to the antibody amino acid sequence described herein by conventional methods, inserted into an expression vector, and transfected into a host cell. The transfected host cell is then cultured in a medium to produce the monoclonal antibody. In some embodiments, the vector expressing the antibody comprises at least one promoter element, an antibody coding sequence, a transcription termination signal, and a polyA tail. Other elements include enhancers, Kozak sequences, and donor and recipient sites flanking the inserted sequence for RNA splicing. Efficient transcription can be obtained by early and late promoters of SV40, long terminal repeats from retroviruses such as RSV, HTLV1 and HIVI, and early promoters of cytomegalovirus, and promoters from other cells such as actin promoter can also be used. Suitable expression vectors may include pIRES1neo, pRetro-Off, pRetro-On, PLXSN, or Plncx, pcDNA3.1 (+/-), pcDNA/Zeo (+/-), pcDNA3.1/Hygro(+/-), PSVL, PMSG, pRSVcat, pSV2dhfr, pBC12MI, pCS2, and the like. Commonly used mammalian host cells include HEK293 cells, Cos1 cells, Cos7 cells, CV1 cells, murine L cells, CHO cells, and the like.

In one or more embodiments, the inserted gene fragment should comprise a screening label, common ones of which include screening genes such as dihydrofolate reductase, glutamine synthetase, neomycin resistance and hygromycin resistance, to facilitate the screening and separation of cells that have been successfully transfected. The constructed plasmid is transfected to a host cell without the above genes, and the successfully transfected cells are cultured in a large quantity in a selective culture medium to produce the desired target protein. Antibodies will be purified by one or more purification steps. Purification can be carried out by conventional methods, such as centrifuging the cell suspension first, collecting the supernatant, and centrifuging again to further remove impurities. Methods such as Protein A affinity column and ion exchange column can be used to purify the antibody protein.

In one or more embodiments of the present invention, the number of small-molecule drugs binding to an antibody in an antibody-drug conjugate, i.e., the drug binding number of an antibody, is referred to as drug-antibody conjugation ratio (DAR). In some embodiments, the value is selected from 1, 2, 3, 4, 5, 6, 7, 8, 9, and 10. In considering the mean binding number of small molecule drugs, i.e., the mean drug binding number of an antibody, or the mean drug-antibody conjugation ratio, the value is selected from about 0 to 10, or 2 to 8. In some embodiments, the drug-antibody conjugation ratio is 3-6; in other embodiments, the drug-antibody conjugation ratio is about 6 to about 8, or about 7 to about 8.

Synthesis Method

The present invention also provides preparation methods for the drug conjugates, such as antibody-drug conjugates, and intermediates. The drug conjugates, such as the antibody-drug conjugates, and intermediates of the present invention can be prepared using known reagents and methods. In some embodiments, the preparation method is as follows.

Preparation method for

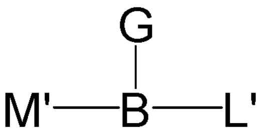

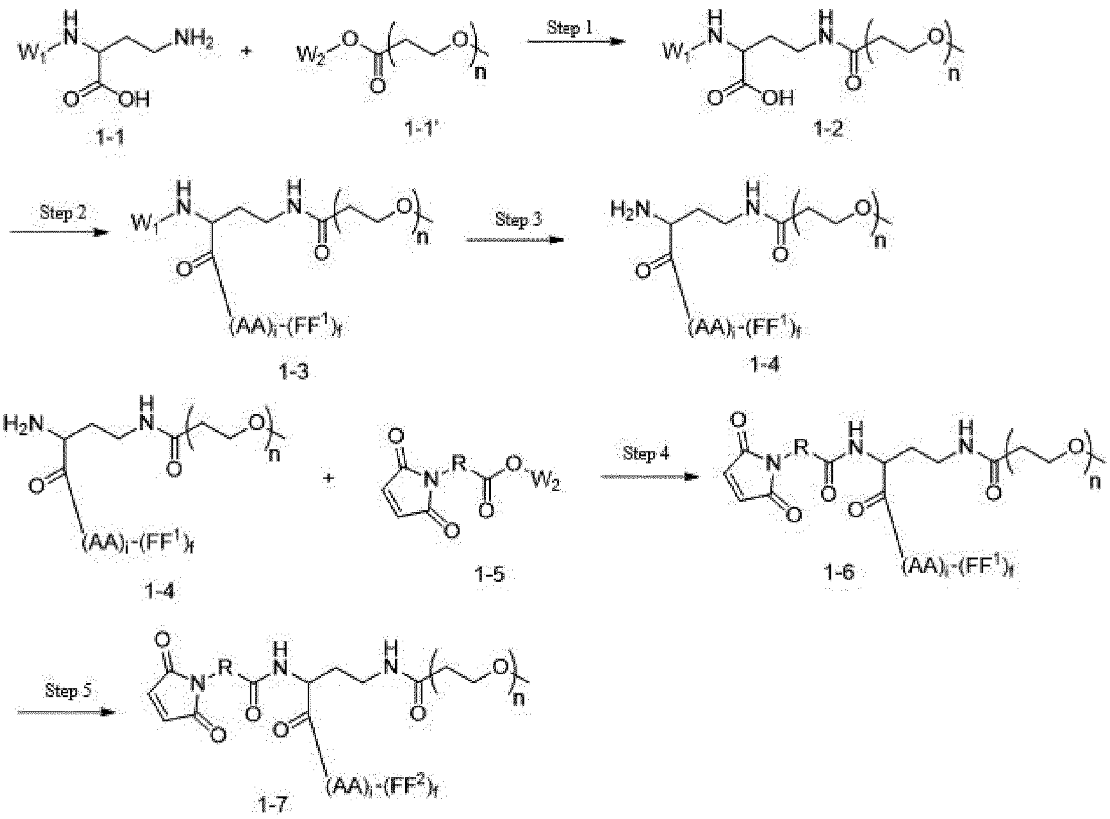


1-1 1-1' 1-2 1-3 1-4 1-4 1-5 1-6 1-7

Step 1: reacting a compound of general Formula 1-1 with a compound of general Formula 1-1' under a basic condition to obtain a compound of general Formula 1-2;

Step 2: reacting the compound of general Formula 1-2 with general Formula (AA)i-($FF^1$)f in the presence of a condensing agent under a basic condition to obtain a compound of general Formula 1-3;

Step 3: removing the amino-protecting group $W_1$ of the compound of general Formula 3 to obtain a compound of general Formula 1-4;

Step 4: reacting the compound of general Formula 1-4 with a compound of general Formula 1-5 under a basic condition to obtain a compound of general Formula 1-6; and

Step 5: reacting the compound of general Formula 1-6 with bis(p-nitrophenyl)carbonate under a basic condition to obtain a compound of general Formula 1-7;

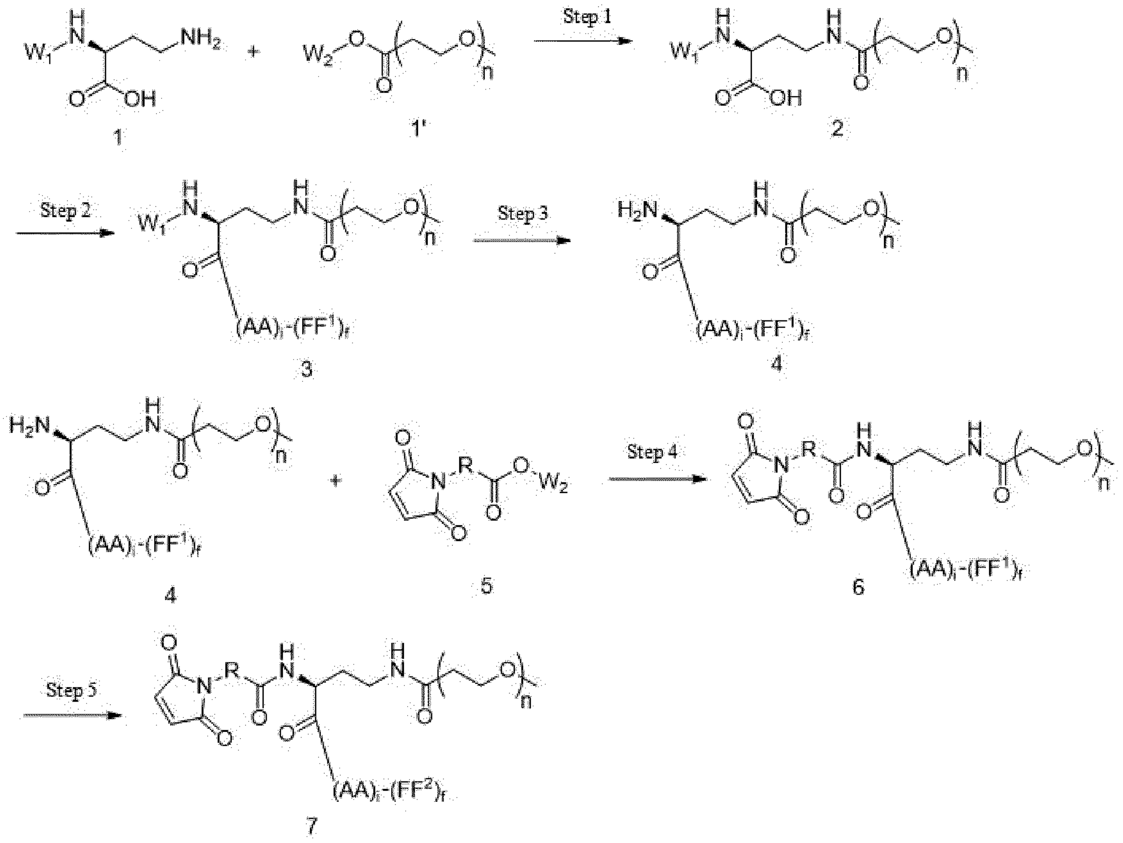


Step 1: reacting a compound of general Formula 1 with a compound of general Formula 1' under a basic condition to obtain a compound of general Formula 2;

Step 2: reacting the compound of general Formula 2 with general Formula $(AA)_i$-$(FF^1)_f$ in the presence of a condensing agent under a basic condition to obtain a compound of general Formula 3;

Step 3: removing the amino-protecting group $W_1$ of the compound of general Formula 3 to obtain a compound of general Formula 4;

Step 4: reacting the compound of general Formula 4 with a compound of general Formula 5 under a basic condition to obtain a compound of general Formula 6; and Step 5: reacting the compound of general Formula 6 with bis(p-nitrophenyl)carbonate under a basic condition to obtain a compound of general Formula 7;

wherein

$W_1$ is an amino-protecting group, e.g., 9-fluorenylmethyloxycarbonyl; $W_2$ is a carboxylic acid active ester, for example, a succinimidyl ester;

wherein n, AA, R, i, f are described as in Formula II herein; $FF^1$ is

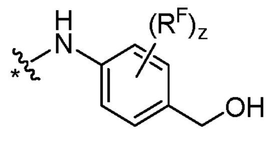 , 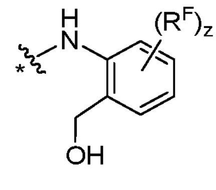 or 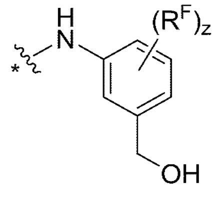 ,

and $FF^2$ is

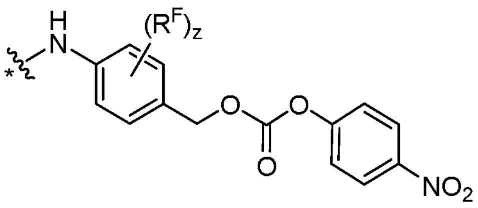

,

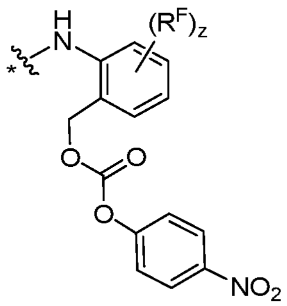

or

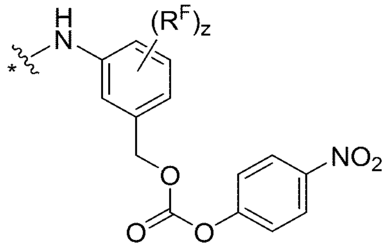

,

wherein * links to AA.

The basic conditions described above can be provided using reagents including organic bases and inorganic bases, wherein the organic bases include, but are not limited to, triethylamine, diethylamine, N-methylmorpholine, pyridine, piperidine, *N,N*-diisopropylethylamine, *n*-butyllithium, lithium diisopropylamide, potassium acetate, sodium *tert*-butoxide, or potassium *tert*-butoxide, and the inorganic bases include, but are not limited to, sodium hydride, potassium phosphate, sodium carbonate, potassium carbonate, cesium carbonate, sodium hydroxide, and lithium hydroxide.

The condensing agent described above may be selected from *N,N,N',N'*-tetramethyl-O-(7-azabenzotriazol-1-yl)urea hexafluorophosphate, 4-(4,6-dimethoxy-1,3,5-triazin-2-yl)-4-methylmorpholinium chloride, 1-hydroxybenzotriazole and 1-(3-dimethylaminopropyl)-3-ethylcarbodiimide hydrochloride, *N,N'*-dicyclohexylcarbodiimide, *N,N'*-diisopropylcarbodiimide, *O*-benzotriazol-*N,N,N',N'*-tetramethyluronium tetrafluoroborate, 1-hydroxybenzotriazole, 1-hydroxy-7-azobenzotriazol, *O*-benzotriazol-*N,N,N',N'*-tetramethyluronium hexafluorophosphate, 2-(7-azobenzotriazol)-*N,N,N',N'*-tetramethyluronium hexafluorophosphate, benzotriazol-1-yloxytris(dimethylamino)phosphonium hexafluorophosphate, and benzotriazol-1-yl-oxytripyrrolidinophosphonium hexafluorophosphate.

Preparation method for

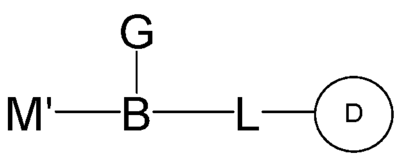

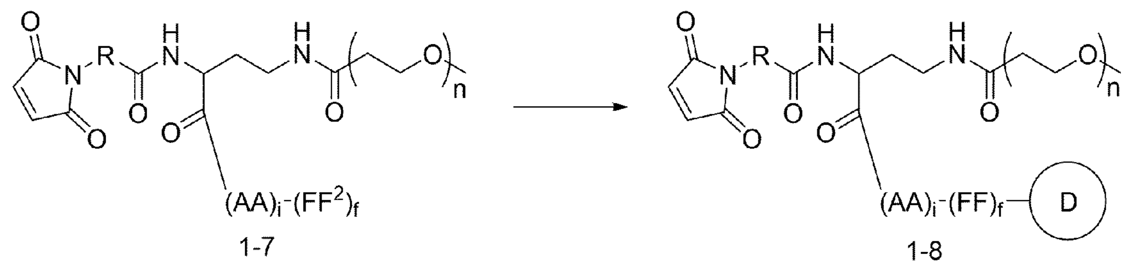

1-7 1-8

The compound of general Formula 1-7 and D are reacted in the presence of a condensing agent under a basic condition to obtain a compound of general Formula 1-8,

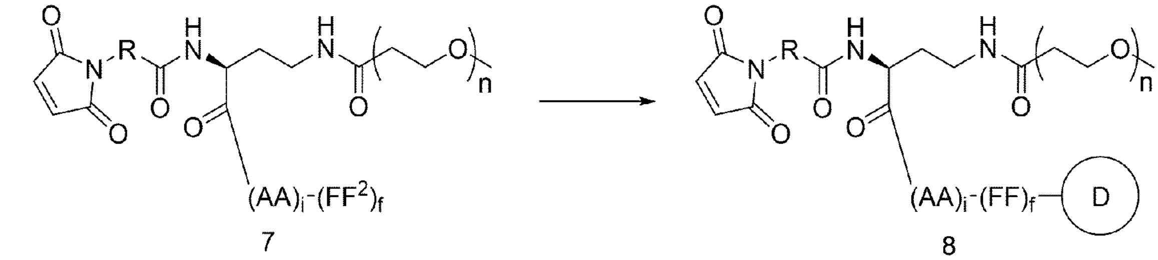

7 8

and the compound of general Formula 7 and D are reacted in the presence of a condensing agent under a basic condition to obtain a compound of general Formula 8, wherein n, AA, R, i, f, FF and D are described as in Formula III, and $FF^2$ is

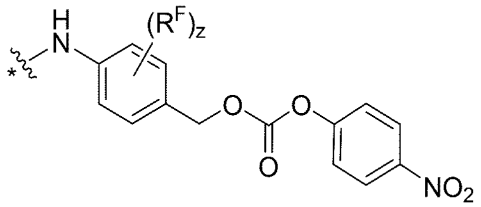

,

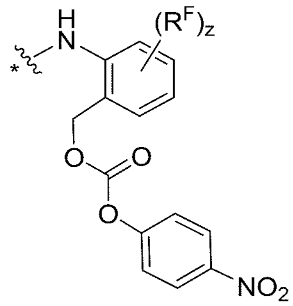

or

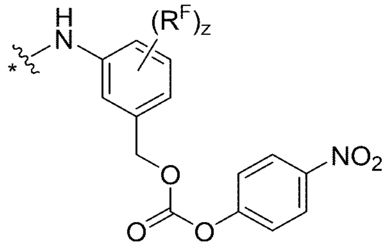

,

wherein * links to AA.

The basic conditions described above can be provided using reagents including organic bases and inorganic bases, wherein the organic bases include, but are not limited to, triethylamine, diethylamine, *N*-methylmorpholine, pyridine, piperidine, *N,N*-diisopropylethylamine, *n*-butyllithium, lithium diisopropylamide, potassium acetate, sodium *tert*-butoxide, or potassium *tert*-butoxide, and the inorganic bases include, but are not limited to, sodium hydride, potassium phosphate, sodium carbonate, potassium carbonate, cesium carbonate, sodium hydroxide, and lithium hydroxide.

The condensing agent described above may be selected from *N,N,N',N'*-tetramethyl-O-(7-azabenzotriazol-1-yl)urea hexafluorophosphate, 4-(4,6-dimethoxy-1,3,5-triazin-2-yl)-4-methylmorpholinium chloride, 1-hydroxybenzotriazole and 1-(3-dimethylaminopropyl)-3-ethylcarbodiimide hydrochloride, *N,N'*-dicyclohexylcarbodiimide, *N,N'*-diisopropylcarbodiimide, *O*-benzotriazol-*N,N,N',N'*-tetramethyluronium tetrafluoroborate, 1-hydroxybenzotriazole, 1-hydroxy-7-azobenzotriazol, *O*-benzotriazol-*N,N,N',N'*-tetramethyluronium hexafluorophosphate, 2-(7-azobenzotriazol)-*N,N,N',N'*-tetramethyluronium hexafluorophosphate, benzotriazol-1-yloxytris(dimethylamino)phosphonium hexafluorophosphate, and benzotriazol-1-yl-oxytripyrrolidinophosphonium hexafluorophosphate.

Preparation method for

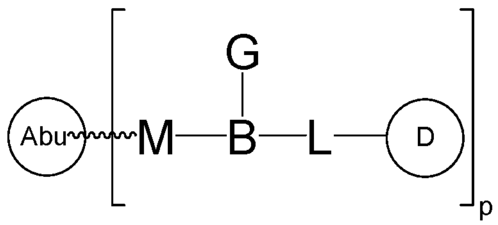

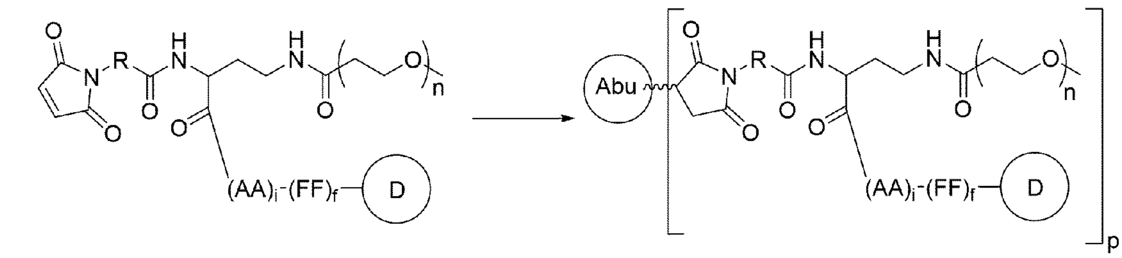

1-8 1-9

The compound of general Formula 1-8 is conjugated to Abu under a weakly acidic condition to obtain a compound of Formula 1-9,

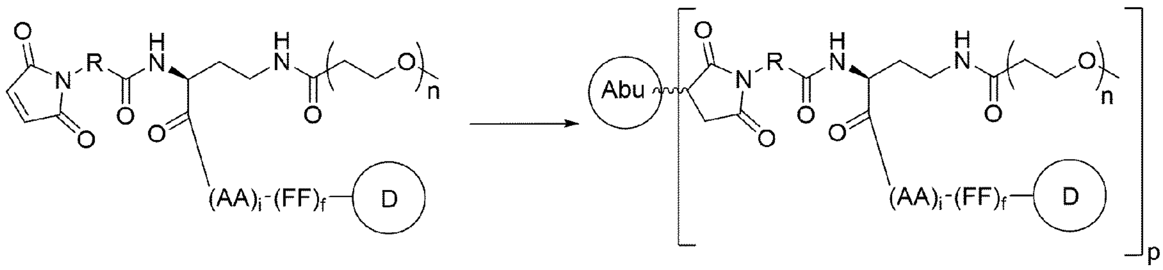

8 9

and the compound of general Formula 8 is conjugated to Abu under a weakly acidic condition to obtain a compound of Formula 9,

wherein n, AA, R, i, f, FF, D and Abu are described as in Formula I.

The weakly acidic condition described above may be provided using reagents including organic acids and inorganic acids, wherein the organic acids include, but are not limited to, acetic acid, benzoic acid, tartaric acid, oxalic acid, malic acid, citric acid, ascorbic acid, citric acid, salicylic acid, caffeic acid, sorbic acid, quinic acid, oleanolic acid, succinic acid, chlorogenic acid, formic acid, and propionic acid, and the inorganic acids include, but are not limited to, carbonic acid, nitrous acid, acetic acid, hypochlorous acid, hydrofluoric acid, sulfurous acid, bisulfic acid, silicic acid, metasilicic acid, phosphoric acid, metaphosphoric acid, sodium bicarbonate, and sodium bisulfite.

The drug conjugate can be purified by conventional methods, such as preparative high-performance liquid chromatography (prep-HPLC) and other methods.

## Examples

Example 1. Preparation of Antibodies

The following antibodies were prepared using conventional methods. After vector construction, eukaryotic cells such as HEK293 cells (Life Technologies Cat. No. 11625019) were transiently transfected, or CHO cells were stably transfected, and stable cell lines were selected and purified for expression.

Preparation and purification of trastuzumab antibody

Referring to the method of Wood et al., J Immunol., 145: 3011 (1990), etc., anti-HER2 antibody, a monoclonal antibody that specifically binds to the extracellular domain of HER2, was produced in CHO cells. Expression vectors

OptiCHO™ Antibody Express System (invitrogen) containing antibody genes were constructed using conventional molecular biological methods, with CHO cells as host cells.

Preparation and purification of hRS9 antibody

Referring to the method of Wood et al., J Immunol., 145: 3011 (1990), etc., an anti-Trop2 specific monoclonal antibody was produced in CHO cells. Expression vectors containing antibody genes were constructed using conventional molecular biological methods, wherein the amino acid sequences of the heavy chain and the light chain of the recombinant humanized anti-Trop2 monoclonal antibody hRS9 antibody are set forth in SEQ ID NO: 5 and SEQ ID NO: 6, respectively.

Preparation and purification of anti-CLND18.2 antibody

Expression vectors containing the anti-CLND18.2 antibody genes were constructed using conventional molecular biology methods, transfected into HEK293F cells, cultured and purified to obtain antibodies. The relevant sequences of the anti-CLDN18.2 antibody are shown in Tables 4 and 5; wherein the light chain amino acid sequence of the H239H-2b-K-6a-1 antibody is set forth in SEQ ID NO:20, and the heavy chain amino acid sequence is set forth in SEQ ID NO:18; the amino acid sequence of the light chain of the H239H-2b-K-6a-2 antibody set forth in SEQ ID NO:20, and the amino acid sequence of the heavy chain set forth in SEQ ID NO: 19.

Table 4 Amino acid sequences of the anti-CLDN18.2 antibodies

| Name | Sequence No. | Amino acid sequence |
|---|---|---|
| VH CDR1 | SEQ ID NO:7 | DYGMH |
| VH CDR2 | SEQ ID NO:8 | YISRGRSTTYSTDTVKG |
| VH CDR3 | SEQ ID NO:9 | GSYYGNALDY |
| VL CDR1 | SEQ ID NO:10 | KSSQSLLNSGNQRNYLT |
| VL CDR2 | SEQ ID NO:11 | WASTRES |
| VL CDR3 | SEQ ID NO:12 | QSAYSYPFT |
| heavy chain variable region | SEQ ID NO:13 | EVQLLESGGGLVQPGGSLRLSCAASGFTFSDYGMHW VRQAPGKGLEWVAYISRGRSTTYSTDTVKGRFTISRDN SKNTLYLQMNSLRAEDTAVYYCARGSYYGNALDYW GQGTLVTVSS |
| light chain variable region | SEQ ID NO:14 | DIVMTQSPDSLAVSLGERATINCKSSQSLLNSGNQRNY LTWYQQKPGQPPKLLIYWASTRESGVPDRFSGSGSGT DFTLTISSLQAEDVAVYYCQSAYSYPFTFGGGTKLEIK |

(continued)

| Name | Sequence No. | Amino acid sequence |
|---|---|---|
| heavy chain constant region | SEQ ID NO:15 | ASTKGPSVFPLAPSSKSTSGGTAALGCLVKDYFPEPVT VSWNSGALTSGVHTFPAVLQSSGLYSLSSVVTVPSSSL GTQTYICNVNHKPSNTKVDKKVEPKSCDKTHTCPPCP APELLGGPSVFLFPPKPKDTLMISRTPEVTCVVVDVSH EDPEVKFNWYVDGVEVHNAKTKPREEQYNSTYRVVS VLTVLHQDWLNGKEYKCKVSNKALPAPIEKTISKAKG QPREPQVYTLPPSRDELTKNQVSLTCLVKGFYPSDIAVE WESNGQPENNYKTTPPVLDSDGSFFLYSKLTVDKSRW QQGNVFSCSVMHEALHNHYTQKSLSLSPGK |
| | SEQ ID NO:16 | ASTKGPSVFPLAPSSKSTSGGTAALGCLVKDYFPEPVT VSWNSGALTSGVHTFPAVLQSSGLYSLSSVVTVPSSSL GTQTYICNVNHKPSNTKVDKKVEPKSCDKTHTCPPCP APELLGGPSVFLFPPKPKDTLMISRTPEVTCVVVDVSH EDPEVKFNWYVDGVEVHNAKTKPREEQYASTYRVVS VLTVLHQDWLNGKEYKCKVSNKALPAPIEKTISKAKG QPREPQVYTLPPSRDELTKNQVSLTCLVKGFYPSDIAVE WESNGQPENNYKTTPPVLDSDGSFFLYSKLTVDKSRW QQGNVFSCSVMHEALHNHYTQKSLSLSPGK |
| light chain constant region | SEQ ID NO:17 | RTVAAPSVFIFPPSDEQLKSGTASVVCLLNNFYPREAK VQWKVDNALQSGNSQESVTEQDSKDSTYSLSSTLTLS KADYEKHKVYACEVTHQGLSSPVTKSFNRGEC |
| heavy chain | SEQ ID NO:18 | EVQLLESGGGLVQPGGSLRLSCAASGFTFSDYGMHW VRQAPGKGLEWVAYISRGRSTTYSTDTVKGRFTISRDN SKNTLYLQMNSLRAEDTAVYYCARGSYYGNALDYW GQGTLVTVSSASTKGPSVFPLAPSSKSTSGGTAALGCL VKDYFPEPVTVSWNSGALTSGVHTFPAVLQSSGLYSLS SVVTVPSSSLGTQTYICNVNHKPSNTKVDKKVEPKSC DKTHTCPPCPAPELLGGPSVFLFPPKPKDTLMISRTPEV TCVVVDVSHEDPEVKFNWYVDGVEVHNAKTKPREE QYNSTYRVVSVLTVLHQDWLNGKEYKCKVSNKALPA PIEKTISKAKGQPREPQVYTLPPSRDELTKNQVSLTCLV KGFYPSDIAVEWESNGQPENNYKTTPPVLDSDGSFFLY SKLTVDKSRWQQGNVFSCSVMHEALHNHYTQKSLSL SPGK |
| | SEQ ID NO:19 | EVQLLESGGGLVQPGGSLRLSCAASGFTFSDYGMHW VRQAPGKGLEWVAYISRGRSTTYSTDTVKGRFTISRDN SKNTLYLQMNSLRAEDTAVYYCARGSYYGNALDYW GQGTLVTVSSASTKGPSVFPLAPSSKSTSGGTAALGCL VKDYFPEPVTVSWNSGALTSGVHTFPAVLQSSGLYSLS SVVTVPSSSLGTQTYICNVNHKPSNTKVDKKVEPKSC DKTHTCPPCPAPELLGGPSVFLFPPKPKDTLMISRTPEV TCVVVDVSHEDPEVKFNWYVDGVEVHNAKTKPREE QYASTYRVVSVLTVLHQDWLNGKEYKCKVSNKALPA PIEKTISKAKGQPREPQVYTLPPSRDELTKNQVSLTCLV KGFYPSDIAVEWESNGQPENNYKTTPPVLDSDGSFFLY SKLTVDKSRWQQGNVFSCSVMHEALHNHYTQKSLSL SPGK |

(continued)

| Name | Sequence No. | Amino acid sequence |
|---|---|---|
| light chain | SEQ ID NO:20 | DIVMTQSPDSLAVSLGERATINCKSSQSLLNSGNQRNY LTWYQQKPGQPPKLLIYWASTRESGVPDRFSGSGSGT DFTLTISSLQAEDVAVYYCQSAYSYPFTFGGGTKLEIKR TVAAPSVFIFPPSDEQLKSGTASVVCLLNNFYPREAKV QWKVDNALQSGNSQESVTEQDSKDSTYSLSSTLTLSK ADYEKHKVYACEVTHQGLSSPVTKSFNRGEC |

Table 5 Composition of the anti-CLDN18.2 antibodies

| Name of antibody | Sequence number of VH | Sequence number of CH | Sequence number of VL | Sequence number of CL |
|---|---|---|---|---|
| H239H-2b-K-6a-1 | SEQ ID NO:13 | SEQIDNO:15 | SEQ ID NO:14 | SEQ ID NO:17 |
| H239H-2b-K-6a-2 | SEQ ID NO:13 | SEQ ID NO:16 | SEQ ID NO:14 | SEQIDNO:17 |

Preparation and purification of anti-B7H3 antibody

Expression vectors containing the anti-B7H3 antibody A genes were constructed using conventional molecular biology methods, transfected into HEK293F cells, cultured and purified to obtain antibodies. The relevant sequences of the anti-B7H3 antibody A are shown in Tables 6; wherein the heavy chain amino acid sequence of the antibody is set forth in SEQ ID NO:21 and the light chain amino acid sequence is set forth in SEQ ID NO:22.

Table 6 Amino acid sequences of the anti-B7H3 antibody A

| Name | Sequence No. | Amino acid sequence |
|---|---|---|
| heavy chain | SEQ ID NO:21 | EVQLVQSGAEVKKSGESLKISCKASGYTFTDYDINW VRQMPGKGLEWIGWIFPGDDTTKYNEKFKGQVTLSA DKSTNTAYMQWSSLKASDTAMYYCARSPSFDYWGQ GTLVTVSSASTKGPSVFPLAPSSKSTSGGTAALGCLVK DYFPEPVTVSWNSGALTSGVHTFPAVLQSSGLYSLSS VVTVPSSSLGTQTYICNVNHKPSNTKVDKKVEPKSC DKTHTCPPCPAPELLGGPSVFLFPPKPKDTLMISRTPE VTCVVVDVSHEDPEVKFNWYVDGVEVHNAKTKPRE EQYNSTYRVVSVLTVLHQDWLNGKEYKCKVSNKAL PAPIEKTISKAKGQPREPQVYTLPPSRDELTKNQVSLT CLVKGFYPSDIAVEWESNGQPENNYKTTPPVLDSDGS FFLYSKLTVDKSRWQQGNVFSCSVMHEALHNHYTQ KSLSLSPGK |
| light chain | SEQ ID NO:22 | QIVLTQSPGTLSLSPGERATLSCSASSTIGFMYWYQQK PGQAPRRWIYDTSKLASGVPDRFSGSGSGTDYTLTISR LEPEDFAVYYCHQRSSYPTFGQGTKVEIKRTVAAPSV FIFPPSDEQLKSGTASVVCLLNNFYPREAKVQWKVD NALQSGNSQESVTEQDSKDSTYSLSSTLTLSKADYEK HKVYACEVTHQGLSSPVTKSFNRGEC |

Preparation and purification of anti-FRα antibody

Expression vectors containing the anti-FRα antibody A genes were constructed using conventional molecular biology methods, transfected into HEK293F cells, cultured and purified to obtain antibodies. The relevant sequences of

the anti-FRα antibody A are shown in Tables 7; wherein the heavy chain amino acid sequence of the antibody is set forth in SEQ ID NO:23 and the light chain amino acid sequence is set forth in SEQ ID NO:24.

Table 7 Amino acid sequences of the anti-FRα antibody A

| Name | Sequence No. | Amino acid sequence |
|---|---|---|
| heavy chain | SEQ ID NO:23 | QVQLVQSGVEVKKPGASVKVSCKASGYSFTGYFMN WVRQAPGQGLEWIGRIHPYDGDTFYNQNFKDKATLT VDKSTTTAYMELKSLQFDDTAVYYCTRYDGSRAMDY WGQGTTVTVSSASTKGPSVFPLAPSSKSTSGGTAALG CLVKDYFPEPVTVSWNSGALTSGVHTFPAVLQSSGLY SLSSVVTVPSSSLGTQTYICNVNHKPSNTKVDKKVEP KSCDKTHTCPPCPAPELLGGPSVFLFPPKPKDTLMISR TPEVTCVVVDVSHEDPEVKFNWYVDGVEVHNAKTK PREEQYNSTYRVVSVLTVLHQDWLNGKEYKCKVSN KALPAPIEKTISKAKGQPREPQVYTLPPSRDELTKNQV SLTCLVKGFYPSDIAVEWESNGQPENNYKTTPPVLDS DGSFFLYSKLTVDKSRWQQGNVFSCSVMHEALHNHY TQKSLSLSPGK |
| light chain | SEQ ID NO:24 | EIVLTQSPATLSLSPGERATLSCKASQSVSFAGTSLMH WYQQKPGQAPRLLIYRASNLEAGVPARFSGSGSKTD FTLTISSLEPEDFAVYYCQQSREYPYTFGGGTKVEIKR TVAAPSVFIFPPSDEQLKSGTASVVCLLNNFYPREAKV QWKVDNALQSGNSQESVTEQDSKDSTYSLSSTLTLS KADYEKHKVYACEVTHQGLSSPVTKSFNRGEC |

Example 2. Synthesis Procedures for Compound

(1*S*,9*S*)-1-amino-9-ethyl-4,5-difluoro-9-hydroxy-1,2,3,9,12,15-hexahydro-10*H*,13*H*-b enzo[*de*]pyrano[3',4':6,7]indolo[1,2-*b*]quinoline-10,13-dione hydrochloride (D-1) 1. Synthesis of *N*,*N'*-(3,4-difluoro-8-oxo-5,6,7,8-tetrahydronaphthalene-1,7-diyl)diacetamide

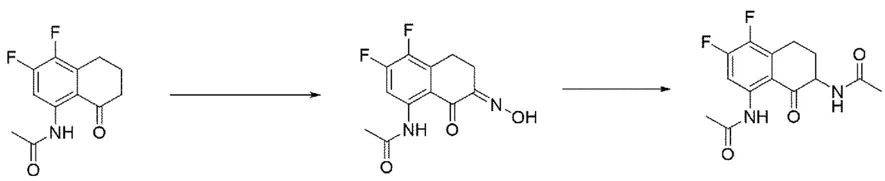

A solution of potassium *tert*-butoxide in tetrahydrofuran (42 mL, 1 M) was added to a dry reaction flask under a nitrogen atmosphere, stirred, and cooled to 0-5 °C. *N*-(3,4-difluoro-8-oxo-5,6,7,8-tetrahydronaphthalen-1-yl)acetamide (CAS No. : 143655-49-6, 5 g, 21 mmol) dissolved in tetrahydrofuran (25 mL) was slowly added dropwise to the reaction flask, followed by *tert*-butyl nitrite (4.32 g, 2 eq) (with temperature controlled at 0-5 °C). The resulting mixture was warmed to 15-20 °C and stirred for 2 hours (h). After the completion of the reaction, the mixture was cooled to 0-5 °C. Acetic acid (25 mL) and acetic anhydride (25 mL) were added dropwise (with temperature controlled at no more than 10 °C). After the dropwise addition, the mixture was stirred for 20 minutes (min). With temperature maintained at 5-10 °C, zinc powder (8 eq) was added according to the amount of *N*-(3,4-difluoro-8-oxo-5,6,7,8-tetrahydronaphthalen-1-yl)acetamide. The mixture was stirred at 20-25 °C for 1 h. The mixture was filtered, and the solid was rinsed with ethyl acetate (50 mL). The temperature was reduced to 0-5 °C, and 15% aqueous solution of $NaCO_3$ (50 mL) was added dropwise for three washings. Ethyl acetate (25 mL) was added for extraction. The organic phases were pooled and washed with a saturated aqueous solution of NaCl. Ethyl acetate (10 mL) was added, and the mixture was stirred at 40 °C for 30 min, slowly cooled to 0-5 °C, and stirred for 2 h. The mixture was filtered, and the solid was washed with ethyl acetate/petroleum ether (1/2, 10 mL). Drying was performed *in vacuo* to obtain a gray powder (2.1 g, 33.7%). LC-MS: $[M+H]^+$ = 297.

2. Synthesis of *N*-(8-amino-5,6-difluoro-1-oxo-1,2,3,4-tetrahydronaphthalen-2-yl)acetamide

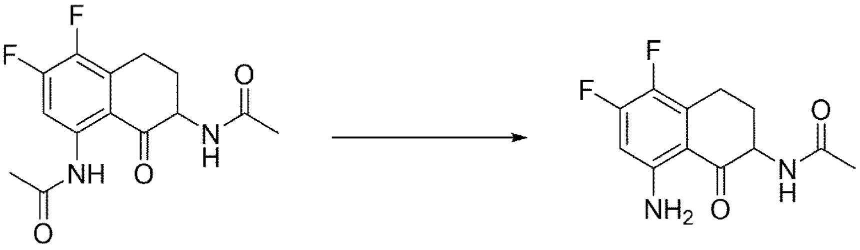

*N',N'*-(3,4-difluoro-8-oxo-5,6,7,8-tetrahydronaphthalene-l,7-diyl)diethylamide (500 mg, 1.68 mmol) was added to a 2 M solution of hydrochloric acid in ethanol (5 mL). The resulting mixture was stirred at 50 °C for 4 h. After the completion of the reaction as detected, water (7.5 mL) was added. The mixture was cooled to 0-5 °C, and triethylamine (1.03 g) was added dropwise. The mixture was stirred for 3 h. The mixture was filtered, and washing was performed successively with 40% cold aqueous solution of ethanol (3 mL) and water (3 mL). Drying was performed *in vacuo* to obtain a gray powder (320 mg, 74.6%). LC-MS: $[M+H]^+$ = 255.

3. Synthesis of *N*-((9*S*)-9-ethyl-4,5-difluoro-9-hydroxy-10,13-dioxo-2,3,9,10,13,15-hexahydro-1*H*,12 *H*-benzo[*de*]pyrano[3';4':6,7]indolo[l,2-*b*]quinoline-1-yl)acetamide

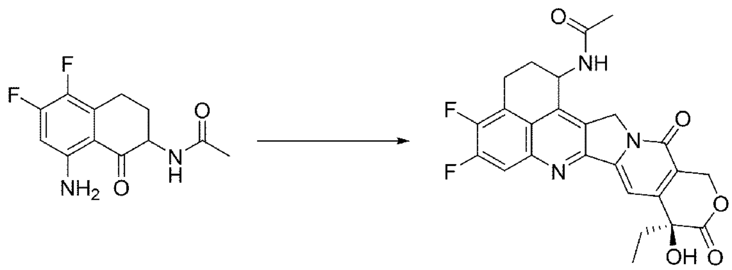

*N*-(8-amino-5,6-difluoro-1-oxo-1,2,3,4-tetrahydronaphthalen-2-yl)acetamide (1.1 g, 1 eq), (*S*)-4-ethyl-4-hydroxy-7,8-dihydro-1*H*-pyrano-[3,4-*f*]indolizine-3,6,10(4*H*)-trione (1.15 g, 1 eq) and toluene (50 ml) were added to a reaction flask under a nitrogen atmosphere. The mixture was warmed to reflux and stirred for 1 h. Then pyridinium 4-toluenesulfonate (100 mg) was added, and the mixture was stirred at reflux for another 20 h. The mixture was cooled to room temperature and stirred for 1 h. The mixture was filtered, and the solid was successively washed with acetone (10 mL) and cold ethanol (5 mL). Drying was performed *in vacuo* to obtain a taupe powder (1.1 g, 53%). LC-MS: $[M+H]^+$= 482.

4. Synthesis of (1*S*,9*S*)-1-amino-9-ethyl-4,5-difluoro-9-hydroxy-1,2,3,9,12,15-hexahydro-10*H*,13*H*-b enzo[*de*]pyrano[3',4':6,7]indolo[1,2-*b*]quinoline-10,13-dione hydrochloride

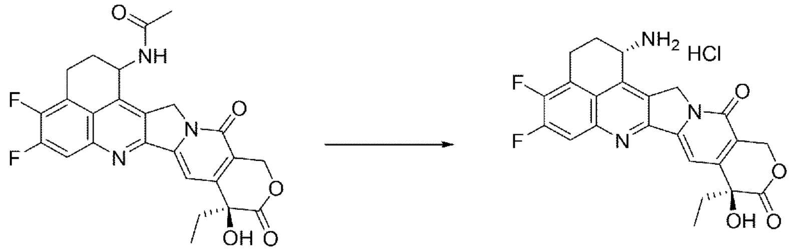

*N*-((9*S*)-9-ethyl-4,5-difluoro-9-hydroxy-10,13-dioxo-2,3,9,10,13,15-hexahydro-1*H*,12 *H*-benzo[*de*]pyrano[3',4':6,7]indolo[1,2-*b*]quinoline-1-yl)acetamide (1.1 g, 2.28 mmol) and a 6 M aqueous solution of hydrochloric acid (44 mL) were added to a reaction flask. The mixture was refluxed with stirring under a nitrogen atmosphere for 4 h. The mixture was concentrated to remove the solvent, and the residue was purified by HPLC to obtain a white powder (200 mg, 18%). LC-MS: $[M+H]^+$ = 440.

Example 3. Synthesis Procedures for 4-((30*S*,33*S*,36*S*)-30-(2-(2,5-dioxo-2,5-dihydro-1*H*-pyrrol-1-yl)acetamido)-33-isopropyl-26,31,34-trioxo-36-(3-ureidopropyl)-2,5,8, 11, 14, 17,20,23-octaoxo-27,32,35 -triazaheptatriacontan-37-amino)benzyl (4-nitrophenyl) carbonate (CB07)

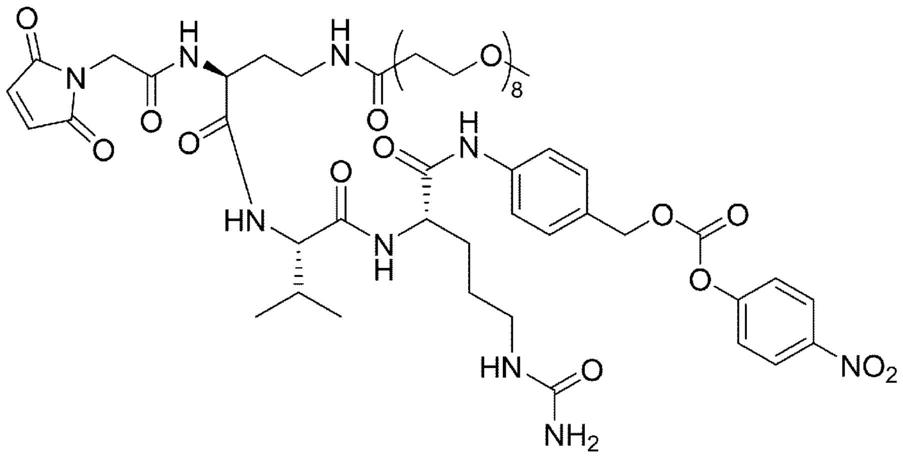

1) Synthesis of (*S*)-30-((((9*H*-fluoren-9-yl)methoxy)carbonyl)amino)-26-oxo-2,5,8,11,14,17,20,23-octaoxa-27-aza-hentriacontan-31-oic acid (CB01).

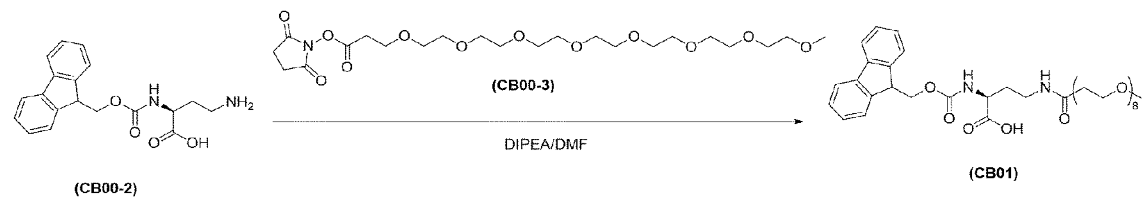


Under a nitrogen atmosphere at 0-5 °C, 8.14 g of *N*2-fluorenylmethoxycarbonyl-L-2,4-diaminobutyric acid (CB00-2) was dissolved in 40 mL of dimethylformamide(DMF), and 10 g of *N*-succinimidyl 4,7,10,13,16,19,22,25-octaoxahexacosanoate (CB00-3) and 10 mL of DMF were added. 6.5 mL of DIPEA was dropwise added with the temperature maintained at 0-5 °C. 1 h after the addition, the mixture was stirred at room temperature for 4 h. After the completion of the reaction, DMF was removed under reduced pressure, and the residue was purified by silica gel column chromatography (with dichloromethane and methanol volume ratio 20:1 as elution solvents) to obtain CB01 in the form of a pale yellow oil (14.2 g).

2) Synthesis of (9*H*-fluoren-9-yl)methyl [(*S*)-1-[[(*S*)-1-[[4-(hydroxymethyl)phenyl] amino]-1-oxo-5-ureidopentan-2-yl]amino]-3-methyl-1-oxobutan-2-yl]carbamate (CB02).

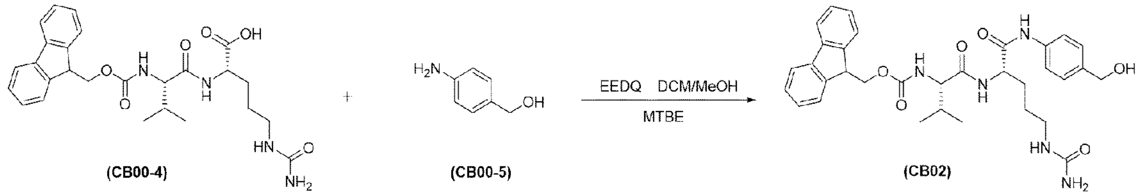


(CB00-4) (CB00-5) (CB02)

11 g of (*S*)-2-((*S*)-2-((((9*H*-fluoren-9-yl)methoxy)carbonyl)amino) -3-methylbutanamido)-5-ureidopentanoic acid (CB00-4) and 5.5 g of (4-aminophenyl)methanol (CB00-5) were dissolved in 400 mL of dichloromethane and 200 mL of methanol at room temperature under a nitrogen atmosphere, and 17 g of 2-ethoxy-1-ethoxycarbonyl-1,2-dihydroquinoline (EEDQ) were added in portions with mechanical stirring. The resulting mixture was allowed to react for 15 h in the absence of light. After the completion of the reaction, the solvent was removed under reduced pressure to obtain a paste solid, which was subjected to silica gel column chromatography (with dichloromethane and methanol volume ratio 20:1 as elution solvents) to obtain an off-white solid (11.9 g).

3) Synthesis of (*S*)-2-((*S)*-2-amino-3-methylbutanamido)-*N*-(4-(hydroxymethyl)phenyl)-5-ureidopentanamide (CB03).

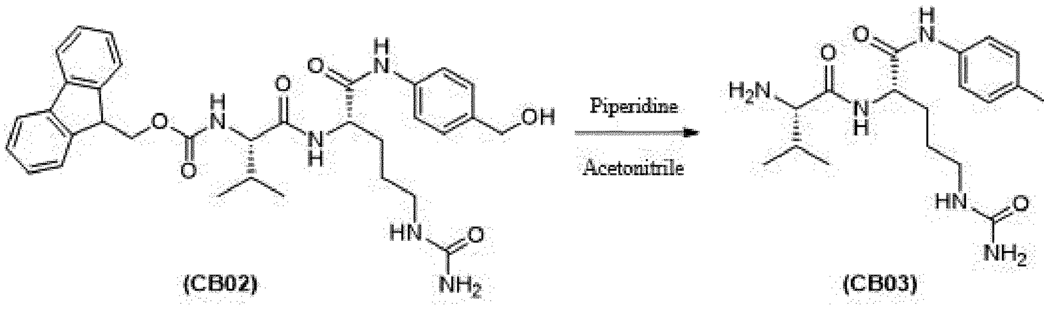


(CB02) (CB03)

Under a nitrogen atmosphere at room temperature, 300 mL of acetonitrile was added to 11.9 g of (9*H*-fluoren-9-yl)methyl [(*S*)-1-[[(*S*)-1-[[4-(hydroxymethyl)phenyl]amino]-1-oxo-5-ureidopentan-2-yl]amino]-3-methyl-1-oxobutan-2-yl]carbamate (CB02), and 18 mL of piperidine was added dropwise with stirring. After the dropwise addition, the mixture was allowed to react at room temperature for 2 h. After the completion of the reaction, the mixture was distilled under reduced pressure to remove the solvent and piperidine, and the residue was subjected to silica gel column chromatography (with dichloromethane and methanol volume ratio 20:1 as elution solvents) to obtain CB03 in the form of a white solid (7.5 g).

4) Synthesis of (9*H*-fluoren-9-yl)methyl ((30*S*,33*S*,36S)-41-amino-36-((4-(hydroxymethyl)phenyl)carbamoyl)-33-isopropyl-26,31,34,41-tetraoxo-2,5,8,11,14,17, 20,23-octaoxa-27,32,35,40-tetraaza-30-yl)carbamate (CB04).

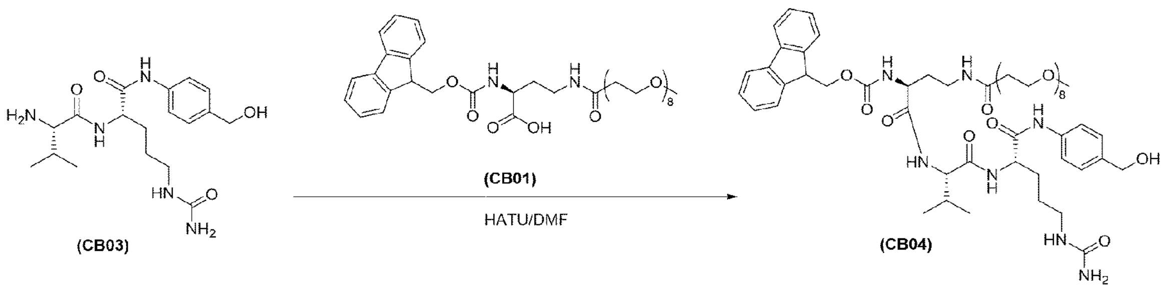


(CB03) (CB04)

At 0 °C under a nitrogen atmosphere, 14.2 g of (*S*)-30-((((9*H*-fluoren-9-yl)methoxy) carbonyl)amino)-26-oxo-2,5,8, 11, 14, 17,20,23-octaoxa-27-aza-hentriacontan-31-oic acid (CB01) was dissolved in 100 mL of DMF, and 11 g of *N,N,N',N'*-tetramethyl-*O*-(7-azabenzotriazol-1-yl)urea hexafluorophosphate (HATU) was added in portions. After 30 min

of stirring, 7.5 g of (*S*)-2-((*S*)-2-amino-3-methylbutanamido)-N-(4-(hydroxymethyl)phenyl)-5-ureidopentanamide (CB03) was added, and the mixture was allowed to react for 2.5 h with 0 °C maintained. After the completion of the reaction, the mixture was distilled under reduced pressure to remove the solvent, and the residue was subjected to silica gel column chromatography (with dichloromethane and methanol volume ratio 10: 1 as elution solvents) to obtain CB04 in the form of a solid (9.66 g).

5) Synthesis of *N*-((3*S*)-3-amino-4-(((2*S*)-1-((1-((4-(hydroxymethyl)phenyl)amino) -1-oxo-5-ureidopentan-2-yl)amino)-3-methyl-1-oxobutan-2-yl)amino)-4-oxy)-2,5,8, 11,14,17,20,23-octaoxahexacosan-26-amide (CB05).

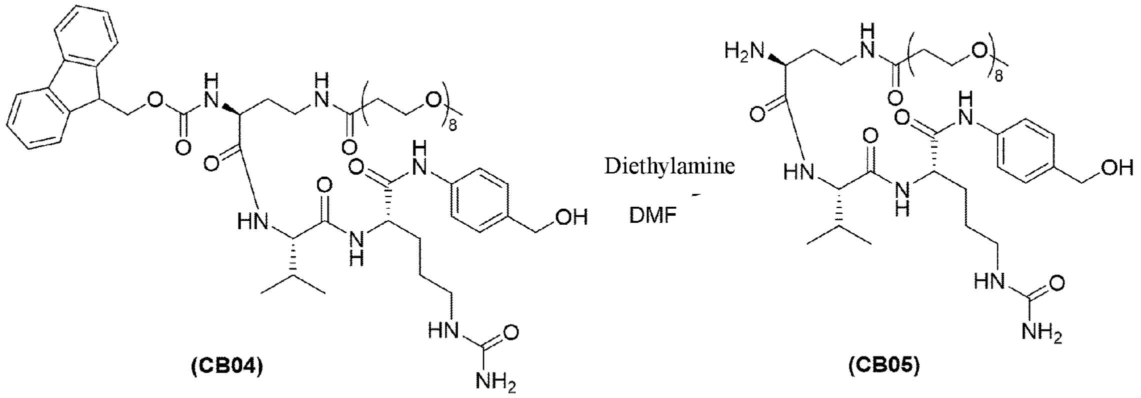


(CB04) (CB05)

9.66 g of (9*H*-fluoren-9-yl)methyl ((30*S*,33*S*,36S)-41-amino-36-((4-(hydroxymethyl) phenyl)carbamoyl)-33-isopropyl-26,31,34,41-tetraoxo-2,5,8,11,14,17,20,23-octaoxa-2 7,32,35,40-tetraaza-30-yl)carbamate (CB04) was dissolved in 50 mL of DMF at room temperature under a nitrogen atmosphere, and 12 mL of diethylamine was added. The mixture was stirred for 1.5 h. After the completion of the reaction, the mixture was distilled under reduced pressure to remove the solvent, and the residue was subjected to silica gel column chromatography (with dichloromethane and methanol volume ratio 7.5:1 as elution solvents) to obtain CB05 in the form of a pale yellow solid (7.7 g).

6) Synthesis of *N*-((3*S*)-3-(2-(2,5-dioxo-2,5-dihydro-1*H*-pyrrol-1-yl)acetamido)-4-(((2*S*)-1-((1-((4-(hydroxymethyl)phenyl)amino)-1-oxo-5-ureidopentan-2-yl)amino)-3-methyl-1-butanone-2-yl)amino)-4-oxo)-2,5,8,11,14,17,20,23-octaoxahexacosan-26-amide (CB06).

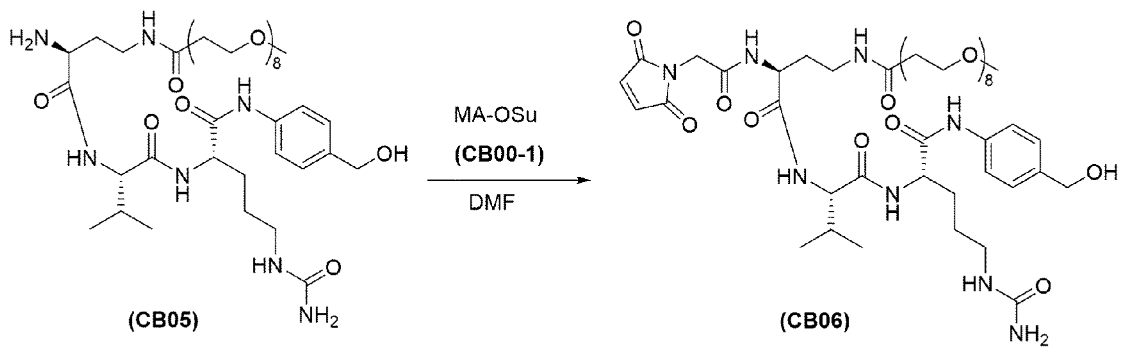


(CB05) (CB06)

7.7 g of N-((3*S*)-3-amino-4-(((2*S*)-1-((1-((4-(hydroxymethyl)phenyl)amino)-1-oxo-5-ureidopentan-2-yl)amino)-3-methyl-1-oxobutan-2-yl)amino)-4-oxy)-2,5,8,11,14,17,20, 23-octaoxahexacosan-26-amide (CB05) was dissolved in 40 mL of DMF, and succinimidyl maleimidoacetate (CB00-1) was added in portions under a nitrogen atmosphere at 0-5 °C. The mixture was allowed to react at 0-5 °C for 4 h. After the completion of the reaction, the mixture was distilled under reduced pressure to remove the solvent, and the residue was subjected to silica gel column chromatography (with dichloromethane and methanol as volume ratio 10:1 elution solvents) to obtain CB06 in the form of a pale yellow solid (9.5 g).

7) Synthesis of 4-((30*S*,33*S*,36S)-30-(2-(2,5-dioxo-2,5-dihydro-1*H*-pyrrol-1-yl) acetamido)-33-isopropyl-26,31,34-trioxo-36-(3-ureidopropyl)-2,5,8,11,14,17,20,23-octaoxo-27,32,35-triazaheptatriacontan-37-amino)benzyl (4-nitrophenyl) carbonate (CB07).

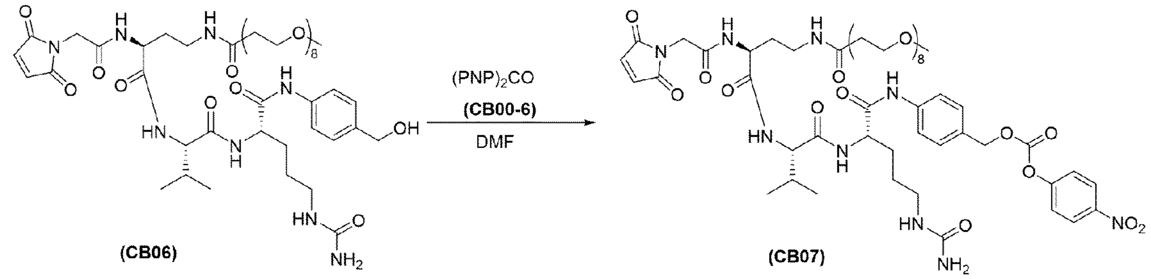


9.5 g of *N*-((3*S*)-3-(2-(2,5-dioxo-2,5-dihydro-1*H*-pyrrol-1-yl)acetamido)-4-(((2*S*-1-((1-((4-(hydroxymethyl)phenyl)amino)-1-oxo-5-ureidopentan-2-yl)amino)-3-methyl-1-butanone-2-yl)amino)-4-oxo)-2,5,8,11,14,17,20,23-octaoxahexacosan-26-amide (CB06) was dissolved in 50 mL of DMF, and 14.0 g of bis(*p*-nitrophenyl)carbonate ($(PNP)_2CO$) was added under a nitrogen atmosphere at 0 °C, followed by 8.2 mL of *N,N*-diisopropylethylamine (DIPEA) after dissolution. The mixture was allowed to react for 4 h with the 0 °C maintained. After the completion of the reaction, the mixture was distilled under reduced pressure to remove the solvent, and the residue was subjected to silica gel column chromatography (with dichloromethane and methanol volume ratio 10: 1 as elution solvents) to obtain CB07 in the form of a white solid (2.6 g).

Example 4. Synthesis of 4-((18*S*,21S,24S)-18-(2-(2,5-dioxo-2,5-dihydro-1*H*-pyrrol-1-yl)acetamido)-21-isopropyl-14,19,22-trioxo-24-(3-ureidopropyl)-2,5,8,11-tetraoxo-15, 20,23-triazapentacosan-25-amino)benzyl(4-nitrophenyl)carbonate (CB14)

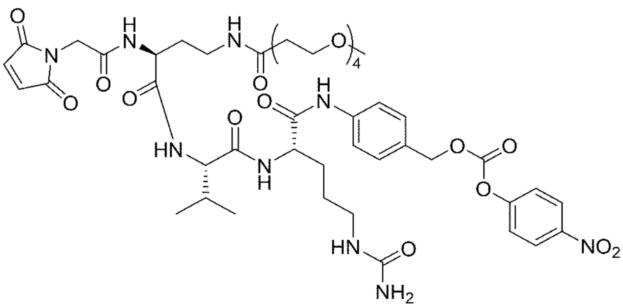

CB14 was prepared by referring to the synthesis of CB07 in Example 3, with *N*-succinimidyl 4,7,10,13,16,19,22,25-octaoxahexacosanoate replaced by *N*-succinimidyl 4,7,10,13-tetraoxatetradecanoate. CB14 was eventually obtained in the form of a white solid.

Example 5. Synthesis of Intermediate (CB07-Exatecan)

Synthesis of 4-((30*S*,33*S*,36S)-30-(2-(2,5-dioxo-2,5-dihydro-1*H*-pyrrol-1-yl) acetamido)-33-isopropyl-26,31,34-trioxo-36-(3-ureidopropyl)-2,5,8,11,14,17,20,23-octaoxo-27,32,35-triazaheptatriacontan-37-amido)benzyl ((1*S*,9*S*)-9-ethyl-5-fluoro-9-hydroxy-4-methyl-10,13-dioxo-2,3,9,10,13,15-hexahydro-1*H*,12*H*-benzo[*de*]pyrano [3',4':6,7]indoline[1,2-*b*]quinoline-1-yl)carbamate.

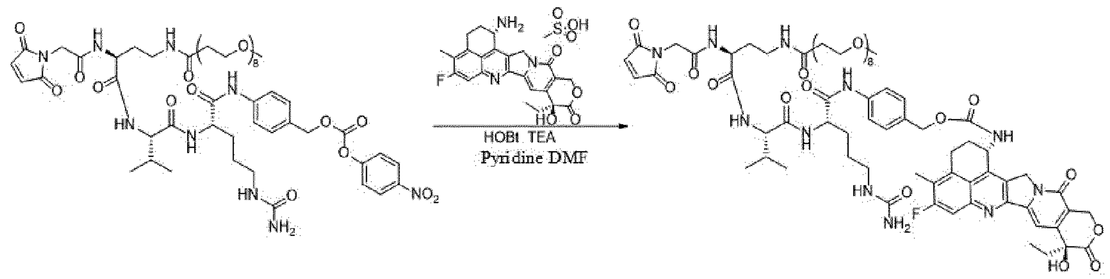


4-((30,*S*,33*S*,36S)-30-(2-(2,5-dioxo-2,5-dihydro-1*H*-pyrrol-l-yl)acetamido)-33-isopropyl-26,31,34-trioxo-36-(3-ureidopropyl)-2,5,8,11,14,17,20,23-octaoxo-27,32,35 -triazaheptatriacontan-37-amido)benzyl (4-nitrophenyl) carbonate (CB07) (2.6 g, 2.21 mmol) and *N,N*-dimethylformamide (23 mL) were added to a reaction flask R1, stirred under a nitrogen atmosphere and cooled to 0-5 °C. At the same time, (1*S*,9*S)*-1-amino-9-ethyl-5-fluoro-9-hydroxy-4-methyl-1,2,3,9,12,15-hexahydro-10*H*, 13*H*-bnzo[3',4':6,7]indoline[1,2-*b*]quinoline-10,13-dione methanesulfonate (Exatecan, 0.98 g, 1.84 mmol, Advanced ChemBlocks) and *N,N*-dimethylformamide (5 mL) were taken and added to another reaction flask R2. Triethylamine (230 mg, 2.27 mmol) was added dropwise at 0-5 °C. The mixture was stirred until complete dissolution was achieved. The solution in reaction flask R2 was added dropwise to reaction flask R1. Reaction flask R2 was then washed with *N,N*-dimethylformamide (2 mL), and the washing solution was added to reaction flask R1. 1-Hydroxybenzotriazole (497 mg, 3.68 mmol) and pyridine (1.45 g, 18.4 mmol) were weighed into reaction flask R1. The mixture was stirred at 0-5 °C for 10 min, warmed to room temperature, and stirred for 5.5 h. After the reaction was completed, the mixture was concentrated at 35 °C under reduced pressure to remove the solvent. The residue was purified by preparative high performance liquid chromatography (prep-HPLC) and lyophilized to obtain a white powder (1.6 g, 59%). LC-MS: $[1/2M+H]^+$= 737.

Example 6. Synthesis of Intermediate (CB07-D-1)

Synthesis of 4-((30*S*,3*3*S,36S)-30-(2-(2,5-dioxo-2,5-dihydro-1*H*-pyrrol-1-yl) acetamido)-33-isopropyl-26,31,34-trioxo-36-(3-ureidopropyl)-2,5,8,11,14,17,20,23-octaoxo-27,32,35-triazaheptatriacontan-37-amido)benzyl ((1*S*,9*S*)-9-ethyl-4,5-difluoro-9-hydroxy-10,13-dioxo-2,3,9,10,13,15-hexahydro-1*H*,12*H*-benzo[*de*]pyrano[ 3',4':6,7]indoline[1,2-*b*]quinoline-1-yl)carbamate

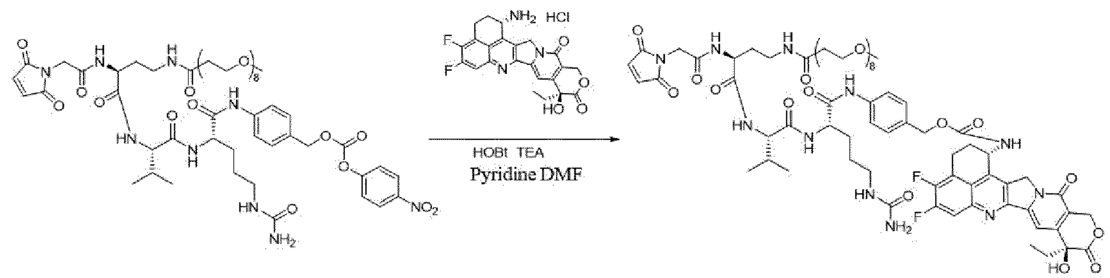


4-((30*S*,33*S*,36S)-30-(2-(2,5-dioxo-2,5-dihydro-1*H*-pyrrol-l-yl)acetamido)-33-isopropyl-26,31,34-trioxo-36-(3-ureidopropyl)-2,5,8,11,14,17,20,23-octaoxo-27,32,35 -triazaheptatriacontan-37-amido)benzyl(4-nitrocarbonate) (CB07) (220 mg, 0.189 mmol) and *N,N*-dimethylformamide (5 mL) were added to a reaction flask R1, stirred under a nitrogen atmosphere and cooled to 0-5 °C. At the same time, (1*S*,9*S*)-1-amino-9-ethyl-4,S-difluoro-9-hydroxy-1,2,3,9,12,15-hexahydro-10*H*,13*H*-b enzo[*de*]pyrano[3',4':6,7]indolo[1,2-*b*]quinoline-10,13-dione hydrochloride (D-1) (90 mg, 0.189 mmol) and *N,N*-dimethylformamide (5 mL) were taken and added to another reaction flask R2. Three drops of triethylamine were added at 0-5 °C. The mixture was stirred until complete dissolution was achieved. The solution in reaction flask R2 was added dropwise to reaction flask R1, and 1-hydroxybenzotriazole (60 mg, 0.44 mmol) and pyridine (0.5 mL) were weighed into reaction flask R1. The mixture was stirred at 0-5 °C for 10 min, warmed to room temperature, and stirred for 3 h. After the reaction was completed, the mixture was concentrated under reduced pressure to remove the solvent. The residue was purified by preparative high performance liquid chromatography (prep-HPLC) and lyophilized to obtain a white powder (55 mg, 20%). LC-MS: $[1/2M+H]^+$ = 739.

Example 7. Synthesis of Intermediate (CB14-Exatecan)

Synthesis of 4-((18*S*,21*S*,24*S*)-18-(2-(2,5-dioxane-2,5-dihydro-1*H*-pyrrol-1-yl) acetamido)-21-isopropyl-14,19,22-trioxo-24-(3-ureidopropyl)-2,5,8,11-tetraoxo-15,20, 23-triazapentacosan-25-amino)-(1*S*,9*S*)-9-ethyl-5-fluoro-9-hydroxy-4-methyl-10,13-dioxo-2,3,9,10,13,15-hexahydro-1*H*,12*H*-benzo[*de*]pyran[3',4':6,7]indoline[1,2-*b*] quinoline-1-carbamate

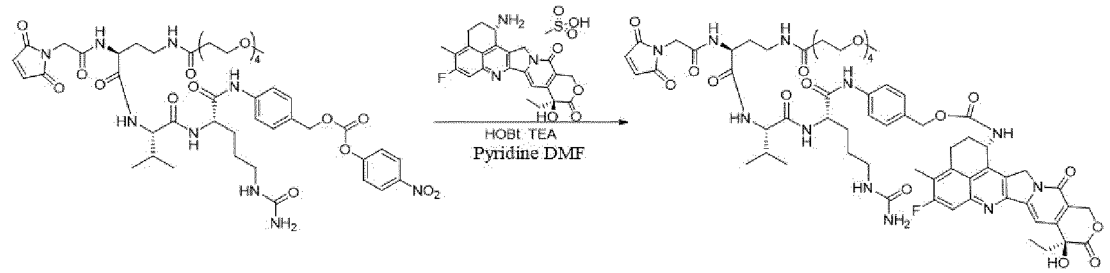


Similarly, 4-((18*S*,21*S*,24*S*)-18-(2-(2,5-dioxo-2,5-dihydro-1*H*-pyrrol-1-yl)acetamido)-21-isopropyl-14,19,22-trioxo-24-(3-ureidopropyl)-2,5,8,11-tetraoxo-15,20,23-triazapentacosan-25-amino)benzyl(4-nitrophenyl)carbonate (190 mg, 0.19 mmol)and *N,N*-dimethylformamide (23 mL) were added to a reaction flask R1, stirred under a nitrogen atmosphere, and cooled to 0-5 °C. At the same time, (1*S*,9*S)*-1-amino-9-ethyl-5-fluoro-9-hydroxy-4-methyl-1,2,3,9,12,15-hexahydro-10*H*, 13*H*-benzo[3',4':6,7]indoline[1,2-*b*]quinoline-10,13-dione methanesulfonate (Exatecan methanesulfonate; 101 mg, 0.19 mmol; Advanced ChemBlocks) and *N*,*N*-dimethylformamide (5 mL) were taken and added to another reaction flask R2. Triethylamine (3 drops) was added dropwise at 0-5 °C. The mixture was stirred until complete dissolution was achieved. The solution in reaction flask R2 was added dropwise to reaction flask R1. Reaction flask R2 was then washed with *N*,*N*-dimethylformamide (1 mL), and the washing solution was added to reaction flask R1. 1-hydroxybenzotriazole (60 mg, 0.44 mmol) and pyridine (0.5 mL) were weighed into reaction flask R1. The mixture was stirred at 0-5 °C for 10 min, warmed to room temperature, and stirred for 5.5 h. After the reaction was completed, the mixture was concentrated at 35 °C under reduced pressure to remove the solvent. The residue was purified by preparative high performance liquid chromatography (prep-HPLC) and lyophilized to obtain a white powder.

Example 8: Synthesis of ADC1

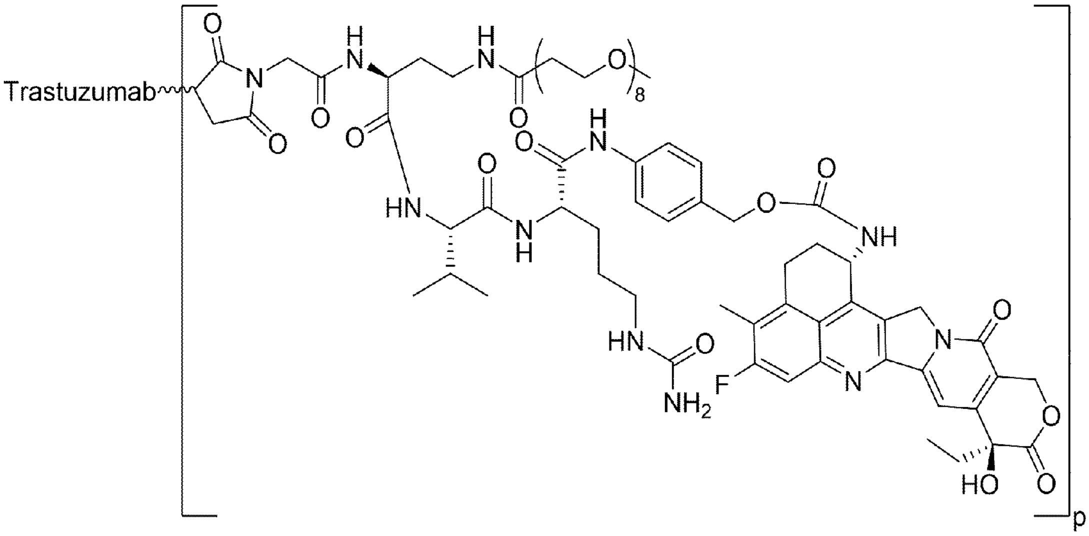

ADC1

4.5 molar equivalents of TCEP (tris(2-carboxyethyl)phosphine) was added to Trastuzumab according to the

amount of the antibody substance, and then the system was adjusted to pH 8 with 1 M Tris base. The system was incubated at 25 °C for 1.5 h for reduction. TCEP was removed by ultrafiltration for buffer exchange using 10 mM succinic acid. The sulfhydryl antibody value was determined by absorbance measurement, and the sulfhydryl concentration was determined by reacting sulfhydryl with DTNB (5,5'-dithiobis(2-nitrobenzoic acid), Aldrich) and then measuring the absorbance at 412 nm.

In the conjugation reaction, 12 molar equivalents of CB07-Exatecan was added according to the amount of the antibody substance, and after stirring at 25 °C for 1 h, 0.1 M N-acetylcysteine was added until a final concentration of 2 mM was reached. The mixture was stirred for another 15 min, and the reaction was terminated. The reaction mixture was filtered through a 0.22 micron filter and eluted through Sephadex G-25 resin with 10 mM succinic acid for exchange.

The final concentration of ADC1 obtained was 7.5 mg/mL, and the DAR, i.e., p, was 8 as measured by reversed-phase chromatography.

Example 9. Synthesis of ADC2

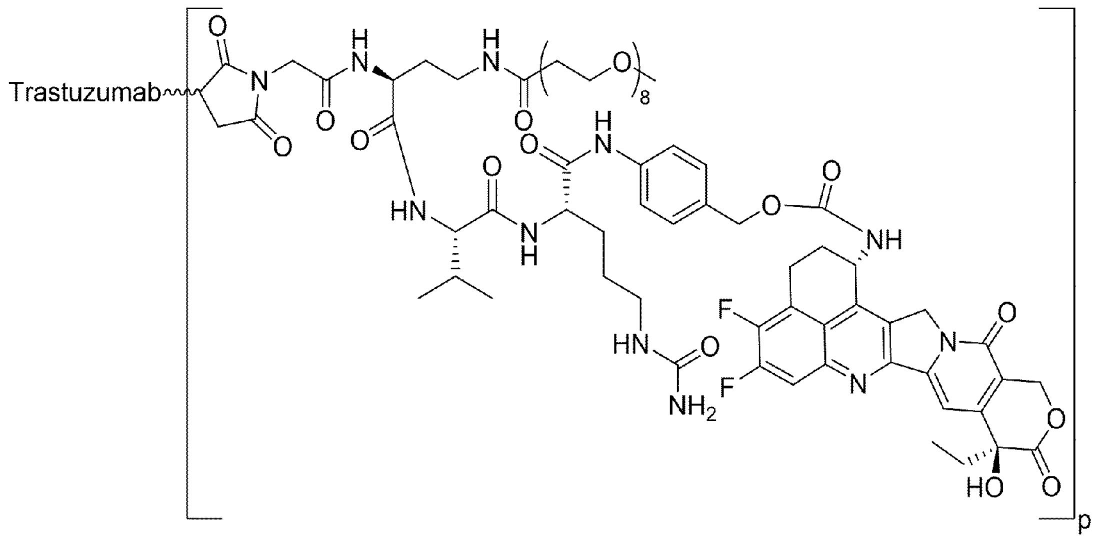

ADC2

To Trastuzumab was added 4.5 molar equivalents of TCEP according to the amount of the antibody substance, and then the system was adjusted to pH 8 with 1 M Tris base. The system was incubated at 25 °C for 1.5 h for reduction. TCEP was removed by ultrafiltration for buffer exchange using 10 mM succinic acid. The sulfhydryl antibody value was determined by absorbance measurement, and the sulfhydryl concentration was determined by reacting sulfhydryl with DTNB (5,5'-dithiobis(2-nitrobenzoic acid), Aldrich) and then measuring the absorbance at 412 nm.

In the conjugation reaction, 12 molar equivalents of CB07-D-1 was added according to the amount of the antibody substance, and after stirring at 25 °C for 2 h, 0.1 M N-acetylcysteine was added until a final concentration of 2 mM was reached. The mixture was stirred for another 15 min, and the reaction was terminated. The reaction mixture was filtered through a 0.22 micron filter and eluted through Sephadex G-25 resin with 10 mM succinic acid for exchange.

The concentration of ADC2 was 3.61 mg/mL, and the DAR, i.e., p, was 7.4 as measured by reversed-phase chromatography.

Example 10. Synthesis of ADC3 (Control Drug)

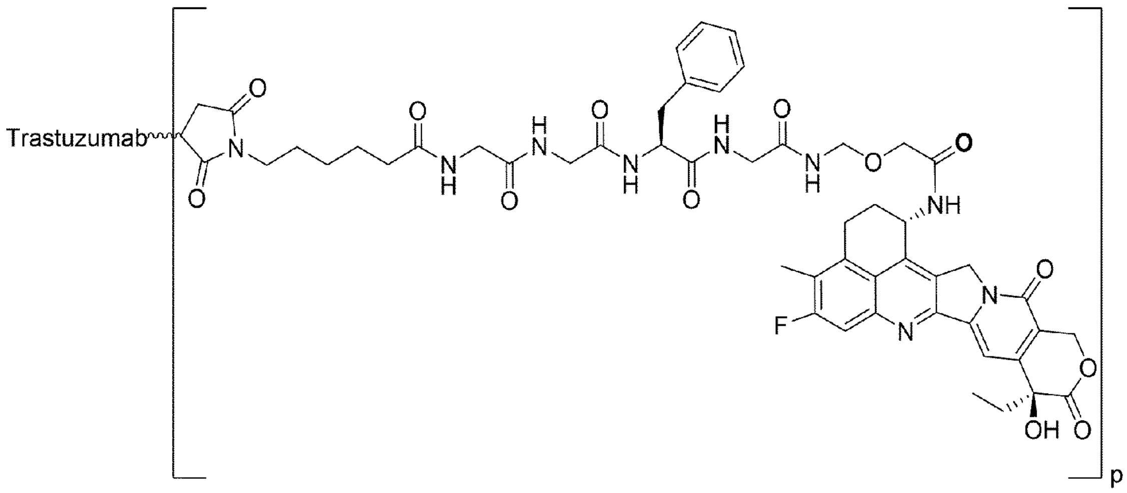

ADC3

According to the amount of the antibody substance, to Trastuzumab was added 4.5 molar equivalents of TCEP, and then the system was adjusted to pH 8 with 1 M Tris base. The system was incubated at 25 °C for 1.5 h for reduction. TCEP was removed by ultrafiltration for buffer exchange using 10 mM succinic acid. The sulfhydryl antibody value was determined by absorbance measurement, and the sulfhydryl concentration was determined by reacting sulfhydryl with DTNB (5,5'-dithiobis(2-nitrobenzoic acid), Aldrich) and then measuring the absorbance at 412 nm.

In the conjugation reaction, 12 molar equivalents of Deuxtecan (MCE, Cat.#HY-13631E) was added according to the amount of the antibody substance, and after stirring at 25 °C for 2 h, 0.1 M N-acetylcysteine was added until a final concentration of 2 mM was reached. The mixture was stirred for another 15 min, and the reaction was terminated. The reaction mixture was filtered through a 0.22 micron filter and eluted through Sephadex G-25 resin with 10 mM succinic acid for exchange.

The concentration of ADC3 was 4.19 g/L, and the DAR, i.e., p, was 7.6 as measured by RP-HPLC.

Example 11. Synthesis of ADC4

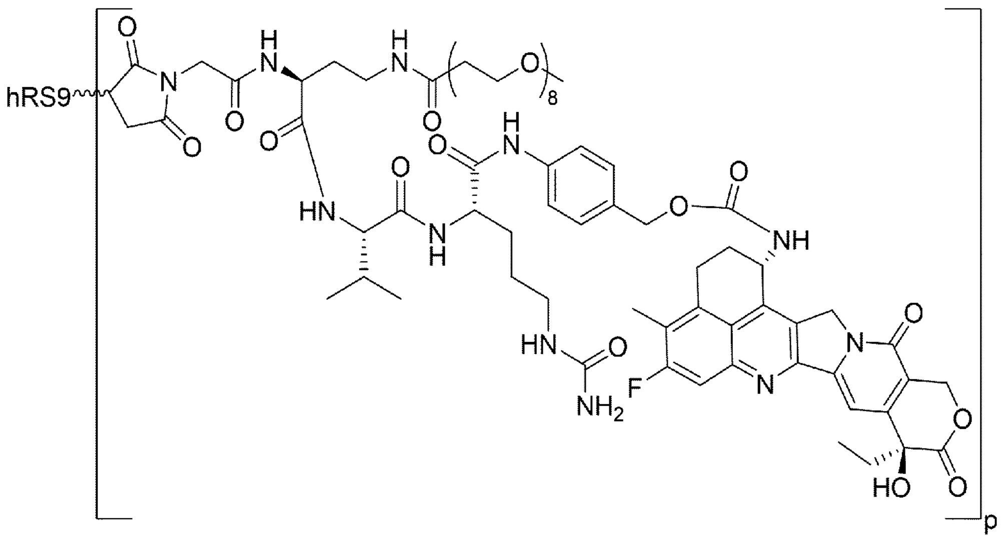

ADC4

To hRS9 antibody was added 2.7 molar equivalents of TCEP according to the amount of the antibody substance, and then the system was adjusted to pH 7 with 1 M Tris base. The system was incubated at 25 °C for 2 h for reduction. TCEP was removed by ultrafiltration for buffer exchange using 10 mM succinic acid. The sulfhydryl antibody value was

determined by absorbance measurement, and the sulfhydryl concentration was determined by reacting sulfhydryl with DTNB (5,5'-dithiobis(2-nitrobenzoic acid), Aldrich) and then measuring the absorbance at 412 nm.

In the conjugation reaction, 6 molar equivalents of CB07-Exatecan was added according to the amount of the antibody substance, and after stirring at 25 °C for 2 h, 0.1 M N-acetylcysteine was added until a final concentration of 2 mM was reached. The mixture was stirred for another 15 min, and the reaction was terminated. The reaction mixture was filtered through a 0.22 micron filter and eluted through Sephadex G-25 resin with 10 mM succinic acid for exchange.

The protein concentration of ADC4 was 8.46 mg/mL, and the DAR, i.e., p, was 4.2 as measured by RP-HPLC.

Example 12. Synthesis of ADC5

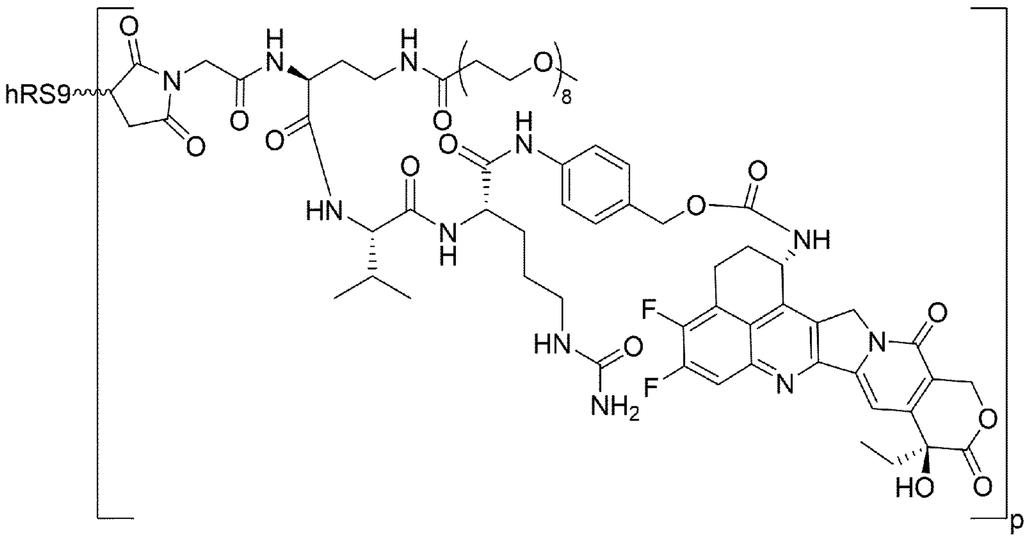

ADC5

To hRS9 antibody was added 2.7 molar equivalents of TCEP according to the amount of the antibody substance, and then the system was adjusted to pH 7 with 1 M Tris base. The system was incubated at 25 °C for 2 h for reduction. TCEP was removed by ultrafiltration for buffer exchange using 10 mM succinic acid. The sulfhydryl antibody value was determined by absorbance measurement, and the sulfhydryl concentration was determined by reacting sulfhydryl with DTNB (5,5'-dithiobis(2-nitrobenzoic acid), Aldrich) and then measuring the absorbance at 412 nm.

In the conjugation reaction, 6 molar equivalents of CB07-D-1 was added according to the amount of the antibody substance, and after stirring at 25 °C for 2 h, 0.1 M N-acetylcysteine was added until a final concentration of 2 mM was reached. The mixture was stirred for another 15 min, and the reaction was terminated. The reaction mixture was filtered through a 0.22 micron filter and eluted through Sephadex G-25 resin with 10 mM succinic acid for exchange.

The protein concentration of ADC5 was 6.5 mg/mL, and the DAR, i.e., p, was 4.8 as measured by RP-HPLC.

Example 13. Synthesis of ADC6

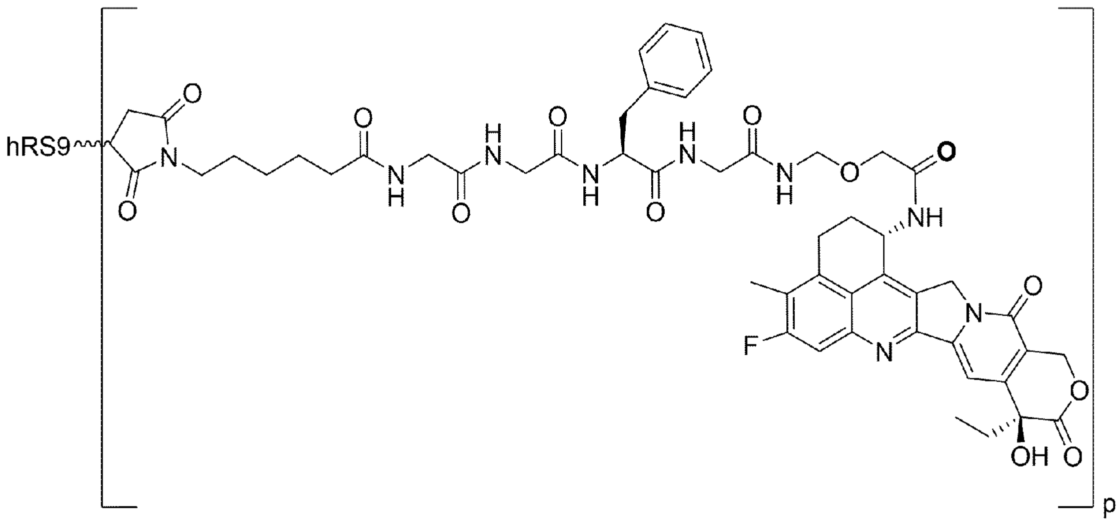

ADC6

To hRS9 antibody was added 2.7 molar equivalents of TCEP according to the amount of the antibody substance, and then the system was adjusted to pH 7 with 1 M Tris base. The system was incubated at 25 °C for 2 h for reduction. TCEP was removed by ultrafiltration for buffer exchange using 10 mM succinic acid. The sulfhydryl antibody value was determined by absorbance measurement, and the sulfhydryl concentration was determined by reacting sulfhydryl with DTNB (5,5'-dithiobis(2-nitrobenzoic acid), Aldrich) and then measuring the absorbance at 412 nm.

In the conjugation reaction, 6 molar equivalents of MC-GGFG-Dxd (Deruxtecan, MCE, Cat.#HY-13631E/CS-0045125) was added according to the amount of the antibody substance, and after stirring at 25 °C for 2 h, 0.1 M N-acetylcysteine was added until a final concentration of 2 mM was reached. The mixture was stirred for another 15 min, and the reaction was terminated. The reaction mixture was filtered through a 0.22 micron filter and eluted through Sephadex G-25 resin with 10 mM succinic acid for exchange.

The protein concentration of ADC6 was 2.83 mg/mL, and the DAR, i.e., p, was 4.6 as measured by RP-HPLC.

Example 14. Synthesis of ADC7

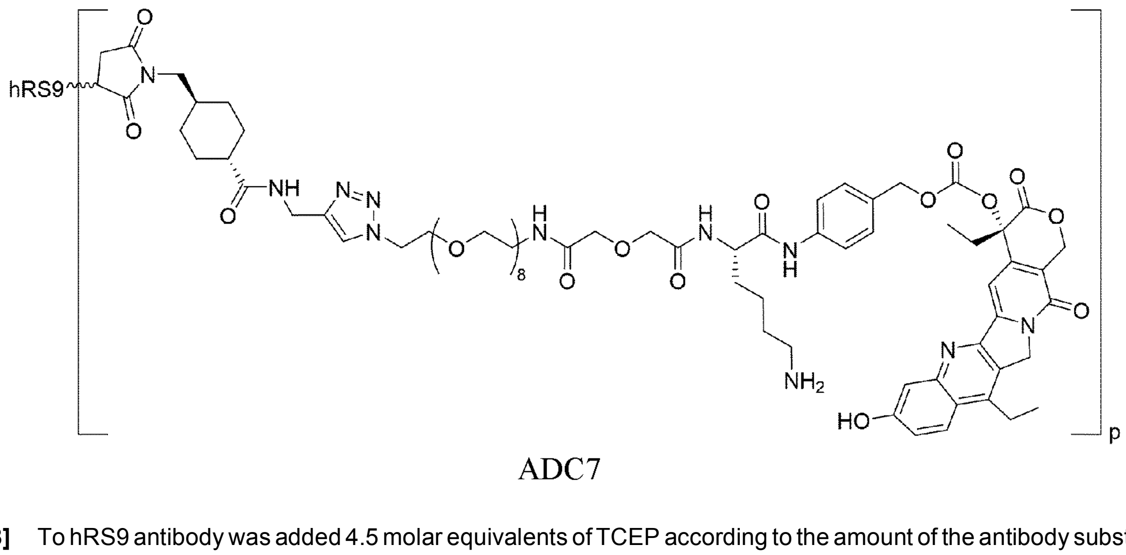

ADC7

To hRS9 antibody was added 4.5 molar equivalents of TCEP according to the amount of the antibody substance, and then the system was adjusted to pH 8 with 1 M Tris base. The system was incubated at 25 °C for 2 h for reduction. TCEP was removed by ultrafiltration for buffer exchange using 10 mM succinic acid. The sulfhydryl antibody value was determined by absorbance measurement, and the sulfhydryl concentration was determined by reacting sulfhydryl with DTNB (5,5'-dithiobis(2-nitrobenzoic acid), Aldrich) and then measuring the absorbance at 412 nm.

In the conjugation reaction, 12 molar equivalents of CL2A-SN38 (see patent US2018161440A1 for details) was

added according to the amount of the antibody substance, and after stirring at 25 °C for 2 h, 0.1 M N-acetylcysteine was added until a final concentration of 2 mM was reached. The mixture was stirred for another 15 min, and the reaction was terminated. The reaction mixture was filtered through a 0.22 micron filter and eluted through Sephadex G-25 resin with 10 mM succinic acid for exchange.

The protein concentration of ADC7 was 6.79 mg/mL, and the DAR, i.e., p, was about 8.3 as measured by RP-HPLC.

Example 15. Synthesis of ADC8

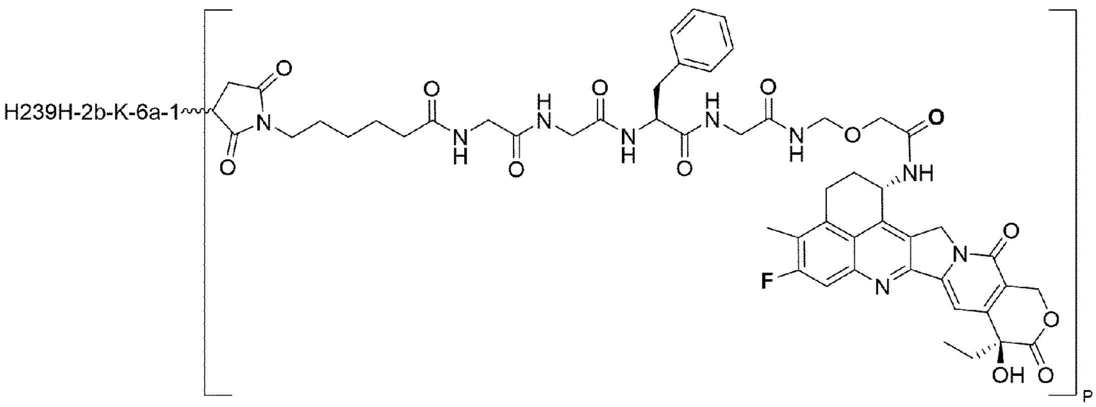

ADC8

To 5ml of 11.5mg/ml H239H-2b-K-6a-1 antibody was added 100μL of 0.1M EDTA aqueous solution and 2.7 molar equivalent of 10mM TCEP aqueous solution according to the amount of antibody substance, and reacted in a thermostat at 25 °C for 2h. Ultrafiltration to removed the reducing agent TCEP from the system. Subsequently, according to the amount of the antibody substance, 6 molar equivalents of 10 mM MC-GGFG-DXD (Deruxtecan, MCE, Cat. #HY-13631E/CS-0045125) dimethylacetamide (DMA) solution was added for conjugating, and organic solvent DMA was added until its volume accounted for 20% of the conjugating system. After reacting in a thermostat at 25 °C for 2h, 0.1M NAC (N-acetylcysteine) aqueous solution was added to a final concentration of 1.5mM, and terminated the reaction after 15 minutes of reaction.

After purification, the DAR, i.e., p, of ADC8 was about 3.43 as measured by RP-UPLC.

Example 16. Synthesis of ADC9

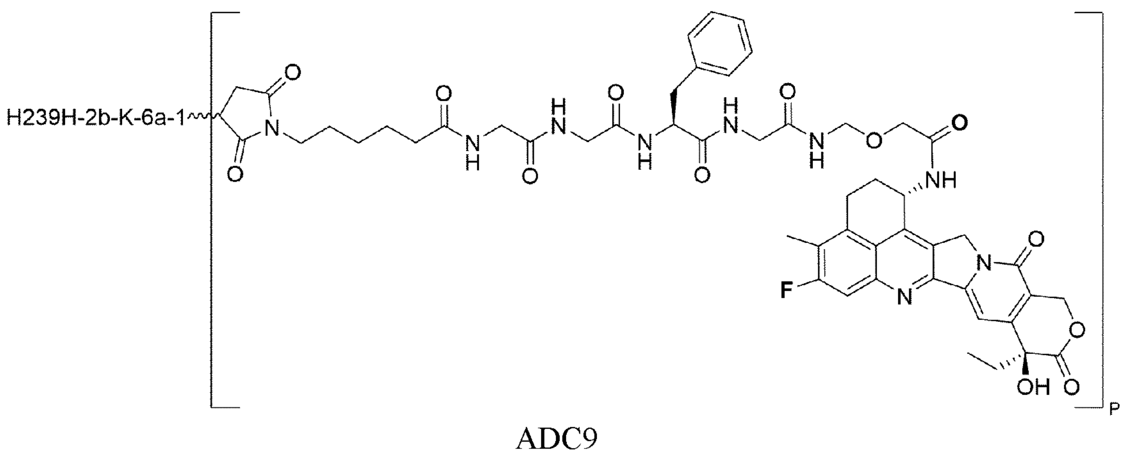

ADC9

To 5ml of 11.5mg/ml H239H-2b-K-6a-1 antibody was added 100μL of 0.1M EDTA aqueous solution and 7 molar equivalent of 10mM TCEP aqueous solution according to the amount of antibody substance, and reacted in a thermostat at 25 °C for 2h. Ultrafiltration to removed the reducing agent TCEP from the system. Subsequently, according to the amount of the antibody substance, 12 molar equivalents of 10 mM MC-GGFG-DXD (Deruxtecan, MCE, Cat. #HY-13631E/CS-0045125) dimethylacetamide (DMA) solution was added for conjugating, and organic solvent DMA was added until its volume accounted for 20% of the conjugating system. After reacting in a thermostat at 25 °C for 2h, 0.1M NAC (N-acetylcysteine) aqueous solution was added to a final concentration of 1.5mM, and terminated the reaction after 15 minutes of reaction.

After purification, the DAR, i.e., p, of ADC9 was about 7.67 as measured by RP-UPLC.

Example 17. Synthesis of ADC10

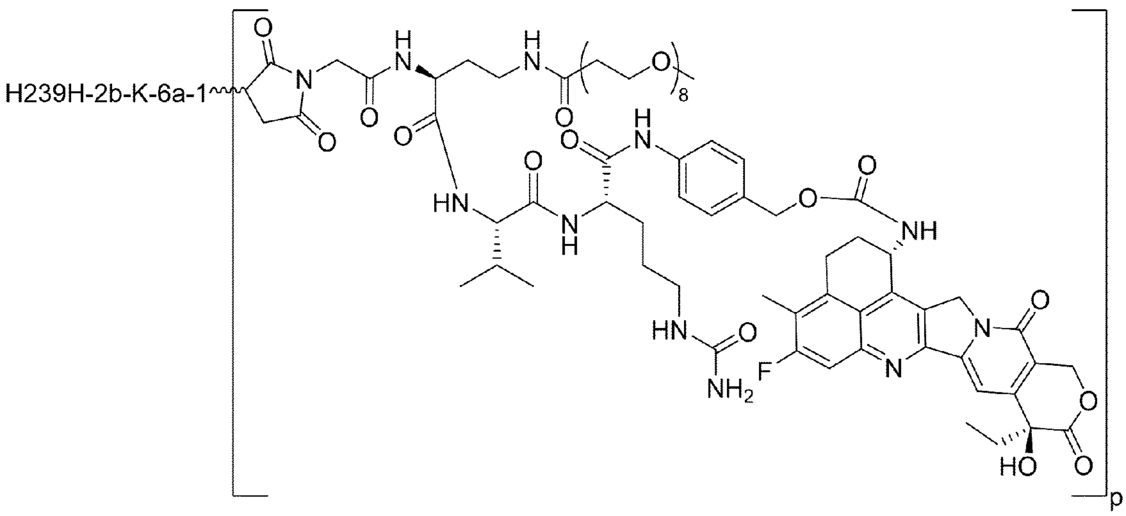

ADC10

To 5ml of 11.5mg/ml H239H-2b-K-6a-1 antibody was added 100μL of 0.1M EDTA aqueous solution and 2.7 molar equivalent of 10mM TCEP aqueous solution according to the amount of antibody substance, and reacted in a thermostat at 25 °C for 2h. Ultrafiltration to removed the reducing agent TCEP from the system. Subsequently, according to the amount of the antibody substance, 6 molar equivalents of 10 mM CB07-Exatecan dimethylacetamide (DMA) solution was added for conjugating, and organic solvent DMA was added until its volume accounted for 20% of the conjugating system. After reacting in a thermostat at 25 °C for 2h15min, 0.1M NAC (N-acetylcysteine) aqueous solution was added to a final concentration of 1.5mM, and terminated the reaction after 15 minutes of reaction.

After purification, the DAR, i.e., p, of ADC10 was about 4.05 as measured by RP-UPLC.

Example 18. Synthesis of ADC11

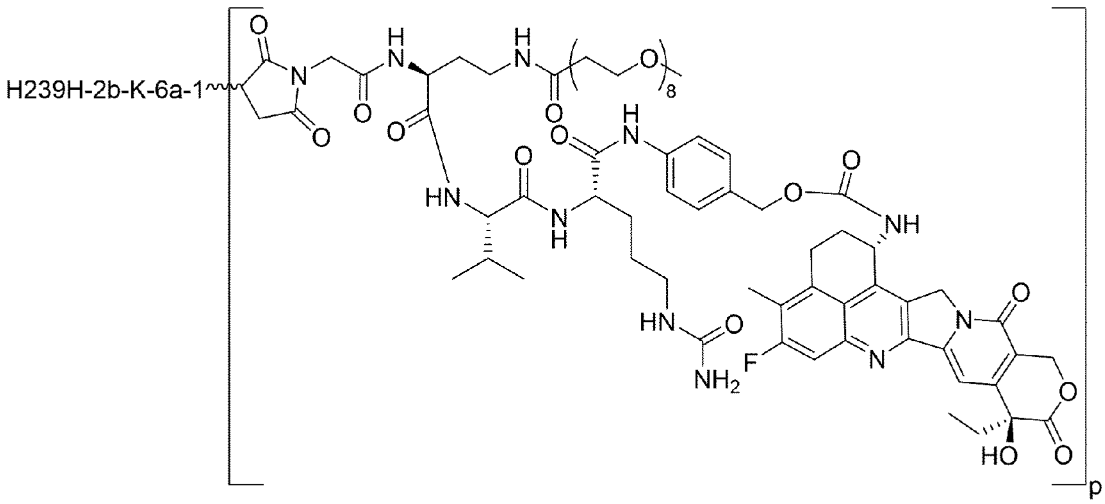

ADC11

To 15.2ml of 11.5mg/ml H239H-2b-K-6a-1 antibody was added 326 μL of 0.1M EDTA aqueous solution and 7 molar equivalent of 10mM TCEP aqueous solution according to the amount of antibody substance, and reacted in a thermostat at 25 °C for 2h. Ultrafiltration to removed the reducing agent TCEP from the system. Subsequently, according to the amount of the antibody substance, 12 molar equivalents of 10 mM CB07-Exatecan dimethylacetamide (DMA) solution was added for conjugating, and organic solvent DMA was added until its volume accounted for 20% of the conjugating system. After reacting in a thermostat at 25 °C for 2.5h, 0.1M NAC (N-acetylcysteine) aqueous solution was added to a final concentration of 2mM, and terminated the reaction after 15 minutes of reaction.

After purification, the DAR, i.e., p, of ADC11 was about 7.49 as measured by RP-UPLC.

Example 19. Synthesis of ADC12

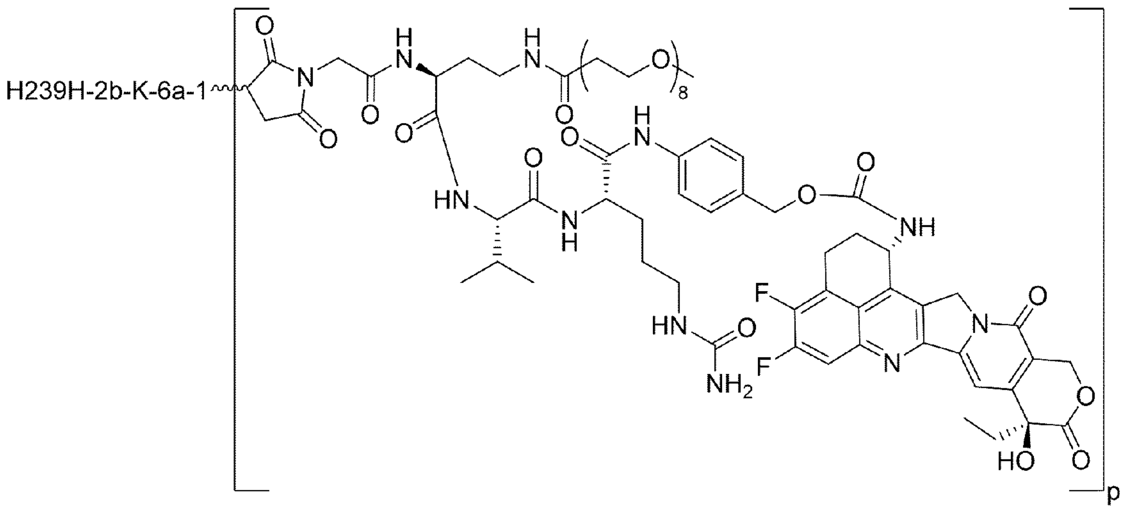

ADC12

To 2.5ml of 11.5mg/ml H239H-2b-K-6a-1 antibody was added 50μL of 0.1M EDTA aqueous solution and 6 molar equivalent of 15mM TCEP aqueous solution according to the amount of antibody substance, and reacted in a thermostat at 25 °C for 2h. Ultrafiltration to removed the reducing agent TCEP from the system. Subsequently, according to the amount of the antibody substance, 12 molar equivalents of 10 mM CB07-D-1 dimethylacetamide (DMA) solution was added for conjugating, and organic solvent DMA was added until its volume accounted for 20% of the conjugating system. After reacting in a thermostat at 25 °C for 2.5h, 0.1M NAC (N-acetylcysteine) aqueous solution was added to a final concentration of 2mM, and terminated the reaction after 15 minutes of reaction.

After purification, the DAR, i.e., p, of ADC12 was about 7.65 as measured by RP-UPLC.

Example 20. Synthesis of ADC13

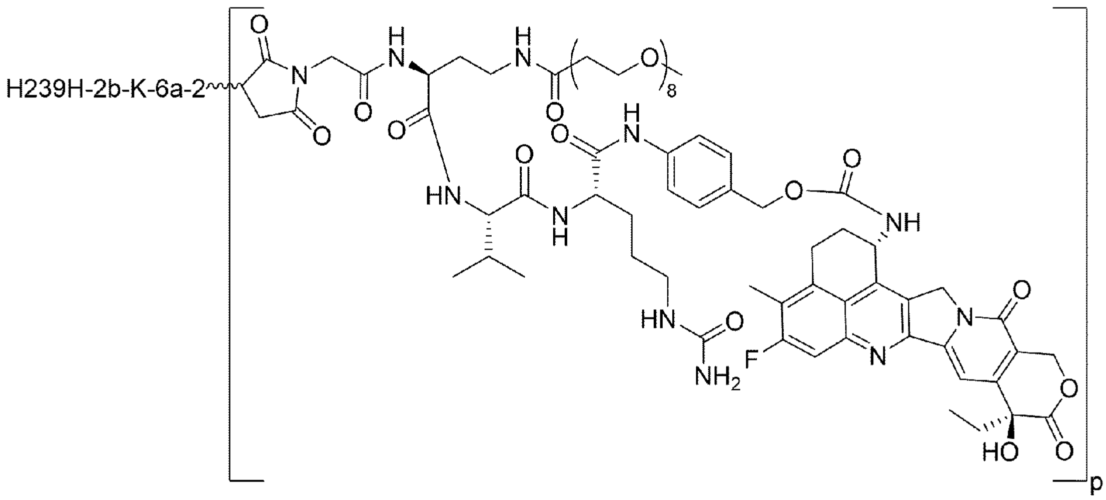

ADC13

To 3ml of 7.89mg/ml H239H-2b-K-6a-2 antibody was added 60 μL of 0.1M EDTA aqueous solution and 7 molar equivalent of 10mM TCEP aqueous solution according to the amount of antibody substance, and reacted in a thermostat at 25 °C for 2h. Ultrafiltration to removed the reducing agent TCEP from the system. Subsequently, according to the amount of the antibody substance, 15 molar equivalents of 100 mM CB07-Exatecan dimethylacetamide (DMA) solution was added for conjugating. After reacting in a thermostat at 25 °C for 2h, 0.1M NAC (N-acetylcysteine) aqueous solution was added to a final concentration of 2mM, and terminated the reaction after 15 minutes of reaction.

After purification, the DAR, i.e., p, of ADC13 was about 7.19 as measured by RP-UPLC.

Example 21. Synthesis of ADC14

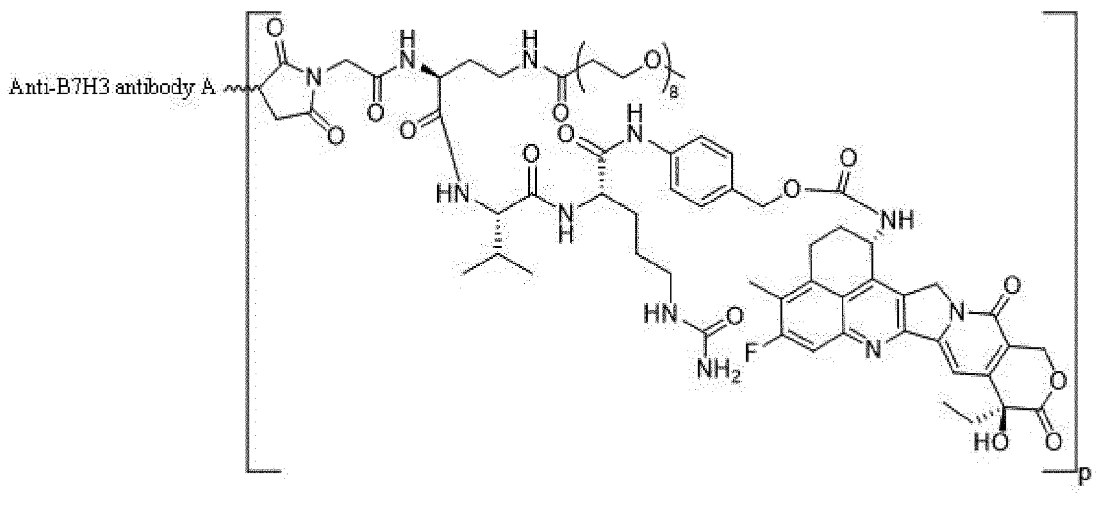

ADC14

10 mM aqueous succinic acid solution was added to 7.0 L of 23.9 g/L anti-B7H3 antibody A solution to adjust the concentration of the antibody A to 18 g/L, thus obtaining an antibody solution. 0.1 M aqueous EDTA solution was

added until the final concentration of EDTA reached 2 mM, the pH was adjusted to 7, and then 5.4 molar equivalents of 0.2 M aqueous TCEP (tris(2-carboxyethyl)phosphine) solution (30.2 mL) was added according to the amount of the antibody substance. The system was stirred at 25 °C and 180 rpm for 2 h and then buffer exchanged by ultrafiltration for 10 volumes using a 10 mM aqueous succinic acid solution and an ultrafiltration membrane with a molecular weight cut-off of 30 KD to remove TCEP.

In the conjugation reaction, 15-fold molar equivalents of a 100 mM solution of CB07-Exatecan in dimethylacetamide (DMA) was added according to the amount of the antibody substance. The system was stirred at 25 °C and 180 rpm for 2 h, and then a 0.4 M aqueous N-acetylcysteine solution was added until its final concentration reached 2 mM. The system was then stirred for 15 min to terminate the conjugation reaction. The reaction mixture was filtered through a 0.22 μ m filter and eluted through Sephadex G-25 resin with 10 mM aqueous succinic acid solution for exchange.

The final concentration of ADC14 obtained was 18.8 mg/mL, and the DAR, i.e., p, was 8.0 as measured by reversed-phase chromatography.

Example 22. Synthesis of ADC15(Control Drug)

0.1 M aqueous EDTA solution was added to 5 mL of 3.08 g/L anti-B7H3 antibody A solution until the final concentration of EDTA reached 2 mM, 2.7 molar equivalents of 10 mM aqueous TCEP (tris(2-carboxyethyl)phosphine) solution (27.7 μL) was added according to the amount of the antibody substance, and then the pH was adjusted to 7. The system was shaken on a shaker at 25 °C for 2 h and then buffer exchanged by ultrafiltration for 10 volumes using a 10 mM aqueous succinic acid solution and an ultrafiltration membrane with a molecular weight cut-off of 30 KD to remove TCEP.

In the conjugation reaction, 6-fold molar equivalents of MC-GGFG-DXD (MedChemExpress, Lot#41557, CAS: 1599440-13-7, product name: Deruxtecan) was added according to the amount of the antibody substance. The system was shaken on a shaker at 25 °C for 2 h, and then a 0.1 M aqueous N-acetylcysteine solution was added until its final concentration reached 1.5 mM. The system was then shaken for 15 min to terminate the conjugation reaction. The reaction mixture was filtered through a 0.22 μm filter and eluted through Sephadex G-25 resin with 10 mM aqueous succinic acid solution for exchange.

The final concentration of ADC15 obtained was 6.3 mg/mL, and the DAR, i.e., p, was 3.7 as measured by reversed-phase chromatography.

Example 23. Synthesis of ADC 16

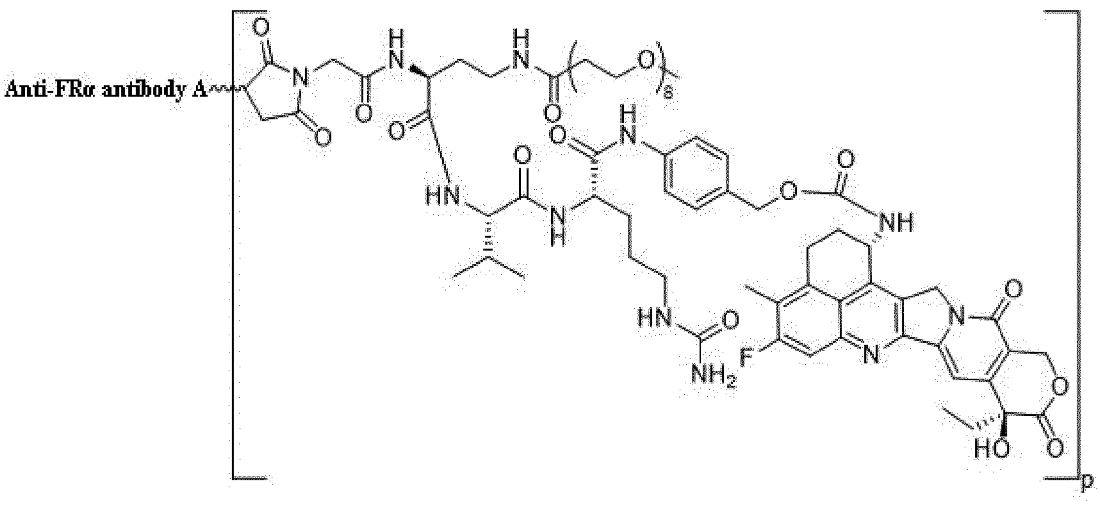

ADC16

Using conjugation buffer (10mM succinic acid aqueous solution), the concentration of anti-FRα antibody A was adjusted to 18g/L, 0.1 M aqueous EDTA solution was added until the final concentration of EDTA reached 2 mM, and 5.4 molar equivalents of 0.2 mM aqueous TCEP was added according to the amount of the antibody substance, and then the pH was adjusted to 7. The system was stirred at 25 °C and 180 rpm for 2 h, so that the disulfide bonds between

the antibody chains were reduced to free thiol groups. The buffer exchanged by ultrafiltration for 10 volumes using a 30 KD ultrafiltration membrane to remove agent TCEP in the system.

15-fold molar equivalents of a 100 mM solution of CB07-Exatecan in dimethylacetamide (DMA) was added according to the amount of the antibody substance. The system was stirred at 25 °C and 180 rpm for 2 h, and then a 0.2 M aqueous N-acetylcysteine solution was added until its final concentration reached 2 mM. The system was then stirred for 15 min to terminate the conjugation reaction. The reaction mixture was purified and ultrafiltered to obtain ADC16 with a concentration of 23.56mg/mL, and the DAR, i.e., p, was 8 as measured by reversed-phase chromatography.

Example 24. Synthesis of ADC17

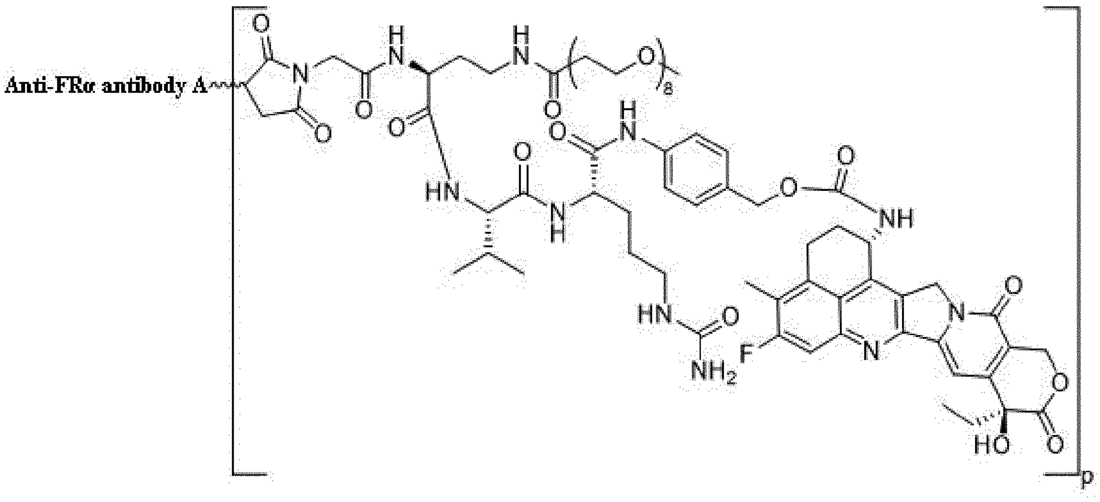

ADC17

Using conjugation buffer (10mM succinic acid aqueous solution), the concentration of anti-FRα antibody A was adjusted to 18g/L, 0.1 M aqueous EDTA solution was added until the final concentration of EDTA reached 2 mM, and 2.5 molar equivalents of 10 mM aqueous TCEP was added according to the amount of the antibody substance, and then the pH was adjusted to 7. The system was shaken on a shaker at 25 °C for 2 h , so that the disulfide bonds between the antibody chains were reduced to free thiol groups. The buffer exchanged by ultrafiltration for 10 volumes to remove agent TCEP in the system.

6-fold molar equivalents of a 100 mM solution of CB07-Exatecan in dimethylacetamide (DMA) was added according to the amount of the antibody substance. The system was shaken on a shaker at 25 °C for 2 h , and then a 0.1 M aqueous N-acetylcysteine solution was added until its final concentration reached 1.5 mM. The system was then shaken for 15 min to terminate the conjugation reaction. The reaction mixture was purified to obtain ADC17 with a concentration of 1.34mg/mL, and the DAR, i.e., p, was 4 as measured by reversed-phase chromatography.

Activity Examples

Example 1

*In Vitro* Bioactivity of ADC 1

The inhibition of the growth of tumor cells by ADC1 antibody was assessed using HER2 positive breast tumor cell lines NCI-N87, MDA-MB-453, SK-BR-3 and BT474, as well as HER2 negative cell line MDA-MB-468 (purchased from Cell Bank of Chinese Academy of Sciences, Shanghai Institutes for Biological Sciences). Briefly, the cells were dissociated by digestion with trypsin after growing in the logarithmic phase and then suspended in 100 μL of complete medium. 4000-8000 cells were seeded in a 96-well plate for culture at 37 °C for 3-5 h or overnight, adhering to the walls. Then 100 μL of media with different concentrations of ADC1 were added. After 120 h, the media in the Petri dish was removed, and relative cell proliferation analysis was performed using cell counting kit-8 (CCK-8, Dojindo, Japan) reagent. The results show that ADC1 has a growth inhibition effect on all of the HER2 positive cells, and the growth inhibition

effect $EC_{50}$ on the negative cells MDA-MB-468 > 10000 ng/mL.

| Cell line | $EC_{50}$ (ng/mL) ADC1 |
|---|---|
| NCI-N87 | 82 |
| MDA-MB-453 | 36 |
| SK-BR-3 | 43 |
| BT474 | 62 |
| MDA-MB-468 | >10000 |

Example 2

*In Vitro* Bioactivity of ADC4

The inhibition of the growth of tumor cells by ADC4 antibody was assessed using Trop2 positive breast tumor cell lines MDA-MB-453, MDA-MB-468 and MX-1, as well as Trop2 negative cell line HGC27 (purchased from Cell Bank of Chinese Academy of Sciences, Shanghai Institutes for Biological Sciences). Briefly, the cells were dissociated by digestion with trypsin after growing in the logarithmic phase and then suspended in 100 μL of complete medium. 4000-8000 cells were seeded in a 96-well plate for culture at 37 °C for 3-5 h or overnight, adhering to the walls. Then 100 μL of media with different concentrations of ADC4 were added. After 120 h, the media in the Petri dish was removed, and relative cell proliferation analysis was performed using cell counting kit-8 (CCK-8, Dojindo, Japan) reagent. The results show that ADC4 has a growth inhibition effect on all of the Trop2 positive cells, and the growth inhibition effect $EC_{50}$ on the negative cells HGC27 > 15000 ng/mL.

| Cell line | $EC_{50}$ (ng/mL) ADC4 |
|---|---|
| MX-1 | 233 |
| MDA-MB-468 | 157.8 |
| MDA-MB-453 | 640 |
| HGC27 | >15000 |

Example 3

1. Construction of CHO-CLDN18.2 stable cell line

The full-length amino acid sequence of human CLDN18.2 (from NCBI, NM_001002026.3) is as follows:

MAVTACQGLGFVVSLIGIAGIIAATCMDQWSTQDLYNNPVTAVFNYQGLWR
SCVRESSGFTECRGYFTLLGLPAMLQAVRALMIVGIVLGAIGLLVSIFALKCIRI
GSMEDSAKANMTLTSGIMFIVSGLCAIAGVSVFANMLVTNFWMSTANMYTG
MGGMVQTVQTRYTFGAALFVGWVAGGLTLIGGVMMCIACRGLAPEETNYK
AVSYHASGHSVAYKPGGFKASTGFGSNTKNKKIYDGGARTEDEVQSYPSKH
DYV（SEQ ID NO: 25）.

The corresponding nucleic acid sequence is as follows:

atggccgtgactgcctgtcagggcttggggttcgtggtttcactgattgggattgcgggcatcattgctgccacctgcatgga
ccagtggagcacccaagacttgtacaacaaccccgtaacagctgttttcaactaccaggggctgtggcgctcctgtgtccg
agagagctctggcttcaccgagtgccggggctacttcaccctgctggggctgccagccatgctgcaggcagtgcgagcc
ctgatgatcgtaggcatcgtcctgggtgccattggcctcctggtatccatctttgccctgaaatgcatccgcattggcagcatg
gaggactctgccaaagccaacatgacactgacctccgggatcatgttcattgtctcaggtctttgtgcaattgctggagtgtct
gtgtttgccaacatgctggtgactaacttctggatgtccacagctaacatgtacaccggcatgggtgggatggtgcagactgt
tcagaccaggtacacatttggtgcggctctgttcgtgggctgggtcgctggaggcctcacactaattgggggtgtgatgatg
tgcatcgcctgccggggcctggcaccagaagaaaccaactacaaagccgtttcttatcatgcctcaggccacagtgttgcc
tacaagcctggaggcttcaaggccagcactggctttgggtccaacaccaaaaacaagaagatatacgatggaggtgcccg
cacagaggacgaggtacaatcttatccttccaagcacgactatgtg （SEQ ID NO: 26）

The nucleic acid sequence corresponding to human CLDN18.2 described above was synthesized, and sequences of enzyme digestion sites HindIII and EcoRI were added at both ends of the sequence. The sequence was then constructed into the pcDNA3.1 expression vector (Invitrogen, Cat. No. V79020). The expression vector was transfected into CHO cells (Life technologies, Cat. No. A13696-01) by electroporation. The electroporation conditions were as follows: voltage 300 V, time 17 ms, and 4 mm electroporation cuvette. After 48 h, 50 μM MSX (methionine iminosulfone) was added as a screening pressure, and positive cells were selected after 2 weeks. High-expression cell lines were selected by FACS. The cells were collected and washed once with PBS (phosphate-buffered saline, 1 L of PBS containing 8.0 g of NaCl, 0.9 g of $Na_2HPO_4$, 0.156 g of $KH_2PO_4$, and 0.125 g of KCl; pH 7.2-7.4). 3 μg/mL anti-CLDN18.2 antibody (IMAB362, the sequence of which is the same as that of the antibody expressed by hybridoma cells 175D10 in Patent No. US20090169547), was added, and the mixture was incubated at 4 °C for 1 h and washed twice with PBS. 100 μL of 1:500 diluted goat anti-human IgG-Fc PE fluorescent secondary antibody (Cat. No. 12-4998-82, eBioscience) was added, and the mixture was incubated at 4 °C for 1 h, washed twice with PBS, and analyzed by a C6 flow cytometer (BD, model: C6 flow cytometer). The cells obtained were named CHO-CLDN18.2 cells, and the FACS results are shown in FIG. 1.

2.Proliferation inhibition experiments of ADC8, ADC10, ADC11 and ADC12

We evaluated the cells growth inhibition by the anti-CLDN18.2 ADC using the CHO-CLDN18.2 cells.The CHO-CLDN18.2 cells in the logarithmic growth phase were collected, centrifuged at 800 rpm for 5 minutes, removed the supernatant, washed once with CD-CHO-AGT (Cat. No. 12490; life technologies) culture medium, centrifuged at 800 rpm for 5 minutes again, and removed the supernatant. The cells were resuspend with 0.5% FBS (Cat. No. FSP500; Excell Bio) CD-CHO-AGT medium, and the cells density were adjusted to $5\times10^4$ cells/ml, 100 μl/well were spread in 96-well cell culture plate (Cat. No. 3599; costar). 100 μl of ADC diluted with 0.5% FBS CD-CHO-AGT gradient was added to each well, and the final concentrations of ADC8, ADC10, ADC11, and ADC12 were: 500, 250, 125, 62.5, 31.25, 15.625, 7.813, 3.9, 1.95 nM. After 5 days of culture in a 37 degree 5% $CO_2$ incubator, 20 μl of CCK-8 (Cat. No. CK04, DojinDo) was added to each well. After incubating for 1 hour at 37 °C, the OD450 nm absorbance value was measured using a microplate reader (SpectraMax M3, Molecular Devices) to calculate the cell inhibition rate. Inhibition rate% = (1-absorbance value of sample well/absorbance value of control well) ×100.

As shown in FIG. 2, ADC8, ADC10, ADC11 and ADC12 have obvious inhibitory effect on the proliferation of CHO-CLDN18.2 cells.

Example 4

Bystander Effects of ADC1 and ADC2

The HER2 positive cells SK-BR-3 and HER2 negative cells MDA-MB-468 were seeded into a 6-well plate in a density ratio of 75 thousand cells/well : 200 thousand cells/well. After 3-5 h of culture, ADC1 and the control drug ADC3 were added at final concentrations of 0.1 nM, 0.5 nM and 5 nM, and 0.3 nM, 2 nM and 20 nM ADC2 was added. After 120 h of culture at 37 °C, the cells were digested with pancreatin and washed once with PBS after termination. The cells

were counted, and then labeled with an FITC-labeled anti-HER2 antibody (different from Trastuzumab binding epitope) at 4 °C for 30 min to 1 h. The proportions of different cells were measured using a flow cytometer, and the cell proportions in the Petri dish after different treatments were calculated. As can be seen from FIG. 3, bystander effects were seen with ADC1, ADC2 and ADC3, among which ADC1 showed stronger bystander effects than ADC2 and the control drug ADC3.

Example 5

Bystander Effects of ADC4 and ADC5

The Trop2 positive cells MDA-MB-468 and Trop2 negative cells HGC27 were seeded into a 6-well plate in a density ratio of 3:1 (150 thousand + 50 thousand). After 3-5 h of culture, ADC4, ADC5 and the control drug ADC6 were added at final concentrations of 0.1 nM, 5 nM and 50 nM, respectively. After 120 h of culture at 37 °C, the cells were digested with pancreatin and washed once with PBS after termination. The cells were counted, and then labeled with an FITC-labeled anti-HER2 antibody at 4 °C for 30 min to 1 h. The proportions of different cells were measured using a flow cytometer, and the cell proportions in the Petri dish after different treatments were calculated. As can be seen from FIG. 4, bystander effects were seen with ADC4, ADC5 and ADC6, among which ADC4 showed stronger bystander effects than ADC5 and the control drug ADC6.

Example 6

Bystander Effects of ADC9, ADC11 and ADC12

Under the condition of co-cultivating the CLDN18.2 positive cells KATO3 and the negative cells HGC-27 in vitro, the killing effect of different ADC on these two cells was observed. The KATO3 cells (the CLDN18.2 positive cells) and the HGC-27 cells (the CLDN18.2 negative cells) in the logarithmic growth phase were collected, resuspended with 2% FBS RPMI 1640, and spread in 6-well plate (Cat. No. 3516; costar), 120,000 cells per well for KATO3 and 80,000 cells per well for HCG-27. After culture overnight in a 37 degree 5% $CO_2$ incubator, 3ml of ADC9, ADC11, ADC12 were added the next day, and the final concentrations of the ADC were10 nM, 1 nM and 0.1 nM with no ADC wells added as negative controls. After 5 days of culture in a 37 degree 5% $CO_2$ incubator, the cells were digested with trypsin ( Cat. No. 25200-072; gibco), and calculated the total number of living cells.

According to the ratio of protein: FITC=1mg: 150 μg, the FITC (3326-32-7, sigma) dissolved in DMSO (D2650, sigma) was added to the 1 mg/ml hRS9 antibody solution, gently shaked while adding to mix evenly with the antibody, and reacted at 4 °C in the dark for 8 hours. 5M of $NH_4Cl$ (A501569, Sangon Biotech) was added to the final concentrations of 50 nM, and stopped the reaction at 4 °C for 2 hours. The cross-linked product was dialyzed in PBS (B548117-0500, Sangon Biotechnology) for more than four times until the dialysate was clear, and obtained the hRS9-FITC Antibody.

In order to determine the ratio of KATO3 and HGC-27 in the total surviving cells, the cells were stained with the hRS9-FITC antibody (KATO3 was the Trop2 positive cells) and incubated on ice for 30min. After washing, the fluorescent signal of the stained cells was measured using a CytoFLEX flow cytometer (model: AOO- 1-1102, Beckmann). According to the number and ratio of the KATO3-positive and the HGC-27-negative cells in each treatment well, the number of KAOT3 and HGC-27 cells was calculated, and the results are shown in FIG. 5.

As shown in FIG. 5, ADC9, ADC11, and ADC12 have killing effects on both the positive and negative cells, with ADC11 having the strongest bystander effect on negative cells.

Example 7

1. *In-vitro* cytotoxicity of ADC14

B7-H3 positive cells Calu-6 and CHO-K1-B7-H3 and B7-H3 negative cells CHO-K1 were used to evaluate the *in-vitro* cytotoxicity of ADC14 on tumor cells. Briefly, for Calu-6, the cells were resuspended in a DMEM medium containing 2% fetal bovine serum, seeded in a 96-well plate at 100 μL/well (5000 cells), and then placed in an incubator at 37 °C, 5% $CO_2$ for adherence culture overnight, the ADC at different concentrations (initial concentration of 200 μg/mL, 4-fold gradient dilution, a DMEM medium containing 10% fetal bovine serum as the vehicle) was added at an amount of 100 μL, and the cells were then cultured for 7 days.

For CHO-K1 or CHO-K1-B7-H3, the cells were resuspended in a CD CHO AGT medium containing 2% fetal bovine serum (gibco, Lot#12490-003), seeded in a 96-well plate at 100 μL/well (4000 cells), and then placed in an incubator at 37 °C, 5% $CO_2$ for adherence culture overnight, the ADC at different concentrations (initial concentration of 200 μg/mL, 4-fold gradient dilution, a CD CHO AGT medium containing 2% fetal bovine serum (gibco, Lot#12490-003)

as the vehicle) was added at an amount of 100 μL, and the cells were then cultured for 6 days. 100 μL of a CCK8 solution (Cell Counting Kit-8 (purchased from DOJINDO, Lot#CK04)) was added, and the cells were incubated in an incubator at 37 °C, 5% $CO_2$ for 30 min. The absorbance at 450 nm was measured using a microplate reader, and the $IC_{50}$ value was calculated.

Table 8. *In-vitro* cytotoxicity of ADC 14 on tumor cells

| Cell line | ADC14 $IC_{50}$ (nM) | ADC 15 $IC_{50}$ (nM) |
|---|---|---|
| Calu-6 | 16.32 | 68.75 |
| CHO-K1-B7-H3 | 0.35 | 0.77 |
| CHO-K1 | - | - |
| Note: "-" indicates no significant killing effect | | |

The results are shown in Table 8. ADC14 shows strong *in-vitro* cytotoxicity on B7-H3 positive cells Calu-6 and CHO-K1-B7-H3, and ADC14 has no significant killing effect on B7-H3 negative cells CHO-K1.

2. Bystander effect of ADC14

B7-H3 positive cells CHO-K1-B7-H3 and B7-H3 negative cells CHO-K1-FRα were used to evaluate the bystander effect of ADC14. Briefly, the B7-H3 positive cells CHO-K1-B7-H3 or the B7-H3 negative cells CHO-K1-FRα were resuspended in a CD CHO AGT medium containing 2% fetal bovine serum (gibco, Lot# 12490-003), seeded in a 96-well plate at 100 μL/well (10000 cells), and then placed in an incubator at 37 °C, 5% $CO_2$ for adherence culture overnight, the ADC14 at different concentrations (initial concentration of 6.25 μg/mL, 4-fold gradient dilution, a CD CHO AGT medium containing 2% fetal bovine serum (gibco, Lot#12490-003) as the vehicle) was added at an amount of 100 μL, and the cells were then incubated in an incubator at 37 °C, 5% $CO_2$ for 3 days. One day before the end of ADC14 treatment, the B7-H3 negative cells CHO-K1-FRα were resuspended in a CD CHO AGT medium containing 2% fetal bovine serum (gibco, Lot#12490-003), seeded in a 96-well plate at 100 μL/well (6000 cells), and then placed in an incubator at 37 °C, 5% $CO_2$ for adherence culture overnight. The culture supernatants of B7-H3 positive cells CHO-K1-B7-H3 or B7-H3 negative cells CHO-K1-FRα treated with ADC14 at different concentrations were each directly added to the wells of B7-H3 negative cells CHO-K1-FRα plated on the previous day, and the mixture was then cultured for 4 days. The culture supernatant was then discarded, 100 μL of a CCK8 solution (Cell Counting Kit-8 (purchased from DOJINDO, Lot#CK04)) was added, and the cells were incubated in an incubator at 37 °C, 5% $CO_2$ for 30 min. The absorbance at 450 nm was measured using a microplate reader.
As shown in FIG. 6, the culture supernatant of B7-H3 positive cells CHO-K1-B7-H3 treated with ADC14 has a significant killing effect on B7-H3 negative cells CHO-K1-FRα, while the culture supernatant of B7-H3 negative cells CHO-K1-FRα treated with ADC14 has no significant killing effect on B7-H3 negative cells CHO-K1-FRα.

Example 8

1. *In-vitro* cytotoxicity of ADC 16

The in vitro cytotoxicity mediated by ADC16 was evaluated with the FRα positive cell lines JEG-3 and MCF-7. Cells were harvested by trypsin, cultured with serially diluted ADC16, and then incubated at 37°C. Viability was determined after 5 days using CCK-8. The $IC_{50}$(half maximum inhibitory concentration ) values were determined by reading and analyzing on SpectraMax Gemini (Molecular Devices).
Experiment procedure:

1) JEG-3 cells were adjusted to a cell density of 60,000 cells/mL with DMEM+10% FBS, inoculated in a 96-well plate (manufacturer: Corning, Cat. No. 3599) at 100 μL per well, and placed in a Series II Water Jacketed CO2 Incubator under the condition of standing for 3 hour at 37 °C and 5% $CO_2$.

MCF-7 cells were adjusted to a cell density of 40,000 cells/mL with DMEM+2% FBS, inoculated in a 96-well plate (manufacturer: Corning, Cat. No. 3599) at 100 μL per well, and placed in a Series II Water Jacketed CO2 Incubator under the condition of standing for 2 hours at 37 °C and 5% $CO_2$.
2) Diluted ADC 16 with the medium of the corresponding cells.
JEG-3 cells: The ADC16 concentration was diluted 4 times starting from 1000nM, with the total of 8 concentration

gradients, and then added to the cells at a volume of 100 μ L per well.

MCF-7 cells: The ADC16 concentration was diluted 4 times starting from 250nM, with the total of 8 concentration gradients, and then added to the cells at a volume of 100 μ L per well.

3000nM Exatecan was used as lethal control. The blank control was the culture medium of the corresponding cells.

Three replicate wells were set up for each concentration.

3) Cultured in Series II Water Jacketed CO2 Incubator for 5 days at 37 °C, 5% $CO_2$.

4) Discarded the culture supernatant after 5 days, the RPMI basal medium 1640 (1 ×) containing 10% CCK-8 was added, and placed in Series II Water Jacketed CO2 Incubator under the condition of standing for about 1 hour at 37 °C and 5% $CO_2$.

5) Read on a microplate reader SpectraMax M3 with an absorption wavelength of 450 nm.

6) Data analysis and arrangement: the data of Exatecan-treated wells was used as the complete lethal control, and the blank control was used as the zero killing control. The formula for calculating cell viability is as follows:

$$\text{Cell viability (\%)} = \text{(experimental group - lethal control group)/(blank control group - lethal control group)} \times 100$$

7) Processed and Analyzed data with GraphPad Prism.

The results are shown in Table 9. The results show that ADC16 has a high cytotoxicity to the FRα positive cell lines *In-vitro.*

Table 9. *In-vitro* cytotoxicity of ADC16 to the FRα positive cell lines

| cell line | $IC_{50}$ (nM) |
|---|---|
| JEG-3 | 3.611 |
| MCF-7 | 0.645 |

2. Bystander Effects of ADC 16

In order to confirm the bystander effect induced by ADC16, we conducted *in vitro* co-culture cell killing assays and conditioned medium (CM) cytotoxicity assays.

The FRα positive JEG-3 cells and the FRα negative A549 cells (ADC16 basically has no killing effect on the A549 cells.) were selected for the experiment. The JEG-3 cells were treated with ADC16 for 2, 3, and 4 days, respectively. Then the CM was transferred to the A549 cells, and the changes in cell viability were monitored, and it was found that the viability of A549 cells decreased significantly.

Experiment procedure:

1) JEG-3 cells were adjusted to a cell density of 100,000 cells/mL with DMEM+10% FBS, inoculated in a 96-well plate (manufacturer: Corning, Cat. No. 3599) at 100 μL per well, and placed in a Series II Water Jacketed CO2 Incubator under the condition of standing for 2 hours at 37 °C and 5% $CO_2$.

2) ADC16 was diluted with DMEM+10% FBS medium, the concentration was diluted 4 times starting from 2000nM, with the total of 10 concentration gradients, and then added to the cells at a volume of 100 μL per well.

3) Repeat steps 1) and 2) on the second and third day.

4) On the fifth day, the A549 cell density was adjusted to 40,000 cells/mL with DMEM+2% FBS medium, inoculated 100 μL per well in a 96-well plate subsequently, and placed in a Series II Water Jacketed CO2 Incubator under the condition of standing for 2 hours at 37 °C and 5% $CO_2$. And the culture supernatant of steps 1), 2), and 3), were added with 100 μL per well. 100 μL of 3000 nM Exatecan was added in column 1 as a lethal control, 100 μL of DMEM+2% FBS medium was added in column 12 as a blank control.

5) Cultured in Series II Water Jacketed CO2 Incubator for 3 days. The culture condition was 37 °C , 5% $CO_2$.

6) The culture medium was discarded, and DMEM medium containing 10% CCK-8 was added. Placed in Series II

Water Jacketed CO2 Incubator, and developed color at 37 °C in the dark for 1hour. Then it was read on a microplate reader SpectraMax M3, and the wavelength of light absorbed was 450nm.

7) Organized the data. The formula for calculating cell viability is as follows:

$$\text{Cell viability (\%)} = (\text{experimental well - lethal well})/(\text{blank well - lethal well})*100$$

8) Analyzed data with GraphPad Prism.

The results are shown in FIG. 7, the culture supernatant of ADC16 co-cultured with the FRα positive JEG-3 cells in vitro has a significant killing effect on A549 cells.

Example 9

*In Vivo* Pharmacokinetics Testing in Rats

A 6-8 week-old healthy adult Sprague Dawley rat was subjected to intravenous infusion of ADC4 at the tail for about 1 min ± 10 s. The volume for administration was 5 mL/kg, and the concentration for administration was 20 mg/kg. Blood was collected at 0.083 h, 1 h, 2 h, 8 h, 24 h, 48 h, 72 h, 96 h, 120 h, 144 h and 168 h after the administration was finished, and the serum was centrifugally isolated within 30-120 min. The concentrations of total antibodies (Tab) and ADC in the blood samples were measured by conventional ELISA.

The measurement method for total antibodies was briefly described as follows. Coating with Trop2-His was performed at 4 °C overnight at a concentration of 0.75 μg/mL. Blocking with 5% skim milk powder was performed at 37 °C for 2 h. A standard curve and quality control point were added for incubation for 2 h, with the measuring range for the standard curve being 64 ng/mL to 0.5 ng/mL. Two-fold serial dilution was performed from 64 ng/mL. The quality control concentration points were set to 60 ng/mL, 6 ng/mL and 0.6 ng/mL. The recovery rate for the quality control point should be in 80%-120%. Then 1:8000 diluted anti-human κ light chain peroxidase secondary antibody (Sigma, Cat.# A7164-1ML) produced in goats was added for incubation for 1 h. After 8 washings with PBST, TMB was added for color development, and the reaction was terminated with 0.1 M sulfuric acid. The plate was read using an OD450 microplate reader, and the blood sample concentration was calculated at different time points using the analysis software of the microplate reader, SoftMax Pro.

The measurement method for ADC was briefly described as follows. Coating with Trop2-His was performed at 4 °C overnight at a concentration of 0.75 μg/mL. Blocking with 5% skim milk powder was performed at 37 °C for 2 h. A standard curve and quality control point were added for incubation for 2 h, with the measuring range for the standard curve being 64 ng/mL to 0.5 ng/mL. Two-fold serial dilution was performed from 64 ng/mL. The quality control concentration points were set to 60 ng/mL, 6 ng/mL and 0.6 ng/mL. The recovery rate for the quality control point should be in 80%-120%. Then a secondary antibody (obtained by Genscript immunization) of a rabbit polyclonal antibody small molecule (1:5000 dilution) was added for incubation for 1 h. Fc fragment-specific peroxidase affinipure goat anti-rabbit IgG (Jackson immunono research, 111-035-008) (1:8000 dilution) was added for indubation for 1 h. After 8 washings with PBST, 100 μL of single-component TMB solution (InnoReagents, TMB-S-001) was added for color development, and the reaction was terminated with 0.1 M sulfuric acid. The plate was read using an OD450 microplate reader, and the blood sample concentration was calculated at different time points using the analysis software of the microplate reader, SoftMax Pro. A curve was plotted by ELISA for the change in the drug concentration in the blood, as shown in FIG. 8. The ADC concentration and the total antibody concentration substantially coincide with low detachment and are stable in the blood.

Example 10

*In Vivo* Efficacy of ADC4 in Capan-1

In this experiment, pharmacodynamic studies of ADC4 in a subcutaneous xenograft female BALB/c nude mouse animal model of human pancreatic cancer Capan-1 cell line were assessed. There were 10 mice in each group. The test protocol was designed as follows, with ADC4 as a test drug and ADC6 and ADC7 as control drugs.

Table 10. Administration route, dosage and regimen in the subcutaneous xenograft model of human pancreatic cancer Capan-1

| Group | Number of animals | Administration group | Dosage (mg/kg) | Administration route | Administration period |
|---|---|---|---|---|---|
| 1 | 10 | Vehicle control | -- | *i.v.* | QW×2 weeks |
| 2 | 10 | ADC6 | 5 | *i.v.* | Single administration |
| 3 | 10 | ADC7 | 5 | *i.v.* | QW×2 weeks |
| 4 | 10 | ADC4 | 5 | *i.v.* | Single administration |
| Note: 1. The day of grouping was defined as day 0 and the administration was started at day 0. | | | | | |

All drugs were diluted with PBS to a given concentration and administered. The test animals were 7-9 week-old BALB/c nude female mice (Beijing AniKeeper Biotech Co., Ltd.).

The Capan-1 cells were cultured in IMDM medium containing 20% fetal bovine serum. Capan-1 cells in the exponential phase were collected and resuspended in PBS to an appropriate concentration for subcutaneous tumor grafting in nude mice. The test mice were inoculated subcutaneously at the right anterior scapula with $5 \times 10^6$ Capan-1 cells, which were resuspended in PBS 1:1 mixed with matrigel (0.1 mL/mouse). The growth of the tumors was observed periodically. When the mean volume of the tumors reached 159.77 $mm^3$, the mice were randomly grouped for administration according to the tumor size and the mouse weight. The day of grouping was defined as day 0 and the administration was started at day 0.

After tumor grafting, routine monitoring included the effect of tumor growth and treatment on the normal behavior of the test animals, specifically the activity, food and water intake, weight gain or loss (the weight was measured twice a week), the eyes, the coat and other abnormal conditions. Clinical symptoms observed during the experiment were recorded in the raw data. The calculation formula for tumor volume: Tumor volume ($mm^3$) = $1/2 \times (a \times b^2)$ (where a represents long diameter and b represents short diameter). In the experiment, StudyDirector™ (version 3.1.399.19, Studylog System, Inc., S. San Francisco, CA, USA) software was employed to collect data.

Table 11. Efficacy analysis for each group in the xenograft model of Capan-1

| Experimental group | Mean tumor volume (day 0)$^a$ | Mean tumor volume (day 25)$^a$ | TGI (%)$^b$ | P value (relative to control group)$^c$ |
|---|---|---|---|---|
| Vehicle, 0 mg/kg, | 159.77±4.49 | 525.58±46.30 | - | - |
| ADC6, 5 mg/kg | 159.78±4.69 | 153.57±21.09 | 101.70 | 2.09e-06*** |
| ADC7, 5 mg/kg, | 159.75±4.31 | 601.83±118.59 | -20.85 | 1.00e+00$^{ns}$ |
| ADC4, 5 mg/kg | 159.77±4.34 | 107.08±18.05 | 114.40 | 4.09e-09*** |
| Note: a. Data were expressed as "mean ± standard error";<br>b. TGI% = [1 - (Ti - T0)/(Ci - CO)] × 100, where T0 and C0 are the mean tumor volumes of the administration group and vehicle control group at the day of grouping (day 0), respectively, and Ti and Ci are the mean tumor volumes of the administration group and vehicle control group at day 25, respectively;<br>c. **P* < 0.05, ***P* < 0.01 and ****P* < 0.001 compared to the tumor volume of the vehicle control group. | | | | |

As shown in FIG. 9, at a dosage of 5 mg/kg, ADC4 has significantly greater efficacy than the control drug ADC7 and greater efficacy than ADC6; ADC7 has substantially no tumor growth inhibition effect on this model at a concentration of 5 mg/kg; the TGI of ADC4 on tumors was up to 114% at day 25 after a single administration.

Example 11

Pharmacodynamic Study of Drugs ADC1, ADC2 and ADC3 in Subcutaneous Xenograft Female BALB/c Nude Mouse Animal Model of Human Ovarian Cancer SK-OV-3 Cell Line

Test drugs: ADC1 (5 mg/kg)
ADC2 (5 mg/kg)
ADC3 (5 mg/kg)

Preparation method: all were diluted with PBS

Test animals: BALB/c nude, 6 per group (Jiangsu GemPharmatech Co., Ltd.).

Test method: The SK-OV-3 cells were cultured in McCoy's 5a medium containing 10% fetal bovine serum. SK-OV-3 cells in the exponential phase were collected and resuspended in PBS to an appropriate concentration for subcutaneous tumor grafting in mice. The test mice were inoculated subcutaneously at the right anterior scapula on the right dorsum with $1 \times 10^7$ SK-OV-3 cells, which were resuspended in PBS 1:1 mixed with matrigel (0.1 mL/mouse). The growth of the tumors was observed periodically. When the mean volume of the tumors reached 129.98 $mm^3$, the mice were randomly grouped for administration according to the tumor size and the mouse weight. The day of grouping was defined as day 0 and the administration was started at day 0. A single administration by intravenous injection was adopted. The tumor volume and weight were measured twice a week, and the data were recorded.

Test results: The tumor volume changed as shown in Table 12 and FIG. 10. The test results show that ADC1 and ADC2 have significantly greater efficacy than ADC3 after a single administration. There was no apparent abnormality or weight loss in the groups of mice during the experiment.

Table 12

| Experimental group | Mean tumor volume (day 0)[a] | Mean tumor volume (day 13)[a] | TGI (%)[b] | P value (relative to control group)[c] |
|---|---|---|---|---|
| Vehicle, 0 mg/kg | 130.12±10.29 | 2224.22±358.85 | - | - |
| ADC3, 5 mg/kg | 129.89±8.09 | 903.31±227.77 | 63.07 | 3.14e-01[ns] |
| ADC2, 5 mg/kg | 129.99±9.92 | 160.77±93.32 | 98.53 | 2.05e-05*** |
| ADC1, 5 mg/kg | 129.89±7.59 | 8.53±5.96 | 105.80 | 7.92e-09*** |

Note: a. Data were expressed as "mean ± standard error";
b. TGI% = [1 - (Ti - T0)/(Ci - CO)] × 100, where T0 and C0 are the mean tumor volumes of the administration group and vehicle control group at the day of grouping (day 0), respectively, and Ti and Ci are the mean tumor volumes of the administration group and vehicle control group at day 28, respectively;
c. *$P$ < 0.05, **$P$ < 0.01 and ***$P$ < 0.001 compared to the tumor volume of the vehicle control group.

Example 12

Pharmacodynamic Study of Drugs ADC4 and ADC6 in Subcutaneous Xenograft Female BALB/c Nude Mouse Animal Model of Human Triple-Negative Breast Cancer MX-1 Cell Line

Test drugs: ADC4 (2.5 mg/kg and 5 mg/kg)
ADC6 (2.5 mg/kg and 5 mg/kg)

Preparation method: all were diluted with PBS

Test animals: BALB/c nude, 6 per group (Beijing AniKeeper Biotech Co., Ltd.).

Test method: The MX-1 cells were cultured in RPM1640 medium containing 10% fetal bovine serum. MX-1 in the exponential phase were collected and resuspended in PBS to an appropriate concentration for subcutaneous tumor grafting in nude mice. The test mice were inoculated subcutaneously on the right dorsum with $5 \times 10^6$ MX-1 cells, which were resuspended in PBS (0.1 mL/mouse). The growth of the tumors was observed periodically. When the mean volume of the tumors reached 149.03 $mm^3$, the mice were randomly grouped for administration according to the tumor size and the mouse weight. The day of grouping was defined as day 0 and the administration was started at day 0.

After tumor grafting, routine monitoring included the effect of tumor growth and treatment on the normal behavior of the animals.

Test results: The tumor volume changed as shown in Table 13 and FIG. 11, and the test results show that

ADC4 and ADC6 have similar efficacy after a single administration, and the TGIs are 109.48% and 108.84%, respectively, at an administration concentration of 5 mg/kg. There was no apparent abnormality or weight loss in the groups of mice during the experiment.

Table 13

| Experimental group | Mean tumor volume (day 0)[a] | Mean tumor volume (day 21)[a] | TGI (%)[b] | P value relative to control group)[c] |
|---|---|---|---|---|
| Vehicle, 0 mg/kg | 149.15±10.37 | 1646.06±422.20 | - | - |
| **ADC4,** 2.5 mg/kg | 148.93±10.42 | 351.33±74.53 | 86.48 | 3.66e-03** |
| ADC4, 5 mg/kg | 149.09±10.58 | 7.14±1.82 | 109.48 | 9.65e-11*** |
| ADC6, 2.5 mg/kg | 149.08±10.20 | 279.24±84.24 | 91.30 | 7.72e-05*** |
| **ADC6**, 5 mg/kg, | 149.01±10.31 | 16.69±12.80 | 108.84 | 5.53e-11*** |

Note: a. Data were expressed as "mean ± standard error";
b. TGI% = [1 - (Ti - T0)/(Ci - CO)] × 100, where T0 and C0 are the mean tumor volumes of the administration group and vehicle control group at the day of grouping (day 0), respectively, and Ti and Ci are the mean tumor volumes of the administration group and vehicle control group at day 21, respectively;
**c. *$P$ < 0.05, **$P$ < 0.01 and ***$P$ < 0.001 compared to the tumor volume of the vehicle control group.**

Example 13

Antitumor effects of ADC9, ADC11, ADC12 and ADC13 in a HuPrime® gastric cancer GA0006 xenograft female BALB/c nude mouse animal model.

A tumor mass with a diameter of 2-3 mm was grafted subcutaneously at the right anterior scapula of BALB/c nude mice. When the average tumor volume of tumor-bearing mice reached about 139.15mm$^3$, the mice were randomly divided into groups. The day of grouping was set as the 0th day, and the administration started on the 0th day, with a single administration. The body weight and tumor growth of the mice were monitored. FIG. 12 showed the tumor growth in each treatment group and the control group of the GA0006 xenograft model.

The calculation formula for tumor volume: Tumor volume (mm$^3$) = 1/2 × (a × b$^2$) (where a represents long diameter and b represents short diameter). $TGI_{TV}$%=[1-(Ti-T0)/(Ci-C0)]×100, where T0 and C0 are the mean tumor volumes of the administration group and vehicle control group at the day of grouping (day 0), respectively, and Ti and Ci are the mean tumor volumes of the administration group and vehicle control group at day 14, respectively. $^{ns}P$>0.05, *$P$<0.05, **$P$<0.01 and ***$P$ < 0.001 compared to the tumor volume of the vehicle control group.

The control group (group 1) of this model achieved an average tumor volume of 628.62 mm$^3$ after 14 days of the administration.

After 14 days of the administration of 10mg/kg and 5mg/kg ADC11 (i.e. Group 2 and Group 3), the average tumor volume was 78.17mm$^3$ and 91.47mm$^3$, respectively, the relative tumor inhibition rates were TGI 112.45% (P = 3.43e-09***) and 109.75% (P=6.53e-08***), respectively.

After 14 days of the administration of 10mg/kg and 5mg/kg ADC13 (i.e. Group 4 and Group 5), the average tumor volume was 25.77mm$^3$ and 78.07mm$^3$, respectively, the relative tumor inhibition rates were TGI 123.17% (P =7.58e-12***) and 112.48% (P =1.23e-08***), respectively.

After 14 days of the administration of 10mg/kg and 5mg/kg ADC12 (i.e. Group 6 and Group 7), the average tumor volume was 185.69 mm$^3$ and 284.02 mm$^3$, respectively, the relative tumor inhibition rates were TGI 90.50% (P =5.92e-04***) and 70.40% (P =3.51e-01$^{ns}$), respectively.

After 14 days of the administration of 5mg/kg ADC9 (i.e. Group 8), the average tumor volume was 296.86 mm$^3$, the relative tumor inhibition rate were TGI 67.79% (P =3.51e-0$^{ns}$).

Consistent with the trend of tumor volume data, using changes in the tumor weight as an indicator, each treatment group also showed varying degrees of anti-tumor efficacy, as shown in FIG. 13.

Example 14

*In- Vivo* Tumor Inhibition Activity of ADC14

Pharmacodynamic evaluation of ADC14 in subcutaneous xenograft BALB/c nude mouse models of human liver cancer Hep 3B cell line The number of animals per group and the detailed route, dose and regimen of administration

are shown in Table 14.

Table 14. Route, dose and regimen of administration in subcutaneous xenograft BALB/c nude mouse models of human liver cancer Hep 3B cell line

| Group | Number of animals | Treatment group | Dose (mg/kg) | Route of administration | Administration period |
|---|---|---|---|---|---|
| 1 | 10 | Vehicle (normal saline) control | -- | *i.v.* | Single administration |
| 2 | 10 | Exatecan | 0.136 | *i.v.* | Single administration |
| 3 | 10 | ADC 15 | 5 | *i.v.* | Single administration |
| 4 | 10 | ADC14 | 2.5 | *i.v.* | Single administration |
| 5 | 10 | ADC14 | 5 | *i.v.* | Single administration |
| Note: the day of grouping was defined as Day 0; administration was started on the day of grouping; i.v.: intravenous injection | | | | | |

Hep 3B cells were cultured in a MEM medium (Hyclone, Lot#SH30024.01) containing 10% FBS and 1% NEAA (GIBCO, Lot#11140050). Hep 3B cells in the exponential growth phase were collected and resuspended in PBS mixed with matrigel in a ratio of 1:1. Male BALB/c nude mice aged 6-8 weeks were selected, 10 mice per group. The test mice were inoculated subcutaneously on the right anterior scapula with $5 \times 10^6$ Hep 3B cells (0.1 mL/mouse). The growth of the tumors was observed periodically. When the mean volume of the tumors reached 150 $mm^3$ (100-200 $mm^3$), the mice were randomly grouped for administration according to the tumor size and the mouse weight. The day of grouping was defined as Day 0 and the administration was started on Day 0.

After inoculation with tumor cells, routine monitoring included the effect of tumor growth and treatment on the normal behavior of the animals, specifically the activity, food and water intake, weight gain or loss, the eyes, the coat and other abnormal conditions. After the administration was started, the body weight and tumor size of the mice were measured twice a week. The calculation formula for tumor volume: Tumor volume ($mm^3$) = $1/2 \times (a \times b^2)$ (where a represents long diameter of tumor and b represents short diameter of tumor). On Day 25 after inoculation, the relative tumor growth inhibition (TGI%) was calculated by the following formula: TGI% = (1 - mean relative tumor volume in treatment group/mean relative tumor volume in vehicle control group) $\times$ 100%.

Table 15. Tumor volume and tumor inhibition rate on Day 25

| Group | Mean tumor volume±SEM ($mm^3$) | Tumor volume P-value | TGI (%) |
|---|---|---|---|
| 1 | 2258±208 | -- | -- |
| 2 | 2237±225 | 0.679 | 0.94% |
| 3 | 858±112 | 0.011 | 62.02% |
| 4 | 139±58 | 0.002 | 93.86% |
| 5 | 30±10 | 0.001 | 98.66% |

The tumor inhibition results are shown in Table 15 and FIG. 14. ADC14 has a very strong growth inhibition effect on Hep 3B tumor in a dose-dependent manner; the inhibition of tumor growth by ADC14 is significantly stronger than that by ADC15 (group 5 vs. group 3) in the case of the same small molecular weight.

Example 15

1. Pharmacodynamic evaluation of the test drug in HuPrime® Ovarian Cancer OV3756 Subcutaneous Xenograft Female BALB/c Nude Mouse Model. The grouping and dosing regimens were shown in Table 16.

Table 16. Grouping and dosing regimens

| Group | Number of animals | Administration group | Dosage (mg/kg) | Administration route | Administration period |
|---|---|---|---|---|---|
| 1 | 8 | Vehicle control (normal saline) | -- | i.v. | Single administration |
| 2 | 8 | ADC17 | 5 | i.v. | Single administration |
| 3 | 8 | ADC16 | 2.5 | i.v. | Single administration |

The tumor tissues were harvested from HuPrime® Ovarian Cancer Xenograft Model OV3756 bearing mice , cuted into tumor masses with a diameter of 2-3 mm, and grafted subcutaneously in the right anterior scapula of 6-7 week old female BALB/c nude mice.

When the mean tumor volume of tumor bearing mice reached about 137.55mm$^3$, the mice were randomly grouped for administration. The day of grouping was set as day 0, and the administration began on day 0.

After the tumor grafting, routine monitoring included the effect of tumor growth and treatment on the normal behavior of the test animals, specifically the activity, food and water intake, weight gain or loss (the weight was measured twice a week), the eyes, the coat and other abnormal conditions. The calculation formula for tumor volume: Tumor volume (mm$^3$) = 1/2 $\times$ (a $\times$ b$^2$) (where a represents long diameter and b represents short diameter).

Table 17. Efficacy analysis for each group in the OV3756 Xenograft Model

| Experimental group | Mean tumor volume (Day 0)[a] | Mean tumor volume (Day 28)[a] | TGI (%)[b] | P value (relative to control group)[c] |
|---|---|---|---|---|
| 1 | 137.46±9.66 | 1198.78±115.80 | - | - |
| 2 | 137.46±9.84 | 439.00±149.55 | 71.59 | 1.15e-04*** |
| 3 | 137.62±11.18 | 277.59±68.82 | 86.81 | 3.59e-06*** |

Note: a. Data were expressed as "mean ± standard error";
b. TGI% = [1 - (Ti - T0)/(Ci - CO)] × 100, where T1 and C1 are the mean tumor volumes of the administration group and vehicle control group at the day of grouping (day 0), respectively, and Ti and Ci are the mean tumor volumes of the administration group and vehicle control group at day 25, respectively;
c. *$P$ < 0.05, **$P$ < 0.01 and ***$P$ < 0.001 compared to the tumor volume of the vehicle control group.

The tumor growth status of the each experimental group and control group in the OV3756 Xenograft Model is shown in Table 17 and FIG. 15. From Table 17 and FIG. 15, it can be seen that ADC16 and ADC17 can significantly inhibit tumor growth of the OV3756 Xenograft Model.

2. The study on the anti-tumor efficacy of experimental drugs in the Balb/c nude mice JEG-3 subcutaneous model aimed to investigate the in vivo efficacy of ADC16 in the JeG-3 model. The grouping and dosing regimens were shown in Table 18

Table 18. Grouping and dosing regimens

| Group | Administratio n group | Administrat ion volume (μL/g) | Dosage (mg/kg) | Administra tion route | Administration period | Numbe r of animal s | Treat ment Time |
|---|---|---|---|---|---|---|---|
| 1 | blank control (normal saline) | 10 μL/g | - | i.v. | Single administration | 8 | 28 days |
| 2 | Exatecan Mesylate | 10 μL/g | 0.136 | i.v. | Single administration | 8 | 28 days |
| 3 | ADC16 | 10 μL/g | 2.5 | i.v. | Single administration | 8 | 28 days |

(continued)

| Group | Administration group | Administrat ion volume ($\mu$L/g) | Dosage (mg/kg) | Administra tion route | Administration period | Numbe r of animal s | Treat ment Time |
|---|---|---|---|---|---|---|---|
| 4 | ADC16 | 10 $\mu$L/g | 5 | i.v. | Single administration | 8 | 28 days |

The 6-7 week old female BALB/c nude mice were selected, and the 1 $\times$ $10^6$ JEG-3 cells suspending in 100 $\mu$L EMEM medium containing 50% Matrigel were grafted subcutaneously on the right side of the mice. When the mean tumor volume reached about 118 $mm^3$ , the mice were randomly grouped for administration according to the animal weight and the tumor volume, and 8 per group.

After Administrating, the tumor volumes were measured twice a week. The calculation formula for tumor volume: Tumor volume ($mm^3$) = 0.5a $\times$ $b^2$ (where a represents long diameter and b represents short diameter of the tumor).

The results are shown in FIG. 16, compared with the control group, the treatment with ADC16 showed a significant tumor inhibitory effect in a dose-dependent manner (p<0.001).On the 8th day after administrating, the TGI was 98.98% (2.5 mg/kg) and 100.00% (5 mg/kg), respectively. Notably, ADC16 caused complete the tumor regression and maintained it to the end of the experiment.

## Claims

**1.** A drug conjugate of Formula I or a stereoisomer thereof or a pharmaceutically acceptable salt or solvate thereof:

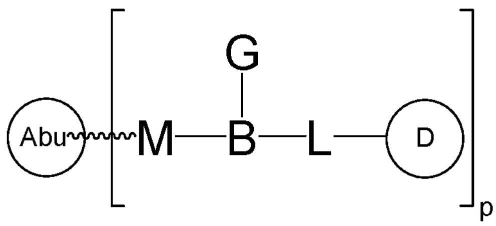

Formula I

wherein

Abu is a polypeptide;
D is a drug;
M is

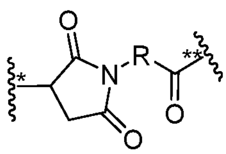

,

wherein * links to Abu, ** links to B, and R is selected from: $-(CH_2)_r$, $-(CHR^m)_r$, C3-C8 carbocyclyl, $-O-(CH_2)_r-$, arylene, $-(CH_2)_r$-arylene-, -arylene-$(CH_2)$r-, $-(CH_2)_r$-(C3-C8 carbocyclyl)-, -(C3-C8 carbocyclyl)-$(CH_2)_r$-, C3-C8 heterocyclyl, $-(CH_2)_r$-(C3-C8 heterocyclyl)-, -(C3-C8 heterocyclyl)-$(CH_2)_r$-, $-(CH_2)_rC(O)NR^m(CH_2)_r$, $-(CH_2CH_2O)_r-$, $-(CH_2CH_2O)_r-CH_2-$, $-(CH_2)_rC(O)NR^m(CH_2CH_2O)_r-$, $-(CH_2)_rC(O)NR^m(CH_2CH_2O)_r-CH_2-$, $-(CH_2CH_2O)_rC(O)NR^m(CH_2CH_2O)_r-$, $-(CH_2CH_2O)_rC(O)NR^m(CH_2CH_2O)_r-CH_2-$ and $-(CH_2CH_2O)_rC(O)NR^m(CH_2)_r-$; wherein each $R^m$ is independently H, C1-C6 alkyl, C3-C8 carbocyclyl, phenyl or benzyl, and each r is independently 1, 2, 3, 4, 5, 6, 7, 8, 9 or 10;
B is

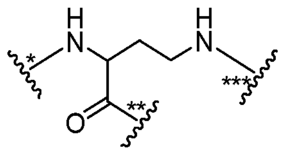

,

wherein * links to M, ** links to L, and *** links to G;
L is -$(AA)_i$-$(FF)_f$-, wherein AA is an amino acid or polypeptide, and i is 1, 2, 3, 4, 5, 6, 7, 8, 9, 10, 11, 12, 13, 14, 15, 16, 17, 18, 19 or 20; each FF is independently

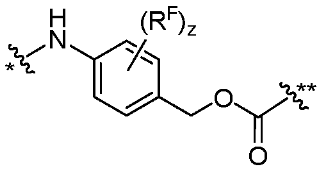

,

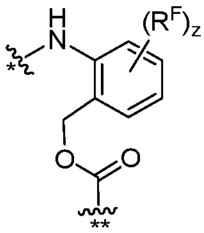

or

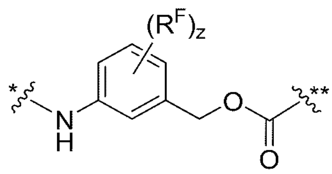

,

wherein
each $R^F$ is independently C1-C6 alkyl, C1-C6 alkoxy, -$NO_2$ or halogen, * links to AA, and ** links to D; z is 0, 1, 2, 3 or 4; f is 1, 2, 3, 4, 5, 6, 7, 8, 9 or 10;
Gis

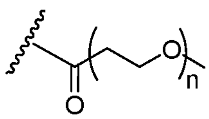

,

wherein n is 1-24;
p is 1-10.

**2.** A drug conjugate of Formula I-1 or a stereoisomer thereof or a pharmaceutically acceptable salt or solvate thereof, wherein Formula I-1 is:

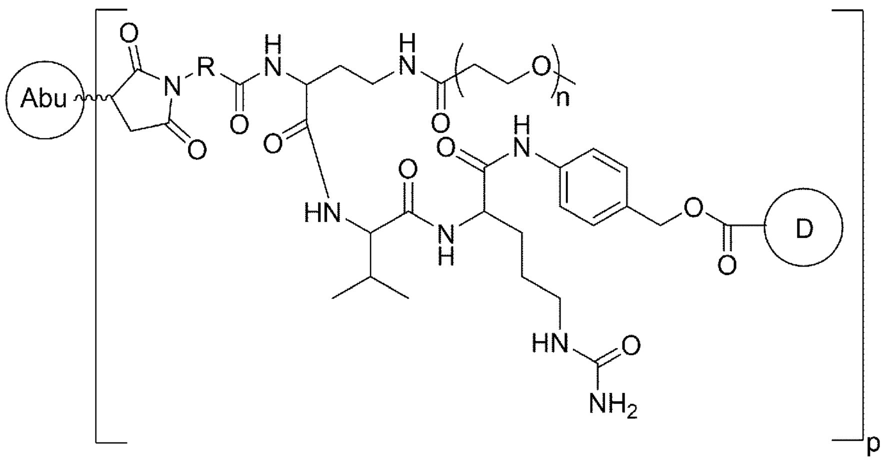

Formula I-1

wherein

Abu is a polypeptide;
R is selected from: $-(CH_2)_r-$, $-(CHR^m)_r$, C3-C8 carbocyclyl, $-O-(CH_2)_r-$, arylene, $-(CH_2)_r$-arylene-, -arylene-$(CH_2)$r-, $-(CH_2)_r$-(C3-C8 carbocyclyl)-, -(C3-C8 carbocyclyl)-$(CH_2)_r-$, C3-C8 heterocyclyl, $-(CH_2)_r$-(C3-C8 heterocyclyl)-, -(C3-C8 heterocyclyl)-$(CH_2)_r-$, $-(CH_2)_rC(O)NR^m(CH_2)_r-$, $-(CH_2CH_2O)_r-$, $-(CH_2CH_2O)_r-CH_2-$, $-(CH_2)_rC(O)NR^m(CH_2CH_2O)_r-$, $-(CH_2)_rC(O)NR^m(CH_2CH_2O)_r-CH_2-$, $-(CH_2CH_2O)_rC(O)NR^m(CH_2CH_2O)_r-$, $-(CH_2CH_2O)_rC(O)NR^m(CH_2CH_2O)_r-CH_2-$ and
$-(CH_2CH_2O)_rC(O)NR^m(CH_2)_r-$; wherein each $R^m$ is independently H, C1-C6 alkyl, C3-C8 carbocyclyl, phenyl or benzyl, and each r is independently 1, 2, 3, 4, 5, 6, 7, 8, 9 or 10;
D is a drug;
n is an integer from 1 to 24;
p is 1-10.

**3.** A drug conjugate of Formula I-2 or Formula I-2-1 or a stereoisomer thereof a pharmaceutically acceptable salt or solvate thereof, wherein Formula I-2 is:

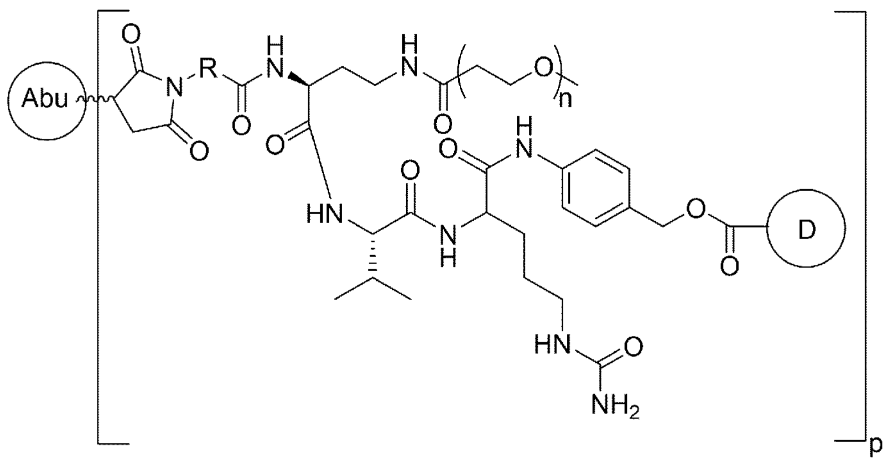

Formula I-2

Formula I-2-1 is:

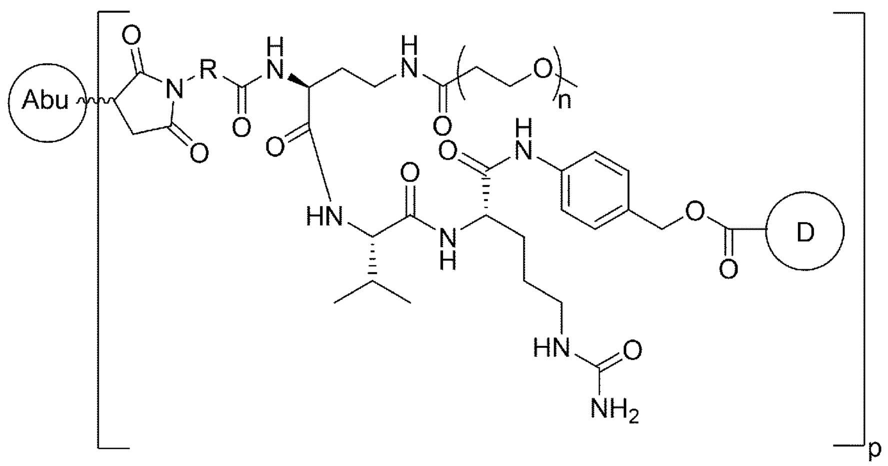

Formula I-2-1

wherein
Abu is a polypeptide;
R is selected from: $-(CH_2)_r-$, $-(CHR^m)_r$, C3-C8 carbocyclyl, $-O-(CH_2)_r-$, arylene, $-(CH_2)_r$-arylene-, -arylene-$(CH_2)$r-, $-(CH_2)_r$-(C3-C8 carbocyclyl)-, -(C3-C8 carbocyclyl)-$(CH_2)_r-$, C3-C8 heterocyclyl, $-(CH_2)_r$-(C3-C8 heterocyclyl)-, -(C3-C8 heterocyclyl)-$(CH_2)_r-$, $-(CH_2)_rC(O)NR^m(CH_2)_r-$, $-(CH_2CH_2O)_r-$, $-(CH_2CH_2O)_r-CH_2-$,

$-(CH_2)_rC(O)NR^m(CH_2CH_2O)_r-$, $-(CH_2)_rC(O)NR^m(CH_2CH_2O)_r-CH_2-$, $-(CH_2CH_2O)_rC(O)NR^m(CH_2CH_2O)_r-$, $-(CH_2CH_2O)_rC(O)NR^m(CH_2CH_2O)_r-CH_2-$ and $-(CH_2CH_2O)_rC(O)NR^m(CH_2)_r-$; wherein each $R^m$ is independently H, C1-C6 alkyl, C3-C8 carbocyclyl, phenyl or benzyl, and each r is independently 1, 2, 3, 4, 5, 6, 7, 8, 9 or 10;
D is a drug;
n is an integer from 1 to 24;
p is 1-10.

**4.** A drug conjugate of Formula I-3 or a stereoisomer thereof or a pharmaceutically acceptable salt or solvate thereof, wherein Formula I-3 is:

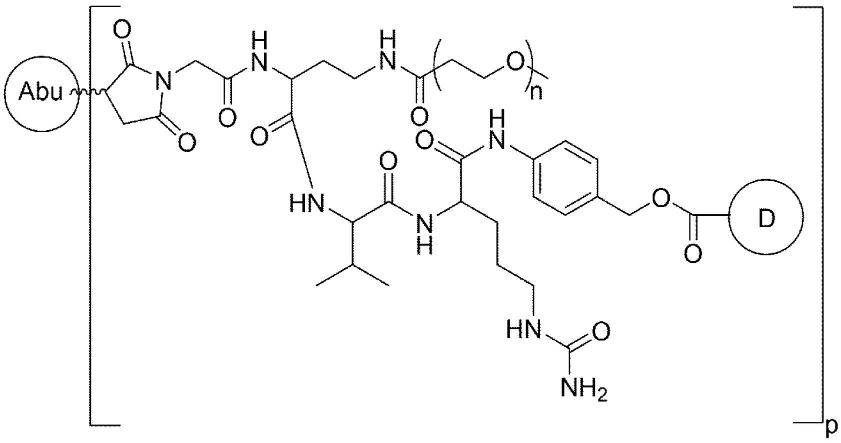

Formula I-3

wherein

Abu is a polypeptide;
D is a drug;
n is an integer from 1 to 24;
p is 1-10.

**5.** A drug conjugate of Formula I-4 or Formula I-4-1 or a stereoisomer thereof a pharmaceutically acceptable salt or solvate thereof, wherein Formula I-4 is:

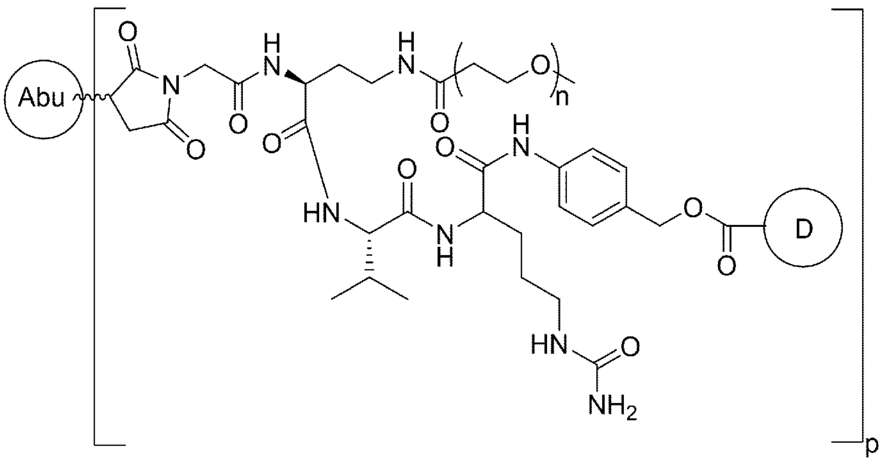

Formula I-4-1 is:

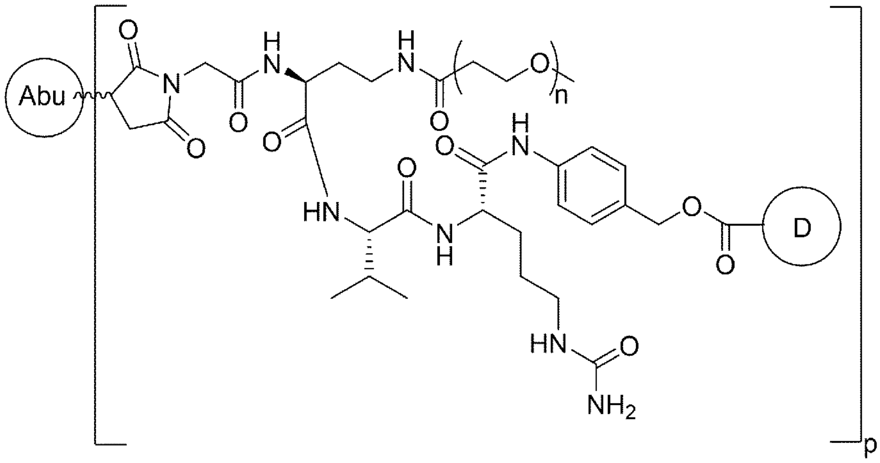

Formula I-4

wherein
Abu is a polypeptide;
D is a drug;
n is an integer from 1 to 24;
p is 1-10.

**6.** A drug conjugate of Formula I-5, I-5-1, I-6, I-6-1, I-7, I-7-1, I-8, I-8-1, I-9, I-9-1, I-10, I-10-1, I-11 or I-11-1 or a stereoisomer thereof or a pharmaceutically acceptable salt or solvate thereof, wherein Formulas I-5, I-5-1, I-6, I-6-1, I-7, I-7-1, I-8, I-8-1, I-9, I-9-1, I-10, I-10-1, I-11 and I-11-1 are:

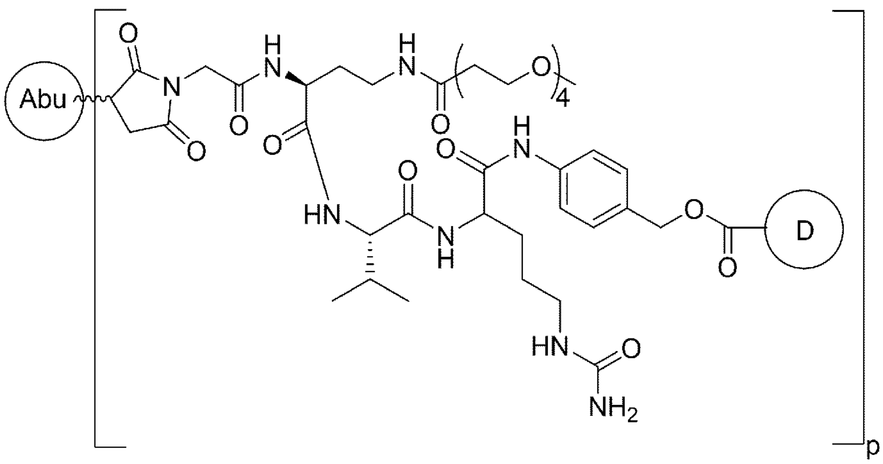

Formula I-5

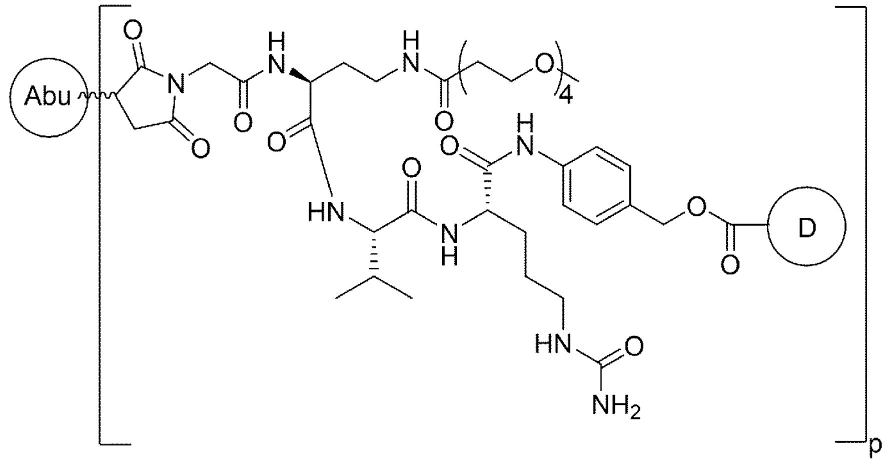

Formula I-5-1

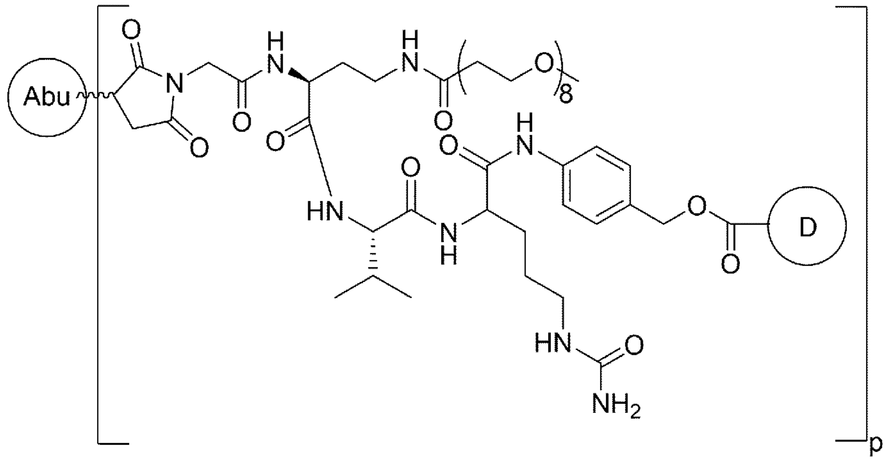

Formula I-6

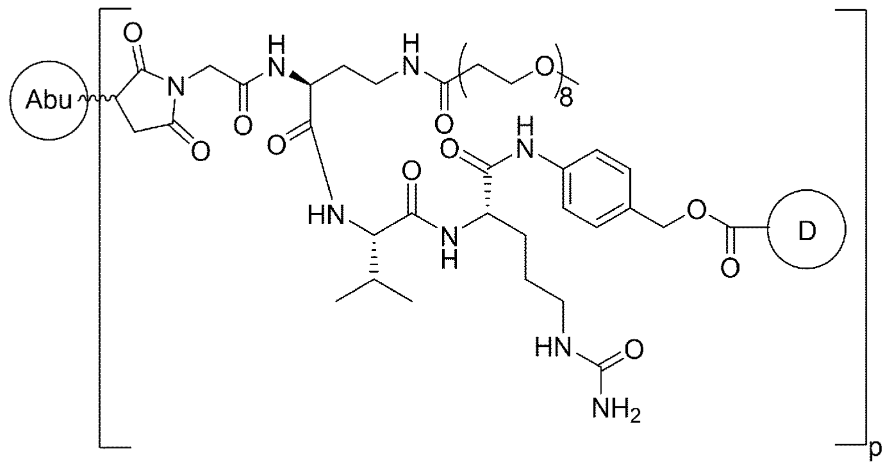

Formula I-6-1

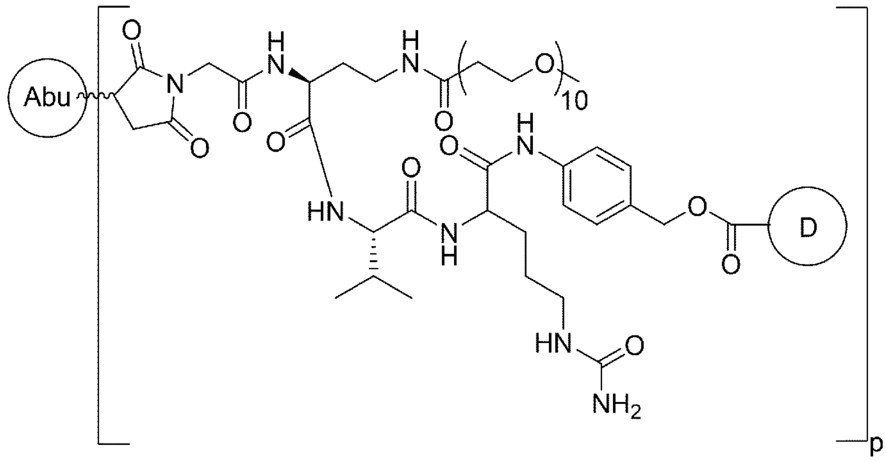

Formula I-7

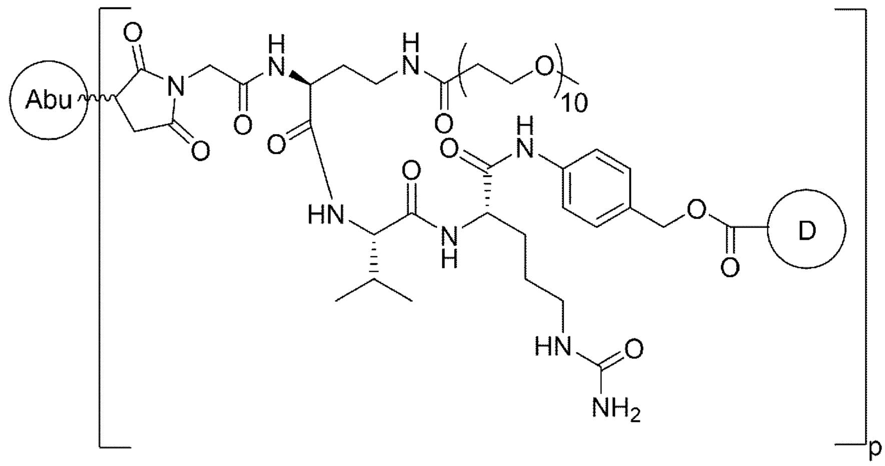

Formula I-7-1

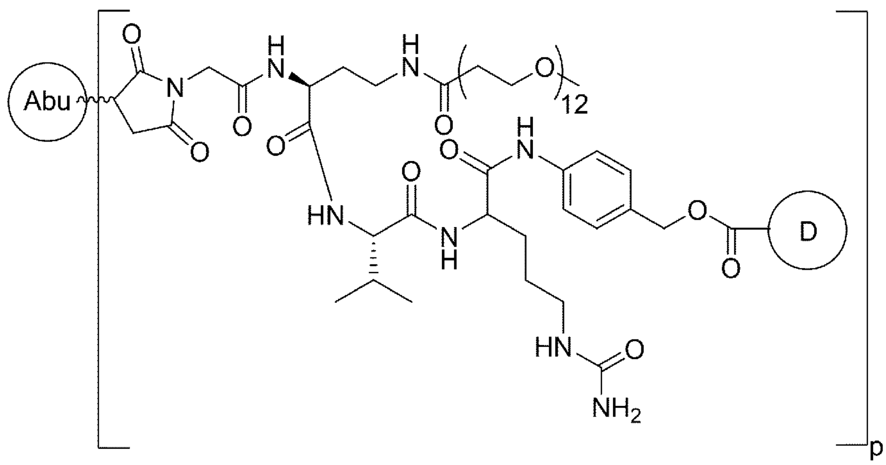

Formula I-8

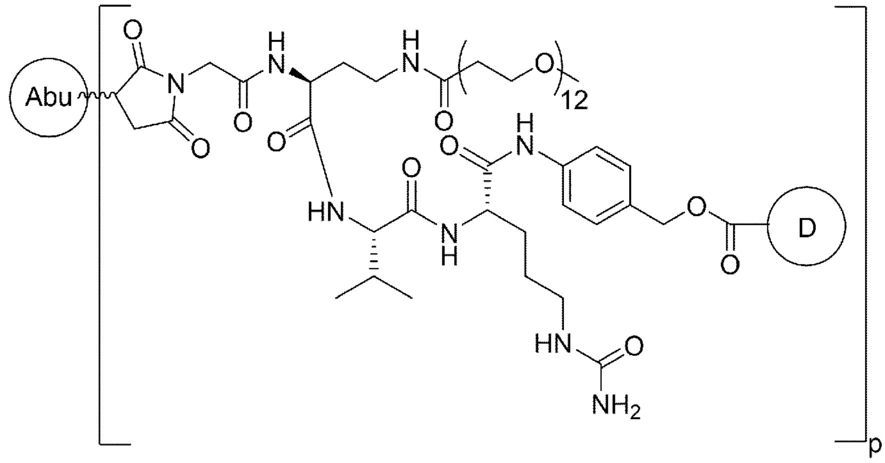

Formula I-8-1

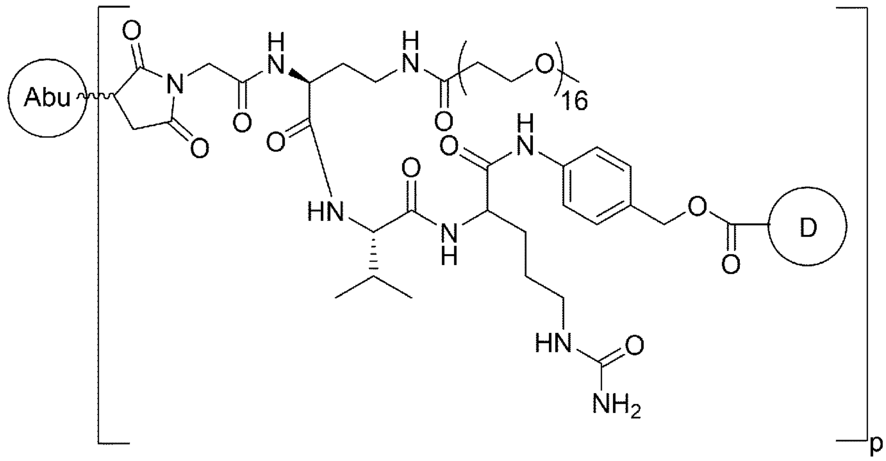

Formula I-9

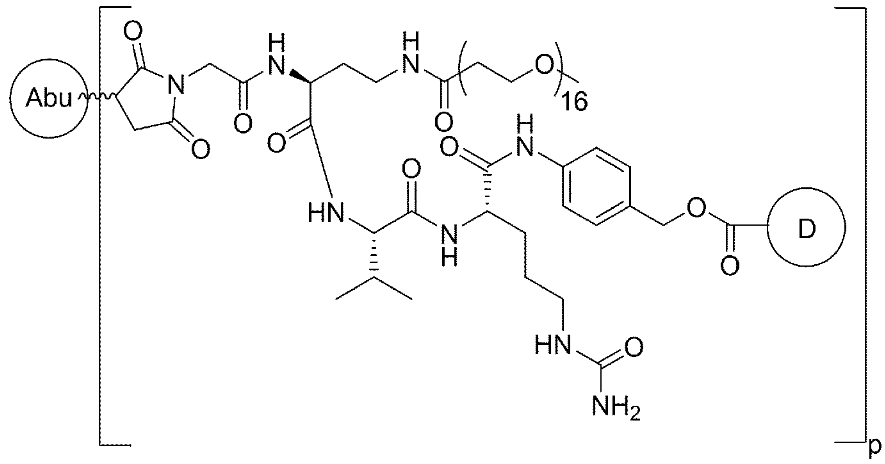

Formula I-9-1

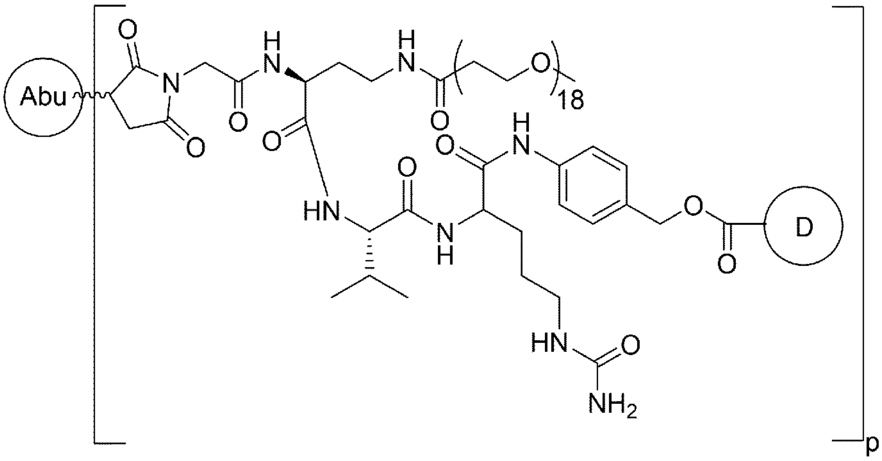

Formula I-10

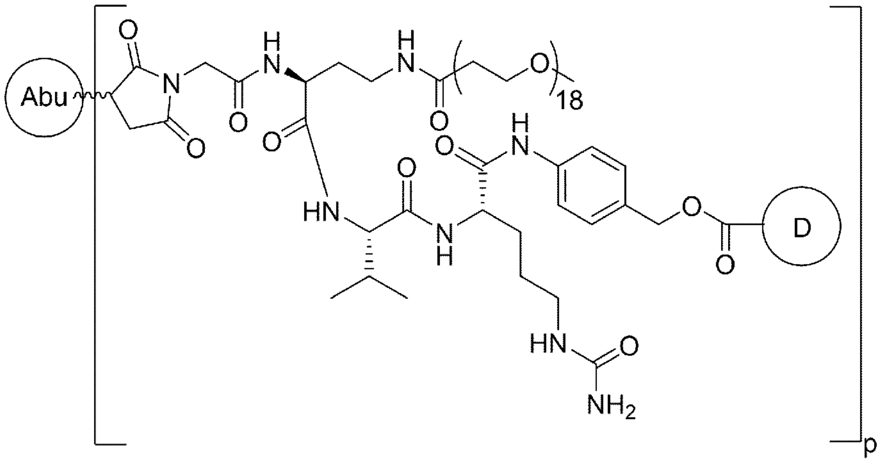

Formula I-10-1

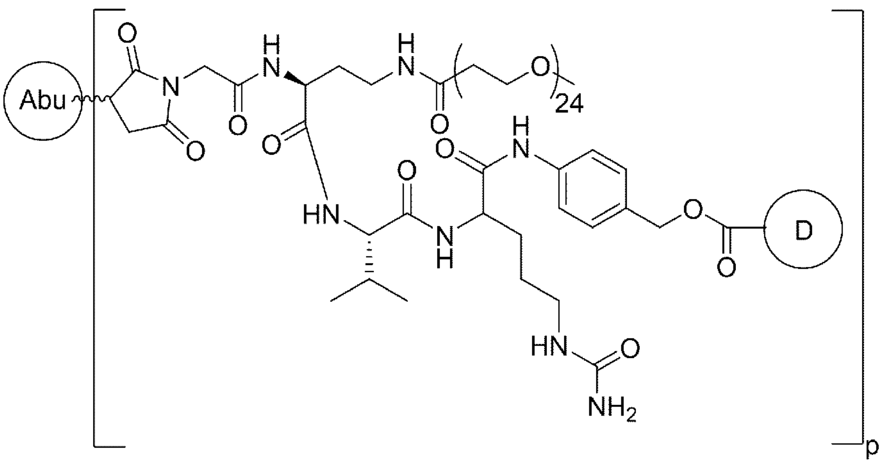

Formula I-11

or

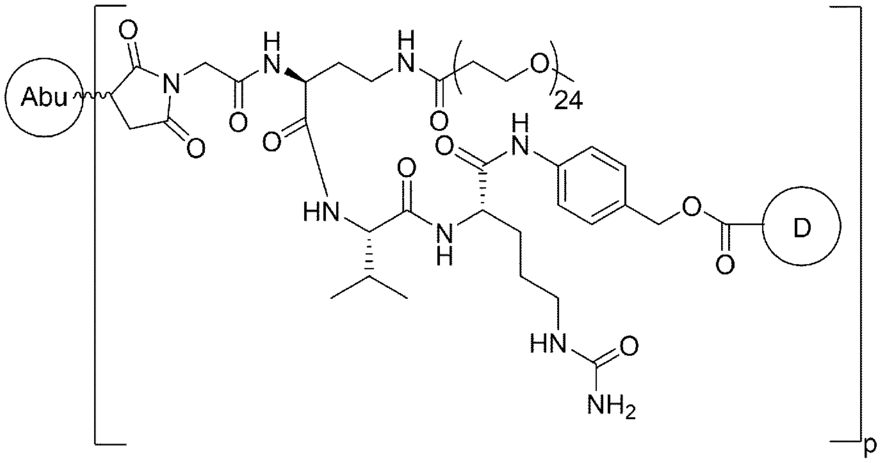

Formula I-11-1

wherein

Abu is a polypeptide;
D is a drug;
p is 1-10.

**7.** A drug conjugate of Formula I-12, I-12-1, I-13, I-13-1, I-14, I-14-1, I-15, I-15-1, I-16, I-16-1, I-17, I-17-1, I-18, I-18-1, I-19, I-19-1, I-20, I-20-1, I-21, I-21-1, I-22, I-22-1, I-23, I-23-1, I-24, I-24-1, I-25 or I-25-1 or a stereoisomer thereof or a pharmaceutically acceptable salt or solvate thereof, wherein Formulas I-12, I-12-1, I-13, I-13-1, I-14, I-14-1, I-15, I-15-1, I-16, I-16-1, I-17, I-17-1, I-18, I-18-1, I-19, I-19-1, I-20, I-20-1, I-21, I-21-1, I-22, I-22-1, I-23, I-23-1, I-24, I-24-1, I-25 and I-2 5-1 are:

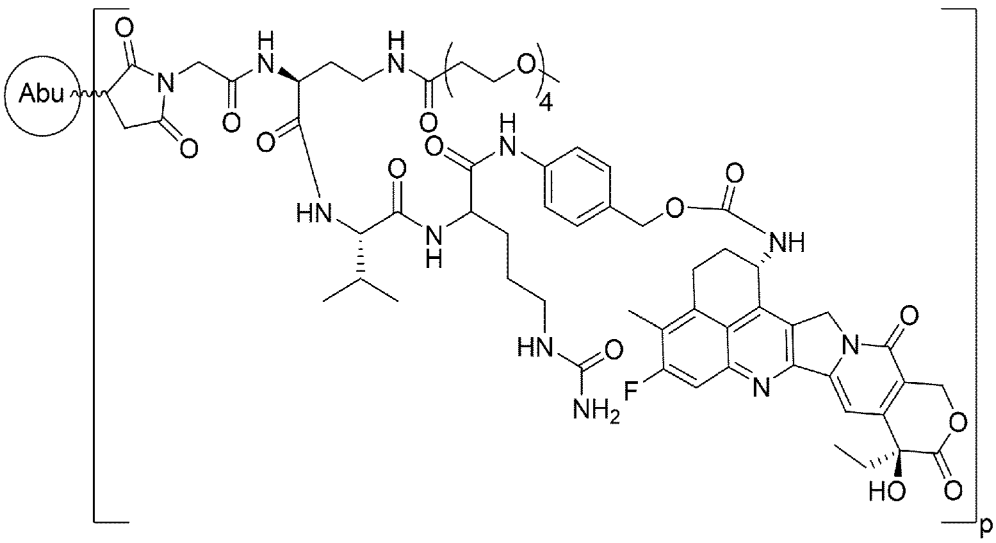

Formula I-12

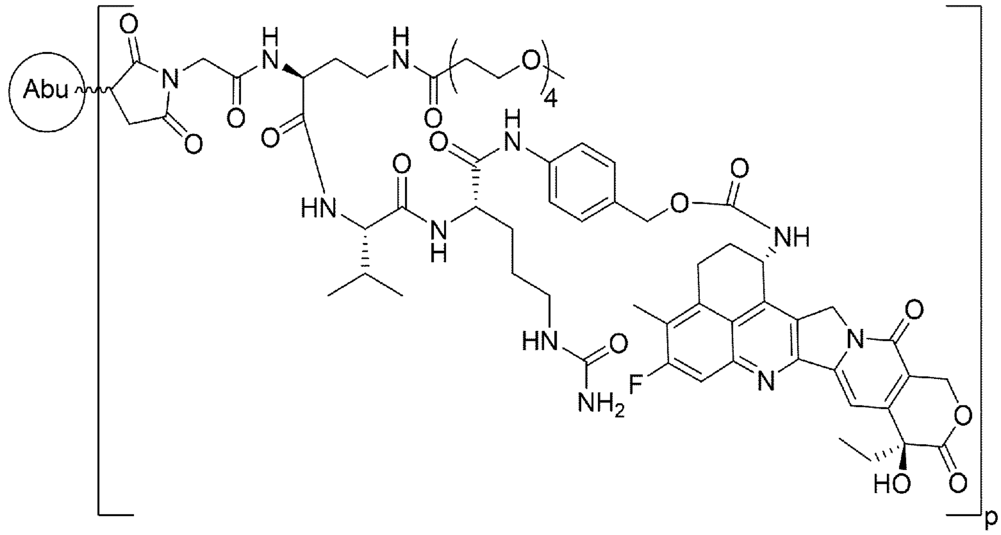

Formula I-12-1

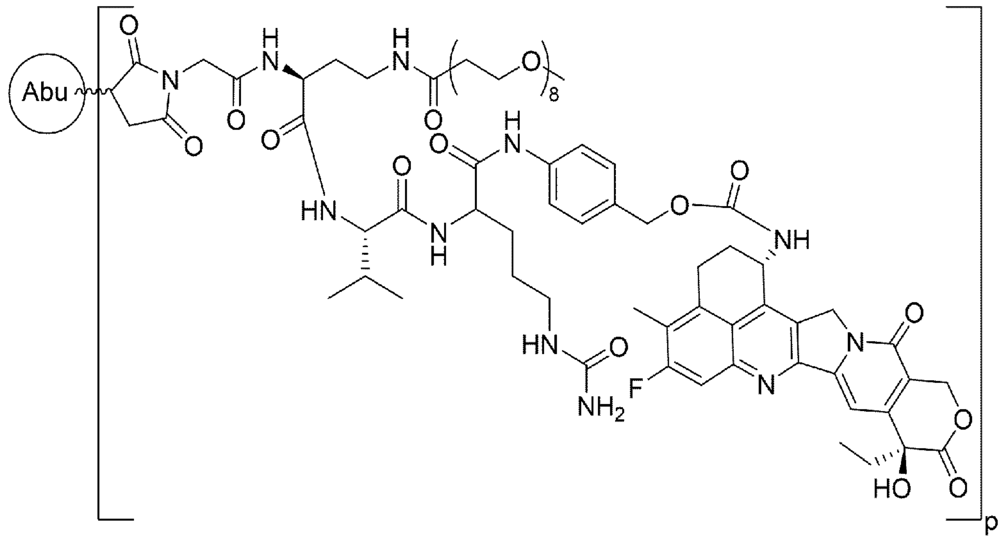

Formula I-13

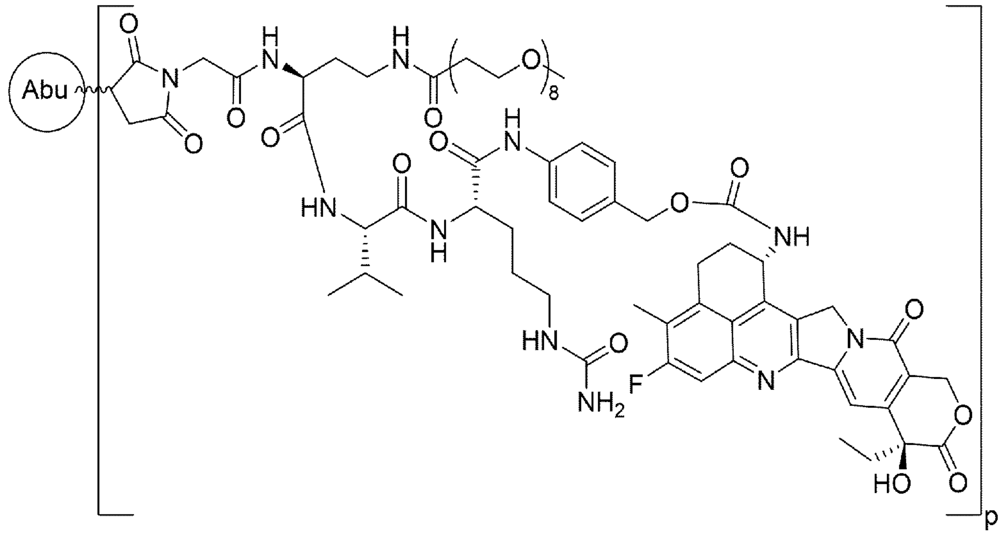

Formula I-13-1

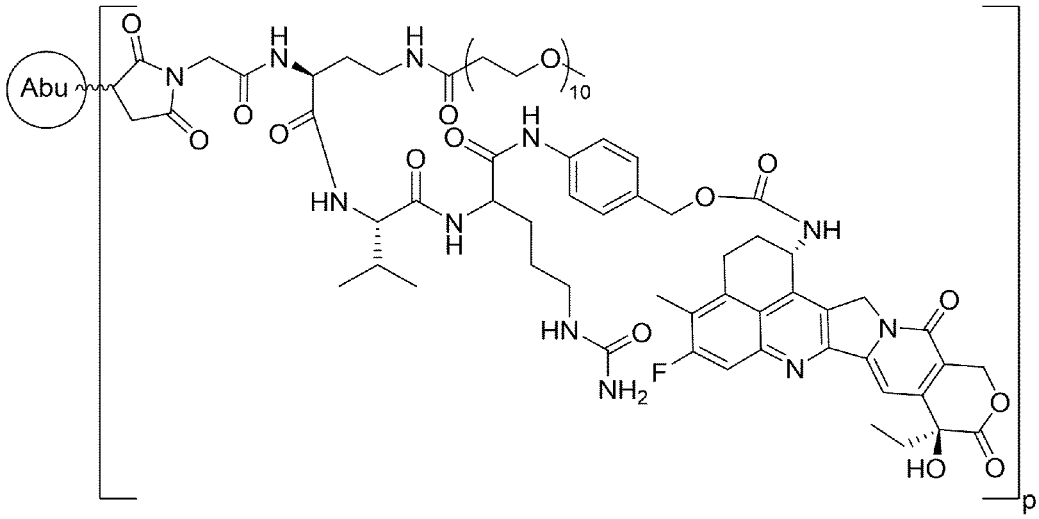

Formula I-14

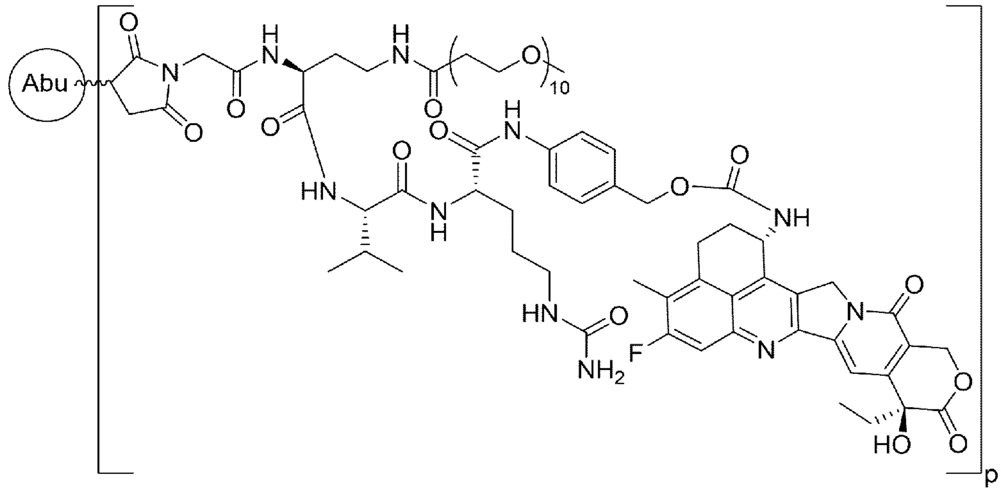

Formula I-14-1

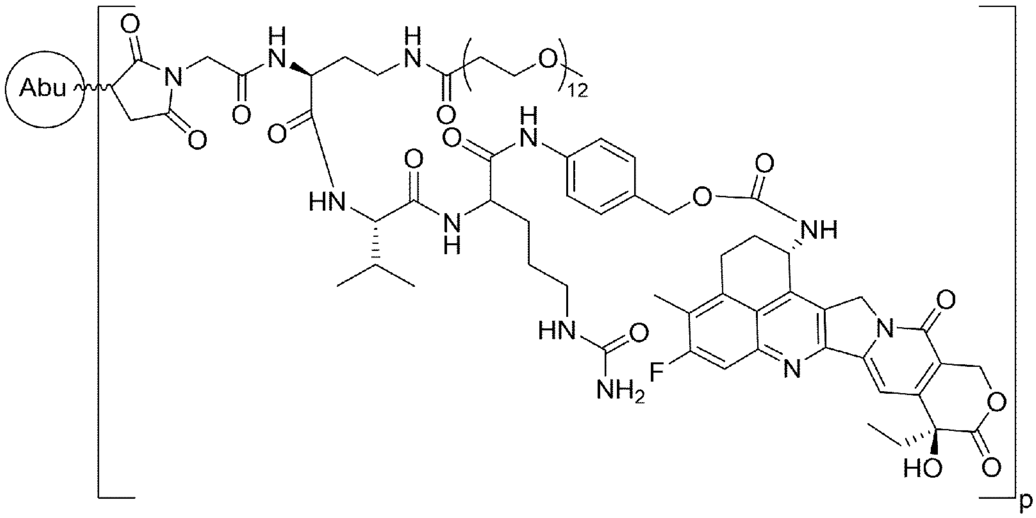

Formula I-15

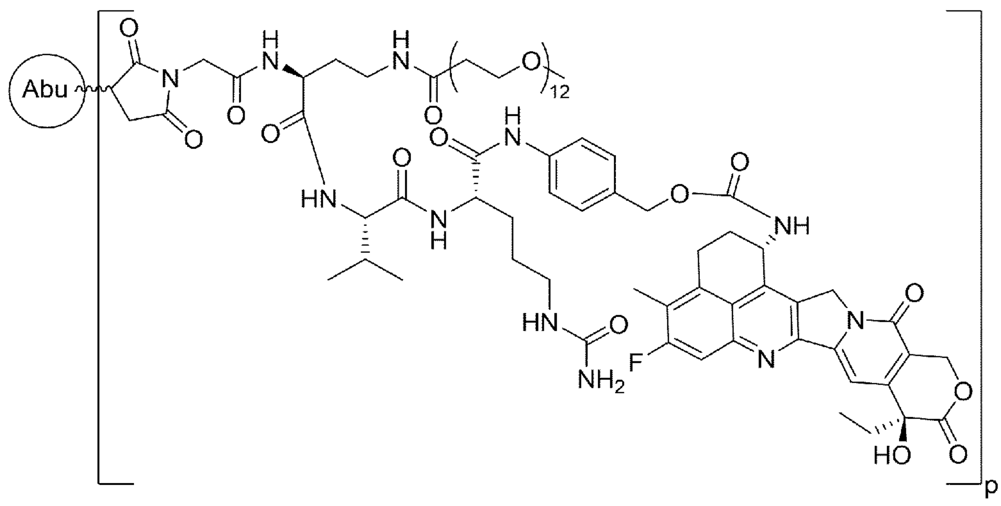

Formula I-15-1

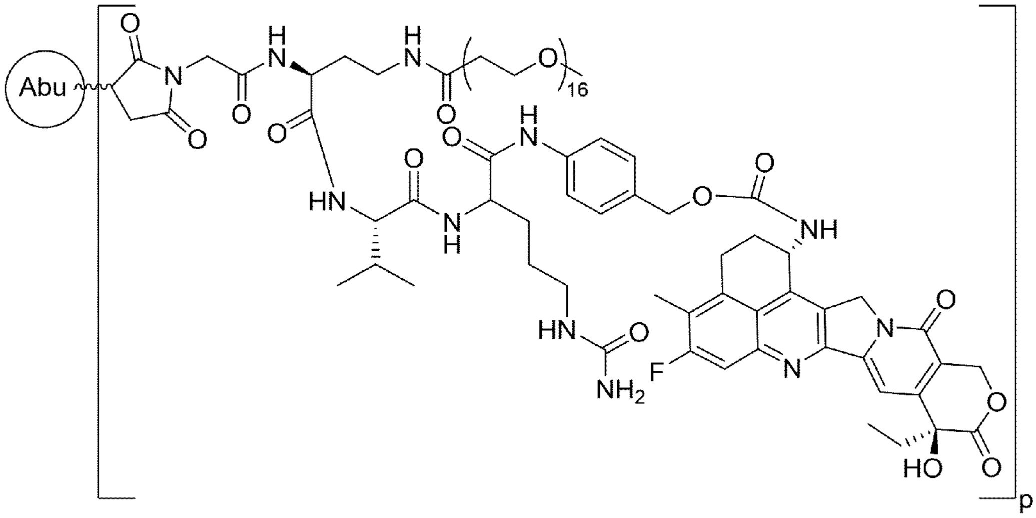

Formula I-16

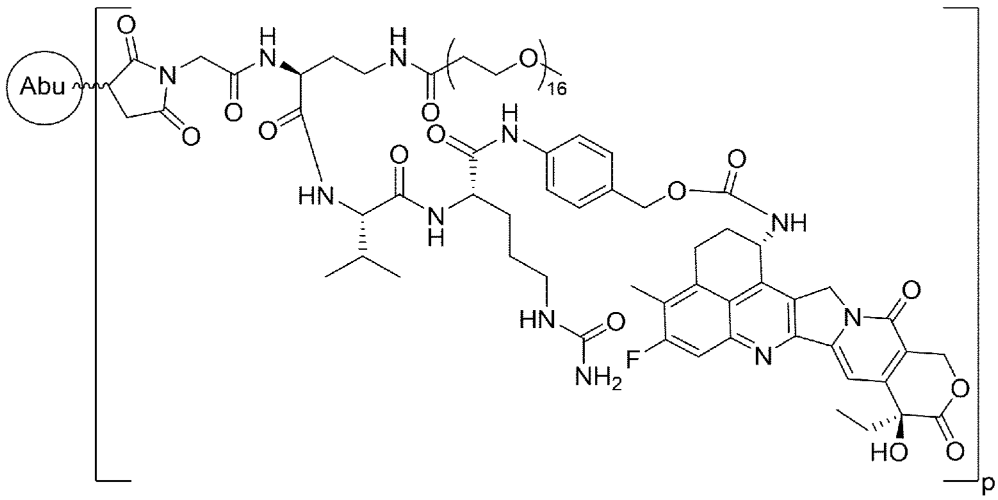

Formula I-16-1

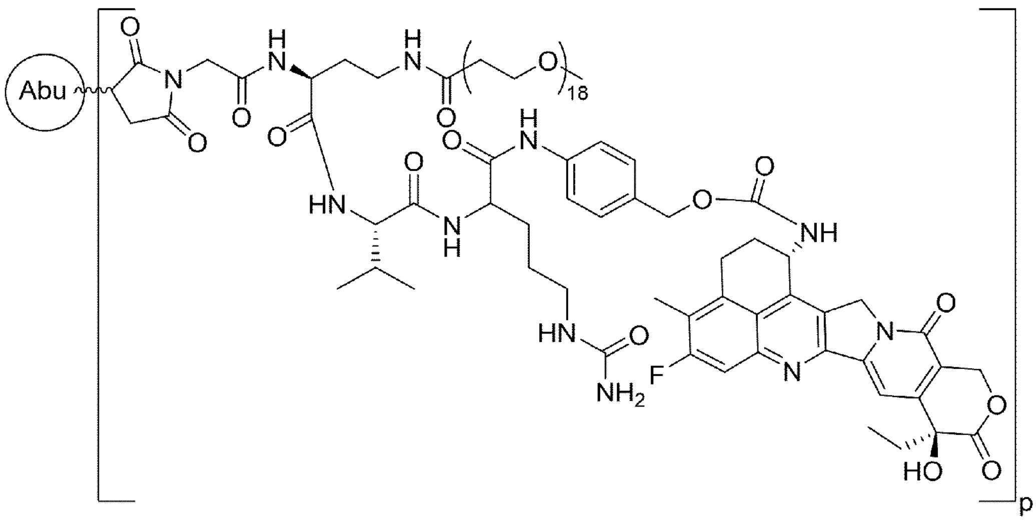

Formula I-17

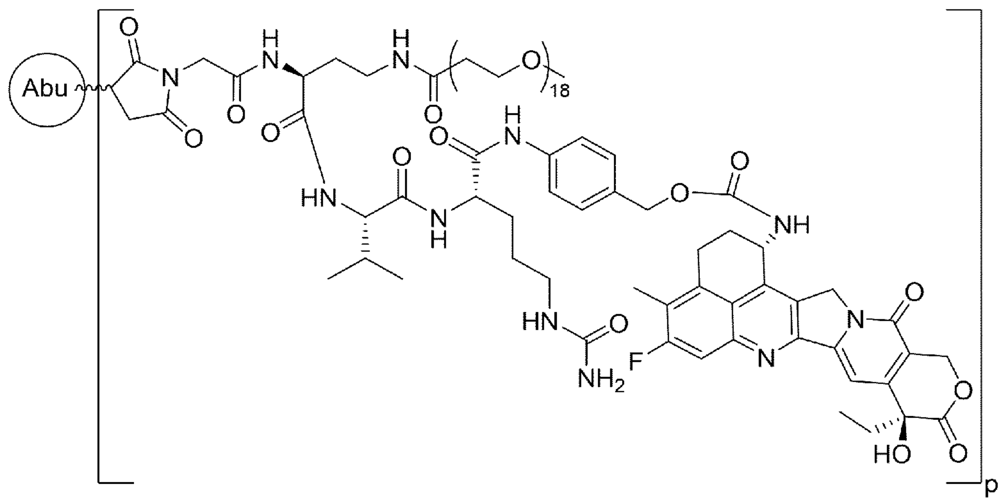

Formula I-17-1

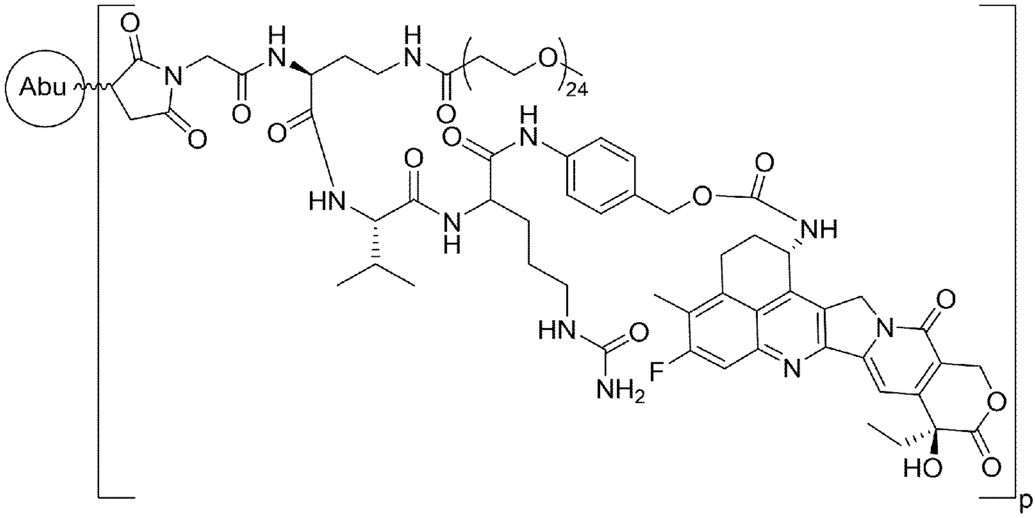

Formula I-18

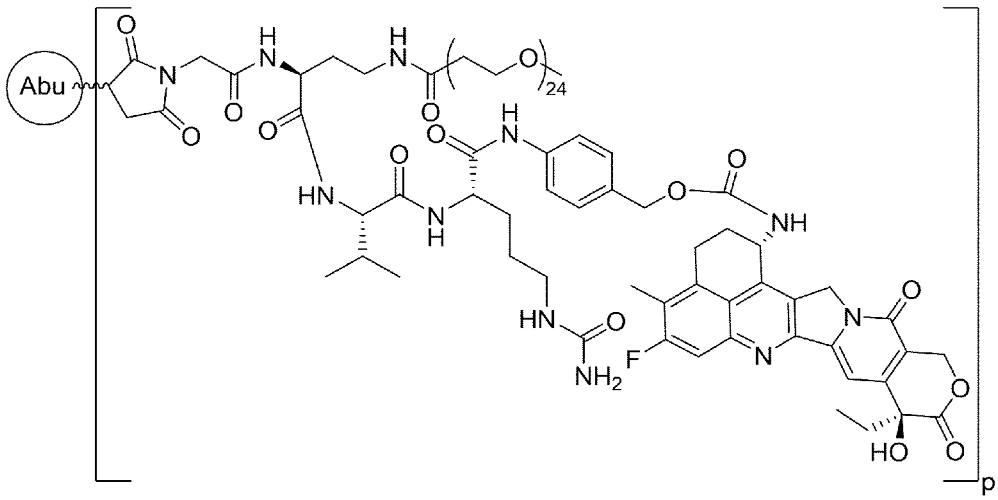

Formula I-18-1

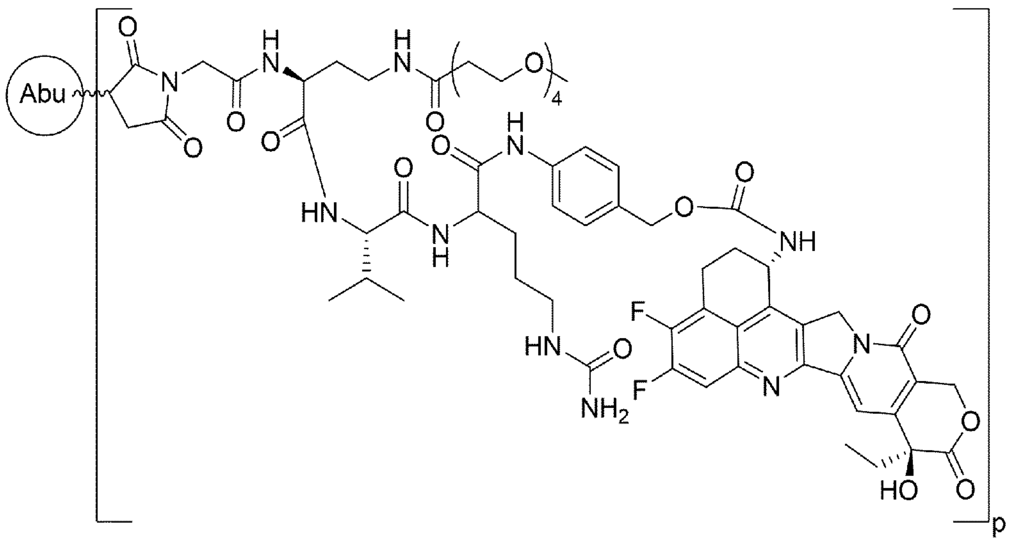

Formula I-19

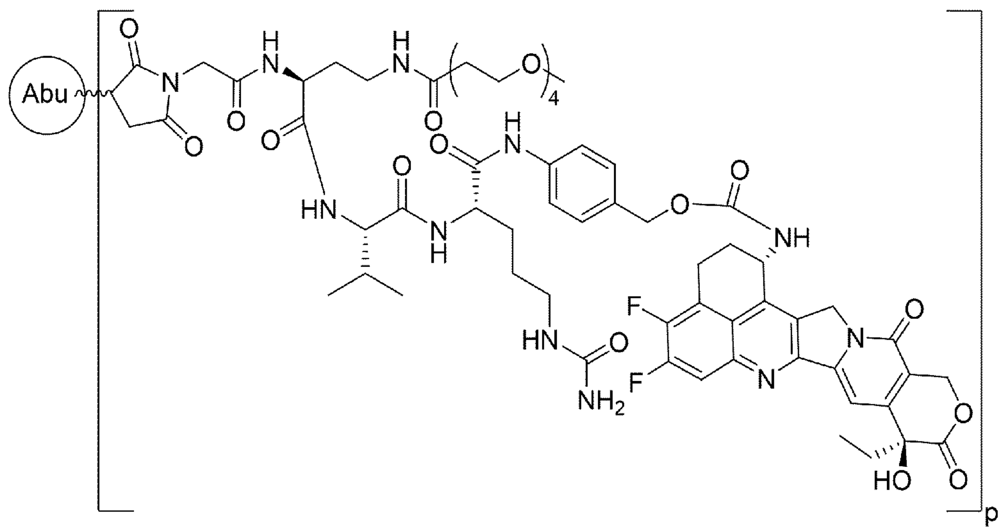

Formula I-19-1

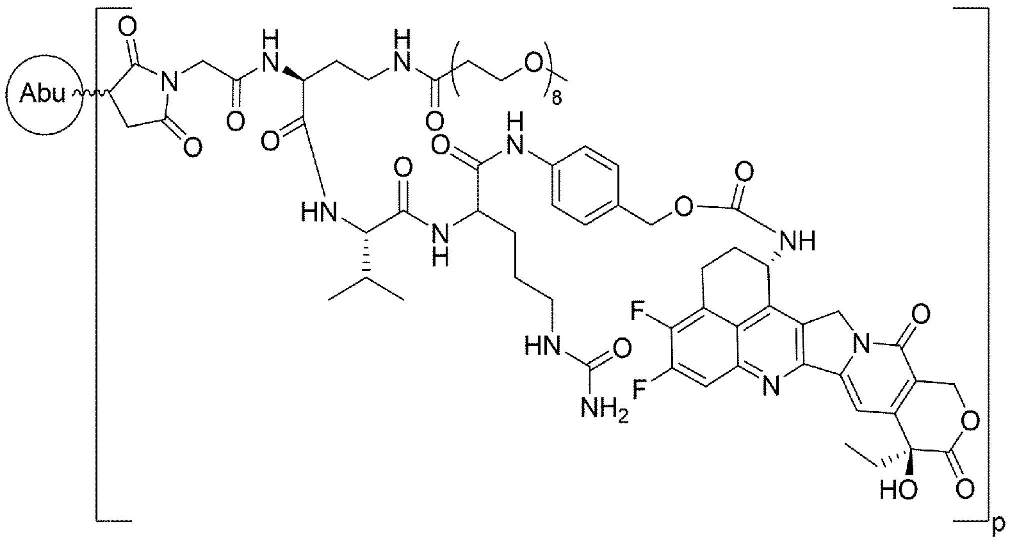

Formula I-20

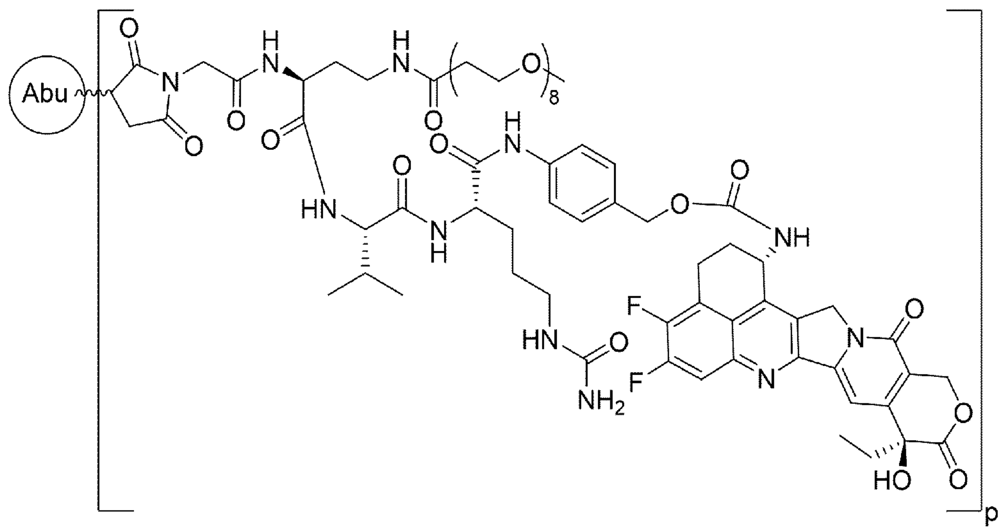

Formula I-20-1

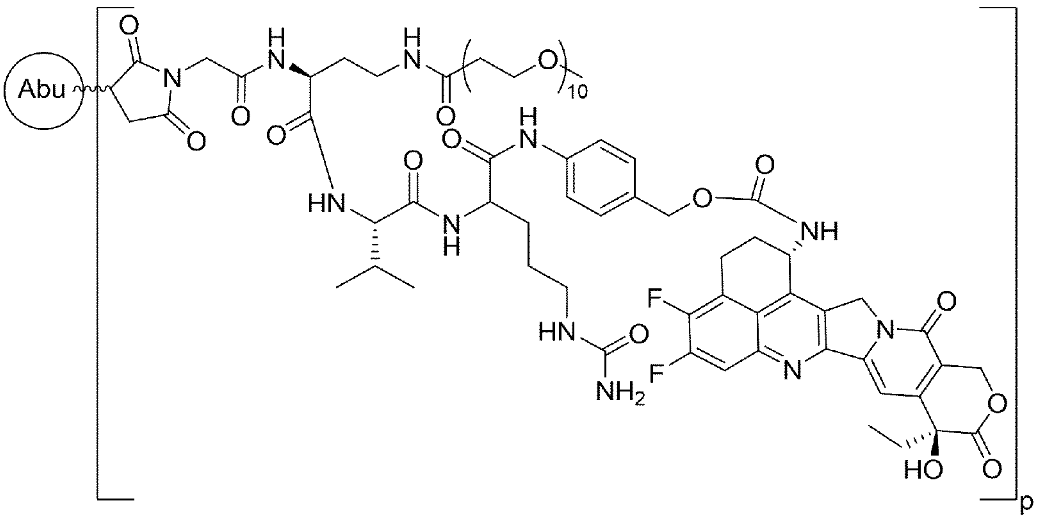

Formula I-21

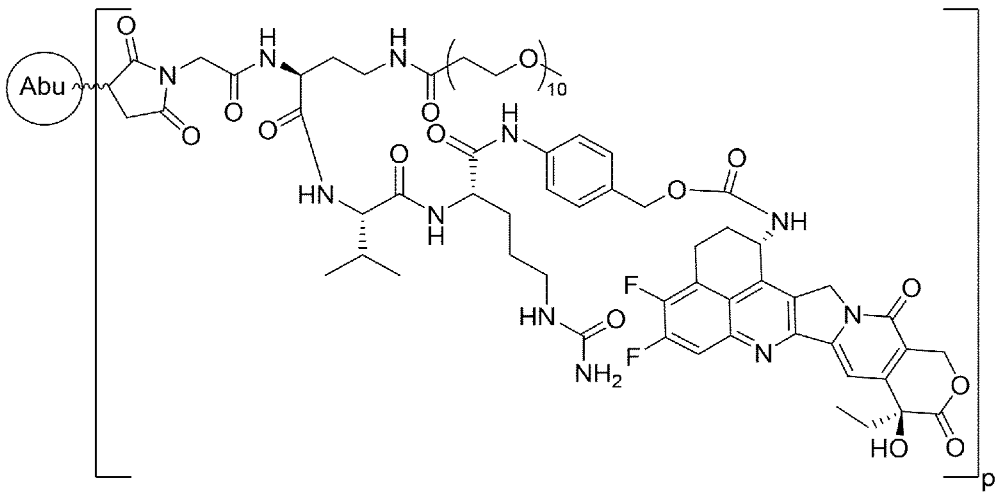

Formula I-21-1

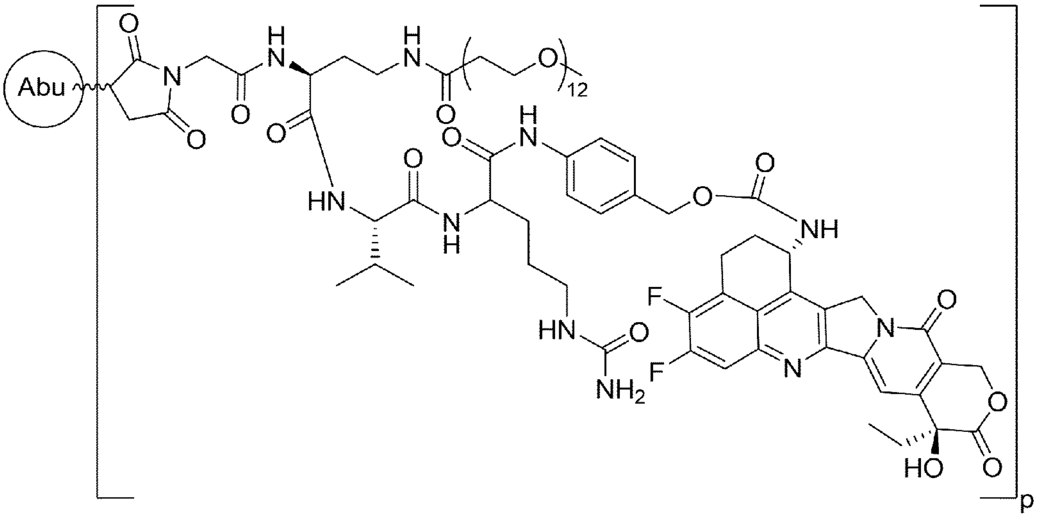

Formula I-22

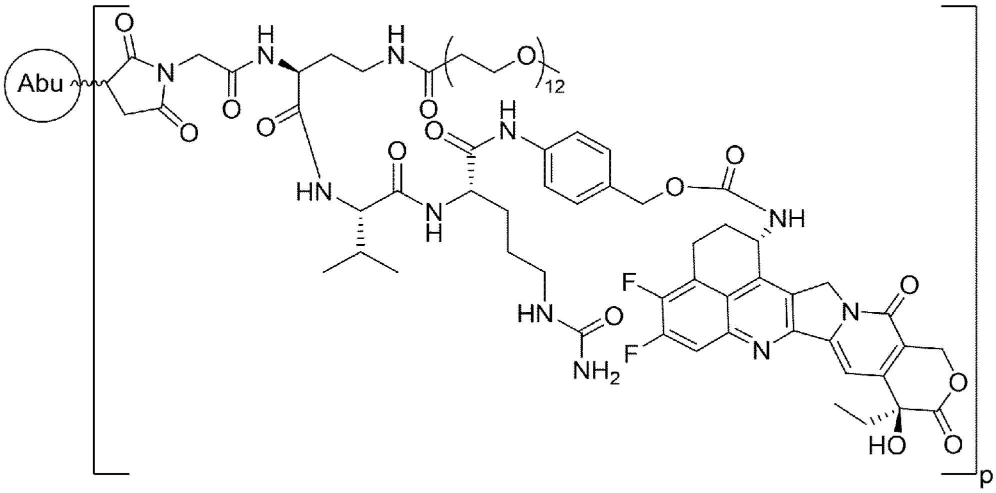

Formula I-22-1

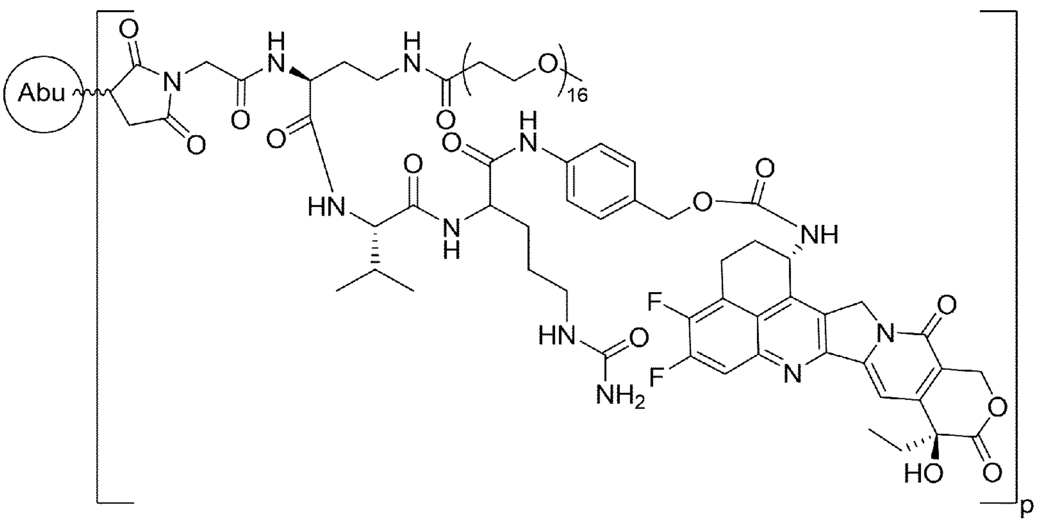

Formula I-23

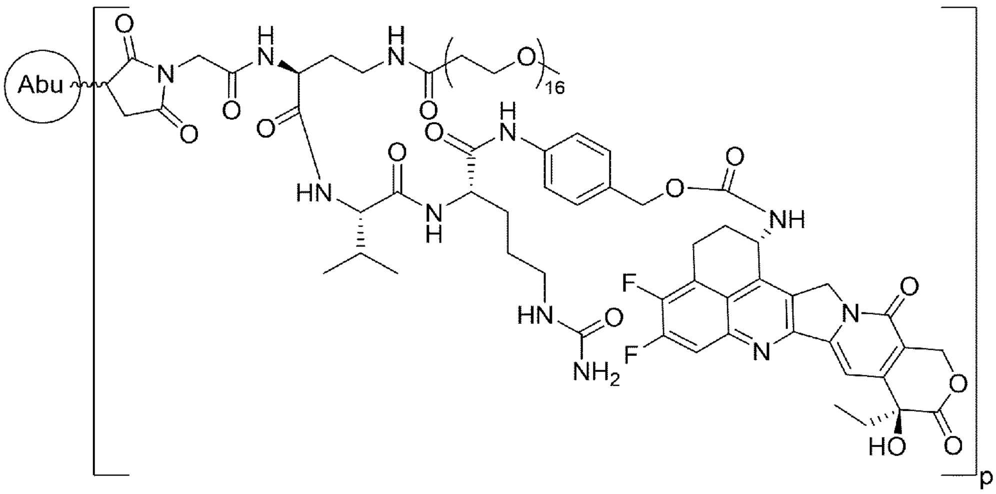

Formula I-23-1

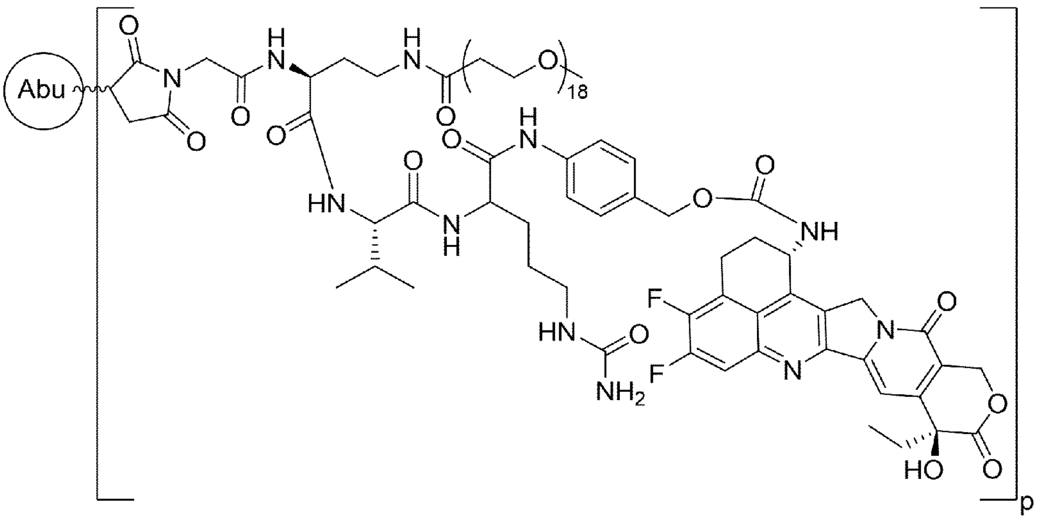

Formula I-24

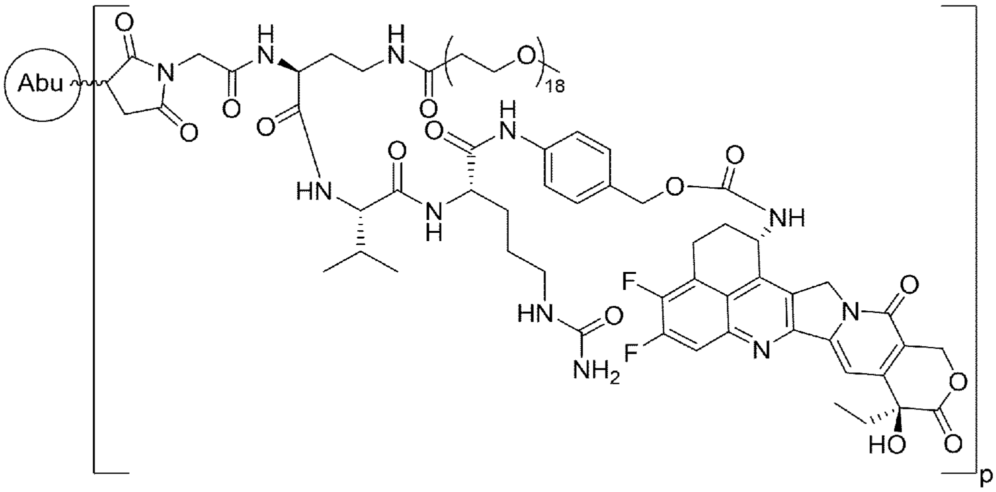

Formula I-24-1

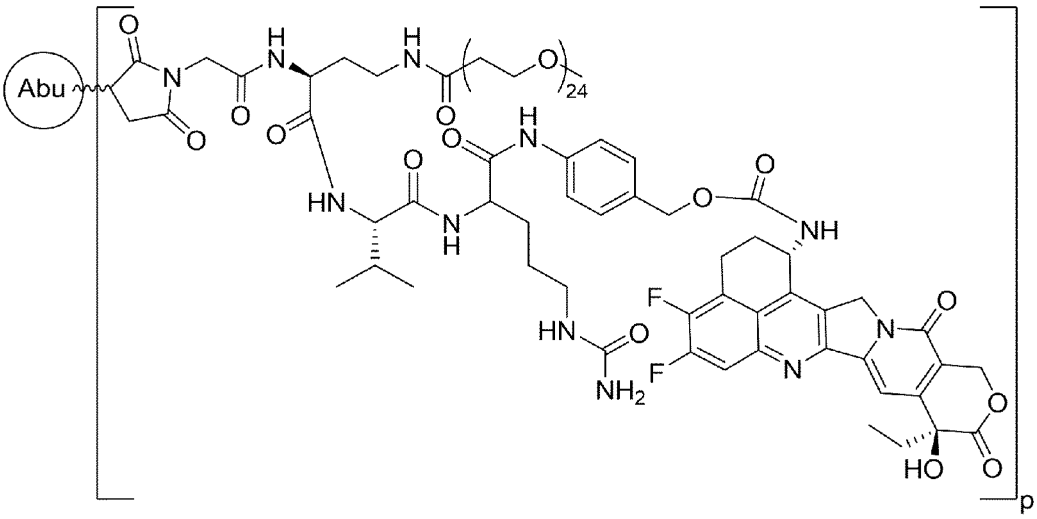

Formula I-25

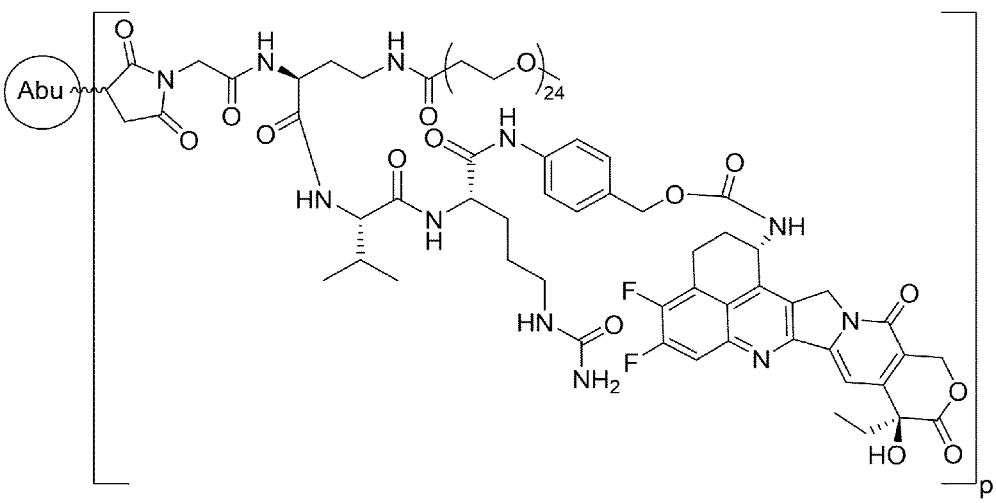

Formula I-25-1

wherein

Abu is a polypeptide;
p is 1-10.

**8.** The drug conjugate according to any one of claims 1-7, wherein the amino acid sequence of Abu contains one or more cysteine, and is connected to other parts of the drug conjugate through the sulfur atom of cysteine.

**9.** The drug conjugate according to claim 8, which is an antibody-drug conjugate, wherein Abu is an antibody or an antigen-binding unit.

**10.** The drug conjugate according to claim 9, wherein the binding target for Abu is selected from: HER2, TROP-2, Nection-4, B7H3, B7H4, CLDN18, BMPR1B, E16, STEAP1, 0772P, MPF, Napi3b, Sema5b, PSCAhlg, ETBR, MSG783, STEAP2, TrpM4, CRIPTO, CD20, CD21, CD22, CD30, FcRH2, NCA, MDP, IL20R α , Brevican, EphB2R, ASLG659, PSCA, GEDA, BAFF-R, CD79a, CD79b, CXCR5, HLA-DOB, P2X5, CD72, LY64, FcRH1, IRTA2, TENB2, PMEL17, TMEFF1, GDNF-Ra1, Ly6E, TMEM46, Ly6G6D, LGR5, RET, LY6K, GPR19, GPR54, ASPHD1, tyrosinase, TMEM118, EpCAM, ROR1, GPR172A, and FRalpha.

**11.** A compound , wherein the compound has a structure shown as Formula II or a stereoisomer thereof or a pharmaceutically acceptable salt or solvate thereof:

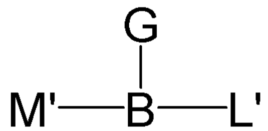

Formula II

wherein

M' is

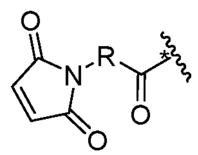

,

wherein * links to B, and R is selected from: $-(CH_2)_r-$, $-(CHR^m)_r-$, C3-C8 carbocyclyl, $-O-(CH_2)_r-$, arylene, $-(CH_2)_r$-arylene-, -arylene-$(CH_2)$r-, $-(CH_2)_r$-(C3-C8 carbocyclyl)-, -(C3-C8 carbocyclyl)-$(CH_2)_r$, C3-C8 heterocyclyl, $-(CH_2)_r$-(C3-C8 heterocyclyl)-, -(C3-C8 heterocyclyl)-$(CH_2)_r-$, $-(CH_2)_rC(O)NR^m(CH_2)_r-$, $-(CH_2CH_2O)_r-$, $-(CH_2CH_2O)_r-CH_2-$, $-(CH_2)_rC(O)NR^m(CH_2CH_2O)_r-$, $-(CH_2)_rC(O)NR^m(CH_2CH_2O)_r-CH_2-$, $-(CH_2CH_2O)_rC(O)NR^m(CH_2CH_2O)_r-$, $-(CH_2CH_2O)_rC(O)NR^m(CH_2CH_2O)_r-CH_2-$ and $-(CH_2CH_2O)_rC(O)NR^m(CH_2)_r-$; wherein each $R^m$ is independently H, C1-C6 alkyl, C3-C8 carbocyclyl, phenyl or benzyl, and each r is independently 1, 2, 3, 4, 5, 6, 7, 8, 9 or 10;
B is

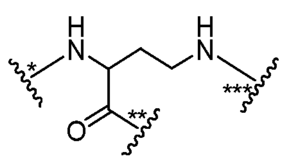

,

wherein * links to M', ** links to L', and *** links to G;
L' is -(AA)i-(FF')f-, wherein AA is an amino acid or polypeptide, and i is 1, 2, 3, 4, 5, 6, 7, 8, 9, 10, 11, 12, 13,

14, 15, 16, 17, 18, 19 or 20; each FF' is independently

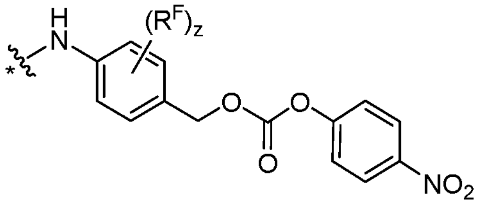,
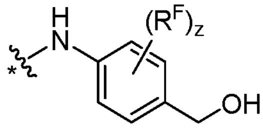,

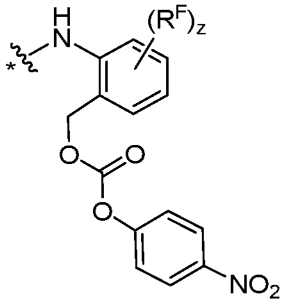
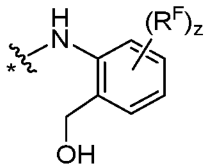,
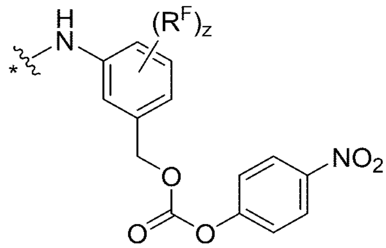

or

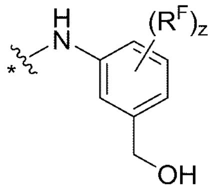,

wherein each $R^F$ is independently C1-C6 alkyl, C1-C6 alkoxy, $-NO_2$ or halogen; or z is 0, 1, 2, 3 or 4, and f is 1, 2, 3, 4, 5, 6, 7, 8, 9 or 10; wherein * links to AA; G is

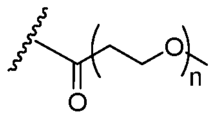,

wherein n is 1-24.

**12.** A compound, wherein the compound has a structure shown as Formula II-1A or Formula II-1B or a stereoisomer thereof or a pharmaceutically acceptable salt or solvate thereof:

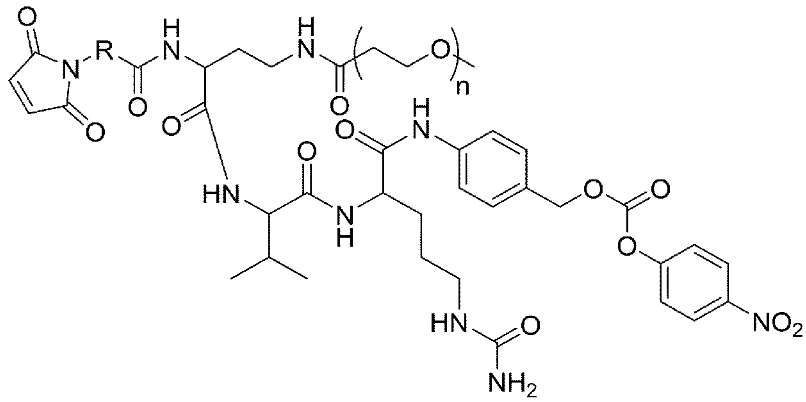

Formula II-1A

or

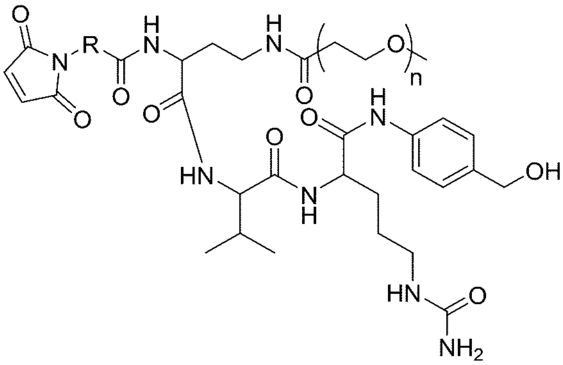

Formula II-1B

wherein

R is selected from: $-(CH_2)_r-$, $-(CHR^m)_r$, C3-C8 carbocyclyl, $-O-(CH_2)_r-$, arylene, $-(CH_2)_r$-arylene-, -arylene-$(CH_2)$r-, $-(CH_2)_r$-(C3-C8 carbocyclyl)-, -(C3-C8 carbocyclyl)-$(CH_2)_r-$, C3-C8 heterocyclyl, $-(CH_2)_r$-(C3-C8 heterocyclyl)-, -(C3-C8 heterocyclyl)-$(CH_2)_r-$, $-(CH_2)_rC(O)NR^m(CH_2)_r-$, $-(CH_2CH_2O)_r-$, $-(CH_2CH_2O)_r-CH_2-$, $-(CH_2)_rC(O)NR^m(CH_2CH_2O)_r-$, $-(CH_2)_rC(O)NR^m(CH_2CH_2O)_r-CH_2-$, $-(CH_2CH_2O)_rC(O)NR^m(CH_2CH_2O)_r-$, $-(CH_2CH_2O)_rC(O)NR^m(CH_2CH_2O)_r-CH_2-$ and $-(CH_2CH_2O)_rC(O)NR^m(CH_2)_r-$; wherein each $R^m$ is independently H, C1-C6 alkyl, C3-C8 carbocyclyl, phenyl or benzyl, and each r is independently 1, 2, 3, 4, 5, 6, 7, 8, 9 or 10; n is 1-24.

**13.** A compound or a stereoisomer thereof or a pharmaceutically acceptable salt or solvate thereof, wherein the compound is:

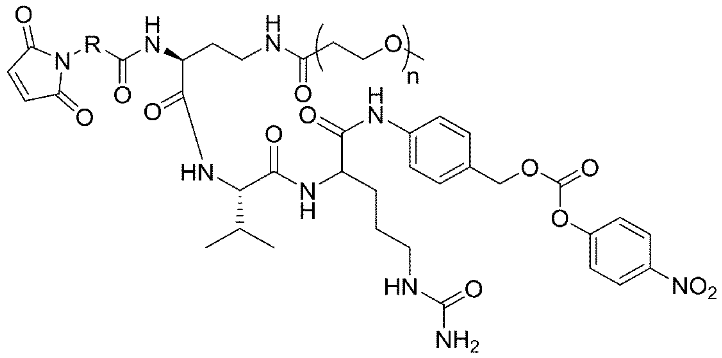

Formula II-2A

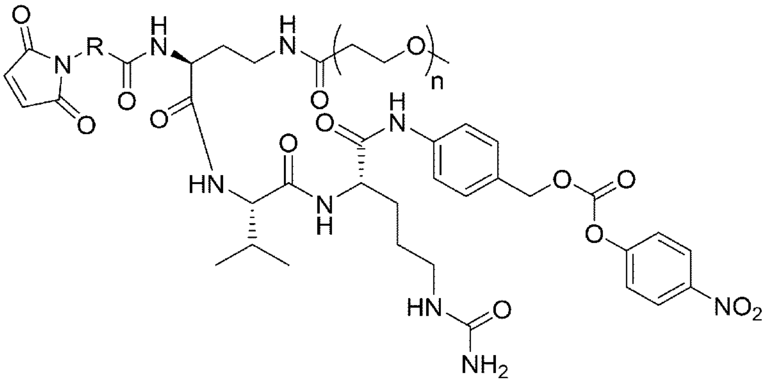

Formula II-2A-1

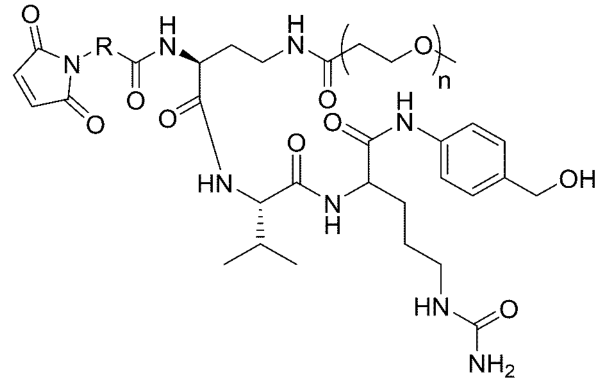

Formula II-2B

or

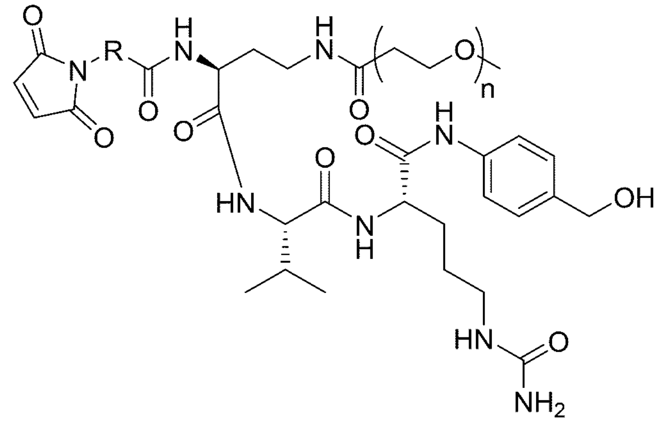

Formula II-2B-1

wherein

R is selected from: $-(CH_2)_r-$, $-(CHR^m)_r$, C3-C8 carbocyclyl, $-O-(CH_2)_r-$, arylene, $-(CH_2)_r$-arylene-, -arylene-$(CH_2)$r-, $-(CH_2)_r$-(C3-C8 carbocyclyl)-, -(C3-C8 carbocyclyl)-$(CH_2)_r$-, C3-C8 heterocyclyl, $-(CH_2)_r$-(C3-C8 heterocyclyl)-, -(C3-C8 heterocyclyl)-$(CH_2)_r$-, $-(CH_2)_rC(O)NR^m(CH_2)_r-$, $-(CH_2CH_2O)_r-$, $-(CH_2CH_2O)_r-CH_2-$, $-(CH_2)_rC(O)NR^m(CH_2CH_2O)_r-$, $-(CH_2)_rC(O)NR^m(CH_2CH_2O)_r-CH_2-$, $-(CH_2CH_2O)_rC(O)NR^m(CH_2CH_2O)_r-$, $-(CH_2CH_2O)_rC(O)NR^m(CH_2CH_2O)_r-CH_2-$ and $-(CH_2CH_2O)_rC(O)NR^m(CH_2)_r-$; wherein each $R^m$ is independently H, C1-C6 alkyl, C3-C8 carbocyclyl, phenyl or benzyl, and each r is independently 1, 2, 3, 4, 5, 6, 7, 8, 9 or 10; n is 1-24.

**14.** A compound or a pharmaceutically acceptable salt or solvate thereof, wherein the compound is:

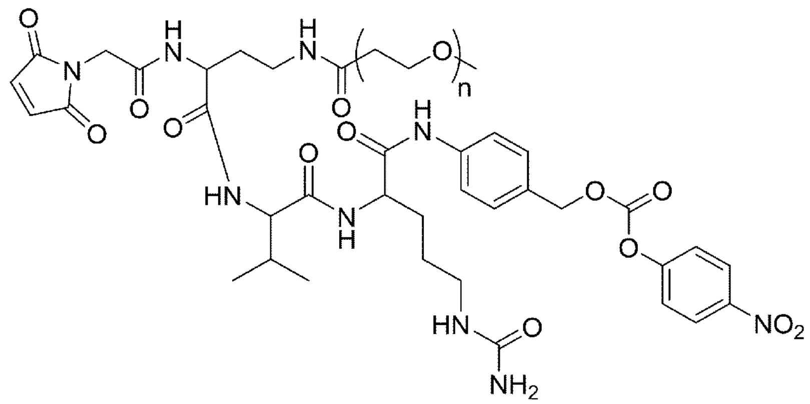

or

Formula II-3A

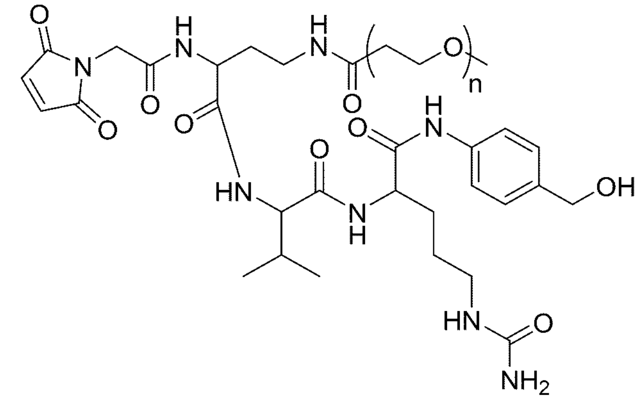

Formula II-3B

wherein n is 1-24.

**15.** A compound or a pharmaceutically acceptable salt or solvate thereof, wherein the compound is:

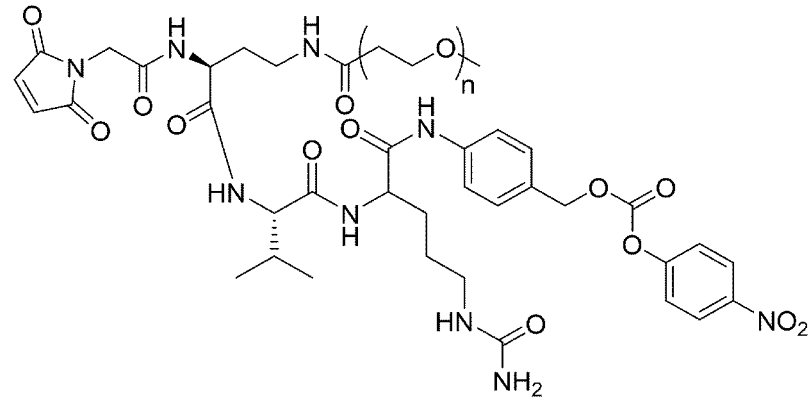

Formula II-4A

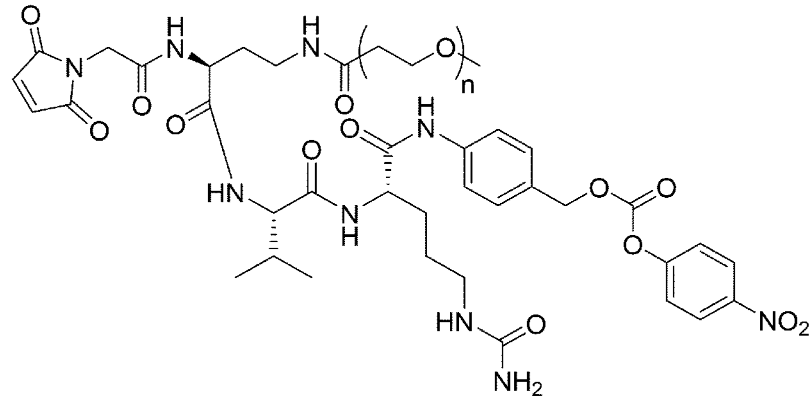

Formula II-4A-1

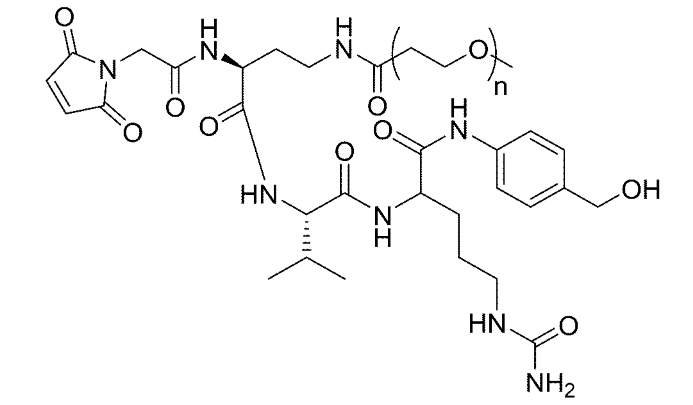

Formula II-4B

or

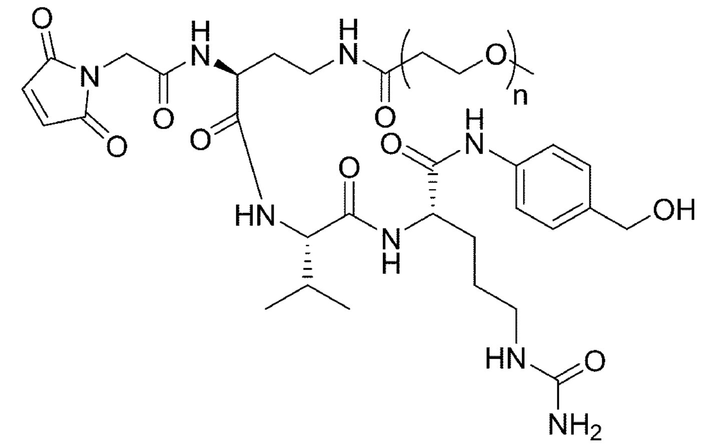

Formula II-4B-1

wherein n is 1-24.

**16.** A compound or a pharmaceutically acceptable salt or solvate thereof, wherein the compound is:

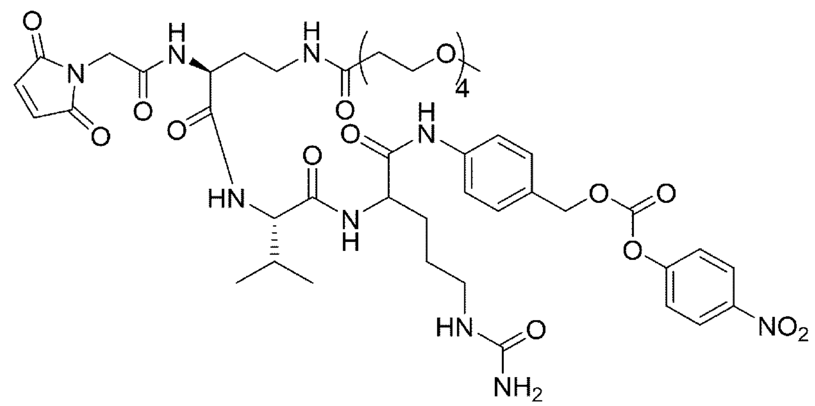

Formula II-5A

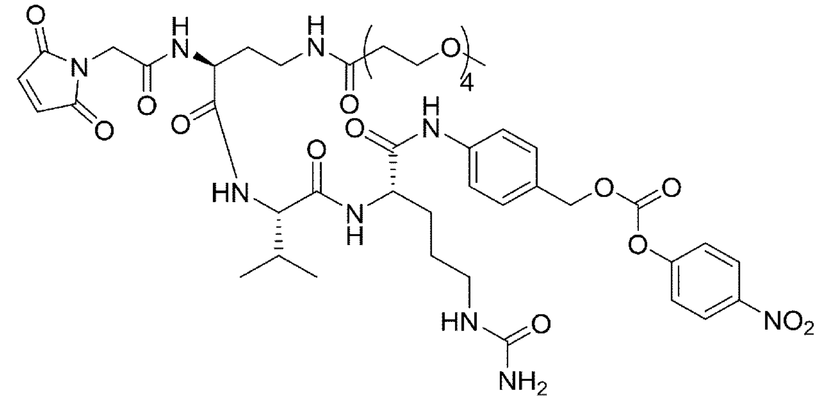

Formula II-5A-1

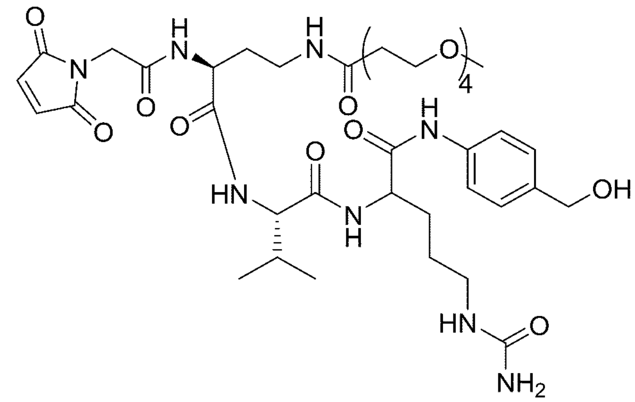

Formula II-5B

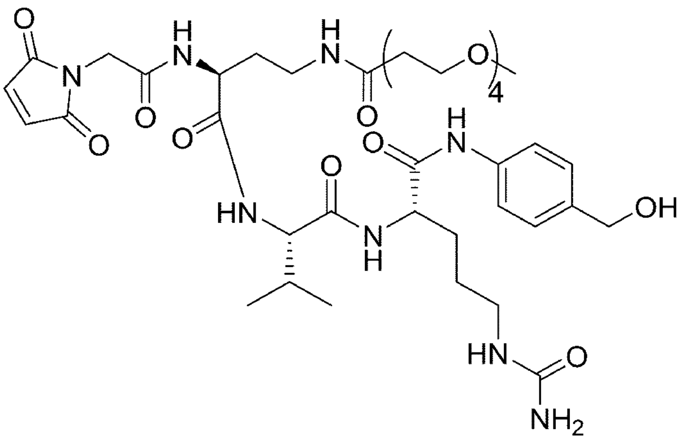

Formula II-5B-1

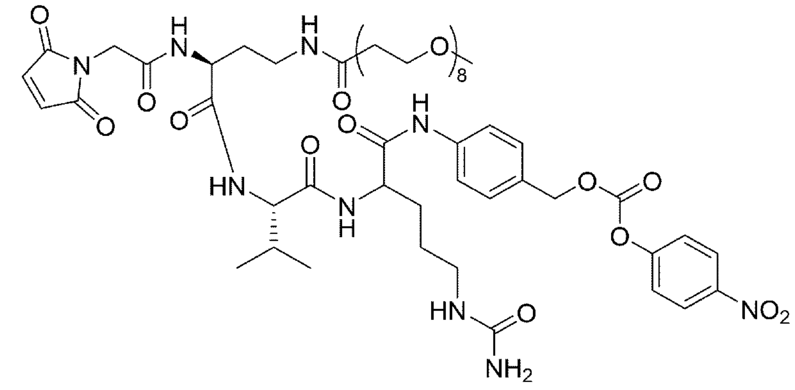

Formula II-6A

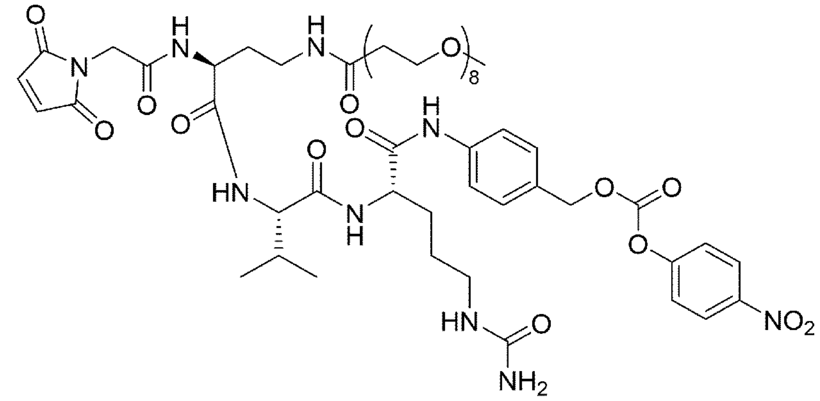

Formula II-6A-1

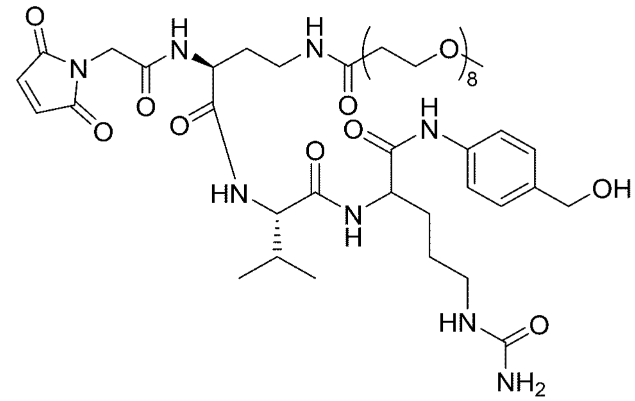

Formula II-6B

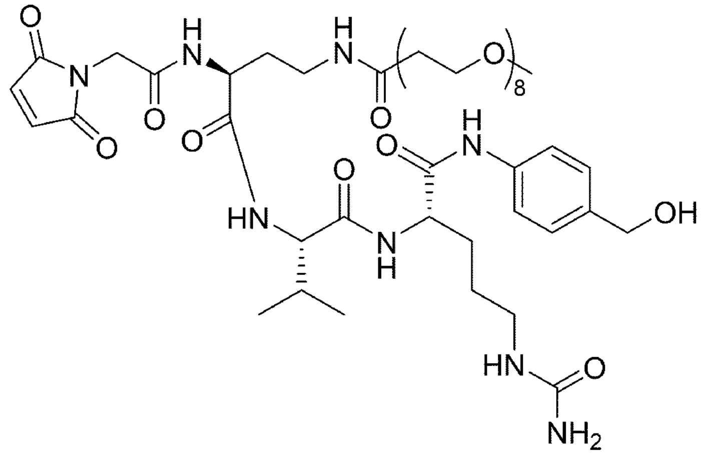

Formula II-6B-1

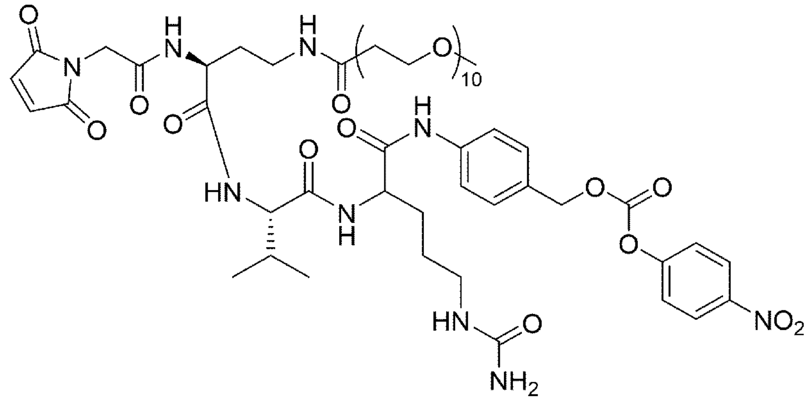

Formula II-7A

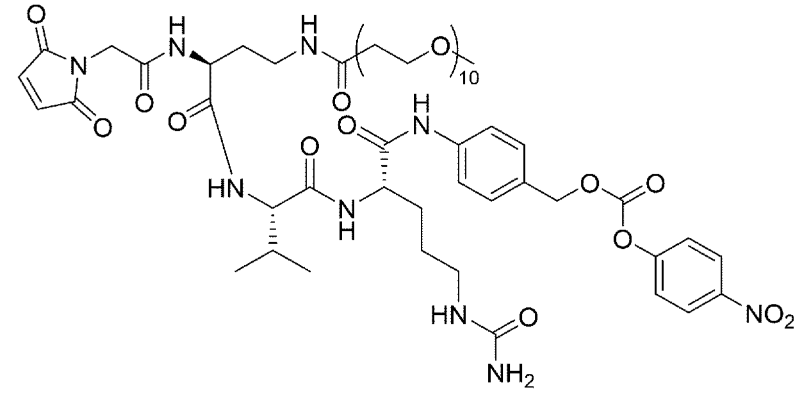

Formula II-7A-1

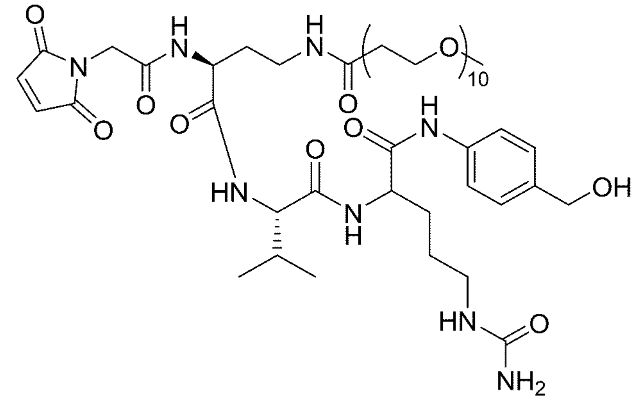

Formula II-7B

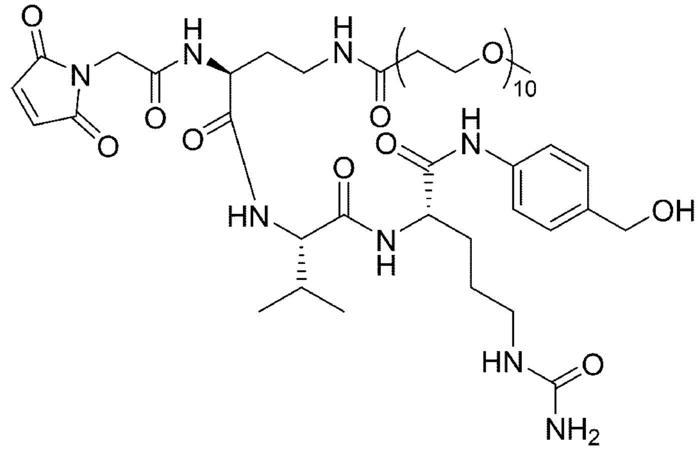

Formula II-7B-1

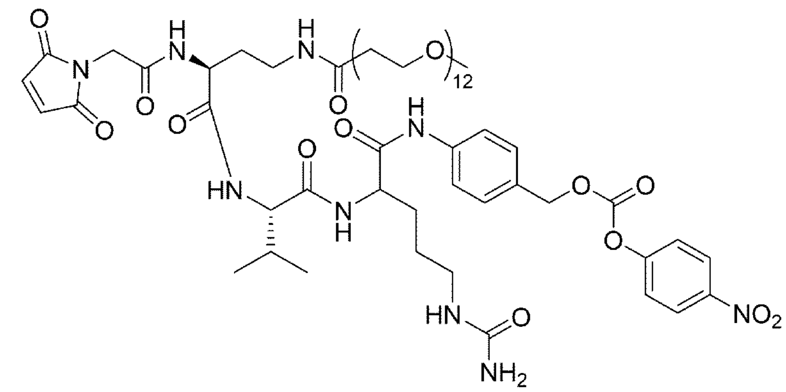

Formula II-8A

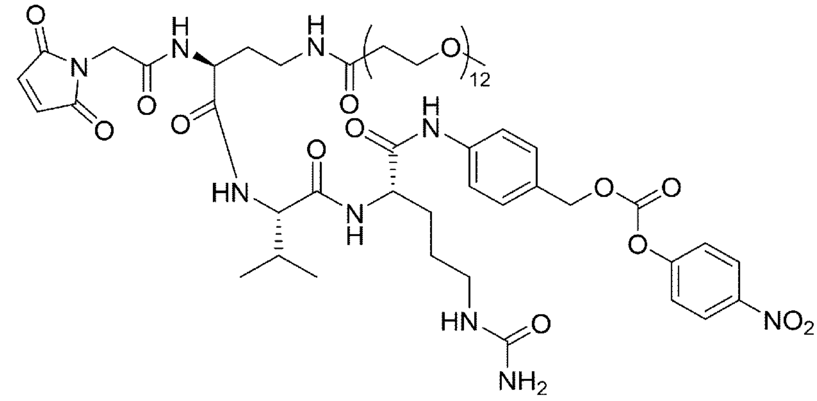

Formula II-8A-1

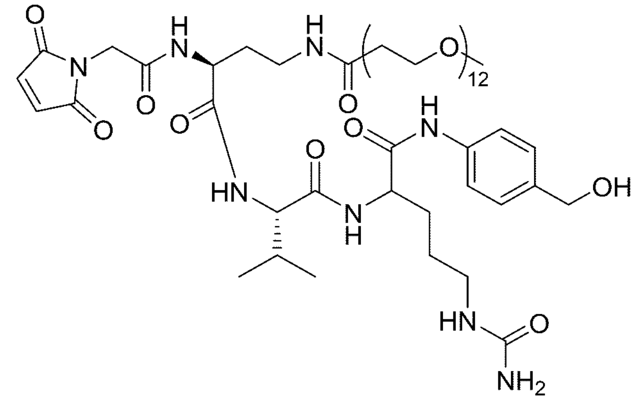

Formula II-8B

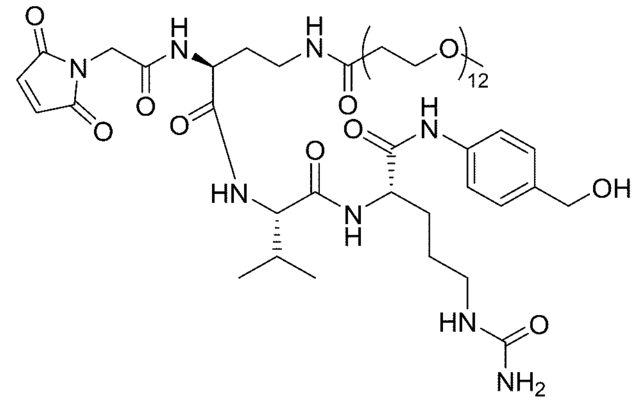

Formula II-8B-1

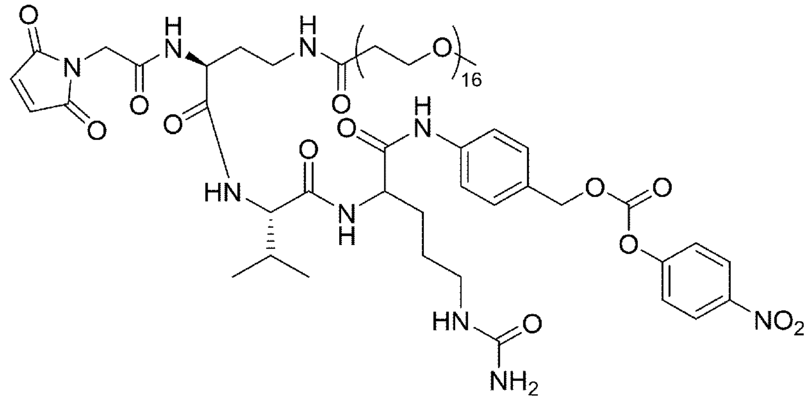

Formula II-9A

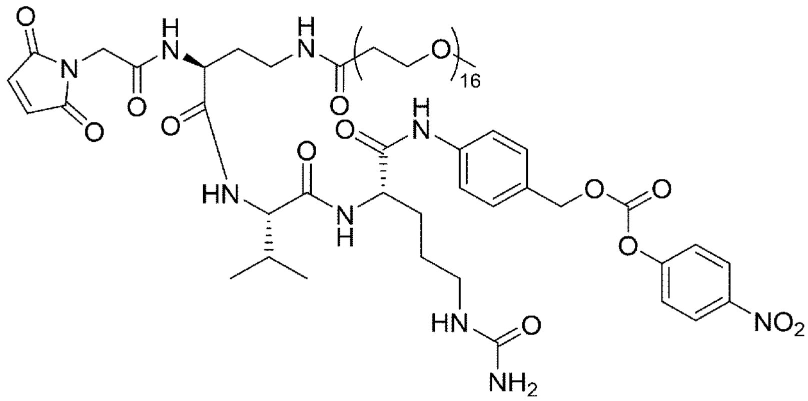

Formula II-9A-1

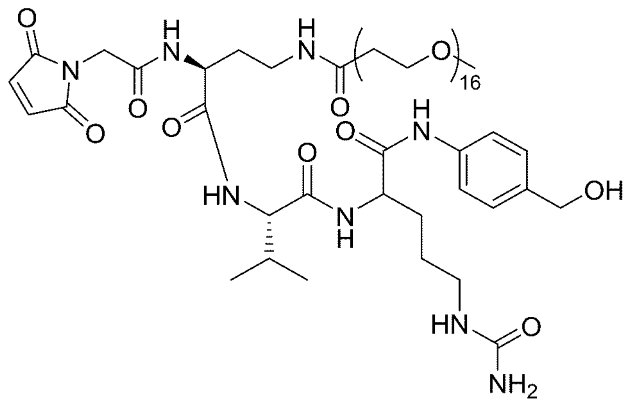

Formula II-9B

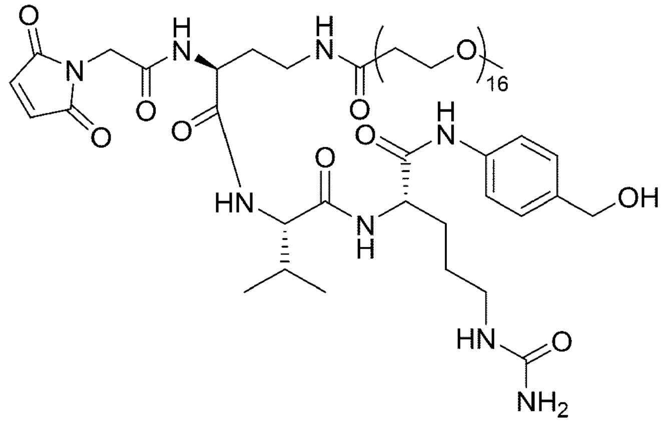

Formula II-9B-1

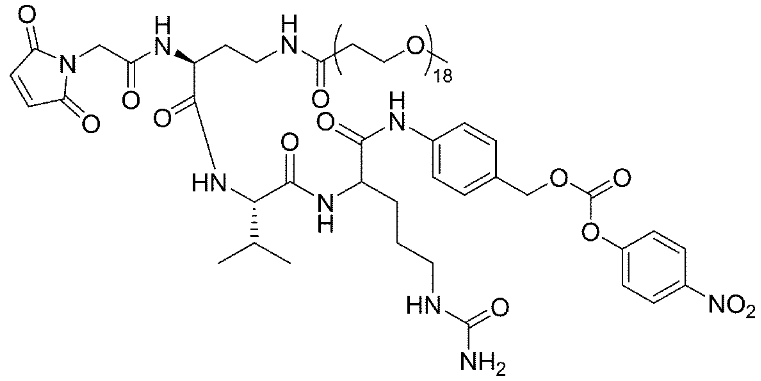

Formula II-10A

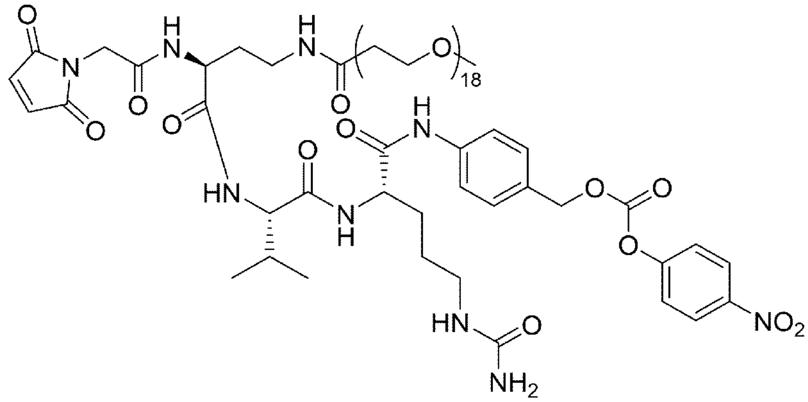

Formula II-10A-1

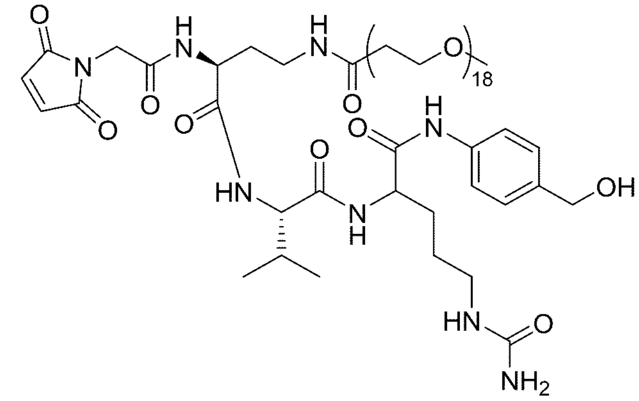

Formula II-10B

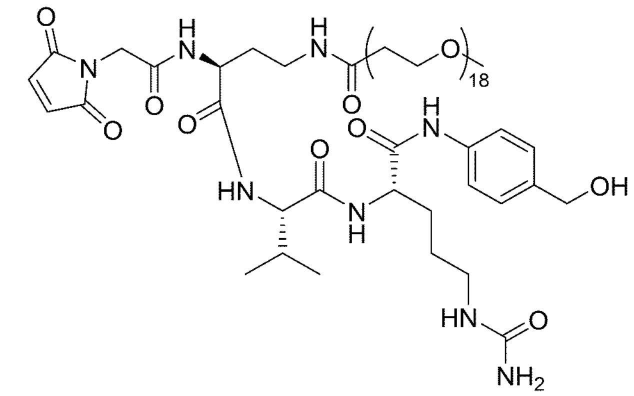

Formula II-10B-1

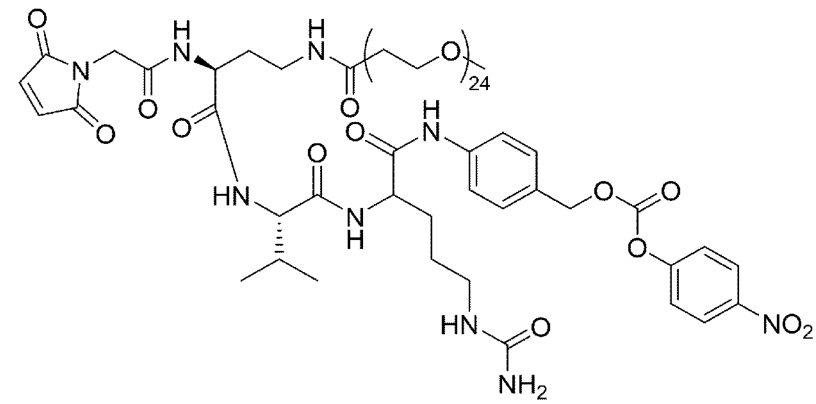

Formula II-11A

or

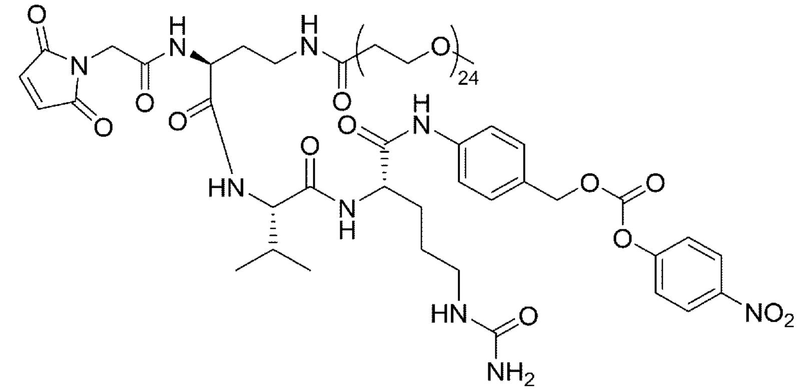

Formula II-11A-1

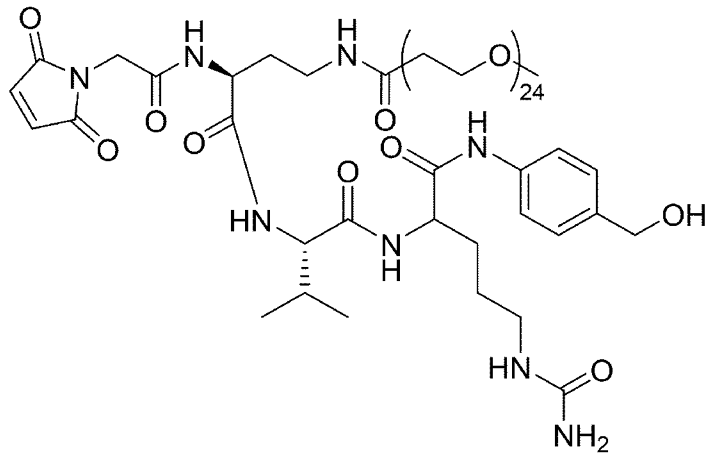

Formula II-11B

or

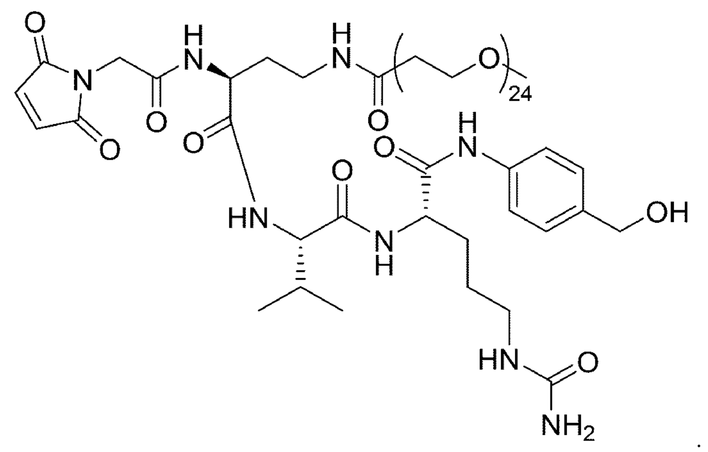

Formula II-11B-1

**17.** A compound of Formula III or a pharmaceutically acceptable salt or solvate thereof:

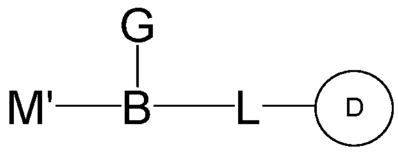

Formula III

D is a drug;

M' is

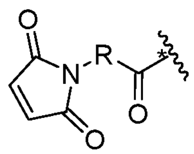

,

wherein * links to B, and R is selected from: $-(CH_2)_r-$, $-(CHR^m)_r-$, C3-C8 carbocyclyl, $-O-(CH_2)_r-$, arylene, $-(CH_2)_r$-arylene-, -arylene-$(CH_2)$r-, $-(CH_2)_r$-(C3-C8 carbocyclyl)-, -(C3-C8 carbocyclyl)-$(CH_2)_r$, C3-C8 heterocyclyl, $-(CH_2)_r$-(C3-C8 heterocyclyl)-, -(C3-C8 heterocyclyl)-$(CH_2)_r$-, $-(CH_2)_rC(O)NR^m(CH_2)_r-$, $-(CH_2CH_2O)_r-$, $-(CH_2CH_2O)_r-CH_2-$, $-(CH_2)_rC(O)NR^m(CH_2CH_2O)_r-$, $-(CH_2)_rC(O)NR^m(CH_2CH_2O)_r-CH_2-$, $-(CH_2CH_2O)_rC(O)NR^m(CH_2CH_2O)_r-$, $-(CH_2CH_2O)_rC(O)NR^m(CH_2CH_2O)_r-CH_2-$ and $-(CH_2CH_2O)_rC(O)NR^m(CH_2)_r-$; wherein each $R^m$ is independently H, C1-C6 alkyl, C3-C8 carbocyclyl, phenyl or benzyl, and each r is independently 1, 2, 3, 4, 5, 6, 7, 8, 9 or 10;

B is

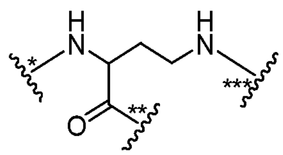

, or

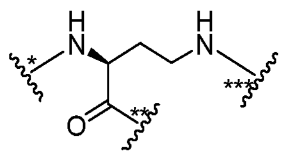

;

wherein * links to M', ** links to L, and *** links to G;

L is $-(AA)_i-(FF)_f-$, wherein AA is an amino acid or polypeptide, and i is 1, 2, 3, 4, 5, 6, 7, 8, 9, 10, 11, 12, 13, 14, 15, 16, 17, 18, 19 or 20; each FF is independently

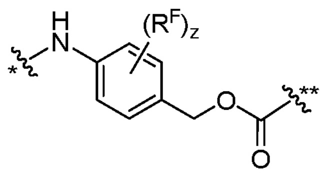

,

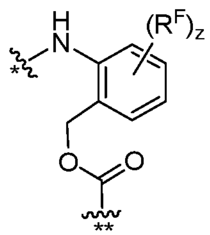

or

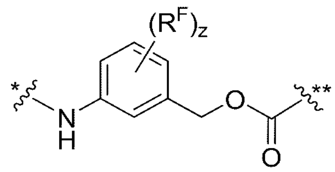

,

wherein

each $R^F$ is independently C1-C6 alkyl, C1-C6 alkoxy, $-NO_2$ or halogen, z is 0, 1, 2, 3 or 4; f is 1, 2, 3, 4, 5, 6, 7, 8, 9 or 10; wherein * links to AA, and ** links to D;

Gis

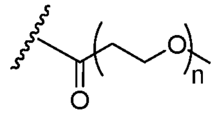

,

wherein n is 1-24; or n is 4-12.

**18.** A compound of Formula III-1 or a pharmaceutically acceptable salt or solvate thereof, wherein Formula III-1 is:

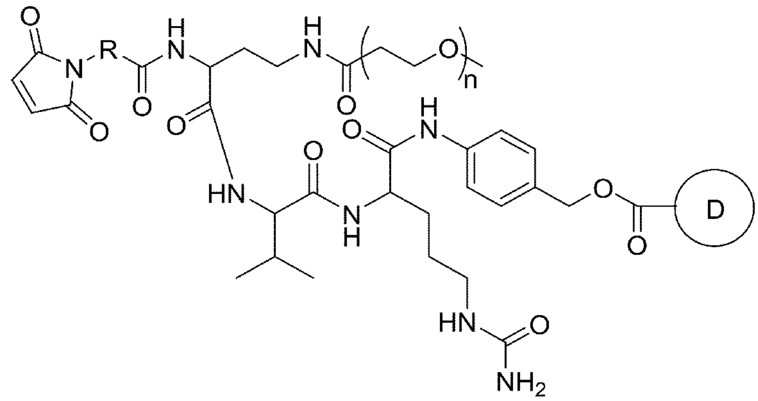

Formula III-1

wherein

D is a drug;
R is selected from: $-(CH_2)_r-$, $-(CHR^m)_r$, C3-C8 carbocyclyl, $-O-(CH_2)_r-$, arylene, $-(CH_2)_r$-arylene-, -arylene-$(CH_2)$r-, $-(CH_2)_r$-(C3-C8 carbocyclyl)-, -(C3-C8 carbocyclyl)-$(CH_2)_r-$, C3-C8 heterocyclyl, $-(CH_2)_r$-(C3-C8 heterocyclyl)-, -(C3-C8 heterocyclyl)-$(CH_2)_r-$, $-(CH_2)_rC(O)NR^m(CH_2)_r-$, $-(CH_2CH_2O)_r-$, $-(CH_2CH_2O)_r-CH_2-$, $-(CH_2)_rC(O)NR^m(CH_2CH_2O)_r-$, $-(CH_2)_rC(O)NR^m(CH_2CH_2O)_r-CH_2-$, $-(CH_2CH_2O)_rC(O)NR^m(CH_2CH_2O)_r-$, $-(CH_2CH_2O)_rC(O)NR^m(CH_2CH_2O)_r-CH_2-$ and $-(CH_2CH_2O)_rC(O)NR^m(CH_2)_r-$; wherein each $R^m$ is independently H, C1-C6 alkyl, C3-C8 carbocyclyl, phenyl or benzyl, and each r is independently 1, 2, 3, 4, 5, 6, 7, 8, 9 or 10;
n is 1-24.

**19.** A compound of Formula III-2 or Formula III-2-1 or a pharmaceutically acceptable salt or solvate thereof, wherein Formula III-2 is:

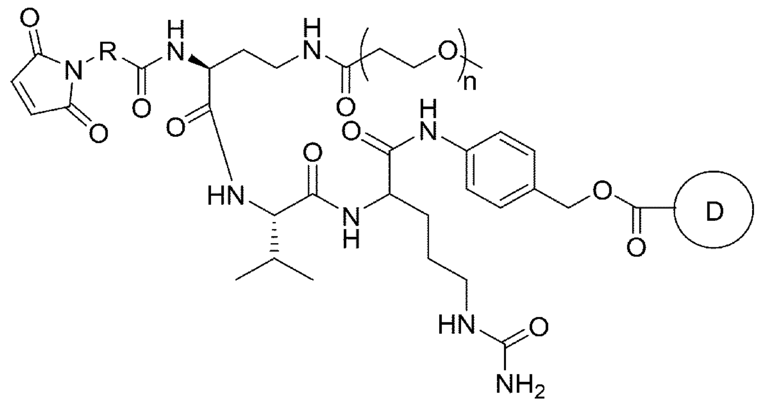

,

Formula III-2

Formula III-2-1 is:

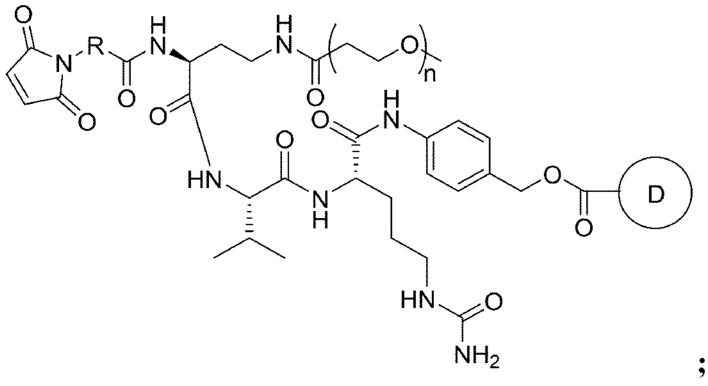

Formula III-2-1

wherein
D is a drug;
R is selected from: $-(CH_2)_r-$, $-(CHR^m)_r$, C3-C8 carbocyclyl, $-O-(CH_2)_r-$, arylene, $-(CH_2)_r$-arylene-, -arylene-$(CH_2)$r-, $-(CH_2)_r$-(C3-C8 carbocyclyl)-, -(C3-C8 carbocyclyl)-$(CH_2)_r-$, C3-C8 heterocyclyl, $-(CH_2)_r$-(C3-C8 heterocyclyl)-, -(C3-C8 heterocyclyl)-$(CH_2)_r-$, $-(CH_2)_rC(O)NR^m(CH_2)_r-$, $-(CH_2CH_2O)_r-$, $-(CH_2CH_2O)_r-CH_2-$, $-(CH_2)_rC(O)NR^m(CH_2CH_2O)_r-$, $-(CH_2)_rC(O)NR^m(CH_2CH_2O)_r-CH_2-$, $-(CH_2CH_2O)_rC(O)NR^m(CH_2CH_2O)_r-$, $-(CH_2CH_2O)_rC(O)NR^m(CH_2CH_2O)_r-CH_2-$ and $-(CH_2CH_2O)_rC(O)NR^m(CH_2)_r-$; wherein each $R^m$ is independently H, C1-C6 alkyl, C3-C8 carbocyclyl, phenyl or benzyl, and each r is independently 1, 2, 3, 4, 5, 6, 7, 8, 9 or 10;
n is 1-24.

**20.** A compound of Formula III-3 or a pharmaceutically acceptable salt or solvate thereof, wherein Formula III-3 is:

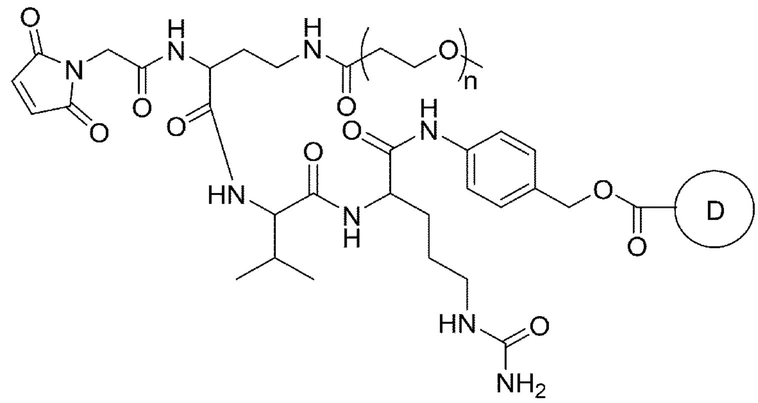

Formula III-3

wherein

D is a drug;
n is 1-24.

**21.** A compound of Formula III-4 or Formula III-4 or a pharmaceutically acceptable salt or solvate thereof, wherein Formula III-4 is:

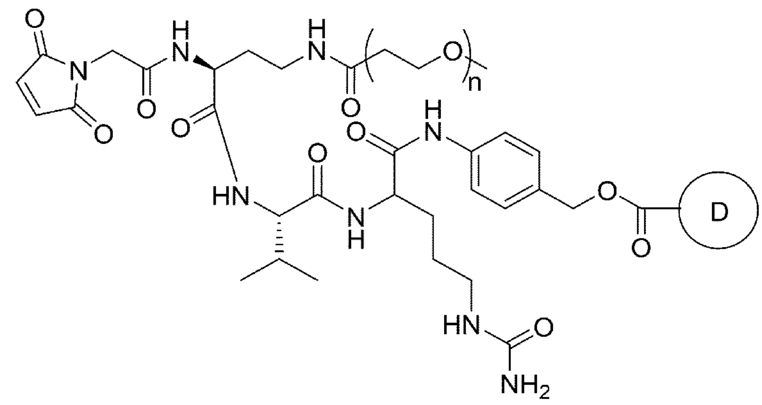

Formula III-4

Formula III-4-1 is:

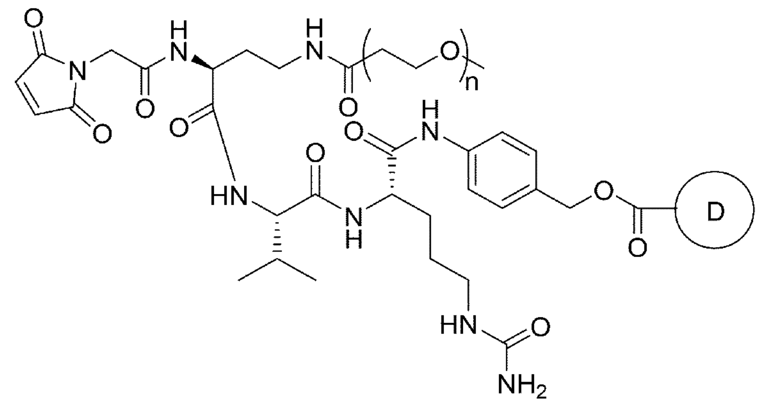

;

Formula III-4-1

wherein
D is a drug;
n is 1-24.

22. A drug conjugate accroding claim 1, or a compound or a pharmaceutically acceptable salt or solvate thereof accroding claim 11 or 17, wherein each AA is independently selected from: Val-Cit, Val-Lys, Phe-Lys, Lys-Lys, Ala-Lys, Phe-Cit, Leu-Cit, Ile-Cit, Trp, Cit, Phe-Ala, Phe-Phe-Lys, D-Phe-Phe-Lys, Gly-Phe-Lys, Leu-Ala-Leu, Ile-Ala-Leu, Val-Ala-Val, Ala-Leu-Ala-Leu, β-Ala-Leu-Ala-Leu, and Gly-Phe-Leu-Gly.

23. A drug conjugate accroding claim 1, or a compound or a pharmaceutically acceptable salt or solvate thereof accroding claim 11 or 17, wherein AA isVal-Cit, and i is 1.

24. A drug conjugate accroding claim 1, and a compound or a pharmaceutically acceptable salt or solvate thereof accroding claim 17, wherein L is

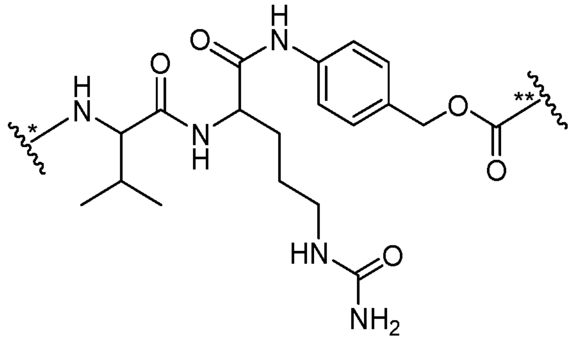

,

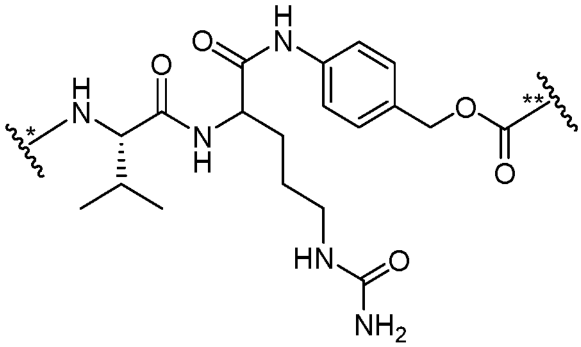

or

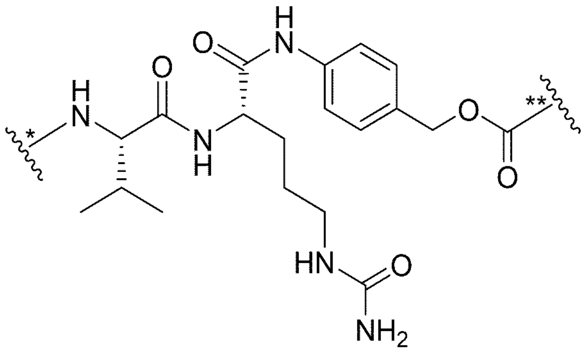

,

wherein * links to B, ** links to D.

**25.** A compound or a pharmaceutically acceptable salt or solvate thereof accroding claim 11, wherein L' is

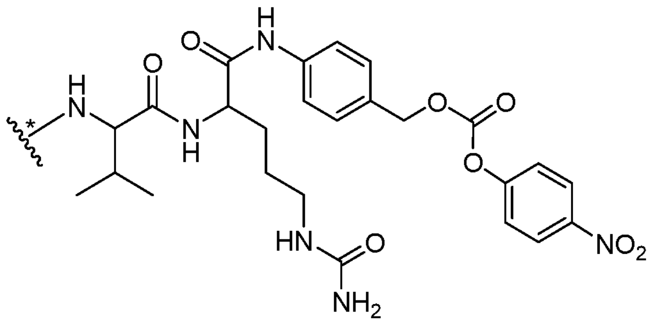

,

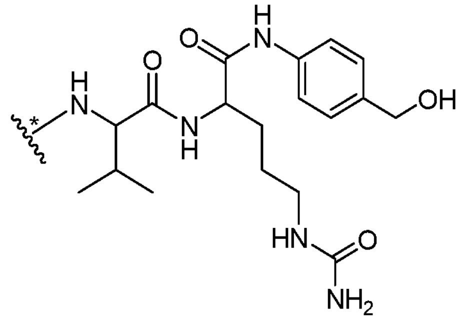

,

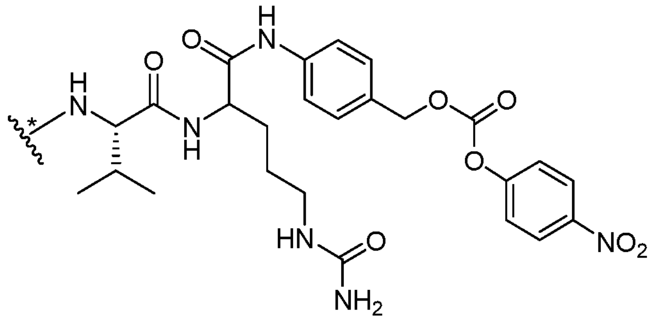

,

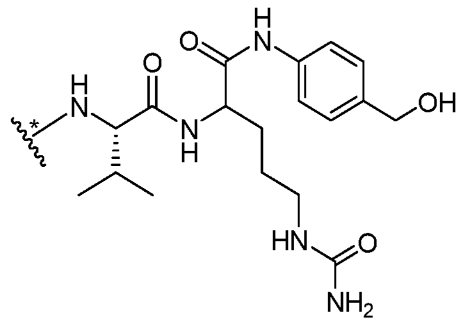

,

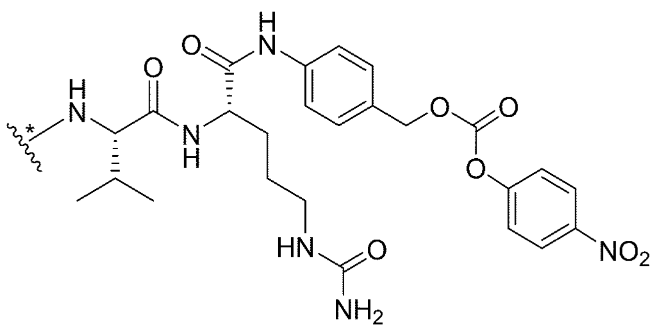

or

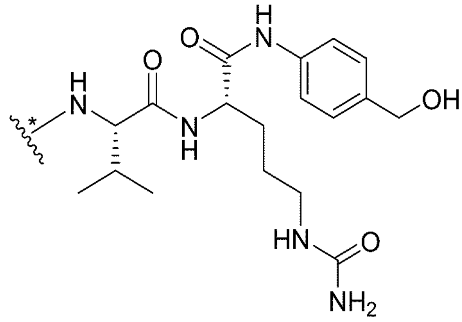

,

wherein * links to B.

**26.** The drug conjugate accroding any one of claims 1-6, 22-24 or the compound accroding any one of claims 17-24 or the pharmaceutically acceptable salt or solvate thereof, wherein D is an anti-cancer drug, a cytotoxic drug, a cell differentiation factor, a stem cell trophic factor, a steroid drug, a drug for treating autoimmune diseases, an anti-inflammatory drug or a drug for treating infectious diseases.

**27.** The drug conjugate accroding any one of claims 1-6, 22-24 or the compound accroding any one of claims 17-24 or the pharmaceutically acceptable salt or solvate thereof, wherein D is an anti-cancer drug.

**28.** The drug conjugate accroding any one of claims 1-6, 22-24 or the compound accroding any one of claims 17-24 or the pharmaceutically acceptable salt or solvate thereof, wherein D is a tubulin inhibitor, a DNA damaging agent, or a DNA topoisomerase inhibitor.

**29.** The drug conjugate accroding any one of claims 1-6, 22-24 or the compound accroding any one of claims 17-24 or the pharmaceutically acceptable salt or solvate thereof, wherein D is selected from MMAE, MMAF, AF, calicheamicin, duocarmycin, anthramycin derivative PBD (pyrrolobenzodiazepine), irinotecan, irinotecan hydrochloride, camptothecin, 9-aminocamptothecin, 9-nitrocamptothecin, 10-hydroxycamptothecin, 9-chloro-10-hydroxycamptothecin, the camptothecin derivative SN-38, 22-hydroxyacuminatine, topotecan, lurtotecan, belotecan, exatecan, homosilatecan, 6,8-dibromo-2-methyl-3-[2-(D-xylopyranosylamino)phenyl]-4(3H)-quinazolinone, 2-cyano-3-(3,4-dihydroxyphenyl)-N-(phenylmethyl)-(2E)-2-propenamide, 2-cyano-3-(3,4-dihydroxyphenyl)-N-(3-hydroxyphenylpropyl)-(E)-2-propenamide, 12-β-D-glucopyranosyl-12,13-dihydro-2,10-dihydroxy-6-[[2-hydroxy-1-(hydroxymethyl)ethyl]amino]-5H-indolo[2,3-a]pyrrolo[3,4-c]carbazole-5,7(6H)-dione, N-[2-(dimethylamino)ethyl]-4-acridinecarboxamide dihydrochloride, N-[2-(dimethylamino)ethyl]-4-acridinecarboxamide, or a pharmaceutically acceptable salts or solvates thereof.

**30.** The drug conjugate accroding any one of claims 1-6, 22-24 or the compound accroding any one of claims 17-24 or the pharmaceutically acceptable salt or solvate

thereof, wherein D is

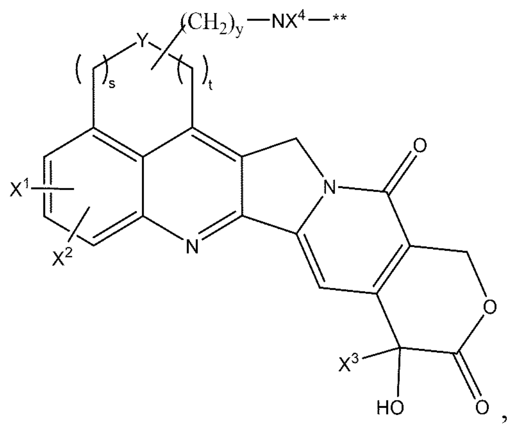

,

wherein

$X^1$ and $X^2$ are each independently:

H,
hydroxy,
C1-C6 alkyl,
C1-C6 alkyl optionally substituted with one or more hydroxy, halogen, nitro or cyano groups,
C2-C6 alkenyl,
C2-C6 alkynyl,
C1-C6 alkoxy,
C1-C6 aminoalkoxy,
halogen,
nitro,
cyano,
thiol,
alkylthio,
amino, amino substituted with an amino-protecting group, C1-C6 aminoalkyl optionally substituted at the amino moiety with an amino-protecting group or C1-C6 alkyl,
C1-C6 aminoalkylamino optionally substituted at the amino moiety with an amino-protecting group or C1-C6 alkyl,
C1-C6 alkyl linking to a heterocycle, wherein the heterocycle is optionally substituted with one or more C1-C6 alkyl, C1-C6 alkoxy, amino, halogen, nitro or cyano groups, C1-C6 alkylamino linking to a heterocycle, wherein the heterocycle is optionally substituted with C1-C6 alkyl or C1-C6 alkoxy, and the amino is optionally substituted with an amino-protecting group, halogen, nitro, cyano or protecting group, amino-substituted heterocyclyl, which is optionally substituted at a nitrogen atom of the heterocyclyl moiety or at the amino moiety with a protecting group or one or more C1-C6 alkyl groups,
heterocyclylamino, which is optionally substituted at a nitrogen atom of the heterocyclic moiety or at the amino moiety with a protecting group or C1-C6 alkyl, carbamoyl optionally substituted with a carbamoyl-protecting group or C1-C6 alkyl, morpholin-1-yl, or
piperidin-1-yl;
$X^3$ is C1-C6 alkyl;
$X^4$ is H, $-(CH_2)_q-CH_3$, $-(CHR^n)_q-CH_3$, C3-C8 carbocyclyl, $-O-(CH_2)_q-CH_3$, arylene-$CH_3$, $-(CH_2)_q$-arylene-$CH_3$, -arylene-$(CH_2)_q-CH_3$, $-(CH_2)_q$-(C3-C8 carbocyclyl)-$CH_3$, -(C3-C8 carbocyclyl)-$(CH_2)_q-CH_3$, C3-C8 heterocyclyl, $-(CH_2)_q$-(C3-C8 heterocyclyl)-$CH_3$, -(C3-C8 heterocyclyl)-$(CH_2)_q-CH_3$, $-(CH_2)_qC(O)NR^n(CH_2)_q-CH_3$, $-(CH_2CH_2O)_q-CH_3$, $-(CH_2CH_2O)_q-CH_2-CH_3$, $-(CH_2)_qC(O)NR^n(CH_2CH_2O)_q-CH_3$, $-(CH_2)_qC(O)NR^n(CH_2CH_2O)_q-CH_2-CH_3$, $-(CH_2CH_2O)_qC(O)NR^n(CH_2CH_2O)_q-CH_3$, $-(CH_2CH_2O)_qC(O)NR^n(CH_2CH_2O)_q-CH_2-CH_3$ or $-(CH_2CH_2O)_qC(O)NR^n(CH_2)_q-CH_3$; wherein each $R^n$ is independently H, C1-C6 alkyl, C3-C8 carbocyclyl, phenyl or
benzyl, and each q is independently 1, 2, 3, 4, 5, 6, 7, 8, 9 or 10;
** is point of connection;
y is 0, 1 or 2;
Y is O, S or $CR^1R^2$, wherein $R^1$ and $R^2$ are each independently H or C1-C6 alkyl;
s and t are each independently 0, 1 or 2, but not both 0.

**31.** The drug conjugate accroding any one of claims 1-6, 22-24 or the compound accroding any one of claims 17-24 or the pharmaceutically acceptable salt or solvate thereof, wherein D is

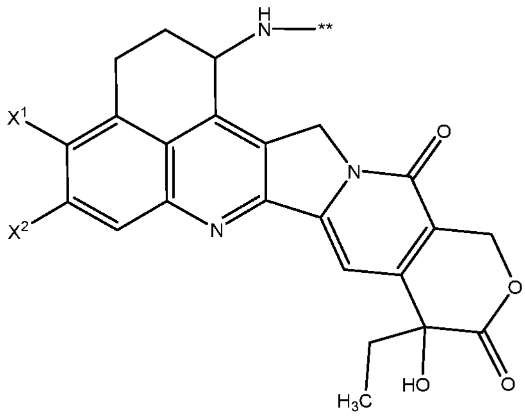

or

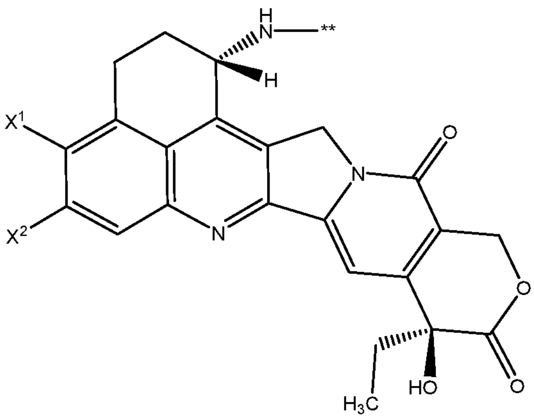

,

wherein $X^1$ and $X^2$ are each independently C1-C6 alkyl, halogen, or -OH; ** is point of connection.

**32.** A compound of Formula III-5, Formula III-5-1, Formula III-6, Formula III-6-1, Formula III-7, Formula III-7-1, Formula III-8, Formula III-8-1, Formula III-9, Formula III-9-1, Formula III-10, Formula III-10-1, Formula III-11, Formula III-11-1, Formula III-12, Formula III-12-1, Formula III-13, Formula III-13-1, Formula III-14, Formula III-14-1, Formula III-15, Formula III-15-1, Formula III-16, Formula III-16-1, Formula III-17, Formula III-17-1, Formula III-18 or Formula III-18-1 or a pharmaceutically acceptable salt or solvate thereof, wherein Formula III-5, Formula III-5-1, Formula III-6, Formula III-6-1, Formula III-7, Formula III-7-1, Formula III-8, Formula III-8-1, Formula III-9, Formula III-9-1, Formula III-10, Formula III-10-1, Formula III-11, Formula III-11-1, Formula III-12, Formula III-12-1, Formula III-13, Formula III-13-1, Formula III-14, Formula III-14-1, Formula III-15, Formula III-15-1, Formula III-16, Formula III-16-1, Formula III-17, Formula III-17-1, Formula III-18 or Formula III-18-1 are:

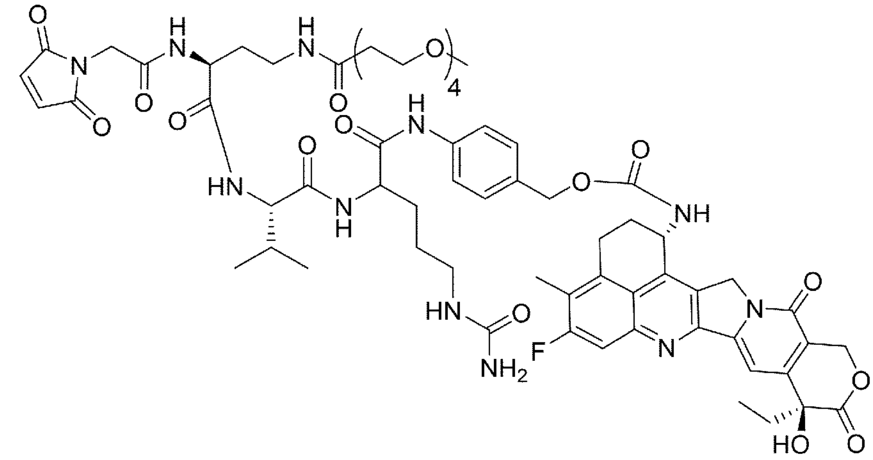

Formula III-5

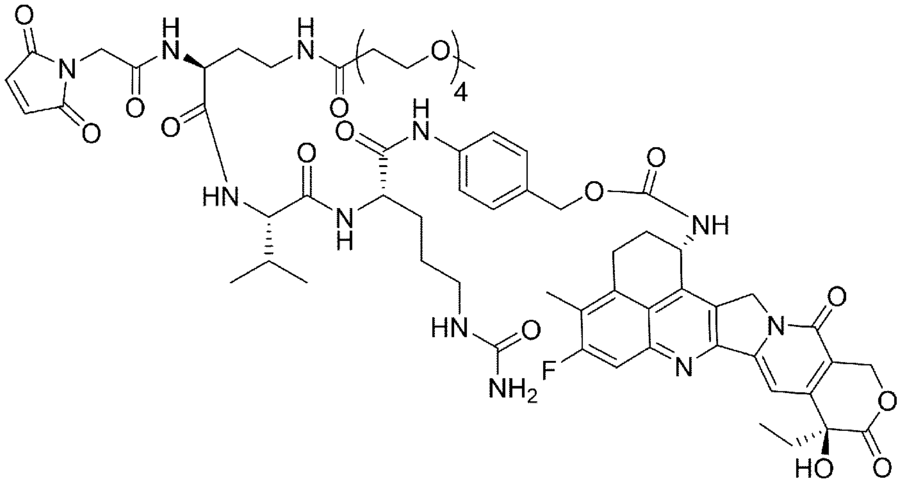

Formula III-5-1

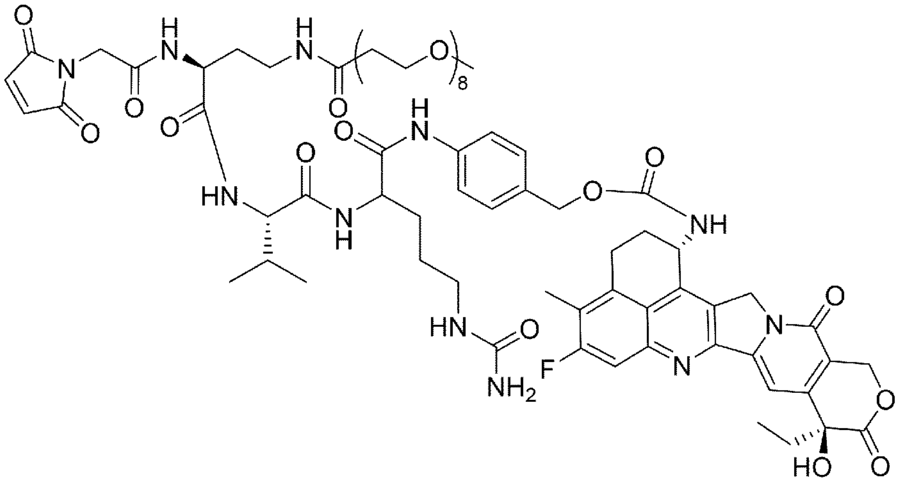

Formula III-6

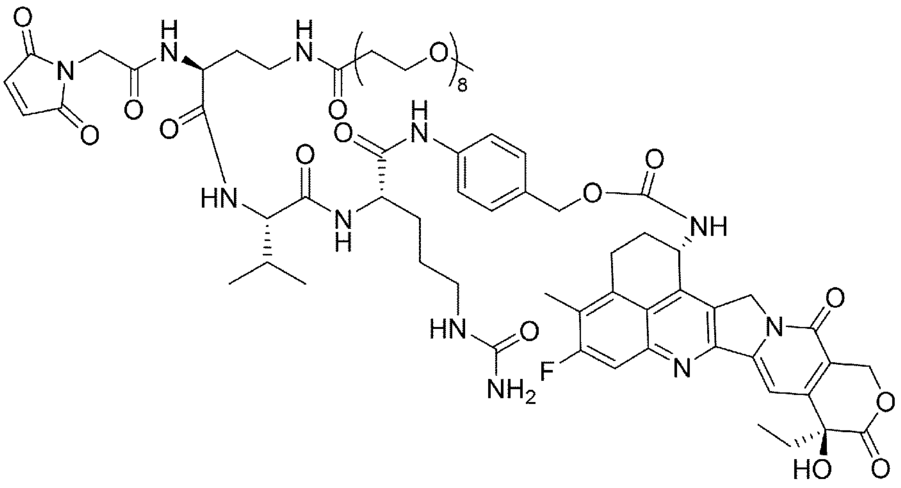

Formula III-6-1

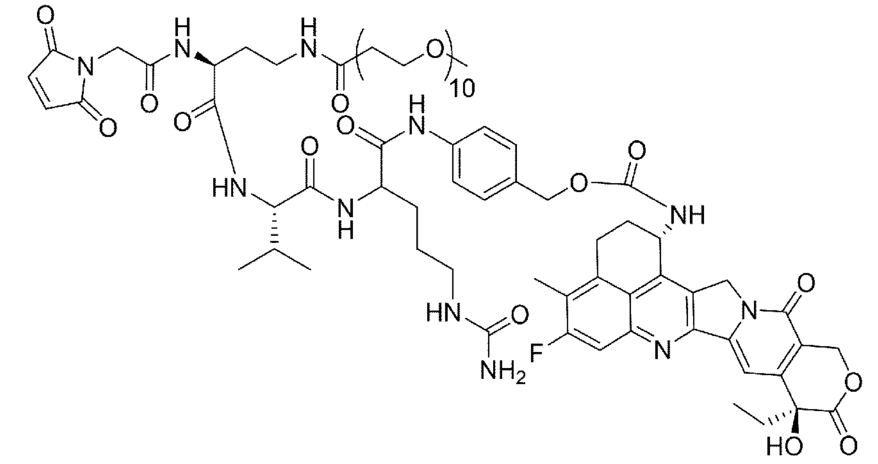

Formula III-7

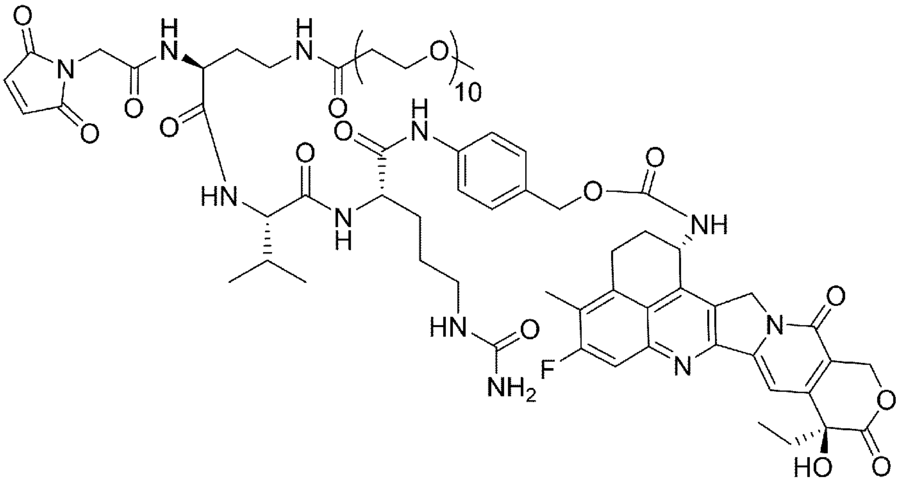

Formula III-7-1

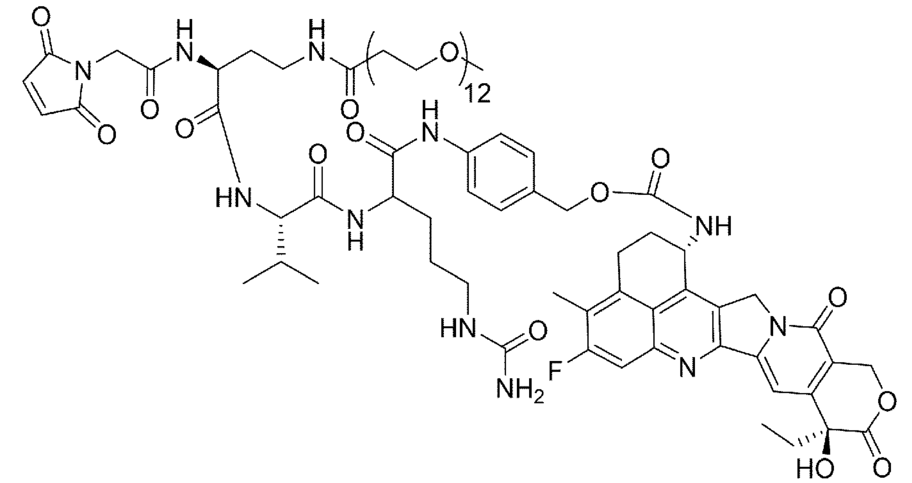

Formula III-8

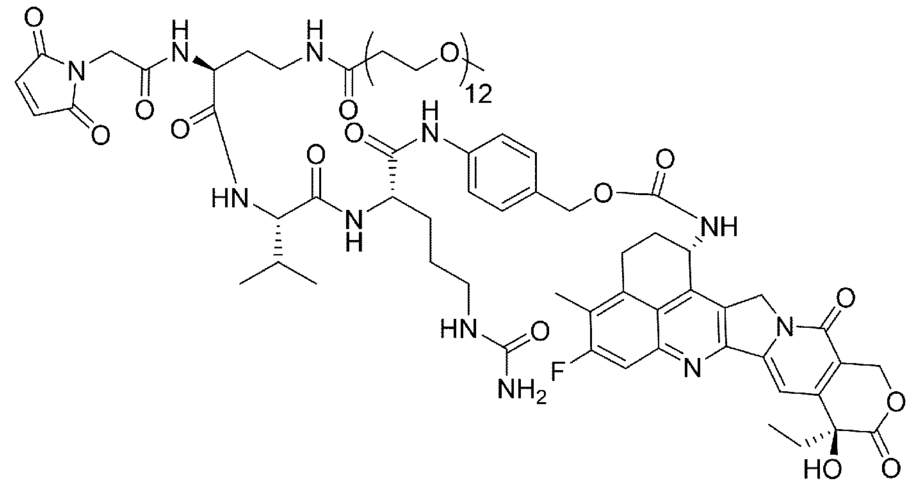

Formula III-8-1

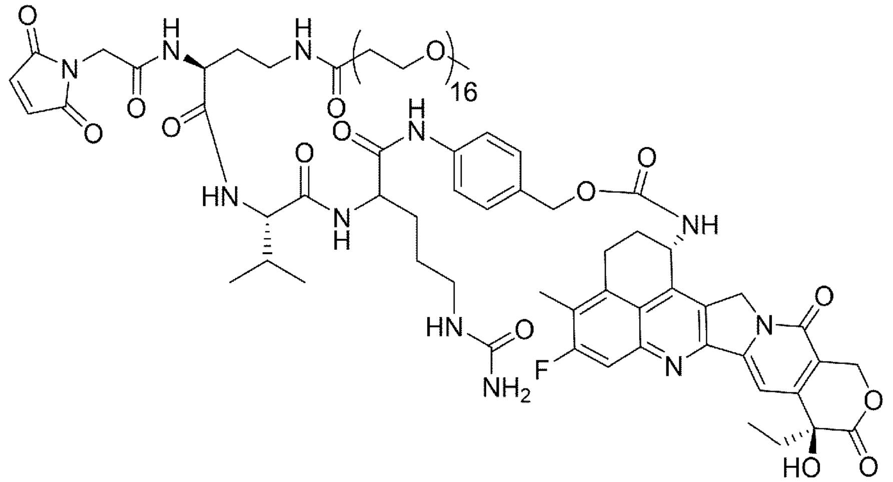

Formula III-9

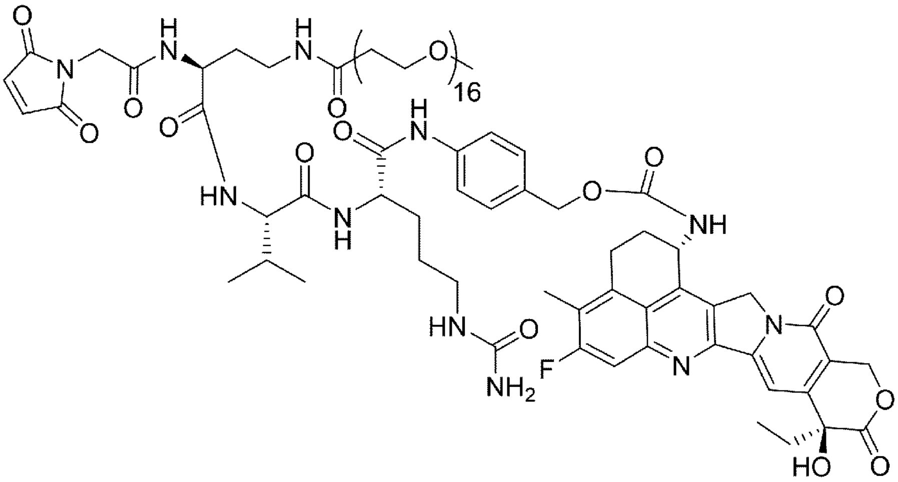

Formula III-9-1

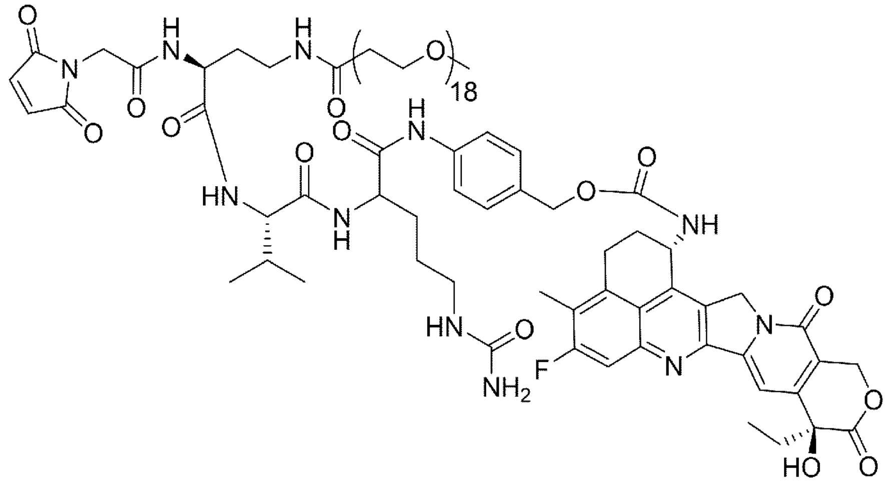

Formula III-10

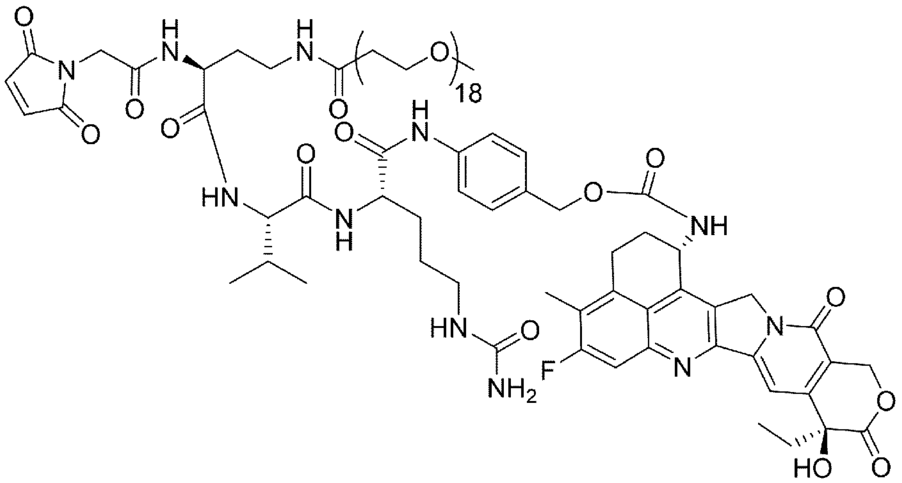

Formula III-10-1

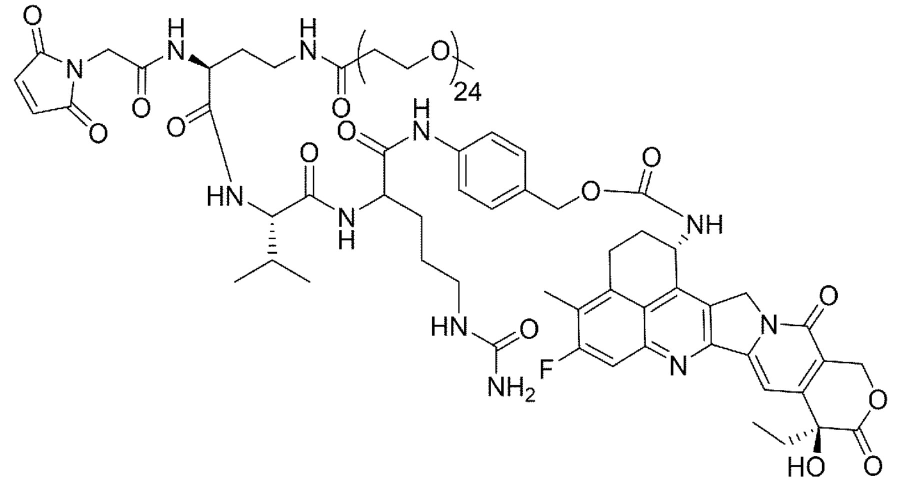

Formula III-11

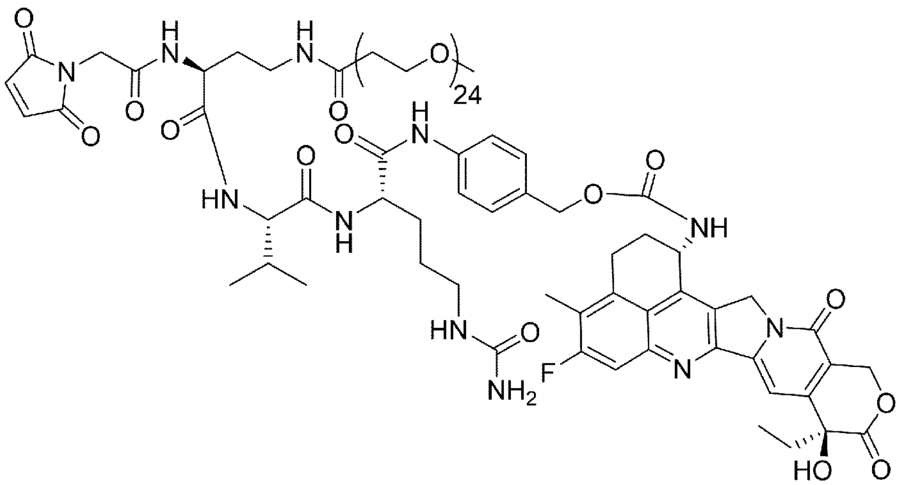

Formula III-11-1

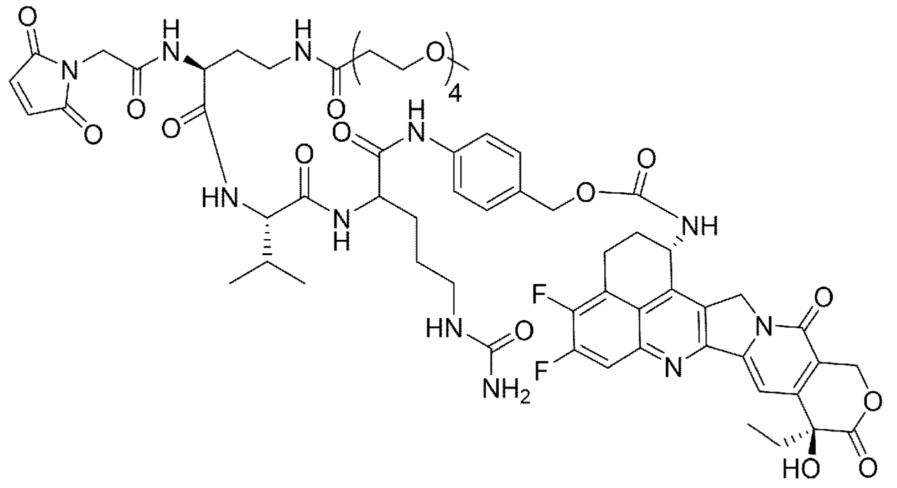

Formula III-12

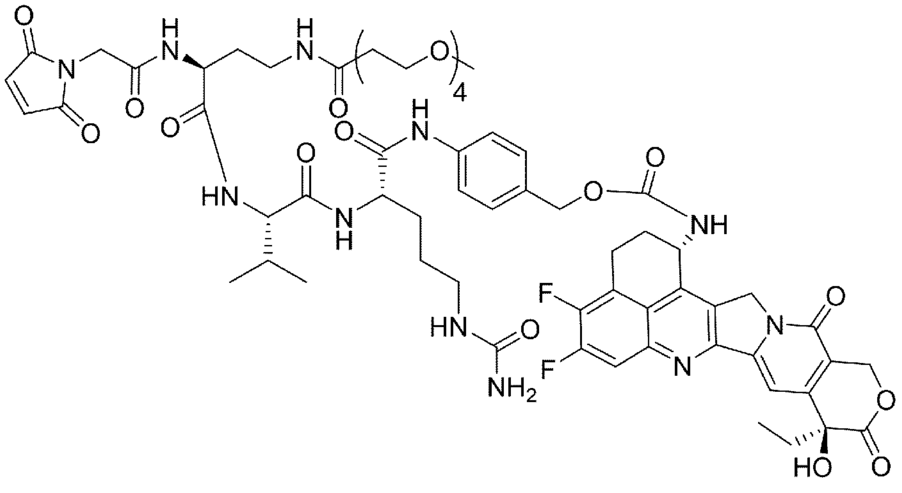

Formula III-12-1

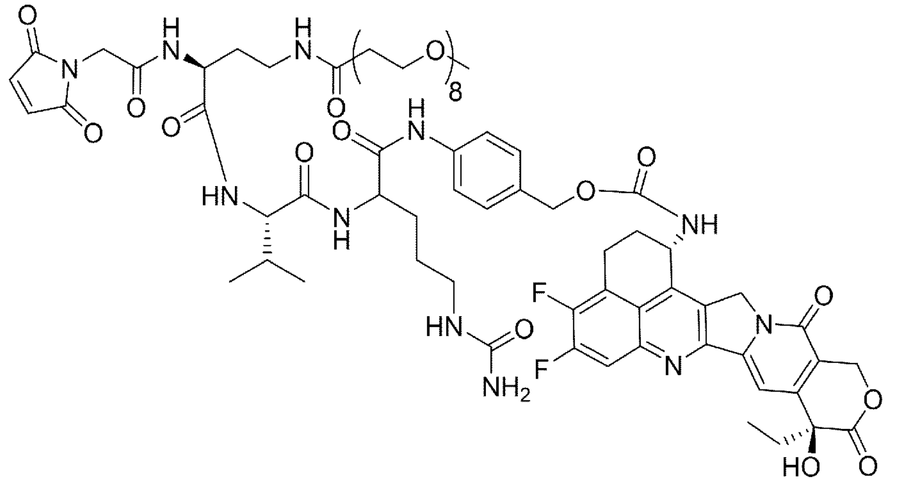

Formula III-13

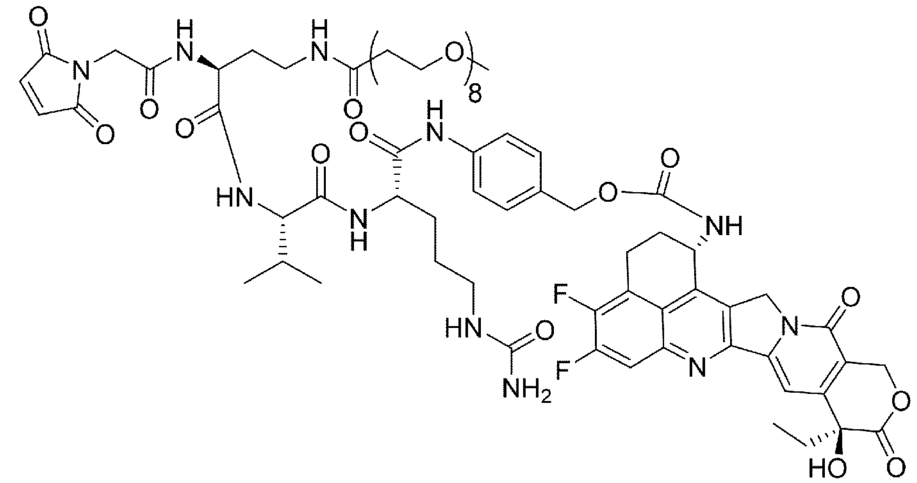

Formula III-13-1

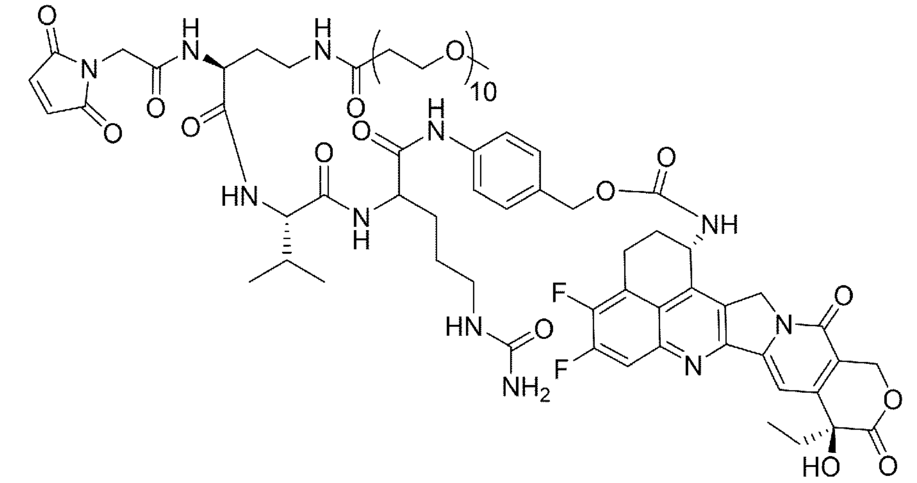

Formula III-14

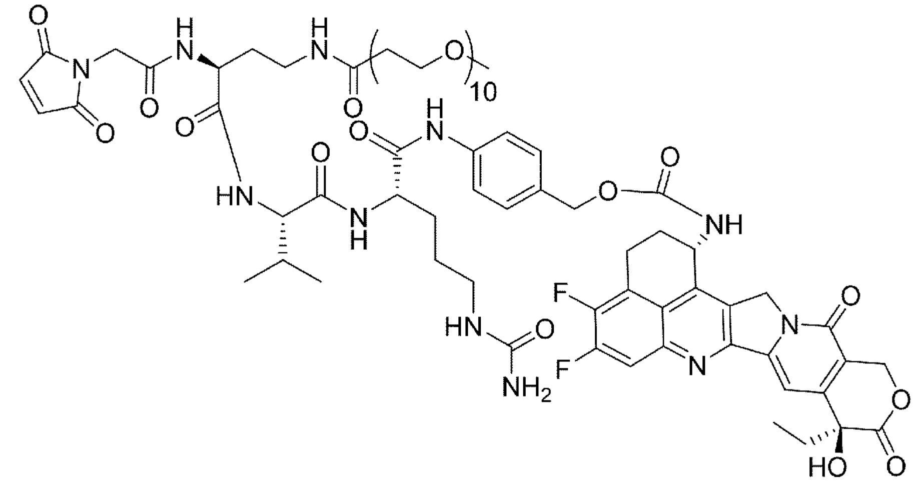

Formula III-14-1

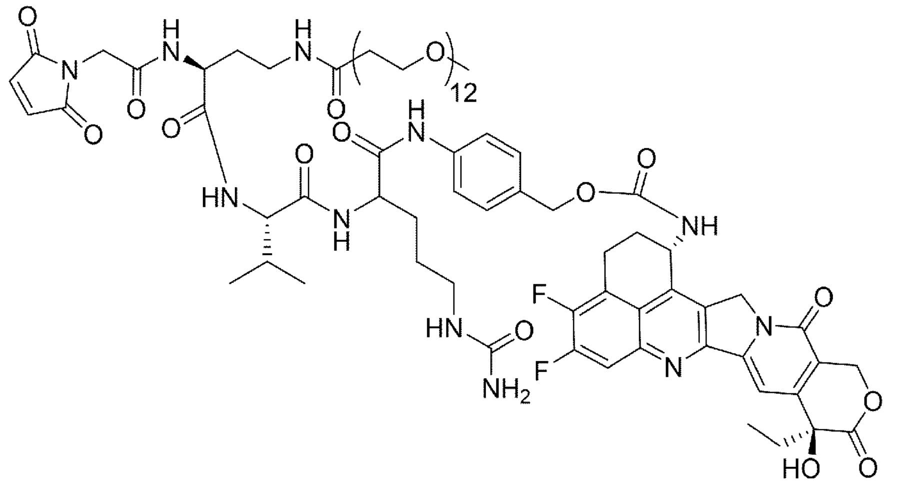

Formula III-15

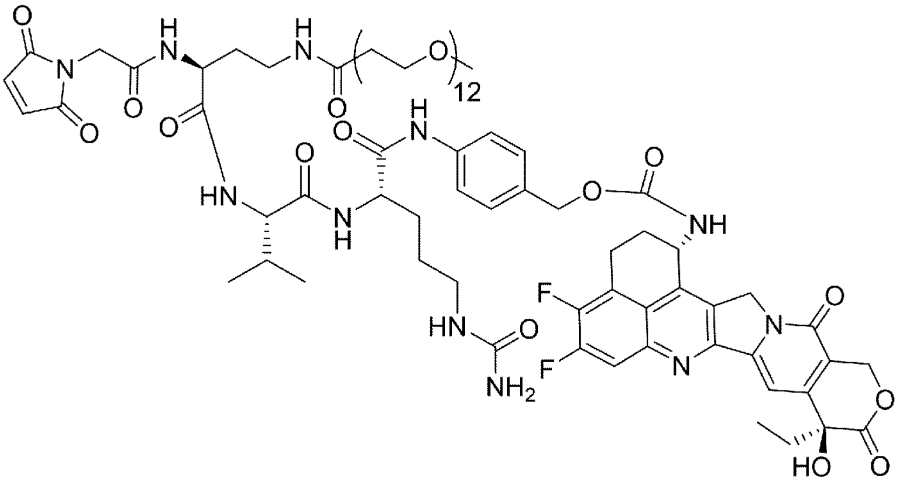

Formula III-15-1

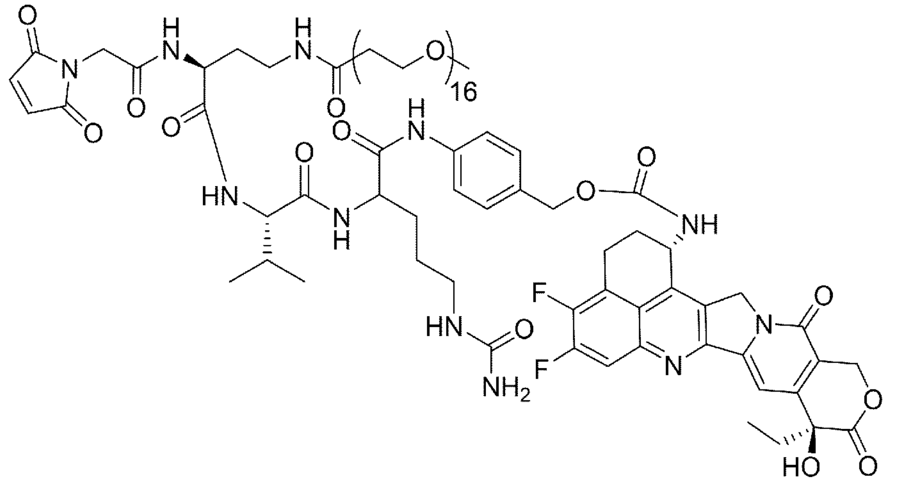

Formula III-16

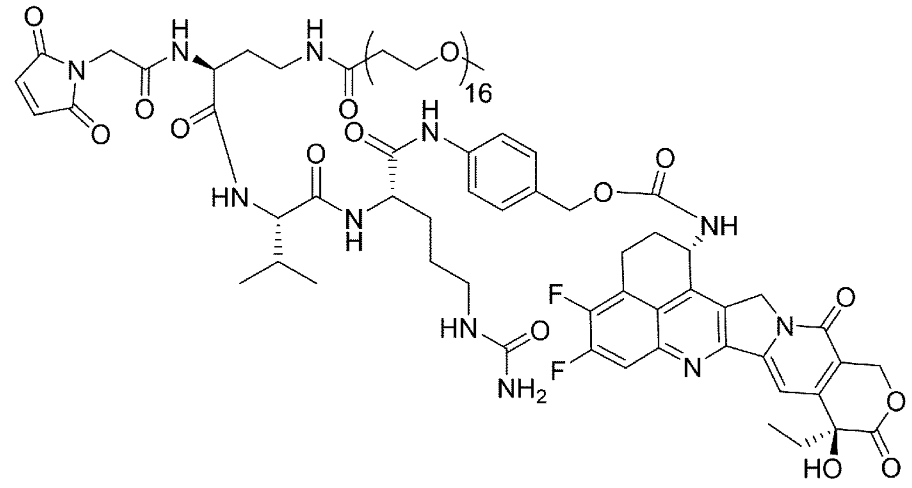

Formula III-16-1

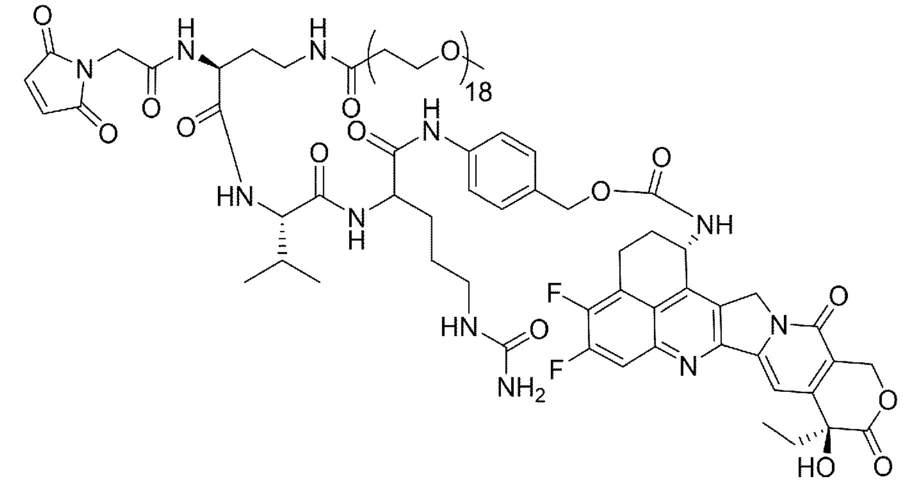

Formula III-17

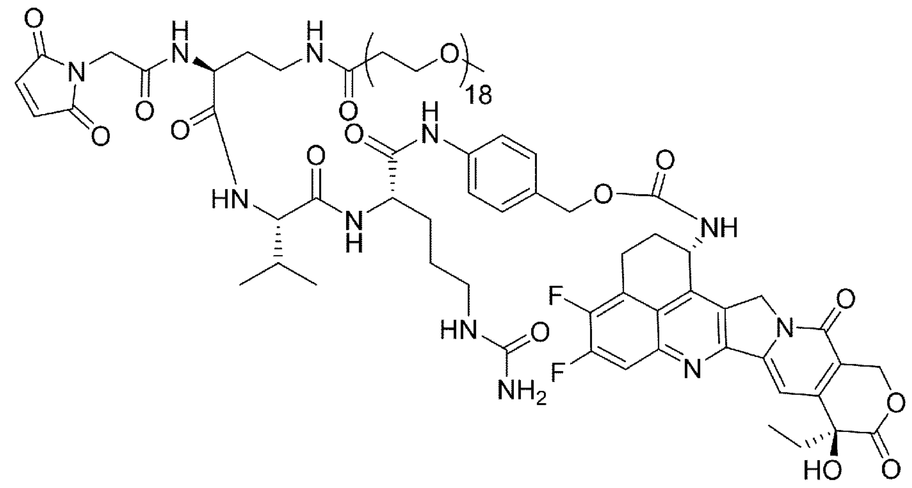

Formula III-17-1

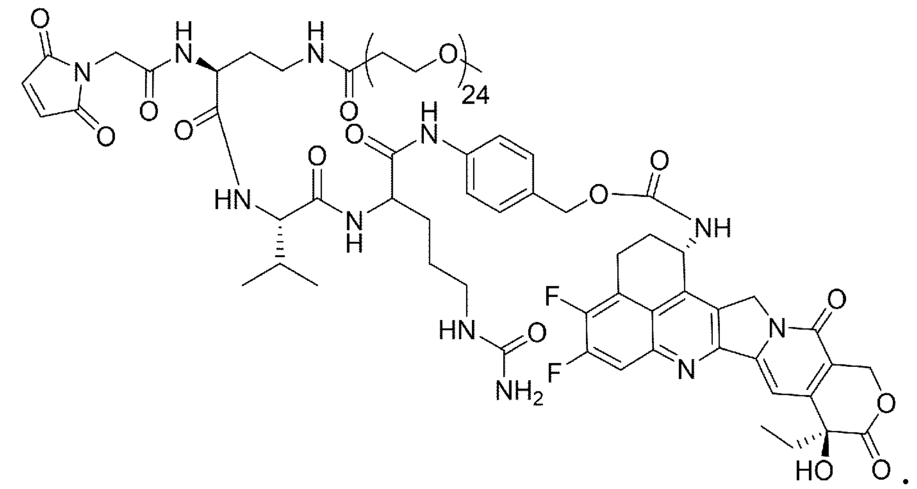

Formula III-18

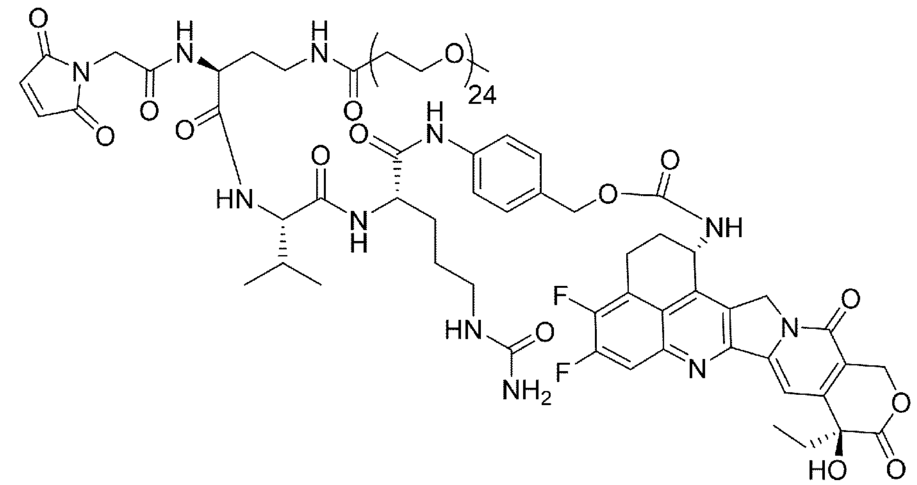

Formula III-18-1

**33.** A pharmaceutical composition comprising the drug conjugate according to any one of claims 1-10, 22-31, and a pharmaceutically acceptable carrier, an excipient and/or an adjuvant, or optionally other anti-cancer drugs.

**34.** Use of the antibody-drug conjugate according to any one of claims 1-10, 22-31, or the pharmaceutical composition according to claim 33, in preparing a medicament for treating cancer, autoimmune diseases, inflammatory diseases or infectious diseases.

**35.** Use of the compound or the pharmaceutically acceptable salt or solvate thereof according to any one of claims 11-32 in preparing a drug conjugate; wherein the drug conjugate is the drug conjugate according to any one of claims 1-10, 22-31.

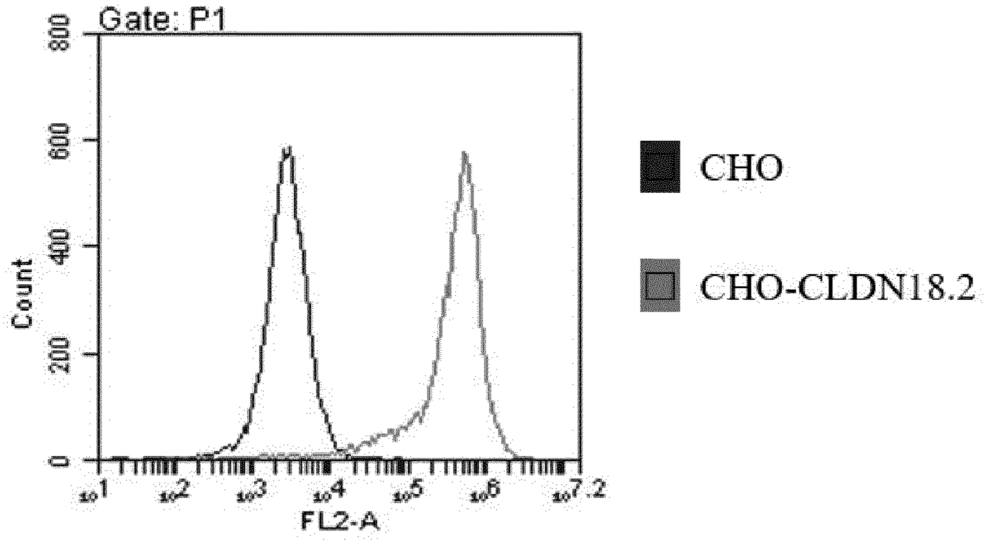


FIG. 1

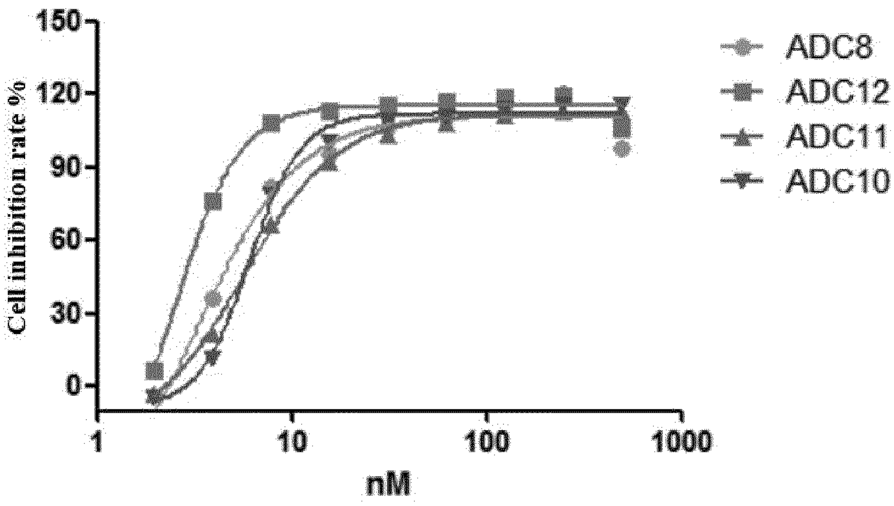


| | ADC8 | ADC12 | ADC11 | ADC10 |
|---|---|---|---|---|
| EC50 | 3.293 | 2.534 | 5.654 | 6.077 |

FIG. 2

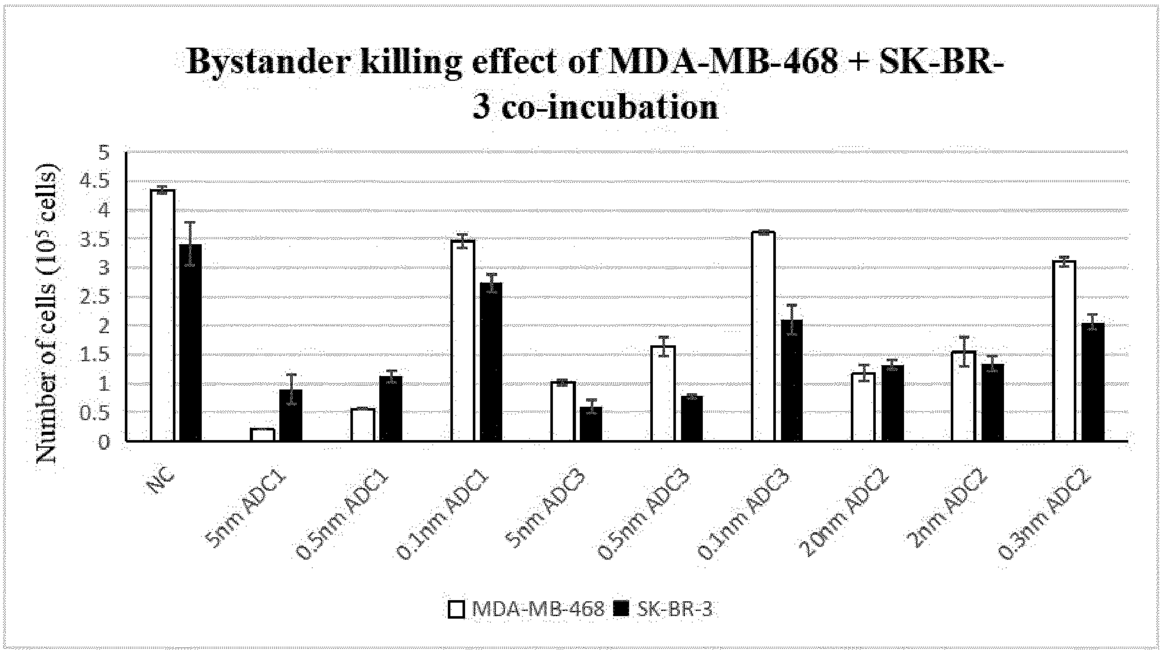
Bystander killing effect of MDA-MB-468 + SK-BR-3 co-incubation
Number of cells (10^5 cells)
0
0.5
1
1.5
2
2.5
3
3.5
4
4.5
5
NC
5nm ADC1
0.5nm ADC1
0.1nm ADC1
5nm ADC3
0.5nm ADC3
0.1nm ADC3
20nm ADC2
2nm ADC2
0.3nm ADC2
MDA-MB-468
SK-BR-3

FIG. 3

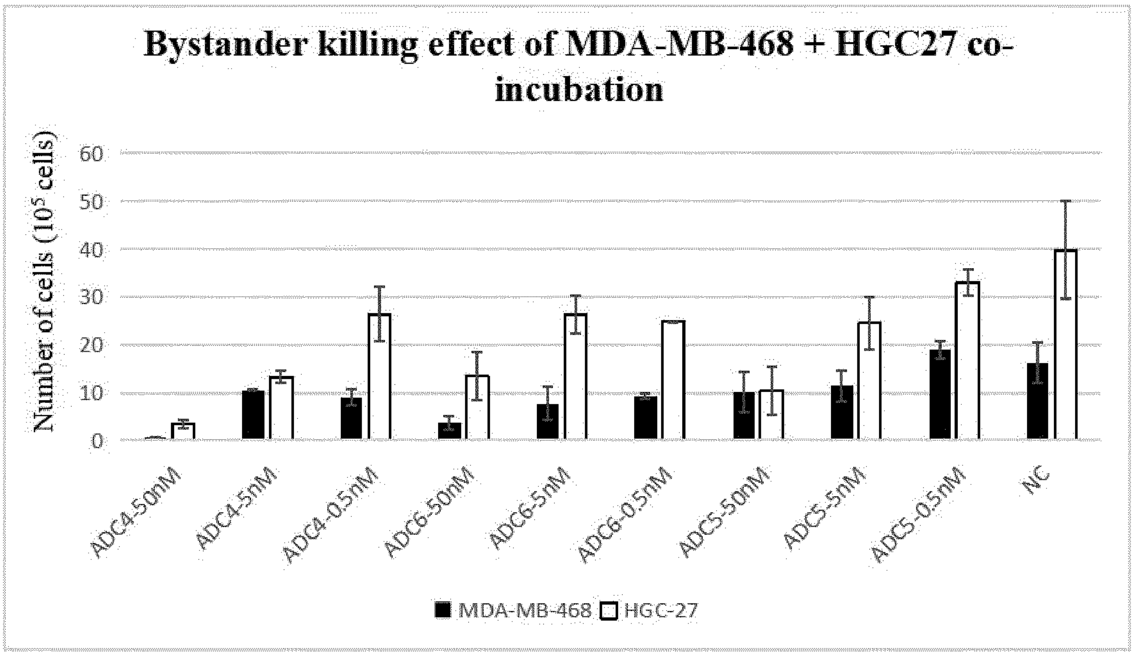
Bystander killing effect of MDA-MB-468 + HGC27 co-incubation
Number of cells (10^5 cells)
0
10
20
30
40
50
60
ADC4-50nM
ADC4-5nM
ADC4-0.5nM
ADC6-50nM
ADC6-5nM
ADC6-0.5nM
ADC5-50nM
ADC5-5nM
ADC5-0.5nM
NC
MDA-MB-468
HGC-27

FIG. 4

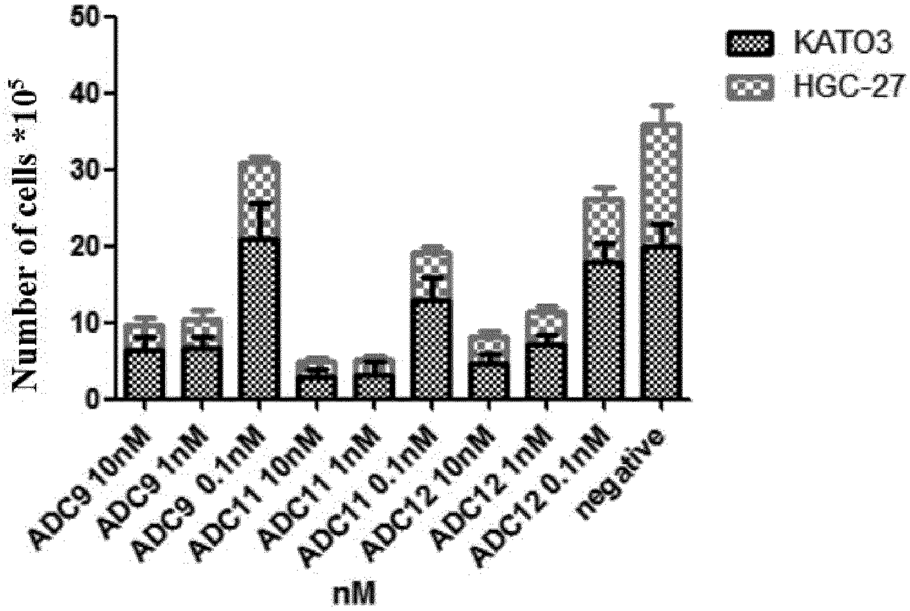
50
40
30
20
10
0
Number of cells *10^5
KATO3
HGC-27
ADC9 10nM
ADC9 1nM
ADC9 0.1nM
ADC11 10nM
ADC11 1nM
ADC11 0.1nM
ADC12 10nM
ADC12 1nM
ADC12 0.1nM
negative
nM

FIG. 5

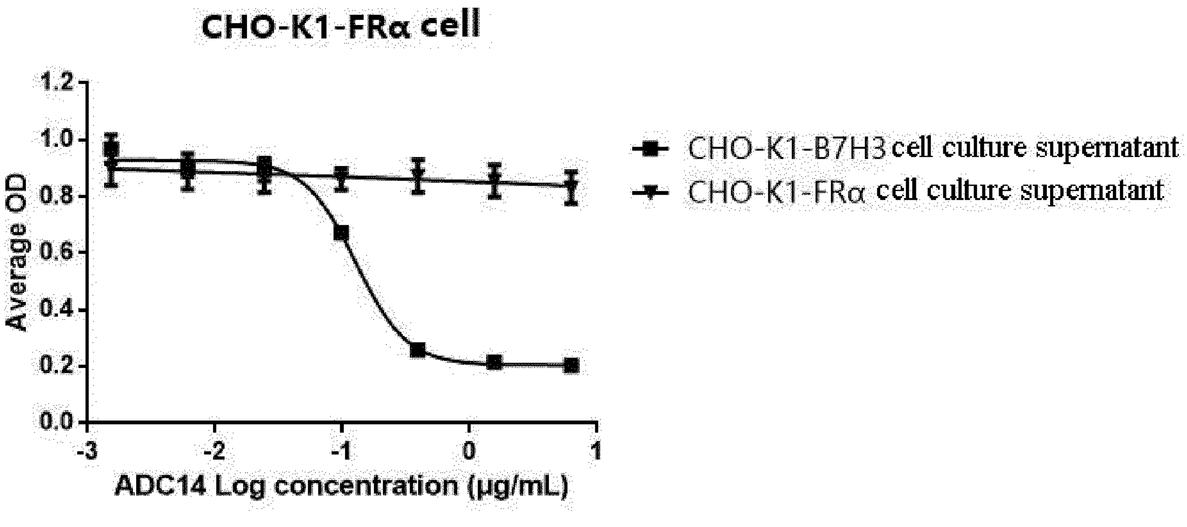
CHO-K1-FRα cell
1.2
1.0
0.8
0.6
0.4
0.2
0.0
Average OD
-3
-2
-1
0
1
ADC14 Log concentration (μg/mL)
CHO-K1-B7H3 cell culture supernatant
CHO-K1-FRα cell culture supernatant

FIG. 6

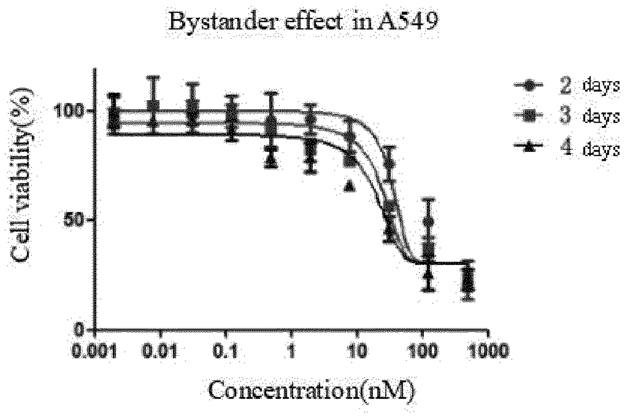


FIG. 7

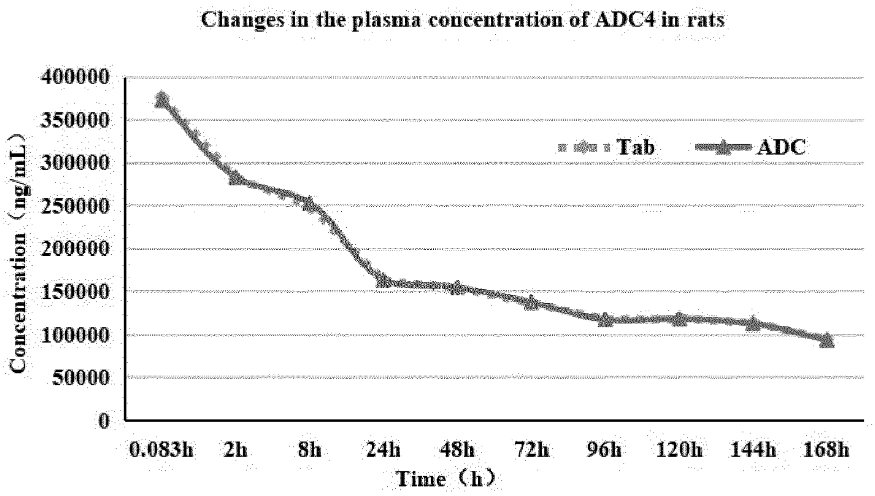


FIG. 8

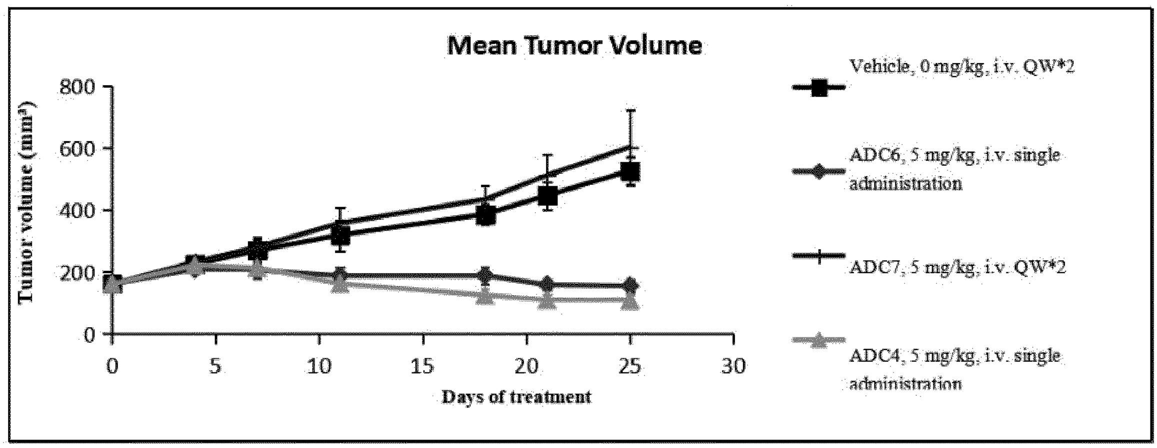


FIG. 9

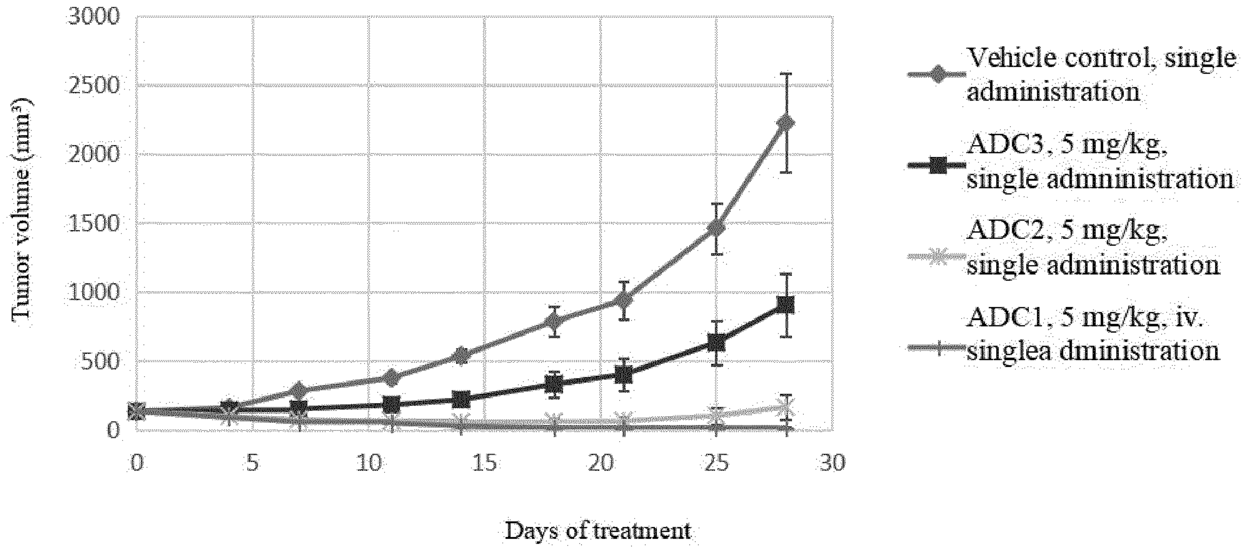
Tumor volume (mm³)
3000
2500
2000
1500
1000
500
0
0
5
10
15
20
25
30
Days of treatment
Vehicle control, single administration
ADC3, 5 mg/kg, single admninistration
ADC2, 5 mg/kg, single administration
ADC1, 5 mg/kg, iv. singlea dministration

FIG. 10

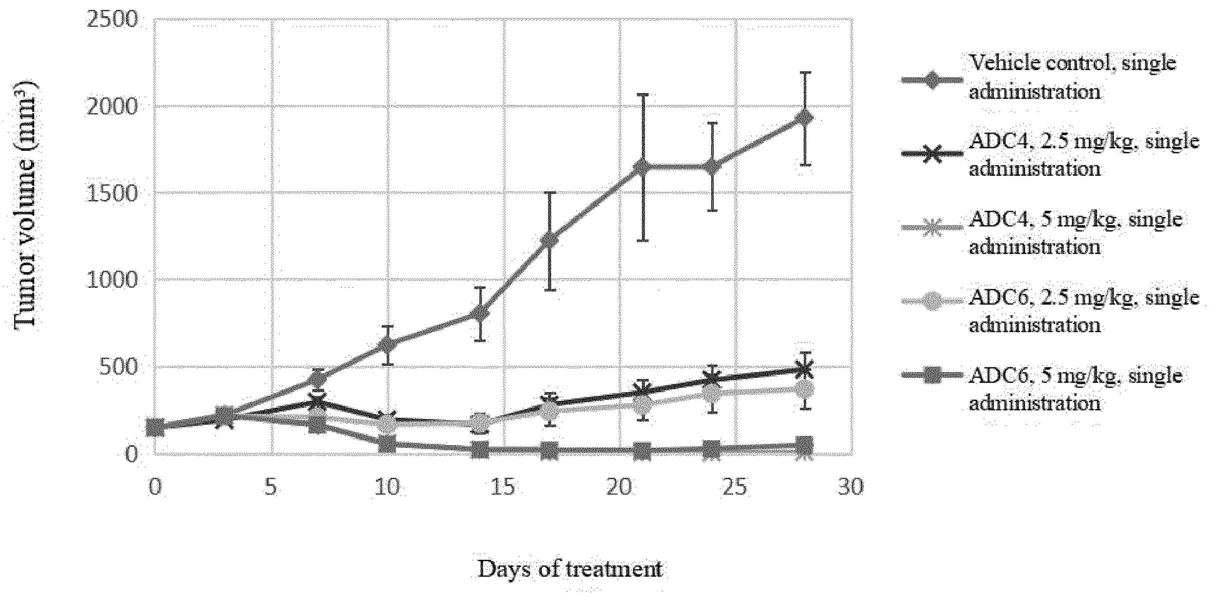
Tumor volume (mm³)
2500
2000
1500
1000
500
0
0
5
10
15
20
25
30
Days of treatment
Vehicle control, single administration
ADC4, 2.5 mg/kg, single administration
ADC4, 5 mg/kg, single administration
ADC6, 2.5 mg/kg, single administration
ADC6, 5 mg/kg, single administration

FIG. 11

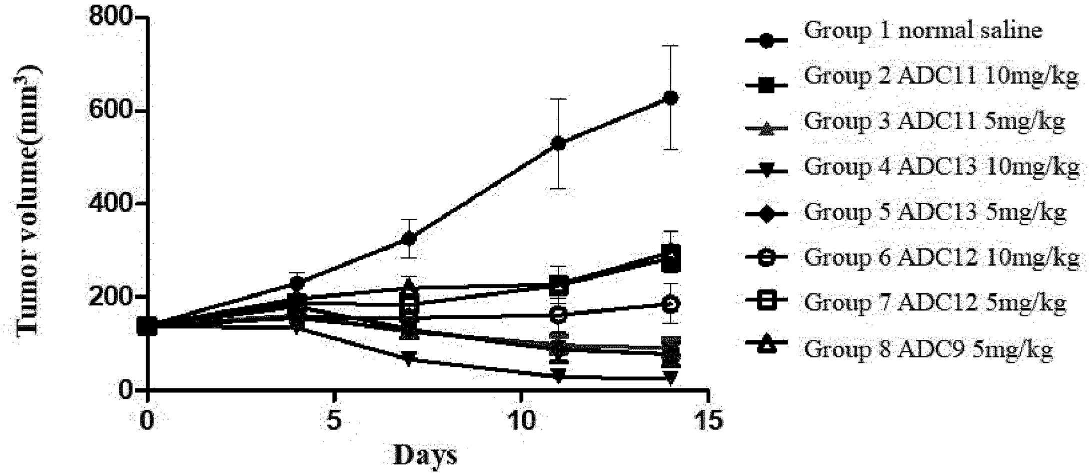
Tumor volume(mm3)
800
600
400
200
0
0
5
10
15
Days
Group 1 normal saline
Group 2 ADC11 10mg/kg
Group 3 ADC11 5mg/kg
Group 4 ADC13 10mg/kg
Group 5 ADC13 5mg/kg
Group 6 ADC12 10mg/kg
Group 7 ADC12 5mg/kg
Group 8 ADC9 5mg/kg

FIG. 12

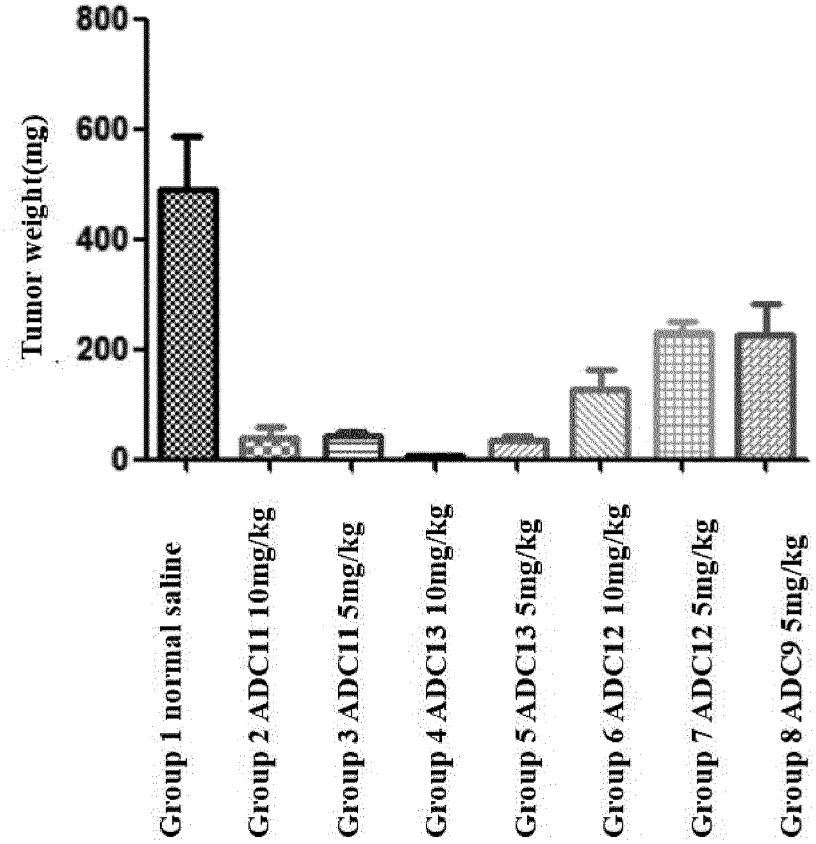
Tumor weight(mg)
800
600
400
200
0
Group 1 normal saline
Group 2 ADC11 10mg/kg
Group 3 ADC11 5mg/kg
Group 4 ADC13 10mg/kg
Group 5 ADC13 5mg/kg
Group 6 ADC12 10mg/kg
Group 7 ADC12 5mg/kg
Group 8 ADC9 5mg/kg

FIG. 13

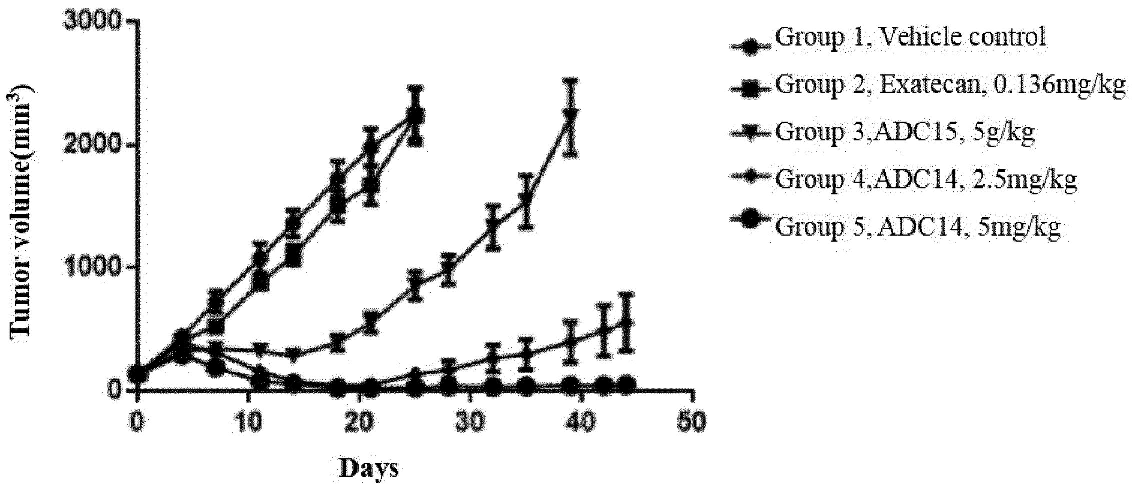


FIG. 14

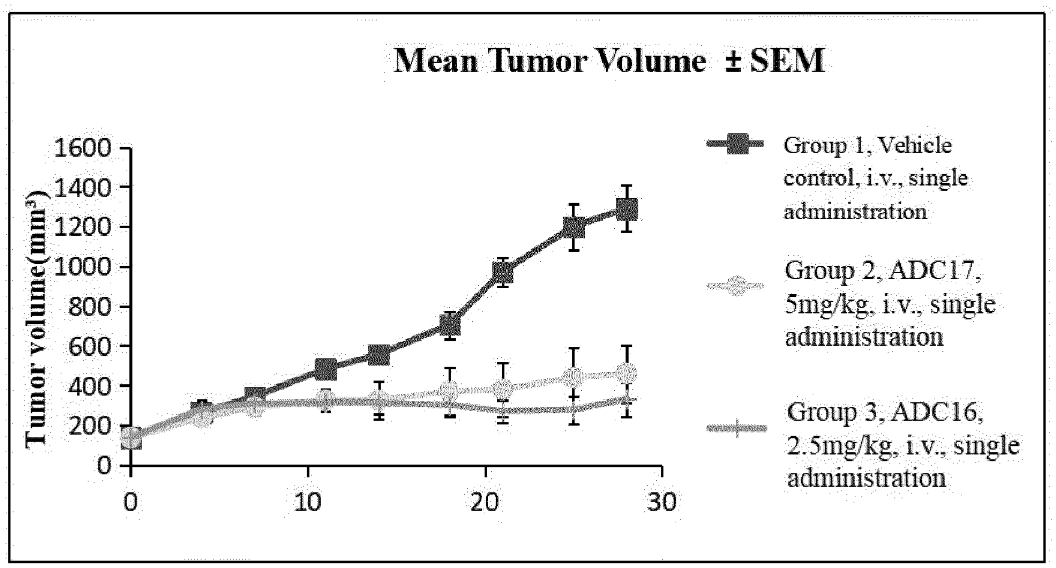


FIG. 15

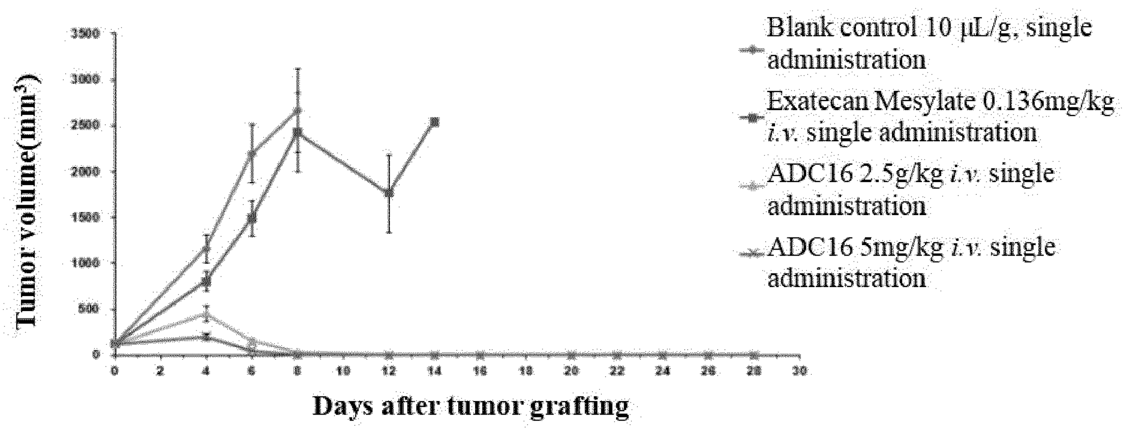


FIG. 16

## INTERNATIONAL SEARCH REPORT

International application No.
**PCT/CN2022/096690**

**A. CLASSIFICATION OF SUBJECT MATTER**

A61K 47/60(2017.01)i; A61K 47/68(2017.01)i; A61K 47/65(2017.01)i; C07K 16/00(2006.01)i; A61P 35/00(2006.01)i

According to International Patent Classification (IPC) or to both national classification and IPC

**B. FIELDS SEARCHED**

Minimum documentation searched (classification system followed by classification symbols)

A61K; C07K; A61P

Documentation searched other than minimum documentation to the extent that such documents are included in the fields searched

Electronic data base consulted during the international search (name of data base and, where practicable, search terms used)

CNTXT; CNABS; DWPI; SIPOABS; STNext caplus/reg; CNKI: 百奥泰; 汤伟佳; 抗体偶联药物; 聚乙二醇; 连接子; 结构检索, structural search; ADC; antibody drug conjugate; PEG; polyethylene glycol; linker

**C. DOCUMENTS CONSIDERED TO BE RELEVANT**

| Category* | Citation of document, with indication, where appropriate, of the relevant passages | Relevant to claim No. |
|---|---|---|
| X | US 2021093733 A1 (OBI PHARMA, INC.) 01 April 2021 (2021-04-01)<br>description, page 58, and claims 53, 59 and 61 | 1-35 |
| X | WO 2019243825 A1 (CURADEV PHARMA LTD.) 26 December 2019 (2019-12-26)<br>description, pp. 128 and 133 | 1-35 |
| A | WO 2020236841 A2 (NOVARTIS AG.) 26 November 2020 (2020-11-26)<br>description, pages 221-222L77-P1, L78-P1, L79-P1 and L80-P1 | 1-35 |
| A | US 2021085799 A1 (MABPLEX INTERNATIONAL, LTD.) 25 March 2021 (2021-03-25)<br>entire document | 1-35 |
| A | CN 108883198 A (EISAI R&D MANAGEMENT CO., LTD.) 23 November 2018 (2018-11-23)<br>entire document | 1-35 |

☐ Further documents are listed in the continuation of Box C.

☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **16 August 2022** | **30 August 2022** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

International application No.
**PCT/CN2022/096690**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      ☑ in the form of an Annex C/ST.25 text file.
      ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      ☐ in the form of an Annex C/ST.25 text file (Rule 13*ter*.1(a)).
      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

**INTERNATIONAL SEARCH REPORT**
**Information on patent family members**

International application No.
**PCT/CN2022/096690**

| Patent document cited in search report | Publication date (day/month/year) | Patent family member(s) | Publication date (day/month/year) |
|---|---|---|---|
| US 2021093733 A1 | 01 April 2021 | None | |
| WO 2019243825 A1 | 26 December 2019 | TW 202016081 A | 01 May 2020 |
| WO 2020236841 A2 | 26 November 2020 | IL 287596 A | 01 December 2021 |
| | | KR 20220010527 A | 25 January 2022 |
| | | AU 2020279731 A1 | 06 January 2022 |
| | | CA 3140063 A1 | 26 November 2020 |
| | | CN 113853219 A | 28 December 2021 |
| | | JP 2022533215 A | 21 July 2022 |
| | | EP 3972650 A2 | 30 March 2022 |
| US 2021085799 A1 | 25 March 2021 | AU 2020204250 A1 | 10 December 2020 |
| | | RU 2745738 C1 | 31 March 2021 |
| | | WO 2020233174 A1 | 26 November 2020 |
| | | JP 2022512519 A | 07 February 2022 |
| | | EP 3760233 A1 | 06 January 2021 |
| | | KR 20200135284 A | 02 December 2020 |
| CN 108883198 A | 23 November 2018 | UA 125024 C2 | 29 December 2021 |
| | | CA 3013791 A1 | 08 September 2017 |
| | | JP 2020019787 A | 06 February 2020 |
| | | US 2017252458 A1 | 07 September 2017 |
| | | EP 3824909 A1 | 26 May 2021 |
| | | US 2020297860 A1 | 24 September 2020 |
| | | SG 10202007520 W A | 29 September 2020 |
| | | LT 3423105 T | 10 September 2021 |
| | | AR 121302 A2 | 04 May 2022 |
| | | IL 261428 A | 31 October 2018 |
| | | SG 11201806515 R A | 27 September 2018 |
| | | CO 2018008667 A2 | 31 August 2018 |
| | | RS 62108 B1 | 31 August 2021 |
| | | BR 112018067379 A2 | 15 January 2019 |
| | | KR 20180115330 A | 22 October 2018 |
| | | HU E054726 T2 | 28 September 2021 |
| | | CN 114191428 A | 18 March 2022 |
| | | CN 114191563 A | 18 March 2022 |
| | | CL 2021000048 A1 | 28 May 2021 |
| | | TW 201800110 A | 01 January 2018 |
| | | JP 2020128413 A | 27 August 2020 |
| | | JP 2019516664 A | 20 June 2019 |
| | | HR P20211125 T1 | 15 October 2021 |
| | | PL 3423105 T3 | 25 October 2021 |
| | | MD 3423105 T2 | 31 October 2021 |
| | | ES 2880402 T3 | 24 November 2021 |
| | | EP 3423105 A1 | 09 January 2019 |
| | | WO 2017151979 A1 | 08 September 2017 |
| | | US 2018193478 A1 | 12 July 2018 |
| | | AR 107787 A1 | 06 June 2018 |
| | | PE 20181953 A1 | 17 December 2018 |
| | | AU 2017225982 A1 | 16 August 2018 |
| | | PH 12018501847 A1 | 15 May 2019 |
| | | SI 3423105 T1 | 31 December 2021 |
| | | AR 121301 A2 | 04 May 2022 |

Form PCT/ISA/210 (patent family annex) (January 2015)

**INTERNATIONAL SEARCH REPORT**
**Information on patent family members**

International application No.
**PCT/CN2022/096690**

| Patent document cited in search report | Publication date (day/month/year) | Patent family member(s) | | | Publication date (day/month/year) |
|---|---|---|---|---|---|
| | | MX | 2018010562 | A | 20 February 2019 |
| | | DK | 3423105 | T3 | 26 July 2021 |
| | | CL | 2018002456 | A1 | 21 December 2018 |
| | | RU | 2018134331 | A | 02 April 2020 |
| | | PT | 3423105 | T | 19 July 2021 |
| | | RU | 2021125492 | A | 05 April 2022 |
| | | MA | 45280 | A | 09 January 2019 |
| | | JP | 2021185176 | A | 09 December 2021 |
| | | CL | 2021000049 | A1 | 28 May 2021 |
| | | CN | 114272389 | A | 05 April 2022 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2021147993 A1
- CN 101264325 B
- CN 105849126 B
- CN 110903395 A
- CN 113896796 A
- US 20130089872 A
- US 5840854 A
- US 20130122020 A
- US 20120237518 A
- WO 2020043044 A
- US 2021403552 A
- WO 2021238831 A
- WO 2021160154 A
- WO 2021111003 A
- US 2018161440 A1
- US 20090169547 A


### Non-patent literature cited in the description


- **ALLEN, T.M. ; CULLIS, P.R.** *Science,* 2004, vol. 303 (5665), 1818-22
- **HU, Q.Y et al.** *Chem Soc Rev,* 2016, vol. 45 (6), 1691-1719
- **THOMAS, A et al.** *Lancet Oncol,* 2016, vol. 17 (6), e254-e262
- **CHARI, R.V.** *Acc Chem Res,* 2008, vol. 41 (1), 98-107
- **SINGH, S.K et al.** *Pharm Res,* 2015, vol. 32 (11), 3541-3571
- **HAMILTON, G.S.** *Biologicals,* 2015, vol. 43 (5), 318-332
- Proteins: Structures and Molecular Principles. W. H. Freeman and Company, 1984
- Introduction to Protein Structure. Garland Publishing, 1991
- **THORNTON et al.** *Nature,* 1991, vol. 354, 105
- These techniques are described in Ouyang. *J. Methods Mol Biol,* 2013, vol. 1045, 275-83
- McGraw-Hill Dictionary of Chemical Terms. McGraw-Hill, 1985
- **LIPINSKI et al.** *Proc Natl. Acad Sci USA,* 1981, vol. 78, 5147-50
- **IKEDA et al.** *Biochem Biophys Res Comm,* 2015, vol. 458, 877-82
- **D. L. HACKER ; F. M. WURM.** *Reference Module in Life Sciences,* 2017
- **WOOD et al.** *J Immunol.,* 1990, vol. 145, 3011
- **WOOD et al.** *J Immunol.,* 1990, vol. 145, 3011
- *CHEMICAL ABSTRACTS,* 1599440-13-7